(19) Europäisches Patentamt
European Patent Office
Office européen des brevets

(11) EP 2 298 886 A2

(12) **EUROPEAN PATENT APPLICATION**

(43) Date of publication:
**23.03.2011 Bulletin 2011/12**

(51) Int Cl.:
***C12N 15/09*** *(2006.01)* ***G06F 17/30*** *(2006.01)*
***G06F 17/40*** *(2006.01)* ***G06F 17/50*** *(2006.01)*

(21) Application number: **10181588.4**

(22) Date of filing: **21.11.2003**

(84) Designated Contracting States:
**AT BE BG CH CY CZ DE DK EE ES FI FR GB GR HU IE IT LI LU MC NL PT RO SE SI SK TR**

(30) Priority: **22.11.2002 JP 2002340053**

(62) Document number(s) of the earlier application(s) in accordance with Art. 76 EPC:
**03774133.7 / 1 571 209**

(71) Applicant: **Bio-Think Tank Co., Ltd.**
**Tokyo 113-0033 (JP)**

(72) Inventors:
- **Saigo, Kaoru**
  **Tokyo 168-0063 (JP)**
- **Tei, Kumiko**
  **Tokyo 113-0023 (JP)**
- **Naito, Yuki**
  **Tokyo 102-0082 (JP)**

(74) Representative: **HOFFMANN EITLE**
**Patent- und Rechtsanwälte**
**Arabellastraße 4**
**81925 München (DE)**

Remarks:
•A request for correction of the description and the drawings has been filed pursuant to Rule 139 EPC. A decision on the request will be taken during the proceedings before the Examining Division (Guidelines for Examination in the EPO, A-V, 3.).
•This application was filed on 29-09-2010 as a divisional application to the application mentioned under INID code 62.

(54) **Method for searching target base sequence of RNA interference, method for designing base sequence of polynucleotide for causing RNA interference, method for producing double-stranded polynucleotide, method for inhibiting gene expression, base sequence processing apparatus, program for running base sequence processing method on computer, recording medium, and base sequence processing system**

(57) In the present invention, a sequence segment conforming to the following rules (a) to (d) is searched from the base sequences of a target gene of RNA interference and, based on the search results, siRNA capable of causing RNAi is designed, synthesized, etc.:
(a) The 3' end base is adenine, thymine, or uracil,
(b) The 5' end base is guanine or cytosine,
(c) A 7-base sequence from the 3' end is rich in one or more types of bases selected from the group consisting of adenine, thymine, and uracil, and
(d) The number of bases is within a range that allows RNA interference to occur without causing cytotoxicity.

*Fig.1*

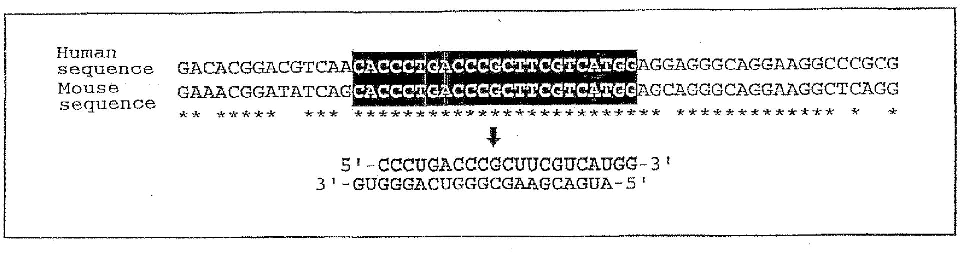


EP 2 298 886 A2

SEQUENCE LISTING

〈110〉 SAIGO, Kaoru; TEI, Kumiko; NAITO, Yuki; NATORI, Yukikazu

〈120〉 METHOD FOR SEARCHING TARGET BASE SEQUENCE OF RNA INTERFERENCE,
METHOD FOR DESIGNING BASE SEQUENCE OF POLYNUCLEOTIDE FOR CAUSING RNA INTERFERENCE,
METHOD FOR PRODUCING DOUBLE-STRANDED POLYNUCLEOTIDE,
METHOD FOR INHIBITING GENE EXPRESSION, BASE SEQUENCE PROCESSING APPARATUS,
PROGRAM FOR RUNNING BASE SEQUENCE PROCESSING METHOD ON COMPUTER,
RECORDING MEDIUM, AND BASE SEQUENCE PROCESSING SYSTEM

〈130〉 PC/B-18-1

〈140〉 PCT/JP2003/014893
〈141〉 2003-11-21

〈150〉 JP 2002-340053
〈151〉 2002-11-22

〈160〉 892

〈210〉 1
〈211〉 37
〈212〉 DNA
〈213〉 Artificial

〈220〉
〈223〉 Inventor: UI-TEI, Kumiko
〈220〉
〈223〉 Inventor: NAITO, Yuki
〈220〉
〈223〉 Inventor: SAIGO, Kaoru

〈220〉
〈223〉 oligomer including Nhe I site, EcoRI site and Xho I site

〈400〉 1
gctagccacc atggaattca cgcgtctcga gtctaga 37

〈210〉 2
〈211〉 18
〈212〉 DNA
〈213〉 Artificial

〈220〉
〈223〉 PCR primer T

〈400〉 2
aggcactggg caggtgtc 18

〈210〉 3

<211> 24
<212> DNA
<213> Artificial

<220>
<223> PCR primer T

<400> 3
tgctcgaagc attaaccctc acta 24

<210> 4
<211> 21
<212> DNA
<213> Artificial

<220>
<223> PCR primer C

<400> 4
atcaggatga tctggacgaa g 21

<210> 5
<211> 21
<212> DNA
<213> Artificial

<220>
<223> PCR primer C

<400> 5
ctcttcagca atatcacggg t 21

<210> 6
<211> 35
<212> DNA
<213> Artificial

<220>
<223> target sequence VIM35

<400> 6
gaattcgcag gatgttcggc ggcccgggcc tcgag 35

<210> 7
<211> 35
<212> DNA
<213> Artificial

<220>

<223> target sequence VIM812

<400> 7
gaattcacgt acgtcagcaa tatgaaagtc tcgag 35

<210> 8
<211> 21
<212> RNA
<213> Artificial

<220>
<223> siRNA as evaluation subject; siVIM35

<400> 8
aggauguucg gcggcccggg c 21

<210> 9
<211> 21
<212> RNA
<213> Artificial

<220>
<223> siRNA as evaluation subject; siVIM812

<400> 9
guacgucagc aauaugaaag u 21

<210> 10
<211> 21
<212> RNA
<213> Artificial

<220>
<223> Control siRNA; siControl

<400> 10
cauucuaucc gcuggaagau g 21

<210> 11
<211> 20
<212> DNA
<213> Artificial

<220>
<223> PCR primer VIM-F3-84

<400> 11
gagctacgtg actacgtcca 20

<210> 12
<211> 21
<212> DNA
<213> Artificial

<220>
<223> PCR primer VIM-R3-274

<400> 12
gttcttgaac tcggtgttga t 21

<210> 13
<211> 20
<212> DNA
<213> Artificial

<220>
<223> PCR primer ACTB-F2-481

<400> 13
cacactgtgc ccatctacga 20

<210> 14
<211> 20
<212> DNA
<213> Artificial

<220>
<223> PCR primer ACTB-R2-664

<400> 14
gccatctctt gctcgaagtc 20

<210> 15
<211> 19
<212> DNA
<213> Artificial

<220>
<223> siRNA target sequence for Firefly luciferase.

<400> 15
gacgccaaaa acataaaga 19

<210> 16
<211> 19
<212> DNA
<213> Artificial

<220>
<223> siRNA target sequence for Firefly luciferase.

<400> 16
gtggcagaa gctatgaaa 19

<210> 17
<211> 19
<212> DNA
<213> Artificial

<220>
<223> siRNA target sequence for Firefly luciferase.

<400> 17
gtgttgggcg cgttattta 19

<210> 18
<211> 19
<212> DNA
<213> Artificial

<220>
<223> siRNA target sequence for Firefly luciferase.

<400> 18
ccgcgaacga catttataa 19

<210> 19
<211> 19
<212> DNA
<213> Artificial

<220>
<223> siRNA target sequence for Firefly luciferase.

<400> 19
ccaatcatcc aaaaaatta 19

<210> 20
<211> 19
<212> DNA
<213> Artificial

<220>
<223> siRNA target sequence for Firefly luciferase.

<400> 20

cctcccggtt ttaatgaat 19

<210> 21
<211> 19
<212> DNA
<213> Artificial

<220>
<223> siRNA target sequence for Firefly luciferase.

<400> 21
gcatgccaga gatcctatt 19

<210> 22
<211> 19
<212> DNA
<213> Artificial

<220>
<223> siRNA target sequence for Firefly luciferase.

<400> 22
ccggatactg cgattttaa 19

<210> 23
<211> 19
<212> DNA
<213> Artificial

<220>
<223> siRNA target sequence for Firefly luciferase.

<400> 23
ggttttggaa tgtttacta 19

<210> 24
<211> 19
<212> DNA
<213> Artificial

<220>
<223> siRNA target sequence for Firefly luciferase.

<400> 24
gatttcgagt cgtcttaat 19

<210> 25
<211> 19

<212> DNA
<213> Artificial

<220>
<223> siRNA target sequence for Firefly luciferase.

<400> 25
gcactctgat tgacaaata 19

<210> 26
<211> 19
<212> DNA
<213> Artificial

<220>
<223> siRNA target sequence for Firefly luciferase.

<400> 26
caaatacgat ttatctaat 19

<210> 27
<211> 19
<212> DNA
<213> Artificial

<220>
<223> siRNA target sequence for Firefly luciferase.

<400> 27
gattatgtcc ggttatgta 19

<210> 28
<211> 19
<212> DNA
<213> Artificial

<220>
<223> siRNA target sequence for Firefly luciferase.

<400> 28
ccgcctgaag tctctgatt 19

<210> 29
<211> 19
<212> DNA
<213> Artificial

<220>
<223> siRNA target sequence for Firefly luciferase.

<400> 29
ctcgacgcaa gaaaaatca 19

<210> 30
<211> 19
<212> DNA
<213> Artificial

<220>
<223> siRNA target sequence for Firefly luciferase.

<400> 30
aacataaaga aaggcccgg 19

<210> 31
<211> 19
<212> DNA
<213> Artificial

<220>
<223> siRNA target sequence for Firefly luciferase.

<400> 31
tatgccggtg ttgggcgcg 19

<210> 32
<211> 19
<212> DNA
<213> Artificial

<220>
<223> siRNA target sequence for Firefly luciferase.

<400> 32
agttgcagtt gcgcccgcg 19

<210> 33
<211> 19
<212> DNA
<213> Artificial

<220>
<223> siRNA target sequence for Firefly luciferase.

<400> 33
acgtgcaaaa aaagctccc 19

<210> 34
<211> 19
<212> DNA
<213> Artificial

<220>
<223> siRNA target sequence for Firefly luciferase.

<400> 34
ttctgattac acccgaggg 19

<210> 35
<211> 19
<212> DNA
<213> Artificial

<220>
<223> siRNA target sequence for SARS coronavirus.

<400> 35
gggcgcggtc ggtaaagtt 19

<210> 36
<211> 19
<212> DNA
<213> Artificial

<220>
<223> siRNA target sequence for SARS coronavirus.

<400> 36
ggaattgccg tcttagata 19

<210> 37
<211> 19
<212> DNA
<213> Artificial

<220>
<223> siRNA target sequence for SARS coronavirus.

<400> 37
gaatggtcgt actatcctt 19

<210> 38
<211> 19
<212> DNA
<213> Artificial

<220>
<223> siRNA target sequence for SARS coronavirus.

<400> 38
ccaagtaatc gttaacaat 19

<210> 39
<211> 19
<212> DNA
<213> Artificial

<220>
<223> siRNA target sequence for SARS coronavirus.

<400> 39
gcttggcgca tatattcta 19

<210> 40
<211> 19
<212> DNA
<213> Artificial

<220>
<223> siRNA target sequence for SARS coronavirus.

<400> 40
cctttcgcga cttgataaa 19

<210> 41
<211> 19
<212> DNA
<213> Artificial

<220>
<223> siRNA target sequence for SARS coronavirus.

<400> 41
gtgcgtactg ctgcaatat 19

<210> 42
<211> 19
<212> DNA
<213> Artificial

<220>
<223> siRNA target sequence for SARS coronavirus.

<400> 42
ctactcgcgt gttaaaaat 19

<210> 43
<211> 19
<212> DNA
<213> Artificial

<220>
<223> siRNA target sequence for SARS coronavirus.

<400> 43
gcagacaacg gtactatta 19

<210> 44
<211> 19
<212> DNA
<213> Artificial

<220>
<223> siRNA target sequence for SARS coronavirus.

<400> 44
ccggtagcaa cgacaatat 19

<210> 45
<211> 19
<212> DNA
<213> Artificial

<220>
<223> siRNA target sequence for SARS coronavirus.

<400> 45
cgtagtcgcg gtaattcaa 19

<210> 46
<211> 19
<212> DNA
<213> Artificial

<220>
<223> siRNA target sequence for SARS coronavirus.

<400> 46
gatcgagggt acagtgaat 19

<210> 47
<211> 19
<212> DNA

<213> Homo sapiens

<220>
<223> siRNA target sequence for FGFR1 (NM_000604).

<400> 47
gtagcaacgt ggagttcat 19

<210> 48
<211> 19
<212> DNA
<213> Homo sapiens

<220>
<223> siRNA target sequence for FGFR1 (NM_000604).

<400> 48
ggtagcaacg tggagttca 19

<210> 49
<211> 19
<212> DNA
<213> Homo sapiens

<220>
<223> siRNA target sequence for FGFR1 (NM_000604).

<400> 49
caacgtggag ttcatgtgt 19

<210> 50
<211> 19
<212> DNA
<213> Homo sapiens

<220>
<223> siRNA target sequence for FGFR1 (NM_000604).

<400> 50
ggtgaatggg agcaagatt 19

<210> 51
<211> 19
<212> DNA
<213> Homo sapiens

<220>
<223> siRNA target sequence for FGFR1 (NM_000604).

<400> 51
gcaagattgg cccagacaa 19

<210> 52
<211> 19
<212> DNA
<213> Homo sapiens

<220>
<223> siRNA target sequence for FGFR1 (NM_000604).

<400> 52
gagttcatgt gtaaggtgt 19

<210> 53
<211> 19
<212> DNA
<213> Homo sapiens

<220>
<223> siRNA target sequence for FGFR2 (NM_000141).

<400> 53
gaggctacaa ggtacgaaa 19

<210> 54
<211> 19
<212> DNA
<213> Homo sapiens

<220>
<223> siRNA target sequence for FGFR2 (NM_000141).

<400> 54
gctacaaggt acgaaacca 19

<210> 55
<211> 19
<212> DNA
<213> Homo sapiens

<220>
<223> siRNA target sequence for FGFR2 (NM_000141).

<400> 55
ctggagcctc attatggaa 19

<210> 56

<211> 19
<212> DNA
<213> Homo sapiens

<220>
<223> siRNA target sequence for FGFR2 (NM_000141).

<400> 56
gaaaaacggg aaggagttt 19

<210> 57
<211> 19
<212> DNA
<213> Homo sapiens

<220>
<223> siRNA target sequence for FGFR2 (NM_000141).

<400> 57
gcaggagcat cgcattgga 19

<210> 58
<211> 19
<212> DNA
<213> Homo sapiens

<220>
<223> siRNA target sequence for FGFR2 (NM_000141).

<400> 58
ccttcagttt agttgagga 19

<210> 59
<211> 19
<212> DNA
<213> Homo sapiens

<220>
<223> siRNA target sequence for FGFR2 (NM_000141).

<400> 59
cttcagttta gttgaggat 19

<210> 60
<211> 19
<212> DNA
<213> Homo sapiens

<220>

<223> siRNA target sequence for FGFR3 (NM_000142).

<400> 60
gacggcacac cctacgtta 19

<210> 61
<211> 19
<212> DNA
<213> Homo sapiens

<220>
<223> siRNA target sequence for FGFR3 (NM_000142).

<400> 61
cacaacctcg actactaca 19

<210> 62
<211> 19
<212> DNA
<213> Homo sapiens

<220>
<223> siRNA target sequence for FGFR3 (NM_000142).

<400> 62
gcacacacga cctgtacat 19

<210> 63
<211> 19
<212> DNA
<213> Homo sapiens

<220>
<223> siRNA target sequence for FGFR3 (NM_000142).

<400> 63
cctgcgtcgt ggagaacaa 19

<210> 64
<211> 19
<212> DNA
<213> Homo sapiens

<220>
<223> siRNA target sequence for FGFR3 (NM_000142).

<400> 64
cacacgacct gtacatgat 19

<210> 65
<211> 19
<212> DNA
<213> Homo sapiens

<220>
<223> siRNA target sequence for FGFR3 (NM_000142).

<400> 65
gagttccact gcaaggtgt 19

<210> 66
<211> 19
<212> DNA
<213> Homo sapiens

<220>
<223> siRNA target sequence for ERBB2 (NM_004448).

<400> 66
ggagacccgc tgaacaata 19

<210> 67
<211> 19
<212> DNA
<213> Homo sapiens

<220>
<223> siRNA target sequence for ERBB2 (NM_004448).

<400> 67
ccttcgacaa cctctatta 19

<210> 68
<211> 19
<212> DNA
<213> Homo sapiens

<220>
<223> siRNA target sequence for ERBB2 (NM_004448).

<400> 68
gggctggctc cgatgtatt 19

<210> 69
<211> 19
<212> DNA
<213> Homo sapiens

<220>
<223> siRNA target sequence for ERBB2 (NM_004448).

<400> 69
ggctggctcc gatgtattt 19

<210> 70
<211> 19
<212> DNA
<213> Homo sapiens

<220>
<223> siRNA target sequence for ERBB2 (NM_004448).

<400> 70
ctggctccga tgtatttga 19

<210> 71
<211> 19
<212> DNA
<213> Homo sapiens

<220>
<223> siRNA target sequence for ERBB3 (NM_001982).

<400> 71
gtgctgggcg tatctatat 19

<210> 72
<211> 19
<212> DNA
<213> Homo sapiens

<220>
<223> siRNA target sequence for ERBB3 (NM_001982).

<400> 72
gctgggcgta tctatataa 19

<210> 73
<211> 19
<212> DNA
<213> Homo sapiens

<220>
<223> siRNA target sequence for ERBB3 (NM_001982).

<400> 73

gcttgtcctg tcgaaatta 19

<210> 74
<211> 19
<212> DNA
<213> Homo sapiens

<220>
<223> siRNA target sequence for ERBB3 (NM_001982).

<400> 74
cttgtcctgt cgaaattat 19

<210> 75
<211> 19
<212> DNA
<213> Homo sapiens

<220>
<223> siRNA target sequence for ERBB3 (NM_001982).

<400> 75
cattcgccca acctttaaa 19

<210> 76
<211> 19
<212> DNA
<213> Homo sapiens

<220>
<223> siRNA target sequence for ERBB4 (NM_005235).

<400> 76
ggagaattta cgcattatt 19

<210> 77
<211> 19
<212> DNA
<213> Homo sapiens

<220>
<223> siRNA target sequence for ERBB4 (NM_005235).

<400> 77
gctcaacttc gtattttga 19

<210> 78
<211> 19

<212> DNA
<213> Homo sapiens

<220>
<223> siRNA target sequence for ERBB4 (NM_005235).

<400> 78
ctcaaagata cctagttat 19

<210> 79
<211> 19
<212> DNA
<213> Homo sapiens

<220>
<223> siRNA target sequence for ERBB4 (NM_005235).

<400> 79
ctcaacttcg tattttgaa 19

<210> 80
<211> 19
<212> DNA
<213> Homo sapiens

<220>
<223> siRNA target sequence for ERBB4 (NM_005235).

<400> 80
ctgacagtag acctaaatt 19

<210> 81
<211> 19
<212> DNA
<213> Homo sapiens

<220>
<223> siRNA target sequence for JAK1 (NM_002227).

<400> 81
ctcagggaca gtatgattt 19

<210> 82
<211> 19
<212> DNA
<213> Homo sapiens

<220>
<223> siRNA target sequence for JAK1 (NM_002227).

<400> 82
cagaatacgc catcaataa 19

<210> 83
<211> 19
<212> DNA
<213> Homo sapiens

<220>
<223> siRNA target sequence for JAK1 (NM_002227).

<400> 83
gatgcggata aataatgtt 19

<210> 84
<211> 19
<212> DNA
<213> Homo sapiens

<220>
<223> siRNA target sequence for JAK1 (NM_002227).

<400> 84
ggatgcggat aaataatgt 19

<210> 85
<211> 19
<212> DNA
<213> Homo sapiens

<220>
<223> siRNA target sequence for JAK1 (NM_002227).

<400> 85
ctttcagaac cttattgaa 19

<210> 86
<211> 19
<212> DNA
<213> Homo sapiens

<220>
<223> siRNA target sequence for JAK1 (NM_002227).

<400> 86
cagctacaag cgatatatt 19

<210> 87
<211> 19
<212> DNA
<213> Homo sapiens

<220>
<223> siRNA target sequence for JAK1 (NM_002227).

<400> 87
caattgaaac cgataagga 19

<210> 88
<211> 19
<212> DNA
<213> Homo sapiens

<220>
<223> siRNA target sequence for JAK1 (NM_002227).

<400> 88
gggttctcgg caatacgtt 19

<210> 89
<211> 19
<212> DNA
<213> Homo sapiens

<220>
<223> siRNA target sequence for JAK2 (NM_004972).

<400> 89
ctggtcggcg taatctaaa 19

<210> 90
<211> 19
<212> DNA
<213> Homo sapiens

<220>
<223> siRNA target sequence for JAK2 (NM_004972).

<400> 90
ggtcggcgta atctaaaat 19

<210> 91
<211> 19
<212> DNA
<213> Homo sapiens

<220>
<223> siRNA target sequence for JAK2 (NM_004972).

<400> 91
gtcggcgtaa tctaaaatt 19

<210> 92
<211> 19
<212> DNA
<213> Homo sapiens

<220>
<223> siRNA target sequence for JAK2 (NM_004972).

<400> 92
ggaatttatg cgtatgatt 19

<210> 93
<211> 19
<212> DNA
<213> Homo sapiens

<220>
<223> siRNA target sequence for JAK2 (NM_004972).

<400> 93
ctgttcgctc agacaatat 19

<210> 94
<211> 19
<212> DNA
<213> Homo sapiens

<220>
<223> siRNA target sequence for JAK2 (NM_004972).

<400> 94
ggaaacggtg gaattcagt 19

<210> 95
<211> 19
<212> DNA
<213> Homo sapiens

<220>
<223> siRNA target sequence for JAK2 (NM_004972).

<400> 95
ctggaaacgg tggaattca 19

<210> 96
<211> 19
<212> DNA
<213> Homo sapiens

<220>
<223> siRNA target sequence for JAK2 (NM_004972).

<400> 96
gatttttgca accattata 19

<210> 97
<211> 19
<212> DNA
<213> Homo sapiens

<220>
<223> siRNA target sequence for JAK3 (NM_000215).

<400> 97
gtcattcgtg acctcaata 19

<210> 98
<211> 19
<212> DNA
<213> Homo sapiens

<220>
<223> siRNA target sequence for JAK3 (NM_000215).

<400> 98
gacccgctag cccacaata 19

<210> 99
<211> 19
<212> DNA
<213> Homo sapiens

<220>
<223> siRNA target sequence for JAK3 (NM_000215).

<400> 99
cccgctagcc cacaataca 19

<210> 100
<211> 19
<212> DNA

<213> Homo sapiens

<220>
<223> siRNA target sequence for JAK3 (NM_000215).

<400> 100
ccatggtgca ggaatttgt 19

<210> 101
<211> 19
<212> DNA
<213> Homo sapiens

<220>
<223> siRNA target sequence for JAK3 (NM_000215).

<400> 101
catgtatctg cgaaaacgt 19

<210> 102
<211> 19
<212> DNA
<213> Homo sapiens

<220>
<223> siRNA target sequence for TYK2 (NM_003331).

<400> 102
gcctgaagga gtataagtt 19

<210> 103
<211> 19
<212> DNA
<213> Homo sapiens

<220>
<223> siRNA target sequence for TYK2 (NM_003331).

<400> 103
cggaccctac ggtttttcca 19

<210> 104
<211> 19
<212> DNA
<213> Homo sapiens

<220>
<223> siRNA target sequence for TYK2 (NM_003331).

<400> 104
ctatatttcc gcataaggt 19

<210> 105
<211> 19
<212> DNA
<213> Homo sapiens

<220>
<223> siRNA target sequence for TYK2 (NM_003331).

<400> 105
ccacaagcgc tatttgaaa 19

<210> 106
<211> 19
<212> DNA
<213> Homo sapiens

<220>
<223> siRNA target sequence for TYK2 (NM_003331).

<400> 106
cacaagcgct atttgaaaa 19

<210> 107
<211> 19
<212> DNA
<213> Homo sapiens

<220>
<223> siRNA target sequence for TYK2 (NM_003331).

<400> 107
gaactggcat ggcatgaat 19

<210> 108
<211> 19
<212> DNA
<213> Homo sapiens

<220>
<223> siRNA target sequence for ZAP70 (NM_001079).

<400> 108
gaggccgagc gcaaacttt 19

<210> 109

<211> 19
<212> DNA
<213> Homo sapiens

<220>
<223> siRNA target sequence for ZAP70 (NM_001079).

<400> 109
ggtacgcacc cgaatgcat 19

<210> 110
<211> 19
<212> DNA
<213> Homo sapiens

<220>
<223> siRNA target sequence for ZAP70 (NM_001079).

<400> 110
gagctctgcg agttctact 19

<210> 111
<211> 19
<212> DNA
<213> Homo sapiens

<220>
<223> siRNA target sequence for ZAP70 (NM_001079).

<400> 111
gacacgagcg tgtatgaga 19

<210> 112
<211> 19
<212> DNA
<213> Homo sapiens

<220>
<223> siRNA target sequence for ZAP70 (NM_001079).

<400> 112
cggcactacg ccaagatca 19

<210> 113
<211> 19
<212> DNA
<213> Homo sapiens

<220>

<223> siRNA target sequence for ZAP70 (NM_001079).

<400> 113
ggagctatgg ggtcaccat 19

<210> 114
<211> 19
<212> DNA
<213> Homo sapiens

<220>
<223> siRNA target sequence for SRC (NM_005417).

<400> 114
ctgttcggag gcttcaact 19

<210> 115
<211> 19
<212> DNA
<213> Homo sapiens

<220>
<223> siRNA target sequence for SRC (NM_005417).

<400> 115
ggtggcctac tactccaaa 19

<210> 116
<211> 19
<212> DNA
<213> Homo sapiens

<220>
<223> siRNA target sequence for SRC (NM_005417).

<400> 116
gggagtcaga gcggttact 19

<210> 117
<211> 19
<212> DNA
<213> Homo sapiens

<220>
<223> siRNA target sequence for SRC (NM_005417).

<400> 117
cagagcggtt actgctcaa 19

<210> 118
<211> 19
<212> DNA
<213> Homo sapiens

<220>
<223> siRNA target sequence for SRC (NM_005417).

<400> 118
cagtgtctga cttcgacaa 19

<210> 119
<211> 19
<212> DNA
<213> Homo sapiens

<220>
<223> siRNA target sequence for SRC (NM_005417).

<400> 119
cctcccgcac ccagttcaa 19

<210> 120
<211> 19
<212> DNA
<213> Homo sapiens

<220>
<223> siRNA target sequence for LYN (NM_002350).

<400> 120
cagcgacatg attaaacat 19

<210> 121
<211> 19
<212> DNA
<213> Homo sapiens

<220>
<223> siRNA target sequence for LYN (NM_002350).

<400> 121
gttattaagc actacaaaa 19

<210> 122
<211> 19
<212> DNA
<213> Homo sapiens

<220>
<223> siRNA target sequence for LYN (NM_002350).

<400> 122
gtatcagcga catgattaa 19

<210> 123
<211> 19
<212> DNA
<213> Homo sapiens

<220>
<223> siRNA target sequence for LYN (NM_002350).

<400> 123
ggatgggtta ctataacaa 19

<210> 124
<211> 19
<212> DNA
<213> Homo sapiens

<220>
<223> siRNA target sequence for LYN (NM_002350).

<400> 124
gaagccatgg gataaagat 19

<210> 125
<211> 19
<212> DNA
<213> Homo sapiens

<220>
<223> siRNA target sequence for LYN (NM_002350).

<400> 125
gcactacaaa attagaagt 19

<210> 126
<211> 19
<212> DNA
<213> Homo sapiens

<220>
<223> siRNA target sequence for ABL1 (NM_005157).

<400> 126

cactctaagc ataactaaa 19

<210> 127
<211> 19
<212> DNA
<213> Homo sapiens

<220>
<223> siRNA target sequence for ABL1 (NM_005157).

<400> 127
gagggcgtgt ggaagaaat 19

<210> 128
<211> 19
<212> DNA
<213> Homo sapiens

<220>
<223> siRNA target sequence for ABL1 (NM_005157).

<400> 128
ccgggtctta ggctataat 19

<210> 129
<211> 19
<212> DNA
<213> Homo sapiens

<220>
<223> siRNA target sequence for ABL1 (NM_005157).

<400> 129
gggtcttagg ctataatca 19

<210> 130
<211> 19
<212> DNA
<213> Homo sapiens

<220>
<223> siRNA target sequence for ABL1 (NM_005157).

<400> 130
catctcgctg agatacgaa 19

<210> 131
<211> 19

<212> DNA
<213> Homo sapiens

<220>
<223> siRNA target sequence for ABL1 (NM_005157).

<400> 131
ggccagtgga gataacact 19

<210> 132
<211> 19
<212> DNA
<213> Homo sapiens

<220>
<223> siRNA target sequence for ABL1 (NM_005157).

<400> 132
gcctggccta caacaagtt 19

<210> 133
<211> 19
<212> DNA
<213> Homo sapiens

<220>
<223> siRNA target sequence for ABL1 (NM_005157).

<400> 133
gtgtccccca actacgaca 19

<210> 134
<211> 19
<212> DNA
<213> Homo sapiens

<220>
<223> siRNA target sequence for ABL2 (NM_005158).

<400> 134
ctcaaactcg caacaaatt 19

<210> 135
<211> 19
<212> DNA
<213> Homo sapiens

<220>
<223> siRNA target sequence for ABL2 (NM_005158).

<400> 135
cctcaaactc gcaacaaat 19

<210> 136
<211> 19
<212> DNA
<213> Homo sapiens

<220>
<223> siRNA target sequence for ABL2 (NM_005158).

<400> 136
ctaaggttta tgaacttat 19

<210> 137
<211> 19
<212> DNA
<213> Homo sapiens

<220>
<223> siRNA target sequence for ABL2 (NM_005158).

<400> 137
gctcagcagt ctaatcaat 19

<210> 138
<211> 19
<212> DNA
<213> Homo sapiens

<220>
<223> siRNA target sequence for ABL2 (NM_005158).

<400> 138
caggccgctg agaaaatct 19

<210> 139
<211> 19
<212> DNA
<213> Homo sapiens

<220>
<223> siRNA target sequence for CLK1 (NM_004071).

<400> 139
ccaggaaacg taaatattt 19

<210> 140
<211> 19
<212> DNA
<213> Homo sapiens

<220>
<223> siRNA target sequence for CLK1 (NM_004071).

<400> 140
catttcgact ggatcatat 19

<210> 141
<211> 19
<212> DNA
<213> Homo sapiens

<220>
<223> siRNA target sequence for CLK1 (NM_004071).

<400> 141
caggaaacgt aaatatttt 19

<210> 142
<211> 19
<212> DNA
<213> Homo sapiens

<220>
<223> siRNA target sequence for CLK1 (NM_004071).

<400> 142
ctttggtagt gcaacatat 19

<210> 143
<211> 19
<212> DNA
<213> Homo sapiens

<220>
<223> siRNA target sequence for CLK1 (NM_004071).

<400> 143
cgtactaagt gcaagatat 19

<210> 144
<211> 19
<212> DNA
<213> Homo sapiens

<220>
<223> siRNA target sequence for CLK2 (NM_001291).

<400> 144
gtatgaccgg cgatactgt 19

<210> 145
<211> 19
<212> DNA
<213> Homo sapiens

<220>
<223> siRNA target sequence for CLK2 (NM_001291).

<400> 145
gctacagacg caacgatta 19

<210> 146
<211> 19
<212> DNA
<213> Homo sapiens

<220>
<223> siRNA target sequence for CLK2 (NM_001291).

<400> 146
ctacagacgc aacgattat 19

<210> 147
<211> 19
<212> DNA
<213> Homo sapiens

<220>
<223> siRNA target sequence for CLK2 (NM_001291).

<400> 147
ggagttaccg tgaacacta 19

<210> 148
<211> 19
<212> DNA
<213> Homo sapiens

<220>
<223> siRNA target sequence for CLK2 (NM_001291).

<400> 148
gagttaccgt gaacactat 19

<210> 149
<211> 19
<212> DNA
<213> Homo sapiens

<220>
<223> siRNA target sequence for CLK3 (NM_001292).

<400> 149
gccgtgacag cgatacata 19

<210> 150
<211> 19
<212> DNA
<213> Homo sapiens

<220>
<223> siRNA target sequence for CLK3 (NM_001292).

<400> 150
cctacagtcg ggaacatga 19

<210> 151
<211> 19
<212> DNA
<213> Homo sapiens

<220>
<223> siRNA target sequence for CLK3 (NM_001292).

<400> 151
ctacagtcgg gaacatgaa 19

<210> 152
<211> 19
<212> DNA
<213> Homo sapiens

<220>
<223> siRNA target sequence for CLK3 (NM_001292).

<400> 152
cgccgtgaca gcgatacat 19

<210> 153
<211> 19
<212> DNA

<213> Homo sapiens

<220>
<223> siRNA target sequence for CLK3 (NM_001292).

<400> 153
gcctccccca cgaagatct 19

<210> 154
<211> 19
<212> DNA
<213> Homo sapiens

<220>
<223> siRNA target sequence for CLK3 (NM_001292).

<400> 154
ggtgaaggca cctttggca 19

<210> 155
<211> 19
<212> DNA
<213> Homo sapiens

<220>
<223> siRNA target sequence for CLK4 (NM_020666).

<400> 155
gtattagagc acttaaata 19

<210> 156
<211> 19
<212> DNA
<213> Homo sapiens

<220>
<223> siRNA target sequence for CLK4 (NM_020666).

<400> 156
gaaaacgcaa gtattttca 19

<210> 157
<211> 19
<212> DNA
<213> Homo sapiens

<220>
<223> siRNA target sequence for CLK4 (NM_020666).

<400> 157
cctggttcga agaatgtta 19

<210> 158
<211> 19
<212> DNA
<213> Homo sapiens

<220>
<223> siRNA target sequence for CLK4 (NM_020666).

<400> 158
cttgaatgag cgagattat 19

<210> 159
<211> 19
<212> DNA
<213> Homo sapiens

<220>
<223> siRNA target sequence for CLK4 (NM_020666).

<400> 159
cagatctgcc agtcaataa 19

<210> 160
<211> 19
<212> DNA
<213> Homo sapiens

<220>
<223> siRNA target sequence for CLK4 (NM_020666).

<400> 160
cgttctaaga gcaagatat 19

<210> 161
<211> 19
<212> DNA
<213> Homo sapiens

<220>
<223> siRNA target sequence for CLK4 (NM_020666).

<400> 161
caaagtggag acgttctaa 19

<210> 162

<211> 19
<212> DNA
<213> Homo sapiens

<220>
<223> siRNA target sequence for CLK4 (NM_020666).

<400> 162
ctaagagcaa gatatgaaa 19

<210> 163
<211> 19
<212> DNA
<213> Homo sapiens

<220>
<223> siRNA target sequence for GSK3B (NM_002093).

<400> 163
gtccgattgc gttatttct 19

<210> 164
<211> 19
<212> DNA
<213> Homo sapiens

<220>
<223> siRNA target sequence for GSK3B (NM_002093).

<400> 164
gctagatcac tgtaacata 19

<210> 165
<211> 19
<212> DNA
<213> Homo sapiens

<220>
<223> siRNA target sequence for GSK3B (NM_002093).

<400> 165
gacgctccct gtgatttat 19

<210> 166
<211> 19
<212> DNA
<213> Homo sapiens

<220>

<223> siRNA target sequence for GSK3B (NM_002093).

<400> 166
cccaatgttt cgtatatct 19

<210> 167
<211> 19
<212> DNA
<213> Homo sapiens

<220>
<223> siRNA target sequence for GSK3B (NM_002093).

<400> 167
cgaggagaac ccaatgttt 19

<210> 168
<211> 19
<212> DNA
<213> Homo sapiens

<220>
<223> siRNA target sequence for GSK3B (NM_002093).

<400> 168
gtatatcaag ccaaacttt 19

<210> 169
<211> 19
<212> DNA
<213> Homo sapiens

<220>
<223> siRNA target sequence for GSK3B (NM_002093).

<400> 169
cattggtgt ggtatatca 19

<210> 170
<211> 19
<212> DNA
<213> Homo sapiens

<220>
<223> siRNA target sequence for GSK3B (NM_002093).

<400> 170
ggtatatcaa gccaaactt 19

<210> 171
<211> 19
<212> DNA
<213> Homo sapiens

<220>
<223> siRNA target sequence for SRPK2 (NM_182691).

<400> 171
gccaaatgga cgacataaa 19

<210> 172
<211> 19
<212> DNA
<213> Homo sapiens

<220>
<223> siRNA target sequence for SRPK2 (NM_182691).

<400> 172
ccaaatggac gacataaaa 19

<210> 173
<211> 19
<212> DNA
<213> Homo sapiens

<220>
<223> siRNA target sequence for SRPK2 (NM_182691).

<400> 173
caaatggacg acataaaat 19

<210> 174
<211> 19
<212> DNA
<213> Homo sapiens

<220>
<223> siRNA target sequence for SRPK2 (NM_182691).

<400> 174
ctgatcccga tgttagaaa 19

<210> 175
<211> 19
<212> DNA
<213> Homo sapiens

<220>
<223> siRNA target sequence for SRPK2 (NM_182691).

<400> 175
ggccggtatc atgttatta 19

<210> 176
<211> 19
<212> DNA
<213> Homo sapiens

<220>
<223> siRNA target sequence for WNT1 (NM_005430).

<400> 176
ggccgtacga ccgtattct 19

<210> 177
<211> 19
<212> DNA
<213> Homo sapiens

<220>
<223> siRNA target sequence for WNT1 (NM_005430).

<400> 177
gcgtctgata cgccaaaat 19

<210> 178
<211> 19
<212> DNA
<213> Homo sapiens

<220>
<223> siRNA target sequence for WNT1 (NM_005430).

<400> 178
cccacgacct cgtctactt 19

<210> 179
<211> 19
<212> DNA
<213> Homo sapiens

<220>
<223> siRNA target sequence for WNT1 (NM_005430).

<400> 179

caaacagcgg cgtctgata 19

<210> 180
<211> 19
<212> DNA
<213> Homo sapiens

<220>
<223> siRNA target sequence for WNT1 (NM_005430).

<400> 180
gagaaatcgc ccaacttct 19

<210> 181
<211> 19
<212> DNA
<213> Homo sapiens

<220>
<223> siRNA target sequence for WNT1 (NM_005430).

<400> 181
ctcgtctact tcgagaaat 19

<210> 182
<211> 19
<212> DNA
<213> Homo sapiens

<220>
<223> siRNA target sequence for WNT1 (NM_005430).

<400> 182
gacctcgtct acttcgaga 19

<210> 183
<211> 19
<212> DNA
<213> Homo sapiens

<220>
<223> siRNA target sequence for WNT2 (NM_003391).

<400> 183
gggtgatgtg cgataatgt 19

<210> 184
<211> 19

<212> DNA
<213> Homo sapiens

<220>
<223> siRNA target sequence for WNT2 (NM_003391).

<400> 184
ggaaaacggg cgattatct 19

<210> 185
<211> 19
<212> DNA
<213> Homo sapiens

<220>
<223> siRNA target sequence for WNT2 (NM_003391).

<400> 185
gctaacgaga ggtttaaga 19

<210> 186
<211> 19
<212> DNA
<213> Homo sapiens

<220>
<223> siRNA target sequence for WNT2 (NM_003391).

<400> 186
ctaacgagag gtttaagaa 19

<210> 187
<211> 19
<212> DNA
<213> Homo sapiens

<220>
<223> siRNA target sequence for WNT2 (NM_003391).

<400> 187
ggtcctactc cgaagtagt 19

<210> 188
<211> 19
<212> DNA
<213> Homo sapiens

<220>
<223> siRNA target sequence for WNT2 (NM_003391).

<400> 188
gacctcgtgt attttgaga 19

<210> 189
<211> 19
<212> DNA
<213> Homo sapiens

<220>
<223> siRNA target sequence for WNT2 (NM_003391).

<400> 189
gaaaaatgac ctcgtgtat 19

<210> 190
<211> 19
<212> DNA
<213> Homo sapiens

<220>
<223> siRNA target sequence for WNT2 (NM_003391).

<400> 190
cgaaaaatga cctcgtgta 19

<210> 191
<211> 19
<212> DNA
<213> Homo sapiens

<220>
<223> siRNA target sequence for WNT7A (NM_004625).

<400> 191
ctgggcgcaa gcatcatct 19

<210> 192
<211> 19
<212> DNA
<213> Homo sapiens

<220>
<223> siRNA target sequence for WNT7A (NM_004625).

<400> 192
gttcacctac gccatcatt 19

<210> 193
<211> 19
<212> DNA
<213> Homo sapiens

<220>
<223> siRNA target sequence for WNT7A (NM_004625).

<400> 193
gccccggactc tcatgaact 19

<210> 194
<211> 19
<212> DNA
<213> Homo sapiens

<220>
<223> siRNA target sequence for WNT7A (NM_004625).

<400> 194
gcttcgccaa ggtctttgt 19

<210> 195
<211> 19
<212> DNA
<213> Homo sapiens

<220>
<223> siRNA target sequence for WNT7A (NM_004625).

<400> 195
cctggacgag tgtcagttt 19

<210> 196
<211> 19
<212> DNA
<213> Homo sapiens

<220>
<223> siRNA target sequence for WNT7A (NM_004625).

<400> 196
gacgagtgtc agtttcagt 19

<210> 197
<211> 19
<212> DNA
<213> Homo sapiens

<220>
<223> siRNA target sequence for WNT7A (NM_004625).

<400> 197
catcatcgtc ataggagaa 19

<210> 198
<211> 19
<212> DNA
<213> Homo sapiens

<220>
<223> siRNA target sequence for WNT11 (NM_004626).

<400> 198
gatcccaagc caataaact 19

<210> 199
<211> 19
<212> DNA
<213> Homo sapiens

<220>
<223> siRNA target sequence for WNT11 (NM_004626).

<400> 199
gacagctgcg accttatgt 19

<210> 200
<211> 19
<212> DNA
<213> Homo sapiens

<220>
<223> siRNA target sequence for WNT11 (NM_004626).

<400> 200
gcgacagctg cgaccttat 19

<210> 201
<211> 19
<212> DNA
<213> Homo sapiens

<220>
<223> siRNA target sequence for WNT11 (NM_004626).

<400> 201
ccggcgtgtg ctatggcat 19

<210> 202
<211> 19
<212> DNA
<213> Homo sapiens

<220>
<223> siRNA target sequence for WNT11 (NM_004626).

<400> 202
gtgtgctatg gcatcaagt 19

<210> 203
<211> 19
<212> DNA
<213> Homo sapiens

<220>
<223> siRNA target sequence for WNT11 (NM_004626).

<400> 203
ctgatgcgtc tacacaaca 19

<210> 204
<211> 19
<212> DNA
<213> Homo sapiens

<220>
<223> siRNA target sequence for WNT11 (NM_004626).

<400> 204
gatgcgtcta cacaacagt 19

<210> 205
<211> 19
<212> DNA
<213> Homo sapiens

<220>
<223> siRNA target sequence for WNT3 (NM_030753).

<400> 205
gctgtgactc gcatcataa 19

<210> 206
<211> 19
<212> DNA

<213> Homo sapiens

<220>
<223> siRNA target sequence for WNT3 (NM_030753).

<400> 206
ctgacttcgg cgtgttagt 19

<210> 207
<211> 19
<212> DNA
<213> Homo sapiens

<220>
<223> siRNA target sequence for WNT3 (NM_030753).

<400> 207
gacttcggcg tgttagtgt 19

<210> 208
<211> 19
<212> DNA
<213> Homo sapiens

<220>
<223> siRNA target sequence for WNT3 (NM_030753).

<400> 208
gaccggactt gcaatgtca 19

<210> 209
<211> 19
<212> DNA
<213> Homo sapiens

<220>
<223> siRNA target sequence for WNT3 (NM_030753).

<400> 209
ctcgctggct acccaattt 19

<210> 210
<211> 19
<212> DNA
<213> Homo sapiens

<220>
<223> siRNA target sequence for WNT3 (NM_030753).

<400> 210
gctggctacc caatttggt 19

<210> 211
<211> 19
<212> DNA
<213> Homo sapiens

<220>
<223> siRNA target sequence for WNT3A (NM_033131).

<400> 211
gccccactcg gatacttct 19

<210> 212
<211> 19
<212> DNA
<213> Homo sapiens

<220>
<223> siRNA target sequence for WNT3A (NM_033131).

<400> 212
ccccactcgg atacttctt 19

<210> 213
<211> 19
<212> DNA
<213> Homo sapiens

<220>
<223> siRNA target sequence for WNT3A (NM_033131).

<400> 213
cccactcgga tacttctta 19

<210> 214
<211> 19
<212> DNA
<213> Homo sapiens

<220>
<223> siRNA target sequence for WNT3A (NM_033131).

<400> 214
gctgttgggc cacagtatt 19

<210> 215

<211> 19
<212> DNA
<213> Homo sapiens

<220>
<223> siRNA target sequence for WNT3A (NM_033131).

<400> 215
gaggcctcgc ccaactict 19

<210> 216
<211> 19
<212> DNA
<213> Homo sapiens

<220>
<223> siRNA target sequence for WNT3A (NM_033131).

<400> 216
ggaactacgt ggagatcat 19

<210> 217
<211> 19
<212> DNA
<213> Homo sapiens

<220>
<223> siRNA target sequence for WNT3A (NM_033131).

<400> 217
ggcagctacc cgatctggt 19

<210> 218
<211> 19
<212> DNA
<213> Homo sapiens

<220>
<223> siRNA target sequence for WNT3A (NM_033131).

<400> 218
gcaggaacta cgtggagat 19

<210> 219
<211> 19
<212> DNA
<213> Homo sapiens

<220>

<223> siRNA target sequence for WNT5A (NM_003392).

<400> 219
gtggtcgcta ggtatgaat 19

<210> 220
<211> 19
<212> DNA
<213> Homo sapiens

<220>
<223> siRNA target sequence for WNT5A (NM_003392).

<400> 220
ggtcgctagg tatgaataa 19

<210> 221
<211> 19
<212> DNA
<213> Homo sapiens

<220>
<223> siRNA target sequence for WNT5A (NM_003392).

<400> 221
ggataacacc tctgttttt 19

<210> 222
<211> 19
<212> DNA
<213> Homo sapiens

<220>
<223> siRNA target sequence for WNT5A (NM_003392).

<400> 222
ccttcgccca ggttgtaat 19

<210> 223
<211> 19
<212> DNA
<213> Homo sapiens

<220>
<223> siRNA target sequence for WNT5A (NM_003392).

<400> 223
cttggtggtc gctaggtat 19

<210> 224
<211> 19
<212> DNA
<213> Homo sapiens

<220>
<223> siRNA target sequence for WNT5A (NM_003392).

<400> 224
ccaactggca ggactttct 19

<210> 225
<211> 19
<212> DNA
<213> Homo sapiens

<220>
<223> siRNA target sequence for WNT5A (NM_003392).

<400> 225
gttcagatgt cagaagtat 19

<210> 226
<211> 19
<212> DNA
<213> Homo sapiens

<220>
<223> siRNA target sequence for WNT5A (NM_003392).

<400> 226
gtatgaataa ccctgttca 19

<210> 227
<211> 19
<212> DNA
<213> Homo sapiens

<220>
<223> siRNA target sequence for CDKL1 (NM_004196).

<400> 227
cgaaacattc cgtgattaa 19

<210> 228
<211> 19
<212> DNA
<213> Homo sapiens

<220>
<223> siRNA target sequence for CDKL1 (NM_004196).

<400> 228
ctcgtgaaga gcataactt 19

<210> 229
<211> 19
<212> DNA
<213> Homo sapiens

<220>
<223> siRNA target sequence for CDKL1 (NM_004196).

<400> 229
ggaccgagtg actactata 19

<210> 230
<211> 19
<212> DNA
<213> Homo sapiens

<220>
<223> siRNA target sequence for CDKL1 (NM_004196).

<400> 230
gttgcatcac ccatatttt 19

<210> 231
<211> 19
<212> DNA
<213> Homo sapiens

<220>
<223> siRNA target sequence for CDKL1 (NM_004196).

<400> 231
cactgcaagc tgtaaattt 19

<210> 232
<211> 19
<212> DNA
<213> Homo sapiens

<220>
<223> siRNA target sequence for CDKL1 (NM_004196).

<400> 232

gatgaccctg tcataaaga 19

<210> 233
<211> 19
<212> DNA
<213> Homo sapiens

<220>
<223> siRNA target sequence for CDKL2 (NM_003948).

<400> 233
gatcagctat atcatatta 19

<210> 234
<211> 19
<212> DNA
<213> Homo sapiens

<220>
<223> siRNA target sequence for CDKL2 (NM_003948).

<400> 234
ccatcaggca tttataaca 19

<210> 235
<211> 19
<212> DNA
<213> Homo sapiens

<220>
<223> siRNA target sequence for CDKL2 (NM_003948).

<400> 235
catcaggcat ttataacat 19

<210> 236
<211> 19
<212> DNA
<213> Homo sapiens

<220>
<223> siRNA target sequence for CDKL2 (NM_003948).

<400> 236
ctgaagtggt gatagattt 19

<210> 237
<211> 19

<212> DNA
<213> Homo sapiens

<220>
<223> siRNA target sequence for CDKL2 (NM_003948).

<400> 237
cagctatatc atattatga 19

<210> 238
<211> 19
<212> DNA
<213> Homo sapiens

<220>
<223> siRNA target sequence for CDKL2 (NM_003948).

<400> 238
gattattaat ggaattgga 19

<210> 239
<211> 19
<212> DNA
<213> Homo sapiens

<220>
<223> siRNA target sequence for CDKL2 (NM_003948).

<400> 239
ggtacaggat accaatgct 19

<210> 240
<211> 19
<212> DNA
<213> Homo sapiens

<220>
<223> siRNA target sequence for CDKL3 (NM_016508).

<400> 240
gagctcccga attagtatt 19

<210> 241
<211> 19
<212> DNA
<213> Homo sapiens

<220>
<223> siRNA target sequence for CDKL3 (NM_016508).

<400> 241
gctcccgaat tagtattaa 19

<210> 242
<211> 19
<212> DNA
<213> Homo sapiens

<220>
<223> siRNA target sequence for CDKL3 (NM_016508).

<400> 242
cacccatcaa tctaactaa 19

<210> 243
<211> 19
<212> DNA
<213> Homo sapiens

<220>
<223> siRNA target sequence for CDKL3 (NM_016508).

<400> 243
ctaactaaca gtaattga 19

<210> 244
<211> 19
<212> DNA
<213> Homo sapiens

<220>
<223> siRNA target sequence for CDKL3 (NM_016508).

<400> 244
ctcccgaatt agtattaaa 19

<210> 245
<211> 19
<212> DNA
<213> Homo sapiens

<220>
<223> siRNA target sequence for CDKL3 (NM_016508).

<400> 245
gttcatgctt gtttacaaa 19

<210> 246
<211> 19
<212> DNA
<213> Homo sapiens

<220>
<223> siRNA target sequence for CDKL3 (NM_016508).

<400> 246
ctttgggctg tatgatcat 19

<210> 247
<211> 19
<212> DNA
<213> Homo sapiens

<220>
<223> siRNA target sequence for CDKL3 (NM_016508).

<400> 247
gcagatatag ttcatgctt 19

<210> 248
<211> 19
<212> DNA
<213> Homo sapiens

<220>
<223> siRNA target sequence for MAPK1 (NM_002745).

<400> 248
gaagacctga attgtataa 19

<210> 249
<211> 19
<212> DNA
<213> Homo sapiens

<220>
<223> siRNA target sequence for MAPK1 (NM_002745).

<400> 249
caaccatcga gcaaatgaa 19

<210> 250
<211> 19
<212> DNA
<213> Homo sapiens

<220>
<223> siRNA target sequence for MAPK1 (NM_002745).

<400> 250
ccaaagctct ggacttatt 19

<210> 251
<211> 19
<212> DNA
<213> Homo sapiens

<220>
<223> siRNA target sequence for MAPK1 (NM_002745).

<400> 251
gacctgaatt gtataataa 19

<210> 252
<211> 19
<212> DNA
<213> Homo sapiens

<220>
<223> siRNA target sequence for MAPK1 (NM_002745).

<400> 252
gtgtgctctg cttatgata 19

<210> 253
<211> 19
<212> DNA
<213> Homo sapiens

<220>
<223> siRNA target sequence for MAPK1 (NM_002745).

<400> 253
cttactgcgc ttcagacat 19

<210> 254
<211> 19
<212> DNA
<213> Homo sapiens

<220>
<223> siRNA target sequence for MAPK1 (NM_002745).

<400> 254
gcttcagaca tgagaacat 19

<210> 255
<211> 19
<212> DNA
<213> Homo sapiens

<220>
<223> siRNA target sequence for MAPK1 (NM_002745).

<400> 255
ctgcgcttca gacatgaga 19

<210> 256
<211> 19
<212> DNA
<213> Homo sapiens

<220>
<223> siRNA target sequence for NLK (NM_016231).

<400> 256
gagtagcgct caaaaagat 19

<210> 257
<211> 19
<212> DNA
<213> Homo sapiens

<220>
<223> siRNA target sequence for NLK (NM_016231).

<400> 257
gcgctaaggc acatatact 19

<210> 258
<211> 19
<212> DNA
<213> Homo sapiens

<220>
<223> siRNA target sequence for NLK (NM_016231).

<400> 258
ctactaggac gaagaatat 19

<210> 259
<211> 19
<212> DNA

<213> Homo sapiens

<220>
<223> siRNA target sequence for NLK (NM_016231).

<400> 259
ctccacacat tgactattt 19

<210> 260
<211> 19
<212> DNA
<213> Homo sapiens

<220>
<223> siRNA target sequence for NLK (NM_016231).

<400> 260
gatttgcga ggtttgaaa 19

<210> 261
<211> 19
<212> DNA
<213> Homo sapiens

<220>
<223> siRNA target sequence for NLK (NM_016231).

<400> 261
gtccgacagg ttaaagaaa 19

<210> 262
<211> 19
<212> DNA
<213> Homo sapiens

<220>
<223> siRNA target sequence for NLK (NM_016231).

<400> 262
ccgacaggtt aaagaaatt 19

<210> 263
<211> 19
<212> DNA
<213> Homo sapiens

<220>
<223> siRNA target sequence for MAPK14 (NM_001315).

<400> 263
ctccgaggtc taaagtata 19

<210> 264
<211> 19
<212> DNA
<213> Homo sapiens

<220>
<223> siRNA target sequence for MAPK14 (NM_001315).

<400> 264
ccgaggtcta aagtatata 19

<210> 265
<211> 19
<212> DNA
<213> Homo sapiens

<220>
<223> siRNA target sequence for MAPK14 (NM_001315).

<400> 265
ggtctgttgg acgtttta 19

<210> 266
<211> 19
<212> DNA
<213> Homo sapiens

<220>
<223> siRNA target sequence for MAPK14 (NM_001315).

<400> 266
ctgcggttac ttaaacata 19

<210> 267
<211> 19
<212> DNA
<213> Homo sapiens

<220>
<223> siRNA target sequence for MAPK14 (NM_001315).

<400> 267
gaggtctaaa gtatataca 19

<210> 268

<211> 19
<212> DNA
<213> Homo sapiens

<220>
<223> siRNA target sequence for MAPK14 (NM_001315).

<400> 268
gtttcctggt acagaccat 19

<210> 269
<211> 19
<212> DNA
<213> Homo sapiens

<220>
<223> siRNA target sequence for MAPK11 (NM_002751).

<400> 269
gcgacgagca cgttcaatt 19

<210> 270
<211> 19
<212> DNA
<213> Homo sapiens

<220>
<223> siRNA target sequence for MAPK11 (NM_002751).

<400> 270
cccgggaagc gactacatt 19

<210> 271
<211> 19
<212> DNA
<213> Homo sapiens

<220>
<223> siRNA target sequence for MAPK11 (NM_002751).

<400> 271
cgggaagcga ctacattga 19

<210> 272
<211> 19
<212> DNA
<213> Homo sapiens

<220>

<223> siRNA target sequence for MAPK11 (NM_002751).

<400> 272
gaggttctgg caaaaatct 19

<210> 273
<211> 19
<212> DNA
<213> Homo sapiens

<220>
<223> siRNA target sequence for MAPK11 (NM_002751).

<400> 273
ctgaggttct ggcaaaaat 19

<210> 274
<211> 19
<212> DNA
<213> Homo sapiens

<220>
<223> siRNA target sequence for MAPK12 (NM_002969).

<400> 274
gaagcgtgtt acttacaaa 19

<210> 275
<211> 19
<212> DNA
<213> Homo sapiens

<220>
<223> siRNA target sequence for MAPK12 (NM_002969).

<400> 275
gctgctggac gtattcact 19

<210> 276
<211> 19
<212> DNA
<213> Homo sapiens

<220>
<223> siRNA target sequence for MAPK12 (NM_002969).

<400> 276
ggaagcgtgt tacttacaa 19

<210> 277
<211> 19
<212> DNA
<213> Homo sapiens

<220>
<223> siRNA target sequence for MAPK12 (NM_002969).

<400> 277
cccgaggtca tcttgaatt 19

<210> 278
<211> 19
<212> DNA
<213> Homo sapiens

<220>
<223> siRNA target sequence for MAPK12 (NM_002969).

<400> 278
gaatggaagc gtgttactt 19

<210> 279
<211> 19
<212> DNA
<213> Homo sapiens

<220>
<223> siRNA target sequence for MAPK13 (NM_002754).

<400> 279
ctgagccgac cctttcagt 19

<210> 280
<211> 19
<212> DNA
<213> Homo sapiens

<220>
<223> siRNA target sequence for MAPK13 (NM_002754).

<400> 280
cctttcagtc cgagatctt 19

<210> 281
<211> 19
<212> DNA
<213> Homo sapiens

<220>
<223> siRNA target sequence for MAPK13 (NM_002754).

<400> 281
ccttagaaca cgagaaact 19

<210> 282
<211> 19
<212> DNA
<213> Homo sapiens

<220>
<223> siRNA target sequence for MAPK13 (NM_002754).

<400> 282
ccctgcgcaa cttctatga 19

<210> 283
<211> 19
<212> DNA
<213> Homo sapiens

<220>
<223> siRNA target sequence for MAPK13 (NM_002754).

<400> 283
ctgcgcaact tctatgact 19

<210> 284
<211> 19
<212> DNA
<213> Homo sapiens

<220>
<223> siRNA target sequence for MAPK8 (NM_139049).

<400> 284
gacttaaagc ccagtaata 19

<210> 285
<211> 19
<212> DNA
<213> Homo sapiens

<220>
<223> siRNA target sequence for MAPK8 (NM_139049).

<400> 285

gagagctagt tcttatgaa 19

<210> 286
<211> 19
<212> DNA
<213> Homo sapiens

<220>
<223> siRNA target sequence for MAPK8 (NM_139049).

<400> 286
cttaaagccc agtaatata 19

<210> 287
<211> 19
<212> DNA
<213> Homo sapiens

<220>
<223> siRNA target sequence for MAPK8 (NM_139049).

<400> 287
caggaacgag ttttatgat 19

<210> 288
<211> 19
<212> DNA
<213> Homo sapiens

<220>
<223> siRNA target sequence for MAPK8 (NM_139049).

<400> 288
gcaggaacga gttttatga 19

<210> 289
<211> 19
<212> DNA
<213> Homo sapiens

<220>
<223> siRNA target sequence for MAPK8 (NM_139049).

<400> 289
gaaatcccta gaagaattt 19

<210> 290
<211> 19

<212> DNA
<213> Homo sapiens

<220>
<223> siRNA target sequence for MAPK9 (NM_002752).

<400> 290
gttgtgctg cattgata 19

<210> 291
<211> 19
<212> DNA
<213> Homo sapiens

<220>
<223> siRNA target sequence for MAPK9 (NM_002752).

<400> 291
gagcttatcg tgaacttgt 19

<210> 292
<211> 19
<212> DNA
<213> Homo sapiens

<220>
<223> siRNA target sequence for MAPK9 (NM_002752).

<400> 292
gccagagatc tgttatcaa 19

<210> 293
<211> 19
<212> DNA
<213> Homo sapiens

<220>
<223> siRNA target sequence for MAPK9 (NM_002752).

<400> 293
ccagagatct gttatcaaa 19

<210> 294
<211> 19
<212> DNA
<213> Homo sapiens

<220>
<223> siRNA target sequence for MAPK9 (NM_002752).

<400> 294
cagagatctg ttatcaaaa 19

<210> 295
<211> 19
<212> DNA
<213> Homo sapiens

<220>
<223> siRNA target sequence for MAPK10 (NM_002753).

<400> 295
gtggtgacac gttattaca 19

<210> 296
<211> 19
<212> DNA
<213> Homo sapiens

<220>
<223> siRNA target sequence for MAPK10 (NM_002753).

<400> 296
cggactccga gcacaataa 19

<210> 297
<211> 19
<212> DNA
<213> Homo sapiens

<220>
<223> siRNA target sequence for MAPK10 (NM_002753).

<400> 297
ggactccgag cacaataaa 19

<210> 298
<211> 19
<212> DNA
<213> Homo sapiens

<220>
<223> siRNA target sequence for MAPK10 (NM_002753).

<400> 298
gtggaataag gtaattgaa 19

<210> 299
<211> 19
<212> DNA
<213> Homo sapiens

<220>
<223> siRNA target sequence for MAPK10 (NM_002753).

<400> 299
ctaaaaatgg tgtagtaaa 19

<210> 300
<211> 19
<212> DNA
<213> Homo sapiens

<220>
<223> siRNA target sequence for MAPK10 (NM_002753).

<400> 300
ggaaagaact tatctacaa 19

<210> 301
<211> 19
<212> DNA
<213> Homo sapiens

<220>
<223> siRNA target sequence for MAPK10 (NM_002753).

<400> 301
gtagtcaagt ctgattgca 19

<210> 302
<211> 19
<212> DNA
<213> Homo sapiens

<220>
<223> siRNA target sequence for MAPK10 (NM_002753).

<400> 302
gaaatggttc gccacaaaa 19

<210> 303
<211> 19
<212> DNA
<213> Homo sapiens

<220>
<223> siRNA target sequence for CDC2 (NM_001786).

<400> 303
gatttgctct cgaaaatgt 19

<210> 304
<211> 19
<212> DNA
<213> Homo sapiens

<220>
<223> siRNA target sequence for CDC2 (NM_001786).

<400> 304
ctctcgaaaa tgttaatct 19

<210> 305
<211> 19
<212> DNA
<213> Homo sapiens

<220>
<223> siRNA target sequence for CDC2 (NM_001786).

<400> 305
gggcactccc aataatgaa 19

<210> 306
<211> 19
<212> DNA
<213> Homo sapiens

<220>
<223> siRNA target sequence for CDC2 (NM_001786).

<400> 306
ctttacagga ctataagaa 19

<210> 307
<211> 19
<212> DNA
<213> Homo sapiens

<220>
<223> siRNA target sequence for CDC2 (NM_001786).

<400> 307
gagtataggc accatattt 19

<210> 308
<211> 19
<212> DNA
<213> Homo sapiens

<220>
<223> siRNA target sequence for CDC2 (NM_001786).

<400> 308
gacaatcaga ttaagaaga 19

<210> 309
<211> 19
<212> DNA
<213> Homo sapiens

<220>
<223> siRNA target sequence for CDK2 (NM_001798).

<400> 309
ctctacctgg tttttgaat 19

<210> 310
<211> 19
<212> DNA
<213> Homo sapiens

<220>
<223> siRNA target sequence for CDK2 (NM_001798).

<400> 310
cttctatgcc tgattacaa 19

<210> 311
<211> 19
<212> DNA
<213> Homo sapiens

<220>
<223> siRNA target sequence for CDK2 (NM_001798).

<400> 311
gatggacgga gcttgttat 19

<210> 312
<211> 19
<212> DNA

<213> Homo sapiens

<220>
<223> siRNA target sequence for CDK2 (NM_001798).

<400> 312
ctacctggtt tttgaattt 19

<210> 313
<211> 19
<212> DNA
<213> Homo sapiens

<220>
<223> siRNA target sequence for CDK2 (NM_001798).

<400> 313
gcacgtacgg agttgtgta 19

<210> 314
<211> 19
<212> DNA
<213> Homo sapiens

<220>
<223> siRNA target sequence for CDK4 (NM_000075).

<400> 314
cctatgggac agtgtacaa 19

<210> 315
<211> 19
<212> DNA
<213> Homo sapiens

<220>
<223> siRNA target sequence for CDK4 (NM_000075).

<400> 315
gatgtttcgt cgaaagcct 19

<210> 316
<211> 19
<212> DNA
<213> Homo sapiens

<220>
<223> siRNA target sequence for CDK4 (NM_000075).

<400> 316
cgtgaggtgg ctttactga 19

<210> 317
<211> 19
<212> DNA
<213> Homo sapiens

<220>
<223> siRNA target sequence for CDK4 (NM_000075).

<400> 317
ggtgtcggtg cctatggga 19

<210> 318
<211> 19
<212> DNA
<213> Homo sapiens

<220>
<223> siRNA target sequence for CDK4 (NM_000075).

<400> 318
cgaactgacc gggagatca 19

<210> 319
<211> 19
<212> DNA
<213> Homo sapiens

<220>
<223> siRNA target sequence for CDK4 (NM_052984).

<400> 319
gaccgggaga tcaagagat 19

<210> 320
<211> 19
<212> DNA
<213> Homo sapiens

<220>
<223> siRNA target sequence for CDK4 (NM_052984).

<400> 320
cgggagatca agagatgtt 19

<210> 321

<211> 19
<212> DNA
<213> Homo sapiens

<220>
<223> siRNA target sequence for CDK7 (NM_001799).

<400> 321
ggacataaat ctaatatta 19

<210> 322
<211> 19
<212> DNA
<213> Homo sapiens

<220>
<223> siRNA target sequence for CDK7 (NM_001799).

<400> 322
caaattgtcg ccattaaga 19

<210> 323
<211> 19
<212> DNA
<213> Homo sapiens

<220>
<223> siRNA target sequence for CDK7 (NM_001799).

<400> 323
ccccaataga gcttataca 19

<210> 324
<211> 19
<212> DNA
<213> Homo sapiens

<220>
<223> siRNA target sequence for CDK7 (NM_001799).

<400> 324
cgggcaaagc gttatgaga 19

<210> 325
<211> 19
<212> DNA
<213> Homo sapiens

<220>

<223> siRNA target sequence for CDK7 (NM_001799).

<400> 325
gggcaaagcg ttatgagaa 19

<210> 326
<211> 19
<212> DNA
<213> Homo sapiens

<220>
<223> siRNA target sequence for CDK7 (NM_001799).

<400> 326
cctacatgtt gatgactct 19

<210> 327
<211> 19
<212> DNA
<213> Homo sapiens

<220>
<223> siRNA target sequence for STK11 (NM_000455).

<400> 327
ggaggttacg gcacaaaaa 19

<210> 328
<211> 19
<212> DNA
<213> Homo sapiens

<220>
<223> siRNA target sequence for STK11 (NM_000455).

<400> 328
gaggttacgg cacaaaaat 19

<210> 329
<211> 19
<212> DNA
<213> Homo sapiens

<220>
<223> siRNA target sequence for STK11 (NM_000455).

<400> 329
ggttacggca caaaaatgt 19

<210> 330
<211> 19
<212> DNA
<213> Homo sapiens

<220>
<223> siRNA target sequence for STK11 (NM_000455).

<400> 330
cccaaggccg tgtgtatga 19

<210> 331
<211> 19
<212> DNA
<213> Homo sapiens

<220>
<223> siRNA target sequence for STK11 (NM_000455).

<400> 331
ccaaggccgt gtgtatgaa 19

<210> 332
<211> 19
<212> DNA
<213> Homo sapiens

<220>
<223> siRNA target sequence for STK11 (NM_000455).

<400> 332
cagctggttc cggaagaaa 19

<210> 333
<211> 19
<212> DNA
<213> Homo sapiens

<220>
<223> siRNA target sequence for CHEK1 (NM_001274).

<400> 333
cagtatttcg gtataataa 19

<210> 334
<211> 19
<212> DNA
<213> Homo sapiens

<220>
<223> siRNA target sequence for CHEK1 (NM_001274).

<400> 334
gcatggtatt ggaataact 19

<210> 335
<211> 19
<212> DNA
<213> Homo sapiens

<220>
<223> siRNA target sequence for CHEK1 (NM_001274).

<400> 335
gcccctcata cattgataa 19

<210> 336
<211> 19
<212> DNA
<213> Homo sapiens

<220>
<223> siRNA target sequence for CHEK1 (NM_001274).

<400> 336
ccacatgtcc tgatcatat 19

<210> 337
<211> 19
<212> DNA
<213> Homo sapiens

<220>
<223> siRNA target sequence for CHEK1 (NM_001274).

<400> 337
ggcaatatcc aatatttat 19

<210> 338
<211> 19
<212> DNA
<213> Homo sapiens

<220>
<223> siRNA target sequence for CHEK1 (NM_001274).

<400> 338

ggtcctgtgg aatagtact 19

<210> 339
<211> 19
<212> DNA
<213> Homo sapiens

<220>
<223> siRNA target sequence for CHEK1 (NM_001274).

<400> 339
gaaagggata acctcaaaa 19

<210> 340
<211> 19
<212> DNA
<213> Homo sapiens

<220>
<223> siRNA target sequence for CHEK1 (NM_001274).

<400> 340
ctgtggaata gtacttact 19

<210> 341
<211> 19
<212> DNA
<213> Homo sapiens

<220>
<223> siRNA target sequence for PIM1 (NM_002648).

<400> 341
ggccaacctt cgaagaaat 19

<210> 342
<211> 19
<212> DNA
<213> Homo sapiens

<220>
<223> siRNA target sequence for PIM1 (NM_002648).

<400> 342
cgatgggacc cgagtgtat 19

<210> 343
<211> 19

<212> DNA
<213> Homo sapiens

<220>
<223> siRNA target sequence for PIM1 (NM_002648).

<400> 343
gatgggaccc gagtgtata 19

<210> 344
<211> 19
<212> DNA
<213> Homo sapiens

<220>
<223> siRNA target sequence for PIM1 (NM_002648).

<400> 344
ggtttctccg gcgtcatta 19

<210> 345
<211> 19
<212> DNA
<213> Homo sapiens

<220>
<223> siRNA target sequence for PIM1 (NM_002648).

<400> 345
caaccttcga agaaatcca 19

<210> 346
<211> 19
<212> DNA
<213> Homo sapiens

<220>
<223> siRNA target sequence for PIM1 (NM_002648).

<400> 346
ccctggagtc gcagtacca 19

<210> 347
<211> 19
<212> DNA
<213> Homo sapiens

<220>
<223> siRNA target sequence for PIM1 (NM_002648).

<400> 347
gtggagaagg accggattt 19

<210> 348
<211> 19
<212> DNA
<213> Homo sapiens

<220>
<223> siRNA target sequence for PIM2 (NM_006875).

<400> 348
ggggacattc cctttgaga 19

<210> 349
<211> 19
<212> DNA
<213> Homo sapiens

<220>
<223> siRNA target sequence for PIM2 (NM_006875).

<400> 349
ctcgaagtcg cactgctat 19

<210> 350
<211> 19
<212> DNA
<213> Homo sapiens

<220>
<223> siRNA target sequence for PIM2 (NM_006875).

<400> 350
gaagtcgcac tgctatgga 19

<210> 351
<211> 19
<212> DNA
<213> Homo sapiens

<220>
<223> siRNA target sequence for PIM2 (NM_006875).

<400> 351
gaacatcctg atagaccta 19

<210> 352
<211> 19
<212> DNA
<213> Homo sapiens

<220>
<223> siRNA target sequence for PIM2 (NM_006875).

<400> 352
gtggagttgt ccatcgtga 19

<210> 353
<211> 19
<212> DNA
<213> Homo sapiens

<220>
<223> siRNA target sequence for TRB2 (NM_021643).

<400> 353
cttgtatcgg gaaatactt 19

<210> 354
<211> 19
<212> DNA
<213> Homo sapiens

<220>
<223> siRNA target sequence for TRB2 (NM_021643).

<400> 354
gaagagttgt cgtctataa 19

<210> 355
<211> 19
<212> DNA
<213> Homo sapiens

<220>
<223> siRNA target sequence for TRB2 (NM_021643).

<400> 355
gtatcgggaa atacttatt 19

<210> 356
<211> 19
<212> DNA
<213> Homo sapiens

<220>
<223> siRNA target sequence for TRB2 (NM_021643).

<400> 356
ctcaagctgc ggaaattca 19

<210> 357
<211> 19
<212> DNA
<213> Homo sapiens

<220>
<223> siRNA target sequence for TRB2 (NM_021643).

<400> 357
gggagatcgc ggaacaaaa 19

<210> 358
<211> 19
<212> DNA
<213> Homo sapiens

<220>
<223> siRNA target sequence for TRB2 (NM_021643).

<400> 358
gttcttgag cgaagctat 19

<210> 359
<211> 19
<212> DNA
<213> Homo sapiens

<220>
<223> siRNA target sequence for TRB2 (NM_021643).

<400> 359
cccgagactc cgaacttgt 19

<210> 360
<211> 19
<212> DNA
<213> Homo sapiens

<220>
<223> siRNA target sequence for TRIO (NM_007118).

<400> 360
caccaatgcg gataaatta 19

<210> 361
<211> 19
<212> DNA
<213> Homo sapiens

<220>
<223> siRNA target sequence for TRIO (NM_007118).

<400> 361
ccaatgcgga taaattact 19

<210> 362
<211> 19
<212> DNA
<213> Homo sapiens

<220>
<223> siRNA target sequence for TRIO (NM_007118).

<400> 362
gaaatctacg aatttcata 19

<210> 363
<211> 19
<212> DNA
<213> Homo sapiens

<220>
<223> siRNA target sequence for TRIO (NM_007118).

<400> 363
gagcagatcg tcatattca 19

<210> 364
<211> 19
<212> DNA
<213> Homo sapiens

<220>
<223> siRNA target sequence for TRIO (NM_007118).

<400> 364
cctatccgta gcattaaaa 19

<210> 365
<211> 19
<212> DNA

<213> Homo sapiens

<220>
<223> siRNA target sequence for DAPK1 (NM_004938).

<400> 365
caatccgttc gcttgatat 19

<210> 366
<211> 19
<212> DNA
<213> Homo sapiens

<220>
<223> siRNA target sequence for DAPK1 (NM_004938).

<400> 366
ggtgtttcgt cgattatca 19

<210> 367
<211> 19
<212> DNA
<213> Homo sapiens

<220>
<223> siRNA target sequence for DAPK1 (NM_004938).

<400> 367
gtgtttcgtc gattatcaa 19

<210> 368
<211> 19
<212> DNA
<213> Homo sapiens

<220>
<223> siRNA target sequence for DAPK1 (NM_004938).

<400> 368
gaaggtactt cgaaatcat 19

<210> 369
<211> 19
<212> DNA
<213> Homo sapiens

<220>
<223> siRNA target sequence for DAPK1 (NM_004938).

<400> 369
gaaacgttag caaatgtat 19

<210> 370
<211> 19
<212> DNA
<213> Homo sapiens

<220>
<223> siRNA target sequence for DAPK1 (NM_004938).

<400> 370
gggtaataac ctatatcct 19

<210> 371
<211> 19
<212> DNA
<213> Homo sapiens

<220>
<223> siRNA target sequence for DAPK1 (NM_004938).

<400> 371
gaggcgagtt tggatatga 19

<210> 372
<211> 19
<212> DNA
<213> Homo sapiens

<220>
<223> siRNA target sequence for DAPK1 (NM_004938).

<400> 372
ggcccataaa attgacttt 19

<210> 373
<211> 19
<212> DNA
<213> Homo sapiens

<220>
<223> siRNA target sequence for PRKAA2 (NM_006252).

<400> 373
gaaacgagca actatcaaa 19

<210> 374

<211> 19
<212> DNA
<213> Homo sapiens

<220>
<223> siRNA target sequence for PRKAA2 (NM_006252).

<400> 374
gaagattcgc agtttagat 19

<210> 375
<211> 19
<212> DNA
<213> Homo sapiens

<220>
<223> siRNA target sequence for PRKAA2 (NM_006252).

<400> 375
gcaaaccgta tgacattat 19

<210> 376
<211> 19
<212> DNA
<213> Homo sapiens

<220>
<223> siRNA target sequence for PRKAA2 (NM_006252).

<400> 376
ctggcaatta cgtgaaaat 19

<210> 377
<211> 19
<212> DNA
<213> Homo sapiens

<220>
<223> siRNA target sequence for PRKAA2 (NM_006252).

<400> 377
ggcaattacg tgaaaatga 19

<210> 378
<211> 19
<212> DNA
<213> Homo sapiens

<220>

<223> siRNA target sequence for PRKCM (NM_002742).

<400> 378
ggtacgtcaa ggtcttaaa 19

<210> 379
<211> 19
<212> DNA
<213> Homo sapiens

<220>
<223> siRNA target sequence for PRKCM (NM_002742).

<400> 379
gattggatag caaatgtat 19

<210> 380
<211> 19
<212> DNA
<213> Homo sapiens

<220>
<223> siRNA target sequence for PRKCM (NM_002742).

<400> 380
gtacgtcaag gtcttaaat 19

<210> 381
<211> 19
<212> DNA
<213> Homo sapiens

<220>
<223> siRNA target sequence for PRKCM (NM_002742).

<400> 381
ggaaggcgat cttattgaa 19

<210> 382
<211> 19
<212> DNA
<213> Homo sapiens

<220>
<223> siRNA target sequence for PRKCM (NM_002742).

<400> 382
caccctggtg ttgtaaatt 19

<210> 383
<211> 19
<212> DNA
<213> Homo sapiens

<220>
<223> siRNA target sequence for PRKCM (NM_002742).

<400> 383
cataacgaag tttttaatt 19

<210> 384
<211> 19
<212> DNA
<213> Homo sapiens

<220>
<223> siRNA target sequence for PRKCM (NM_002742).

<400> 384
ctatcagacc tggttagat 19

<210> 385
<211> 19
<212> DNA
<213> Homo sapiens

<220>
<223> siRNA target sequence for MKNK1 (NM_003684).

<400> 385
gagtatgccg tcaaaatca 19

<210> 386
<211> 19
<212> DNA
<213> Homo sapiens

<220>
<223> siRNA target sequence for MKNK1 (NM_003684).

<400> 386
caaaatcatc gagaaacaa 19

<210> 387
<211> 19
<212> DNA
<213> Homo sapiens

<220>
<223> siRNA target sequence for MKNK1 (NM_003684).

<400> 387
gatgacacaa ggttttact 19

<210> 388
<211> 19
<212> DNA
<213> Homo sapiens

<220>
<223> siRNA target sequence for MKNK1 (NM_003684).

<400> 388
gtgccgtgag cctacagaa 19

<210> 389
<211> 19
<212> DNA
<213> Homo sapiens

<220>
<223> siRNA target sequence for MKNK1 (NM_003684).

<400> 389
gcaaggaggt tccatctta 19

<210> 390
<211> 19
<212> DNA
<213> Homo sapiens

<220>
<223> siRNA target sequence for MAPKAPK2 (NM_004759).

<400> 390
ccatcaccga gtttatgaa 19

<210> 391
<211> 19
<212> DNA
<213> Homo sapiens

<220>
<223> siRNA target sequence for MAPKAPK2 (NM_004759).

<400> 391

cgaatgggcc agtatgaat 19

<210> 392
<211> 19
<212> DNA
<213> Homo sapiens

<220>
<223> siRNA target sequence for MAPKAPK2 (NM_004759).

<400> 392
cctgagaatc tcttataca 19

<210> 393
<211> 19
<212> DNA
<213> Homo sapiens

<220>
<223> siRNA target sequence for MAPKAPK2 (NM_004759).

<400> 393
gttatacacc gtactatgt 19

<210> 394
<211> 19
<212> DNA
<213> Homo sapiens

<220>
<223> siRNA target sequence for MAPKAPK2 (NM_004759).

<400> 394
gatgtgtacg agaatctgt 19

<210> 395
<211> 19
<212> DNA
<213> Homo sapiens

<220>
<223> siRNA target sequence for CAMK2G (NM_172171).

<400> 395
gagtacgcag caaaaatca 19

<210> 396
<211> 19

<212> DNA
<213> Homo sapiens

<220>
<223> siRNA target sequence for CAMK2G (NM_172171).

<400> 396
ctgctgctgg cgagtaaat 19

<210> 397
<211> 19
<212> DNA
<213> Homo sapiens

<220>
<223> siRNA target sequence for CAMK2G (NM_172171).

<400> 397
ggtacacaac gctacagat 19

<210> 398
<211> 19
<212> DNA
<213> Homo sapiens

<220>
<223> siRNA target sequence for CAMK2G (NM_172171).

<400> 398
gcctagccat cgaagtaca 19

<210> 399
<211> 19
<212> DNA
<213> Homo sapiens

<220>
<223> siRNA target sequence for CAMK2G (NM_172171).

<400> 399
ctgctggcga gtaaatgca 19

<210> 400
<211> 19
<212> DNA
<213> Homo sapiens

<220>
<223> siRNA target sequence for CAMK2G (NM_172171).

<400> 400
ctcgtgtttg accttgtta 19

<210> 401
<211> 19
<212> DNA
<213> Homo sapiens

<220>
<223> siRNA target sequence for CAMK2G (NM_172171).

<400> 401
gcggggtcat cctgtatat 19

<210> 402
<211> 19
<212> DNA
<213> Homo sapiens

<220>
<223> siRNA target sequence for CAMK2A (NM_015981).

<400> 402
ccatcgattc tatttttgaa 19

<210> 403
<211> 19
<212> DNA
<213> Homo sapiens

<220>
<223> siRNA target sequence for CAMK2A (NM_015981).

<400> 403
cttccatcga ttctatttt 19

<210> 404
<211> 19
<212> DNA
<213> Homo sapiens

<220>
<223> siRNA target sequence for CAMK2A (NM_015981).

<400> 404
cggaaacagg aaattataa 19

<210> 405
<211> 19
<212> DNA
<213> Homo sapiens

<220>
<223> siRNA target sequence for CAMK2A (NM_015981).

<400> 405
gcggaaacag gaaatiata 19

<210> 406
<211> 19
<212> DNA
<213> Homo sapiens

<220>
<223> siRNA target sequence for CAMK2A (NM_015981).

<400> 406
gaccattaac ccatccaaa 19

<210> 407
<211> 19
<212> DNA
<213> Homo sapiens

<220>
<223> siRNA target sequence for CAMK2A (NM_015981).

<400> 407
gagtcctaca cgaagatgt 19

<210> 408
<211> 19
<212> DNA
<213> Homo sapiens

<220>
<223> siRNA target sequence for CAMK2A (NM_015981).

<400> 408
ggcagatcgt ccacttcca 19

<210> 409
<211> 19
<212> DNA
<213> Homo sapiens

<220>
<223> siRNA target sequence for CAMK2A (NM_015981).

<400> 409
cagatcgtcc acttccaca 19

<210> 410
<211> 19
<212> DNA
<213> Homo sapiens

<220>
<223> siRNA target sequence for CAMK1G (NM_020439).

<400> 410
ggtcatggta ccagttaaa 19

<210> 411
<211> 19
<212> DNA
<213> Homo sapiens

<220>
<223> siRNA target sequence for CAMK1G (NM_020439).

<400> 411
ggagtctgtc tcattatgt 19

<210> 412
<211> 19
<212> DNA
<213> Homo sapiens

<220>
<223> siRNA target sequence for CAMK1G (NM_020439).

<400> 412
gtggataccc cccattcta 19

<210> 413
<211> 19
<212> DNA
<213> Homo sapiens

<220>
<223> siRNA target sequence for CAMK1G (NM_020439).

<400> 413
ctggattgac ggaaacaca 19

<210> 414
<211> 19
<212> DNA
<213> Homo sapiens

<220>
<223> siRNA target sequence for CAMK1G (NM_020439).

<400> 414
gaaacggagt ctaagcttt 19

<210> 415
<211> 19
<212> DNA
<213> Homo sapiens

<220>
<223> siRNA target sequence for CAMK1G (NM_020439).

<400> 415
gggatcagga gctttctca 19

<210> 416
<211> 19
<212> DNA
<213> Homo sapiens

<220>
<223> siRNA target sequence for CAMK1G (NM_020439).

<400> 416
gcaagtggag gcaagcctt 19

<210> 417
<211> 19
<212> DNA
<213> Homo sapiens

<220>
<223> siRNA target sequence for CHEK2 (NM_007194).

<400> 417
ctcttacatt gcatacata 19

<210> 418
<211> 19
<212> DNA

<213> Homo sapiens

<220>
<223> siRNA target sequence for CHEK2 (NM_007194).

<400> 418
ctcaggaact ctattctat 19

<210> 419
<211> 19
<212> DNA
<213> Homo sapiens

<220>
<223> siRNA target sequence for CHEK2 (NM_007194).

<400> 419
gtttaggagt tattctttt 19

<210> 420
<211> 19
<212> DNA
<213> Homo sapiens

<220>
<223> siRNA target sequence for CHEK2 (NM_007194).

<400> 420
gataaatacc gaacataca 19

<210> 421
<211> 19
<212> DNA
<213> Homo sapiens

<220>
<223> siRNA target sequence for CHEK2 (NM_007194).

<400> 421
cagataaata ccgaacata 19

<210> 422
<211> 19
<212> DNA
<213> Homo sapiens

<220>
<223> siRNA target sequence for CHEK2 (NM_007194).

<400> 422
gtagatgatc agtcagttt 19

<210> 423
<211> 19
<212> DNA
<213> Homo sapiens

<220>
<223> siRNA target sequence for CHEK2 (NM_007194).

<400> 423
gatcagtcag tttatccta 19

<210> 424
<211> 19
<212> DNA
<213> Homo sapiens

<220>
<223> siRNA target sequence for CHEK2 (NM_007194).

<400> 424
ctgtagatga tcagtcagt 19

<210> 425
<211> 19
<212> DNA
<213> Homo sapiens

<220>
<223> siRNA target sequence for PDK1 (NM_002610).

<400> 425
gactcccagt gtataacaa 19

<210> 426
<211> 19
<212> DNA
<213> Homo sapiens

<220>
<223> siRNA target sequence for PDK1 (NM_002610).

<400> 426
catgagtcgc atttcaatt 19

<210> 427

<211> 19
<212> DNA
<213> Homo sapiens

<220>
<223> siRNA target sequence for PDK1 (NM_002610).

<400> 427
ggacaccatc cgttcaatt 19

<210> 428
<211> 19
<212> DNA
<213> Homo sapiens

<220>
<223> siRNA target sequence for PDK1 (NM_002610).

<400> 428
gtctttacgc acaatactt 19

<210> 429
<211> 19
<212> DNA
<213> Homo sapiens

<220>
<223> siRNA target sequence for PDK1 (NM_002610).

<400> 429
ggatgctaaa gctatttat 19

<210> 430
<211> 19
<212> DNA
<213> Homo sapiens

<220>
<223> siRNA target sequence for ADRBK1 (NM_001619).

<400> 430
gggacgtgtt ccagaaatt 19

<210> 431
<211> 19
<212> DNA
<213> Homo sapiens

<220>

<223> siRNA target sequence for ADRBK1 (NM_001619).

<400> 431
gagatcttcg actcataca 19

<210> 432
<211> 19
<212> DNA
<213> Homo sapiens

<220>
<223> siRNA target sequence for ADRBK1 (NM_001619).

<400> 432
gacaaaagc gcatcaaga 19

<210> 433
<211> 19
<212> DNA
<213> Homo sapiens

<220>
<223> siRNA target sequence for ADRBK1 (NM_001619).

<400> 433
gccatacatc gaagagatt 19

<210> 434
<211> 19
<212> DNA
<213> Homo sapiens

<220>
<223> siRNA target sequence for ADRBK1 (NM_001619).

<400> 434
gacgtgttcc agaaattca 19

<210> 435
<211> 19
<212> DNA
<213> Homo sapiens

<220>
<223> siRNA target sequence for ADRBK1 (NM_001619).

<400> 435
caaaaggaat caagttact 19

<210> 436
<211> 19
<212> DNA
<213> Homo sapiens

<220>
<223> siRNA target sequence for ADRBK1 (NM_001619).

<400> 436
cacaaaagga atcaagtta 19

<210> 437
<211> 19
<212> DNA
<213> Homo sapiens

<220>
<223> siRNA target sequence for ADRBK1 (NM_001619).

<400> 437
ccggcagcac aagaccaaa 19

<210> 438
<211> 19
<212> DNA
<213> Homo sapiens

<220>
<223> siRNA target sequence for ADRBK2 (NM_005160).

<400> 438
gagagtcccg gcaaaattt 19

<210> 439
<211> 19
<212> DNA
<213> Homo sapiens

<220>
<223> siRNA target sequence for ADRBK2 (NM_005160).

<400> 439
ggagagtccc ggcaaaatt 19

<210> 440
<211> 19
<212> DNA
<213> Homo sapiens

<220>
<223> siRNA target sequence for ADRBK2 (NM_005160).

<400> 440
cagcatgtct acttacaaa 19

<210> 441
<211> 19
<212> DNA
<213> Homo sapiens

<220>
<223> siRNA target sequence for ADRBK2 (NM_005160).

<400> 441
cagaagtcga caaatttat 19

<210> 442
<211> 19
<212> DNA
<213> Homo sapiens

<220>
<223> siRNA target sequence for ADRBK2 (NM_005160).

<400> 442
gcagaagtcg acaaattta 19

<210> 443
<211> 19
<212> DNA
<213> Homo sapiens

<220>
<223> siRNA target sequence for RPS6KB1 (NM_003161).

<400> 443
ccgatcacct cgaagattt 19

<210> 444
<211> 19
<212> DNA
<213> Homo sapiens

<220>
<223> siRNA target sequence for RPS6KB1 (NM_003161).

<400> 444

caccigcgta igaatctat 19

<210> 445
<211> 19
<212> DNA
<213> Homo sapiens

<220>
<223> siRNA target sequence for RPS6KB1 (NM_003161).

<400> 445
gatcacctcg aagatttat 19

<210> 446
<211> 19
<212> DNA
<213> Homo sapiens

<220>
<223> siRNA target sequence for RPS6KB1 (NM_003161).

<400> 446
gtttgggagc attaatgta 19

<210> 447
<211> 19
<212> DNA
<213> Homo sapiens

<220>
<223> siRNA target sequence for RPS6KB1 (NM_003161).

<400> 447
cgatcacctc gaagattta 19

<210> 448
<211> 19
<212> DNA
<213> Homo sapiens

<220>
<223> siRNA target sequence for RPS6KA6 (NM_014496).

<400> 448
gaaggcttac tcattttgt 19

<210> 449
<211> 19

<212> DNA
<213> Homo sapiens

<220>
<223> siRNA target sequence for RPS6KA6 (NM_014496).

<400> 449
ggaggctagt gatatacta 19

<210> 450
<211> 19
<212> DNA
<213> Homo sapiens

<220>
<223> siRNA target sequence for RPS6KA6 (NM_014496).

<400> 450
gaggctagtg atatacata 19

<210> 451
<211> 19
<212> DNA
<213> Homo sapiens

<220>
<223> siRNA target sequence for RPS6KA6 (NM_014496).

<400> 451
gggaggctag tgatatact 19

<210> 452
<211> 19
<212> DNA
<213> Homo sapiens

<220>
<223> siRNA target sequence for RPS6KA6 (NM_014496).

<400> 452
cttgttacgg atttaatga 19

<210> 453
<211> 19
<212> DNA
<213> Homo sapiens

<220>
<223> siRNA target sequence for RPS6KA6 (NM_014496).

<400> 453
gaaatgagac catgaatat 19

<210> 454
<211> 19
<212> DNA
<213> Homo sapiens

<220>
<223> siRNA target sequence for RPS6KA6 (NM_014496).

<400> 454
gatgcgctat ggacaacat 19

<210> 455
<211> 19
<212> DNA
<213> Homo sapiens

<220>
<223> siRNA target sequence for RPS6KA6 (NM_014496).

<400> 455
ggaatccagc aaatagatt 19

<210> 456
<211> 19
<212> DNA
<213> Homo sapiens

<220>
<223> siRNA target sequence for RPS6KA1 (NM_002953).

<400> 456
ctatggggtg ttgatgttt 19

<210> 457
<211> 19
<212> DNA
<213> Homo sapiens

<220>
<223> siRNA target sequence for RPS6KA1 (NM_002953).

<400> 457
gctgtcaagg tcattgata 19

<210> 458
<211> 19
<212> DNA
<213> Homo sapiens

<220>
<223> siRNA target sequence for RPS6KA1 (NM_002953).

<400> 458
ctgtcaaggt cattgataa 19

<210> 459
<211> 19
<212> DNA
<213> Homo sapiens

<220>
<223> siRNA target sequence for RPS6KA1 (NM_002953).

<400> 459
ggtcctatgg ggtgttgat 19

<210> 460
<211> 19
<212> DNA
<213> Homo sapiens

<220>
<223> siRNA target sequence for RPS6KA1 (NM_002953).

<400> 460
cctatggggt gttgatgtt 19

<210> 461
<211> 19
<212> DNA
<213> Homo sapiens

<220>
<223> siRNA target sequence for RPS6KA1 (NM_002953).

<400> 461
gcgggacagt ggagtacat 19

<210> 462
<211> 19
<212> DNA
<213> Homo sapiens

<220>
<223> siRNA target sequence for RPS6KA1 (NM_002953).

<400> 462
gctaggcatg ccccagttt 19

<210> 463
<211> 19
<212> DNA
<213> Homo sapiens

<220>
<223> siRNA target sequence for RPS6KA1 (NM_002953).

<400> 463
caccaacatg gagtatgct 19

<210> 464
<211> 19
<212> DNA
<213> Homo sapiens

<220>
<223> siRNA target sequence for AKT2 (NM_001626).

<400> 464
ctctaccccc cttaaacaa 19

<210> 465
<211> 19
<212> DNA
<213> Homo sapiens

<220>
<223> siRNA target sequence for AKT2 (NM_001626).

<400> 465
cacaagcgtg gtgaataca 19

<210> 466
<211> 19
<212> DNA
<213> Homo sapiens

<220>
<223> siRNA target sequence for AKT2 (NM_001626).

<400> 466
ctaccccct taaacaact 19

<210> 467
<211> 19
<212> DNA
<213> Homo sapiens

<220>
<223> siRNA target sequence for AKT2 (NM_001626).

<400> 467
cgtggtgaat acatcaaga 19

<210> 468
<211> 19
<212> DNA
<213> Homo sapiens

<220>
<223> siRNA target sequence for AKT2 (NM_001626).

<400> 468
gcaaggcacg ggctaaagt 19

<210> 469
<211> 19
<212> DNA
<213> Homo sapiens

<220>
<223> siRNA target sequence for AKT1 (NM_005163).

<400> 469
gactgacacc aggtatttt 19

<210> 470
<211> 19
<212> DNA
<213> Homo sapiens

<220>
<223> siRNA target sequence for AKT1 (NM_005163).

<400> 470
ctgacaccag gtattttga 19

<210> 471
<211> 19
<212> DNA

<213> Homo sapiens

<220>
<223> siRNA target sequence for AKT1 (NM_005163).

<400> 471
gagactgaca ccaggtatt 19

<210> 472
<211> 19
<212> DNA
<213> Homo sapiens

<220>
<223> siRNA target sequence for AKT1 (NM_005163).

<400> 472
cttctatggc gctgagatt 19

<210> 473
<211> 19
<212> DNA
<213> Homo sapiens

<220>
<223> siRNA target sequence for AKT1 (NM_005163).

<400> 473
cagccctgaa gtactcttt 19

<210> 474
<211> 19
<212> DNA
<213> Homo sapiens

<220>
<223> siRNA target sequence for AKT3 (NM_005465).

<400> 474
ccagtggact actgttata 19

<210> 475
<211> 19
<212> DNA
<213> Homo sapiens

<220>
<223> siRNA target sequence for AKT3 (NM_005465).

<400> 475
cattcatagg atataaaga 19

<210> 476
<211> 19
<212> DNA
<213> Homo sapiens

<220>
<223> siRNA target sequence for AKT3 (NM_005465).

<400> 476
cctctacaac ccatcataa 19

<210> 477
<211> 19
<212> DNA
<213> Homo sapiens

<220>
<223> siRNA target sequence for AKT3 (NM_005465).

<400> 477
gagacagata ctagatatt 19

<210> 478
<211> 19
<212> DNA
<213> Homo sapiens

<220>
<223> siRNA target sequence for AKT3 (NM_005465).

<400> 478
ggaccgcaca cgtttctat 19

<210> 479
<211> 19
<212> DNA
<213> Homo sapiens

<220>
<223> siRNA target sequence for AKT3 (NM_005465).

<400> 479
cagctcagac tattacaat 19

<210> 480

<211> 19
<212> DNA
<213> Homo sapiens

<220>
<223> siRNA target sequence for AKT3 (NM_005465).

<400> 480
gctcagacta ttacaataa 19

<210> 481
<211> 19
<212> DNA
<213> Homo sapiens

<220>
<223> siRNA target sequence for SGK (NM_005627).

<400> 481
ggcctgccgc cttttata 19

<210> 482
<211> 19
<212> DNA
<213> Homo sapiens

<220>
<223> siRNA target sequence for SGK (NM_005627).

<400> 482
gggtctgaac gactttatt 19

<210> 483
<211> 19
<212> DNA
<213> Homo sapiens

<220>
<223> siRNA target sequence for SGK (NM_005627).

<400> 483
gtctgaacga ctttatica 19

<210> 484
<211> 19
<212> DNA
<213> Homo sapiens

<220>

<223> siRNA target sequence for SGK (NM_005627).

<400> 484
ggagcctgag cttatgaat 19

<210> 485
<211> 19
<212> DNA
<213> Homo sapiens

<220>
<223> siRNA target sequence for SGK (NM_005627).

<400> 485
gaggagaagc atattatgt 19

<210> 486
<211> 19
<212> DNA
<213> Homo sapiens

<220>
<223> siRNA target sequence for SGK (NM_005627).

<400> 486
catcgtttat agagactta 19

<210> 487
<211> 19
<212> DNA
<213> Homo sapiens

<220>
<223> siRNA target sequence for SGK (NM_005627).

<400> 487
ctatgcagtc aaagtttta 19

<210> 488
<211> 19
<212> DNA
<213> Homo sapiens

<220>
<223> siRNA target sequence for SGK (NM_005627).

<400> 488
gatcggaaag ggcagtttt 19

<210> 489
<211> 19
<212> DNA
<213> Homo sapiens

<220>
<223> siRNA target sequence for SGK2 (NM_170693).

<400> 489
gtctgatggg gcgttctat 19

<210> 490
<211> 19
<212> DNA
<213> Homo sapiens

<220>
<223> siRNA target sequence for SGK2 (NM_170693).

<400> 490
cagactttct tgagattaa 19

<210> 491
<211> 19
<212> DNA
<213> Homo sapiens

<220>
<223> siRNA target sequence for SGK2 (NM_170693).

<400> 491
gactttcttg agattaaga 19

<210> 492
<211> 19
<212> DNA
<213> Homo sapiens

<220>
<223> siRNA target sequence for SGK2 (NM_170693).

<400> 492
gtggtacccc tgagtactt 19

<210> 493
<211> 19
<212> DNA
<213> Homo sapiens

<220>
<223> siRNA target sequence for SGK2 (NM_170693).

<400> 493
cagtgaaggt actacagaa 19

<210> 494
<211> 19
<212> DNA
<213> Homo sapiens

<220>
<223> siRNA target sequence for SGK2 (NM_170693).

<400> 494
gtgggcctgc gctactcct 19

<210> 495
<211> 19
<212> DNA
<213> Homo sapiens

<220>
<223> siRNA target sequence for SGKL (NM_013257).

<400> 495
caggactaaa cgaattcat 19

<210> 496
<211> 19
<212> DNA
<213> Homo sapiens

<220>
<223> siRNA target sequence for SGKL (NM_013257).

<400> 496
gacaccacta ccacatttt 19

<210> 497
<211> 19
<212> DNA
<213> Homo sapiens

<220>
<223> siRNA target sequence for SGKL (NM_013257).

<400> 497

gtatcttctg actattcta 19

<210> 498
<211> 19
<212> DNA
<213> Homo sapiens

<220>
<223> siRNA target sequence for SGKL (NM_013257).

<400> 498
caccactacc acatttttgt 19

<210> 499
<211> 19
<212> DNA
<213> Homo sapiens

<220>
<223> siRNA target sequence for SGKL (NM_013257).

<400> 499
gttttacgct gctgaaatt 19

<210> 500
<211> 19
<212> DNA
<213> Homo sapiens

<220>
<223> siRNA target sequence for SGKL (NM_013257).

<400> 500
caactgaaaa gctttattt 19

<210> 501
<211> 19
<212> DNA
<213> Homo sapiens

<220>
<223> siRNA target sequence for SGKL (NM_013257).

<400> 501
gaatatttgg tgataattt 19

<210> 502
<211> 19

<212> DNA
<213> Homo sapiens

<220>
<223> siRNA target sequence for SGKL (NM_013257).

<400> 502
ccaagtgtaa gcattccca 19

<210> 503
<211> 19
<212> DNA
<213> Homo sapiens

<220>
<223> siRNA target sequence for PRKCZ (NM_002744).

<400> 503
gcggaacccc gaattacat 19

<210> 504
<211> 19
<212> DNA
<213> Homo sapiens

<220>
<223> siRNA target sequence for PRKCZ (NM_002744).

<400> 504
caagccaagc gctttaaca 19

<210> 505
<211> 19
<212> DNA
<213> Homo sapiens

<220>
<223> siRNA target sequence for PRKCZ (NM_002744).

<400> 505
caaagcctcc catgtttta 19

<210> 506
<211> 19
<212> DNA
<213> Homo sapiens

<220>
<223> siRNA target sequence for PRKCZ (NM_002744).

<400> 506
ccaaatttac gccatgaaa 19

<210> 507
<211> 19
<212> DNA
<213> Homo sapiens

<220>
<223> siRNA target sequence for PRKCZ (NM_002744).

<400> 507
cacgagaggg ggatcatct 19

<210> 508
<211> 19
<212> DNA
<213> Homo sapiens

<220>
<223> siRNA target sequence for PRKCD (NM_006254).

<400> 508
gcggcacccc tgactatat 19

<210> 509
<211> 19
<212> DNA
<213> Homo sapiens

<220>
<223> siRNA target sequence for PRKCD (NM_006254).

<400> 509
ctaccgtgcc acgttttat 19

<210> 510
<211> 19
<212> DNA
<213> Homo sapiens

<220>
<223> siRNA target sequence for PRKCD (NM_006254).

<400> 510
gggacctacg gcaagatct 19

<210> 511
<211> 19
<212> DNA
<213> Homo sapiens

<220>
<223> siRNA target sequence for PRKCD (NM_006254).

<400> 511
gttcgacgcc cacatctat 19

<210> 512
<211> 19
<212> DNA
<213> Homo sapiens

<220>
<223> siRNA target sequence for PRKCD (NM_006254).

<400> 512
cagaaagaac gcttcaaca 19

<210> 513
<211> 19
<212> DNA
<213> Homo sapiens

<220>
<223> siRNA target sequence for PRKCD (NM_006254).

<400> 513
gtgaagcagg gattaaagt 19

<210> 514
<211> 19
<212> DNA
<213> Homo sapiens

<220>
<223> siRNA target sequence for PRKCA (NM_002737).

<400> 514
ggcgtcctgt tgtatgaaa 19

<210> 515
<211> 19
<212> DNA
<213> Homo sapiens

<220>
<223> siRNA target sequence for PRKCA (NM_002737).

<400> 515
gtgacacctg cgatatgaa 19

<210> 516
<211> 19
<212> DNA
<213> Homo sapiens

<220>
<223> siRNA target sequence for PRKCA (NM_002737).

<400> 516
gacgactgtc tgtagaaat 19

<210> 517
<211> 19
<212> DNA
<213> Homo sapiens

<220>
<223> siRNA target sequence for PRKCA (NM_002737).

<400> 517
gaactgtatg caatcaaaa 19

<210> 518
<211> 19
<212> DNA
<213> Homo sapiens

<220>
<223> siRNA target sequence for PRKCA (NM_002737).

<400> 518
gctggttatt gctaacata 19

<210> 519
<211> 19
<212> DNA
<213> Homo sapiens

<220>
<223> siRNA target sequence for PRKCA (NM_002737).

<400> 519
gaagggttct cgtatgtca 19

<210> 520
<211> 19
<212> DNA
<213> Homo sapiens

<220>
<223> siRNA target sequence for PRKCA (NM_002737).

<400> 520
ccattcaagc ccaaagtgt 19

<210> 521
<211> 19
<212> DNA
<213> Homo sapiens

<220>
<223> siRNA target sequence for PRKCA (NM_002737).

<400> 521
gctgtacttc gtcatggaa 19

<210> 522
<211> 19
<212> DNA
<213> Homo sapiens

<220>
<223> siRNA target sequence for PRKCB1 (NM_002738).

<400> 522
cagatcccta cgtaaaact 19

<210> 523
<211> 19
<212> DNA
<213> Homo sapiens

<220>
<223> siRNA target sequence for PRKCB1 (NM_002738).

<400> 523
cattttccg gtatattga 19

<210> 524
<211> 19
<212> DNA

<213> Homo sapiens

<220>
<223> siRNA target sequence for PRKCB1 (NM_002738).

<400> 524
catttaccgt gacctaaaa 19

<210> 525
<211> 19
<212> DNA
<213> Homo sapiens

<220>
<223> siRNA target sequence for PRKCB1 (NM_002738).

<400> 525
gatccctacg taaaactga 19

<210> 526
<211> 19
<212> DNA
<213> Homo sapiens

<220>
<223> siRNA target sequence for PRKCB1 (NM_002738).

<400> 526
ggagccccat gctgtattt 19

<210> 527
<211> 19
<212> DNA
<213> Homo sapiens

<220>
<223> siRNA target sequence for PRKCB1 (NM_002738).

<400> 527
gatgaaactg accgatttt 19

<210> 528
<211> 19
<212> DNA
<213> Homo sapiens

<220>
<223> siRNA target sequence for PRKCB1 (NM_002738).

<400> 528
gaattcgaag gattttcct 19

<210> 529
<211> 19
<212> DNA
<213> Homo sapiens

<220>
<223> siRNA target sequence for PRKCB1 (NM_002738).

<400> 529
ccatggaccg cctgtactt 19

<210> 530
<211> 19
<212> DNA
<213> Homo sapiens

<220>
<223> siRNA target sequence for CLASP1 (NM_015282).

<400> 530
gagccgtatg ggatgtatt 19

<210> 531
<211> 19
<212> DNA
<213> Homo sapiens

<220>
<223> siRNA target sequence for CLASP1 (NM_015282).

<400> 531
gccgagctga cgattatga 19

<210> 532
<211> 19
<212> DNA
<213> Homo sapiens

<220>
<223> siRNA target sequence for CLASP1 (NM_015282).

<400> 532
ccgagctgac gattatgaa 19

<210> 533

<211> 19
<212> DNA
<213> Homo sapiens

<220>
<223> siRNA target sequence for CLASP1 (NM_015282).

<400> 533
gcgatctcga agtgatatt 19

<210> 534
<211> 19
<212> DNA
<213> Homo sapiens

<220>
<223> siRNA target sequence for CLASP1 (NM_015282).

<400> 534
cagtcccggt tgaatgtaa 19

<210> 535
<211> 19
<212> DNA
<213> Homo sapiens

<220>
<223> siRNA target sequence for TFPI (NM_006287).

<400> 535
ctcgacagtg cgaagaatt 19

<210> 536
<211> 19
<212> DNA
<213> Homo sapiens

<220>
<223> siRNA target sequence for TFPI (NM_006287).

<400> 536
cgacagtgcg aagaattta 19

<210> 537
<211> 19
<212> DNA
<213> Homo sapiens

<220>

<223> siRNA target sequence for TFPI (NM_006287).

<400> 537
gacagtgcga agaatttat 19

<210> 538
<211> 19
<212> DNA
<213> Homo sapiens

<220>
<223> siRNA target sequence for TFPI (NM_006287).

<400> 538
cagtgcgaag aatttatat 19

<210> 539
<211> 19
<212> DNA
<213> Homo sapiens

<220>
<223> siRNA target sequence for TFPI (NM_006287).

<400> 539
gaatatgtcg aggttatat 19

<210> 540
<211> 19
<212> DNA
<213> Homo sapiens

<220>
<223> siRNA target sequence for CNK (NM_004073).

<400> 540
gttgactact ccaataagt 19

<210> 541
<211> 19
<212> DNA
<213> Homo sapiens

<220>
<223> siRNA target sequence for CNK (NM_004073).

<400> 541
gcgcctacgc tgtcaaagt 19

<210> 542
<211> 19
<212> DNA
<213> Homo sapiens

<220>
<223> siRNA target sequence for CNK (NM_004073).

<400> 542
cgccacatcg tgcgttttt 19

<210> 543
<211> 19
<212> DNA
<213> Homo sapiens

<220>
<223> siRNA target sequence for CNK (NM_004073).

<400> 543
gggttgacta ctccaataa 19

<210> 544
<211> 19
<212> DNA
<213> Homo sapiens

<220>
<223> siRNA target sequence for CNK (NM_004073).

<400> 544
gcgagaagat cctaaatga 19

<210> 545
<211> 19
<212> DNA
<213> Homo sapiens

<220>
<223> siRNA target sequence for CNK (NM_004073).

<400> 545
cgcatcagcg cgagaagat 19

<210> 546
<211> 19
<212> DNA
<213> Homo sapiens

<220>
<223> siRNA target sequence for CNK (NM_004073).

<400> 546
gcgcgagaag atcctaaat 19

<210> 547
<211> 19
<212> DNA
<213> Homo sapiens

<220>
<223> siRNA target sequence for VRK1 (NM_003384).

<400> 547
ccttgggagg ataattga 19

<210> 548
<211> 19
<212> DNA
<213> Homo sapiens

<220>
<223> siRNA target sequence for VRK1 (NM_003384).

<400> 548
cttccttggg aggataatt 19

<210> 549
<211> 19
<212> DNA
<213> Homo sapiens

<220>
<223> siRNA target sequence for VRK1 (NM_003384).

<400> 549
caccttgtgt tgtaaaagt 19

<210> 550
<211> 19
<212> DNA
<213> Homo sapiens

<220>
<223> siRNA target sequence for VRK1 (NM_003384).

<400> 550

ctlgggagga taatttgaa 19

<210> 551
<211> 19
<212> DNA
<213> Homo sapiens

<220>
<223> siRNA target sequence for VRK1 (NM_003384).

<400> 551
gttacaggtt tatgataat 19

<210> 552
<211> 19
<212> DNA
<213> Homo sapiens

<220>
<223> siRNA target sequence for VRK1 (NM_003384).

<400> 552
gcagctaagc ttaagaatt 19

<210> 553
<211> 19
<212> DNA
<213> Homo sapiens

<220>
<223> siRNA target sequence for VRK1 (NM_003384).

<400> 553
ggactaaaag ctataggaa 19

<210> 554
<211> 19
<212> DNA
<213> Homo sapiens

<220>
<223> siRNA target sequence for VRK2 (NM_006296).

<400> 554
gactaggaat agatttaca 19

<210> 555
<211> 19

<212> DNA
<213> Homo sapiens

<220>
<223> siRNA target sequence for VRK2 (NM_006296).

<400> 555
caagacatgt agtaaaagt 19

<210> 556
<211> 19
<212> DNA
<213> Homo sapiens

<220>
<223> siRNA target sequence for VRK2 (NM_006296).

<400> 556
ggtatgtgct catagttta 19

<210> 557
<211> 19
<212> DNA
<213> Homo sapiens

<220>
<223> siRNA target sequence for VRK2 (NM_006296).

<400> 557
ggtttatctt gcagatatat 19

<210> 558
<211> 19
<212> DNA
<213> Homo sapiens

<220>
<223> siRNA target sequence for VRK2 (NM_006296).

<400> 558
ggatttggat tgatatatt 19

<210> 559
<211> 19
<212> DNA
<213> Homo sapiens

<220>
<223> siRNA target sequence for VRK2 (NM_006296).

<400> 559
ggactttcct acagatatt 19

<210> 560
<211> 19
<212> DNA
<213> Homo sapiens

<220>
<223> siRNA target sequence for VRK2 (NM_006296).

<400> 560
cataatggga caatagagt 19

<210> 561
<211> 19
<212> DNA
<213> Homo sapiens

<220>
<223> siRNA target sequence for VRK2 (NM_006296).

<400> 561
ctacagatat tgtcccaat 19

<210> 562
<211> 19
<212> DNA
<213> Homo sapiens

<220>
<223> siRNA target sequence for MAP3K6 (NM_004672).

<400> 562
ctttctcctc cgaactttt 19

<210> 563
<211> 19
<212> DNA
<213> Homo sapiens

<220>
<223> siRNA target sequence for MAP3K6 (NM_004672).

<400> 563
gatgttggag tttgattat 19

<210> 564
<211> 19
<212> DNA
<213> Homo sapiens

<220>
<223> siRNA target sequence for MAP3K6 (NM_004672).

<400> 564
caaagagctc cggctaata 19

<210> 565
<211> 19
<212> DNA
<213> Homo sapiens

<220>
<223> siRNA target sequence for MAP3K6 (NM_004672).

<400> 565
ccctgcggga ggatgtttt 19

<210> 566
<211> 19
<212> DNA
<213> Homo sapiens

<220>
<223> siRNA target sequence for MAP3K6 (NM_004672).

<400> 566
gccgagcagc ataatgtct 19

<210> 567
<211> 19
<212> DNA
<213> Homo sapiens

<220>
<223> siRNA target sequence for MAP3K6 (NM_004672).

<400> 567
ggactactcg gccatcatt 19

<210> 568
<211> 19
<212> DNA
<213> Homo sapiens

<220>
<223> siRNA target sequence for MAP3K6 (NM_004672).

<400> 568
ctatttccgg gagaccatt 19

<210> 569
<211> 19
<212> DNA
<213> Homo sapiens

<220>
<223> siRNA target sequence for MAP3K6 (NM_004672).

<400> 569
ggctgctcaa gatttctga 19

<210> 570
<211> 19
<212> DNA
<213> Homo sapiens

<220>
<223> siRNA target sequence for MAP3K5 (NM_005923).

<400> 570
gatccactga ccgaaaaat 19

<210> 571
<211> 19
<212> DNA
<213> Homo sapiens

<220>
<223> siRNA target sequence for MAP3K5 (NM_005923).

<400> 571
caggaaagct cgtaattta 19

<210> 572
<211> 19
<212> DNA
<213> Homo sapiens

<220>
<223> siRNA target sequence for MAP3K5 (NM_005923).

<400> 572
ggaaagctcg taatttata 19

<210> 573
<211> 19
<212> DNA
<213> Homo sapiens

<220>
<223> siRNA target sequence for MAP3K5 (NM_005923).

<400> 573
gtacctcaag tctattgta 19

<210> 574
<211> 19
<212> DNA
<213> Homo sapiens

<220>
<223> siRNA target sequence for MAP3K5 (NM_005923).

<400> 574
ctggtaccct ccagtatat 19

<210> 575
<211> 19
<212> DNA
<213> Homo sapiens

<220>
<223> siRNA target sequence for MST1 (NM_020998).

<400> 575
ccgatttacg ccagaaaaa 19

<210> 576
<211> 19
<212> DNA
<213> Homo sapiens

<220>
<223> siRNA target sequence for MST1 (NM_020998).

<400> 576
cgatttacgc cagaaaaat 19

<210> 577
<211> 19
<212> DNA

<213> Homo sapiens

<220>
<223> siRNA target sequence for MST1 (NM_020998).

<400> 577
gatttacgcc agaaaaata 19

<210> 578
<211> 19
<212> DNA
<213> Homo sapiens

<220>
<223> siRNA target sequence for MST1 (NM_020998).

<400> 578
ggtctggacg acaactatt 19

<210> 579
<211> 19
<212> DNA
<213> Homo sapiens

<220>
<223> siRNA target sequence for MST1 (NM_020998).

<400> 579
ccaaaggtac gggtaatga 19

<210> 580
<211> 19
<212> DNA
<213> Homo sapiens

<220>
<223> siRNA target sequence for STK24 (NM_003576).

<400> 580
gctccgcact agatctatt 19

<210> 581
<211> 19
<212> DNA
<213> Homo sapiens

<220>
<223> siRNA target sequence for STK24 (NM_003576).

<400> 581
ctccgcacta gatctatta 19

<210> 582
<211> 19
<212> DNA
<213> Homo sapiens

<220>
<223> siRNA target sequence for STK24 (NM_003576).

<400> 582
ccgcactaga tctattaga 19

<210> 583
<211> 19
<212> DNA
<213> Homo sapiens

<220>
<223> siRNA target sequence for STK24 (NM_003576).

<400> 583
cgcactagat ctattagaa 19

<210> 584
<211> 19
<212> DNA
<213> Homo sapiens

<220>
<223> siRNA target sequence for STK24 (NM_003576).

<400> 584
ctccattcgg agaagaaaa 19

<210> 585
<211> 19
<212> DNA
<213> Homo sapiens

<220>
<223> siRNA target sequence for STK24 (NM_003576).

<400> 585
gttcaaaggc attgacaat 19

<210> 586

<211> 19
<212> DNA
<213> Homo sapiens

<220>
<223> siRNA target sequence for MST4 (NM_016542).

<400> 586
ctgatagatc gttttaaga 19

<210> 587
<211> 19
<212> DNA
<213> Homo sapiens

<220>
<223> siRNA target sequence for MST4 (NM_016542).

<400> 587
gcaagtcgtt gctattaaa 19

<210> 588
<211> 19
<212> DNA
<213> Homo sapiens

<220>
<223> siRNA target sequence for MST4 (NM_016542).

<400> 588
gaagaactcg agaaaagta 19

<210> 589
<211> 19
<212> DNA
<213> Homo sapiens

<220>
<223> siRNA target sequence for MST4 (NM_016542).

<400> 589
ggctcctgaa gttattcaa 19

<210> 590
<211> 19
<212> DNA
<213> Homo sapiens

<220>

<223> siRNA target sequence for MST4 (NM_016542).

<400> 590
gggaattact gctattgaa 19

<210> 591
<211> 19
<212> DNA
<213> Homo sapiens

<220>
<223> siRNA target sequence for MST4 (NM_016542).

<400> 591
caatgagagt tctgtttct 19

<210> 592
<211> 19
<212> DNA
<213> Homo sapiens

<220>
<223> siRNA target sequence for MST4 (NM_016542).

<400> 592
gataatcaca cctgcattt 19

<210> 593
<211> 19
<212> DNA
<213> Homo sapiens

<220>
<223> siRNA target sequence for PAK1 (NM_002576).

<400> 593
gcccctccga tgagaaata 19

<210> 594
<211> 19
<212> DNA
<213> Homo sapiens

<220>
<223> siRNA target sequence for PAK1 (NM_002576).

<400> 594
ggcgatccta agaagaaat 19

<210> 595
<211> 19
<212> DNA
<213> Homo sapiens

<220>
<223> siRNA target sequence for PAK1 (NM_002576).

<400> 595
caaataacgg cctagacat 19

<210> 596
<211> 19
<212> DNA
<213> Homo sapiens

<220>
<223> siRNA target sequence for PAK1 (NM_002576).

<400> 596
ccgattttac cgatccatt 19

<210> 597
<211> 19
<212> DNA
<213> Homo sapiens

<220>
<223> siRNA target sequence for PAK1 (NM_002576).

<400> 597
gggttgttat ggaatactt 19

<210> 598
<211> 19
<212> DNA
<213> Homo sapiens

<220>
<223> siRNA target sequence for PAK1 (NM_002576).

<400> 598
catcaagagt gacaatatt 19

<210> 599
<211> 19
<212> DNA
<213> Homo sapiens

<220>
<223> siRNA target sequence for PAK1 (NM_002576).

<400> 599
gctgtgggtt gttatggaa 19

<210> 600
<211> 19
<212> DNA
<213> Homo sapiens

<220>
<223> siRNA target sequence for PAK2 (NM_002577).

<400> 600
cataggtgac cctaagaaa 19

<210> 601
<211> 19
<212> DNA
<213> Homo sapiens

<220>
<223> siRNA target sequence for PAK2 (NM_002577).

<400> 601
cccaacatcg ttaactttt 19

<210> 602
<211> 19
<212> DNA
<213> Homo sapiens

<220>
<223> siRNA target sequence for PAK2 (NM_002577).

<400> 602
ccaacatcgt taactttt t 19

<210> 603
<211> 19
<212> DNA
<213> Homo sapiens

<220>
<223> siRNA target sequence for PAK2 (NM_002577).

<400> 603

ccggatcata cgaaatcaa 19

<210> 604
<211> 19
<212> DNA
<213> Homo sapiens

<220>
<223> siRNA target sequence for PAK2 (NM_002577).

<400> 604
cggatcatac gaaatcaat 19

<210> 605
<211> 19
<212> DNA
<213> Homo sapiens

<220>
<223> siRNA target sequence for PAK3 (NM_002578).

<400> 605
catccttcga gtacaaaaa 19

<210> 606
<211> 19
<212> DNA
<213> Homo sapiens

<220>
<223> siRNA target sequence for PAK3 (NM_002578).

<400> 606
ctgtattccg tgactttt t 19

<210> 607
<211> 19
<212> DNA
<213> Homo sapiens

<220>
<223> siRNA target sequence for PAK3 (NM_002578).

<400> 607
gtattccgtg actttttaa 19

<210> 608
<211> 19

<212> DNA
<213> Homo sapiens

<220>
<223> siRNA target sequence for PAK3 (NM_002578).

<400> 608
cacagatcgg caaagaaaa 19

<210> 609
<211> 19
<212> DNA
<213> Homo sapiens

<220>
<223> siRNA target sequence for PAK3 (NM_002578).

<400> 609
ctgacggtct ggataatga 19

<210> 610
<211> 19
<212> DNA
<213> Homo sapiens

<220>
<223> siRNA target sequence for PAK3 (NM_002578).

<400> 610
cccccttacc ttaatgaaa 19

<210> 611
<211> 19
<212> DNA
<213> Homo sapiens

<220>
<223> siRNA target sequence for PAK3 (NM_002578).

<400> 611
cagactttga gcatacgat 19

<210> 612
<211> 19
<212> DNA
<213> Homo sapiens

<220>
<223> siRNA target sequence for PAK3 (NM_002578).

<400> 612
gcagtcaccg gggaattca 19

<210> 613
<211> 19
<212> DNA
<213> Homo sapiens

<220>
<223> siRNA target sequence for PAK4 (NM_005884).

<400> 613
gggataatgg tgattgaga 19

<210> 614
<211> 19
<212> DNA
<213> Homo sapiens

<220>
<223> siRNA target sequence for PAK4 (NM_005884).

<400> 614
ggataatggt gattgagat 19

<210> 615
<211> 19
<212> DNA
<213> Homo sapiens

<220>
<223> siRNA target sequence for PAK4 (NM_005884).

<400> 615
gccacagcga gtatcccat 19

<210> 616
<211> 19
<212> DNA
<213> Homo sapiens

<220>
<223> siRNA target sequence for PAK4 (NM_005884).

<400> 616
cagcacgagc agaagttca 19

<210> 617
<211> 19
<212> DNA
<213> Homo sapiens

<220>
<223> siRNA target sequence for PAK4 (NM_005884).

<400> 617
ggtcgctggg gataatggt 19

<210> 618
<211> 19
<212> DNA
<213> Homo sapiens

<220>
<223> siRNA target sequence for MAP2K1 (NM_002755).

<400> 618
ggccagaaag ctaattcat 19

<210> 619
<211> 19
<212> DNA
<213> Homo sapiens

<220>
<223> siRNA target sequence for MAP2K1 (NM_002755).

<400> 619
gcgatggcga gatcagtat 19

<210> 620
<211> 19
<212> DNA
<213> Homo sapiens

<220>
<223> siRNA target sequence for MAP2K1 (NM_002755).

<400> 620
gatggcgaga tcagtatct 19

<210> 621
<211> 19
<212> DNA
<213> Homo sapiens

<220>
<223> siRNA target sequence for MAP2K1 (NM_002755).

<400> 621
ctacatgtcg ccagaaaga 19

<210> 622
<211> 19
<212> DNA
<213> Homo sapiens

<220>
<223> siRNA target sequence for MAP2K1 (NM_002755).

<400> 622
ccaccatcgg ccttaacca 19

<210> 623
<211> 19
<212> DNA
<213> Homo sapiens

<220>
<223> siRNA target sequence for MAP2K1 (NM_002755).

<400> 623
gacctcccat ggcaatttt 19

<210> 624
<211> 19
<212> DNA
<213> Homo sapiens

<220>
<223> siRNA target sequence for MAP2K1 (NM_002755).

<400> 624
ctcccatggc aattttga 19

<210> 625
<211> 19
<212> DNA
<213> Homo sapiens

<220>
<223> siRNA target sequence for MAP2K1 (NM_002755).

<400> 625
cgacctccca tggcaattt 19

<210> 626
<211> 19
<212> DNA
<213> Homo sapiens

<220>
<223> siRNA target sequence for MAP2K2 (NM_030662).

<400> 626
gccggctggt tgtgtaaaa 19

<210> 627
<211> 19
<212> DNA
<213> Homo sapiens

<220>
<223> siRNA target sequence for MAP2K2 (NM_030662).

<400> 627
caaggtcggc gaactcaaa 19

<210> 628
<211> 19
<212> DNA
<213> Homo sapiens

<220>
<223> siRNA target sequence for MAP2K2 (NM_030662).

<400> 628
ctcctggact atattgtga 19

<210> 629
<211> 19
<212> DNA
<213> Homo sapiens

<220>
<223> siRNA target sequence for MAP2K2 (NM_030662).

<400> 629
ccaaggtcgg cgaactcaa 19

<210> 630
<211> 19
<212> DNA

<213> Homo sapiens

<220>
<223> siRNA target sequence for MAP2K2 (NM_030662).

<400> 630
ggttgcaggg cacacatta 19

<210> 631
<211> 19
<212> DNA
<213> Homo sapiens

<220>
<223> siRNA target sequence for MAP2K3 (NM_002756).

<400> 631
cgcacggtcg actgtttct 19

<210> 632
<211> 19
<212> DNA
<213> Homo sapiens

<220>
<223> siRNA target sequence for MAP2K3 (NM_002756).

<400> 632
gcacggtcga ctgtttcta 19

<210> 633
<211> 19
<212> DNA
<213> Homo sapiens

<220>
<223> siRNA target sequence for MAP2K3 (NM_002756).

<400> 633
ctacggggca ctattcaga 19

<210> 634
<211> 19
<212> DNA
<213> Homo sapiens

<220>
<223> siRNA target sequence for MAP2K3 (NM_002756).

<400> 634
cctlctacgg ggcactatt 19

<210> 635
<211> 19
<212> DNA
<213> Homo sapiens

<220>
<223> siRNA target sequence for MAP2K3 (NM_002756).

<400> 635
gactcccgga ccttcatca 19

<210> 636
<211> 19
<212> DNA
<213> Homo sapiens

<220>
<223> siRNA target sequence for MAP2K3 (NM_002756).

<400> 636
gagcctatgg ggtggtaga 19

<210> 637
<211> 19
<212> DNA
<213> Homo sapiens

<220>
<223> siRNA target sequence for MAP2K3 (NM_002756).

<400> 637
ctggactccc ggaccttca 19

<210> 638
<211> 19
<212> DNA
<213> Homo sapiens

<220>
<223> siRNA target sequence for MAP2K3 (NM_002756).

<400> 638
gaggctgatg acttggtga 19

<210> 639

<211> 19
<212> DNA
<213> Homo sapiens

<220>
<223> siRNA target sequence for MAP2K6 (NM_002758).

<400> 639
ggatacatca ctagataaa 19

<210> 640
<211> 19
<212> DNA
<213> Homo sapiens

<220>
<223> siRNA target sequence for MAP2K6 (NM_002758).

<400> 640
gatacatcac tagataaat 19

<210> 641
<211> 19
<212> DNA
<213> Homo sapiens

<220>
<223> siRNA target sequence for MAP2K6 (NM_002758).

<400> 641
cttcgattic cctatgatt 19

<210> 642
<211> 19
<212> DNA
<213> Homo sapiens

<220>
<223> siRNA target sequence for MAP2K6 (NM_002758).

<400> 642
cttttatggc gcactgttt 19

<210> 643
<211> 19
<212> DNA
<213> Homo sapiens

<220>

<223> siRNA target sequence for MAP2K6 (NM_002758).

<400> 643
catcactaga taaattcta 19

<210> 644
<211> 19
<212> DNA
<213> Homo sapiens

<220>
<223> siRNA target sequence for MAP2K6 (NM_002758).

<400> 644
ggacggtgga ctgtccatt 19

<210> 645
<211> 19
<212> DNA
<213> Homo sapiens

<220>
<223> siRNA target sequence for MAP2K6 (NM_002758).

<400> 645
caaacaagtt attgataaa 19

<210> 646
<211> 19
<212> DNA
<213> Homo sapiens

<220>
<223> siRNA target sequence for MAP2K6 (NM_002758).

<400> 646
ctacaaacaa gttattgat 19

<210> 647
<211> 19
<212> DNA
<213> Homo sapiens

<220>
<223> siRNA target sequence for MAP2K4 (NM_003010).

<400> 647
ctacctcgtt tgataagtt 19

<210> 648
<211> 19
<212> DNA
<213> Homo sapiens

<220>
<223> siRNA target sequence for MAP2K4 (NM_003010).

<400> 648
gcatgctatg tttgtaaaa 19

<210> 649
<211> 19
<212> DNA
<213> Homo sapiens

<220>
<223> siRNA target sequence for MAP2K4 (NM_003010).

<400> 649
ccaaaaggcc aaagtataa 19

<210> 650
<211> 19
<212> DNA
<213> Homo sapiens

<220>
<223> siRNA target sequence for MAP2K4 (NM_003010).

<400> 650
cgcatgctat gtttgtaaa 19

<210> 651
<211> 19
<212> DNA
<213> Homo sapiens

<220>
<223> siRNA target sequence for MAP2K4 (NM_003010).

<400> 651
caaaaggcca aagtataaa 19

<210> 652
<211> 19
<212> DNA
<213> Homo sapiens

<220>
<223> siRNA target sequence for MAP2K4 (NM_003010).

<400> 652
gtaatgcgga gtagtgatt 19

<210> 653
<211> 19
<212> DNA
<213> Homo sapiens

<220>
<223> siRNA target sequence for IRAK4 (NM_016123).

<400> 653
gccaatgtcg gcatgaaaa 19

<210> 654
<211> 19
<212> DNA
<213> Homo sapiens

<220>
<223> siRNA target sequence for IRAK4 (NM_016123).

<400> 654
gctttgcgtg gagaaataa 19

<210> 655
<211> 19
<212> DNA
<213> Homo sapiens

<220>
<223> siRNA target sequence for IRAK4 (NM_016123).

<400> 655
ctcaatgttg gactaatta 19

<210> 656
<211> 19
<212> DNA
<213> Homo sapiens

<220>
<223> siRNA target sequence for IRAK4 (NM_016123).

<400> 656

cctctgctta gtatatgtt 19

<210> 657
<211> 19
<212> DNA
<213> Homo sapiens

<220>
<223> siRNA target sequence for IRAK4 (NM_016123).

<400> 657
gttattgcta gatattaaa 19

<210> 658
<211> 19
<212> DNA
<213> Homo sapiens

<220>
<223> siRNA target sequence for RAF1 (NM_002880).

<400> 658
gatcttagta agctatata 19

<210> 659
<211> 19
<212> DNA
<213> Homo sapiens

<220>
<223> siRNA target sequence for RAF1 (NM_002880).

<400> 659
gcatgactgc cttatgaaa 19

<210> 660
<211> 19
<212> DNA
<213> Homo sapiens

<220>
<223> siRNA target sequence for RAF1 (NM_002880).

<400> 660
ctatggcatc gtattgtat 19

<210> 661
<211> 19

<212> DNA
<213> Homo sapiens

<220>
<223> siRNA target sequence for RAF1 (NM_002880).

<400> 661
cttagtaagc tatataaga 19

<210> 662
<211> 19
<212> DNA
<213> Homo sapiens

<220>
<223> siRNA target sequence for RAF1 (NM_002880).

<400> 662
cagacaactc ttattgttt 19

<210> 663
<211> 19
<212> DNA
<213> Homo sapiens

<220>
<223> siRNA target sequence for ACVRL1 (NM_000020).

<400> 663
caagaagaca ctacaaaaa 19

<210> 664
<211> 19
<212> DNA
<213> Homo sapiens

<220>
<223> siRNA target sequence for ACVRL1 (NM_000020).

<400> 664
gagactgaga tctataaca 19

<210> 665
<211> 19
<212> DNA
<213> Homo sapiens

<220>
<223> siRNA target sequence for ACVRL1 (NM_000020).

<400> 665
gaagacacta caaaaaatt 19

<210> 666
<211> 19
<212> DNA
<213> Homo sapiens

<220>
<223> siRNA target sequence for ACVRL1 (NM_000020).

<400> 666
gagatctata acacagtat 19

<210> 667
<211> 19
<212> DNA
<213> Homo sapiens

<220>
<223> siRNA target sequence for ACVRL1 (NM_000020).

<400> 667
gctccctcta cgactttct 19

<210> 668
<211> 19
<212> DNA
<213> Homo sapiens

<220>
<223> siRNA target sequence for ACVR1 (NM_001105).

<400> 668
cacagcactg cgtatcaaa 19

<210> 669
<211> 19
<212> DNA
<213> Homo sapiens

<220>
<223> siRNA target sequence for ACVR1 (NM_001105).

<400> 669
gttgctctcc gaaaattta 19

<210> 670
<211> 19
<212> DNA
<213> Homo sapiens

<220>
<223> siRNA target sequence for ACVR1 (NM_001105).

<400> 670
gctctccgaa aatttaaaa 19

<210> 671
<211> 19
<212> DNA
<213> Homo sapiens

<220>
<223> siRNA target sequence for ACVR1 (NM_001105).

<400> 671
gcactgcgta tcaaaaaga 19

<210> 672
<211> 19
<212> DNA
<213> Homo sapiens

<220>
<223> siRNA target sequence for ACVR1 (NM_001105).

<400> 672
cagcactgcg tatcaaaaa 19

<210> 673
<211> 19
<212> DNA
<213> Homo sapiens

<220>
<223> siRNA target sequence for ACVR1 (NM_001105).

<400> 673
caatgaccca agttttgaa 19

<210> 674
<211> 19
<212> DNA
<213> Homo sapiens

<220>
<223> siRNA target sequence for ACVR1 (NM_001105).

<400> 674
gttctcagac ccgacatta 19

<210> 675
<211> 19
<212> DNA
<213> Homo sapiens

<220>
<223> siRNA target sequence for ACVR1 (NM_001105).

<400> 675
caaggggact ggtgtaaca 19

<210> 676
<211> 19
<212> DNA
<213> Homo sapiens

<220>
<223> siRNA target sequence for ACVR1B (NM_004302).

<400> 676
cccgaaccat cgtttaca 19

<210> 677
<211> 19
<212> DNA
<213> Homo sapiens

<220>
<223> siRNA target sequence for ACVR1B (NM_004302).

<400> 677
ccgaaccatc gtttacaa 19

<210> 678
<211> 19
<212> DNA
<213> Homo sapiens

<220>
<223> siRNA target sequence for ACVR1B (NM_004302).

<400> 678
caattgaggg gatgattaa 19

<210> 679
<211> 19
<212> DNA
<213> Homo sapiens

<220>
<223> siRNA target sequence for ACVR1B (NM_004302).

<400> 679
cacgggtccc tgtttgatt 19

<210> 680
<211> 19
<212> DNA
<213> Homo sapiens

<220>
<223> siRNA target sequence for ACVR1B (NM_004302).

<400> 680
cgggtccctg tttgattat 19

<210> 681
<211> 19
<212> DNA
<213> Homo sapiens

<220>
<223> siRNA target sequence for ACVR1B (NM_004302).

<400> 681
gggtggggac caaacgata 19

<210> 682
<211> 19
<212> DNA
<213> Homo sapiens

<220>
<223> siRNA target sequence for ACVR1B (NM_004302).

<400> 682
cctggctgtc cgtcatgat 19

<210> 683
<211> 19
<212> DNA

<213> Homo sapiens

<220>
<223> siRNA target sequence for ACVR1B (NM_004302).

<400> 683
gtggggacca aacgataca 19

<210> 684
<211> 19
<212> DNA
<213> Homo sapiens

<220>
<223> siRNA target sequence for ACVR1C (NM_145259).

<400> 684
ctgctcttcg tattaagaa 19

<210> 685
<211> 19
<212> DNA
<213> Homo sapiens

<220>
<223> siRNA target sequence for ACVR1C (NM_145259).

<400> 685
gctcatcgag acataaaat 19

<210> 686
<211> 19
<212> DNA
<213> Homo sapiens

<220>
<223> siRNA target sequence for ACVR1C (NM_145259).

<400> 686
gctccttata tgactattt 19

<210> 687
<211> 19
<212> DNA
<213> Homo sapiens

<220>
<223> siRNA target sequence for ACVR1C (NM_145259).

<400> 687
catcgagaca taaaatcaa 19

<210> 688
<211> 19
<212> DNA
<213> Homo sapiens

<220>
<223> siRNA target sequence for ACVR1C (NM_145259).

<400> 688
gtaccaattg ccttattat 19

<210> 689
<211> 19
<212> DNA
<213> Homo sapiens

<220>
<223> siRNA target sequence for TGFBR1 (NM_004612).

<400> 689
cgagataggc cgtttgtat 19

<210> 690
<211> 19
<212> DNA
<213> Homo sapiens

<220>
<223> siRNA target sequence for TGFBR1 (NM_004612).

<400> 690
gcattgcgga ttaagaaaa 19

<210> 691
<211> 19
<212> DNA
<213> Homo sapiens

<220>
<223> siRNA target sequence for TGFBR1 (NM_004612).

<400> 691
ccatcgagtg ccaaatgaa 19

<210> 692

<211> 19
<212> DNA
<213> Homo sapiens

<220>
<223> siRNA target sequence for TGFBR1 (NM_004612).

<400> 692
cattagatcg cccttttat 19

<210> 693
<211> 19
<212> DNA
<213> Homo sapiens

<220>
<223> siRNA target sequence for TGFBR1 (NM_004612).

<400> 693
cagcattgcg gattaagaa 19

<210> 694
<211> 19
<212> DNA
<213> Homo sapiens

<220>
<223> siRNA target sequence for TGFBR1 (NM_004612).

<400> 694
gttggtgtca gattatcat 19

<210> 695
<211> 19
<212> DNA
<213> Homo sapiens

<220>
<223> siRNA target sequence for TGFBR1 (NM_004612).

<400> 695
caacatattg ctgcaatca 19

<210> 696
<211> 19
<212> DNA
<213> Homo sapiens

<220>

<223> siRNA target sequence for TGFBR1 (NM_004612).

<400> 696
gattatcatg agcatggat 19

<210> 697
<211> 19
<212> DNA
<213> Homo sapiens

<220>
<223> siRNA target sequence for EIF2AK3 (NM_004836).

<400> 697
catagcaaca acgtttatt 19

<210> 698
<211> 19
<212> DNA
<213> Homo sapiens

<220>
<223> siRNA target sequence for EIF2AK3 (NM_004836).

<400> 698
catatgataa tggttatta 19

<210> 699
<211> 19
<212> DNA
<213> Homo sapiens

<220>
<223> siRNA target sequence for EIF2AK3 (NM_004836).

<400> 699
ggtaatgcga gaagttaaa 19

<210> 700
<211> 19
<212> DNA
<213> Homo sapiens

<220>
<223> siRNA target sequence for EIF2AK3 (NM_004836).

<400> 700
ctaatgaaaa cgcaattat 19

<210> 701
<211> 19
<212> DNA
<213> Homo sapiens

<220>
<223> siRNA target sequence for EIF2AK3 (NM_004836).

<400> 701
ctttgaactt cggtatatt 19

<210> 702
<211> 19
<212> DNA
<213> Homo sapiens

<220>
<223> siRNA target sequence for EIF2AK3 (NM_004836).

<400> 702
cactttgaac ttcggtata 19

<210> 703
<211> 19
<212> DNA
<213> Homo sapiens

<220>
<223> siRNA target sequence for EIF2AK3 (NM_004836).

<400> 703
gaatgggagt accagtttt 19

<210> 704
<211> 19
<212> DNA
<213> Homo sapiens

<220>
<223> siRNA target sequence for ERN1 (NM_001433).

<400> 704
cattgcacga gaattgata 19

<210> 705
<211> 19
<212> DNA
<213> Homo sapiens

<220>
<223> siRNA target sequence for ERN1 (NM_001433).

<400> 705
caggctgcgt ctttacta 19

<210> 706
<211> 19
<212> DNA
<213> Homo sapiens

<220>
<223> siRNA target sequence for ERN1 (NM_001433).

<400> 706
cgtgagcgac agaatagaa 19

<210> 707
<211> 19
<212> DNA
<213> Homo sapiens

<220>
<223> siRNA target sequence for ERN1 (NM_001433).

<400> 707
ccaaacatcg ggaaaatgt 19

<210> 708
<211> 19
<212> DNA
<213> Homo sapiens

<220>
<223> siRNA target sequence for ERN1 (NM_001433).

<400> 708
ggacatctgg tatgttatt 19

<210> 709
<211> 19
<212> DNA
<213> Homo sapiens

<220>
<223> siRNA target sequence for ERN1 (NM_001433).

<400> 709

cccatgccga agttcagat 19

<210> 710
<211> 19
<212> DNA
<213> Homo sapiens

<220>
<223> siRNA target sequence for ERN1 (NM_001433).

<400> 710
ctacacggtg gacatcttt 19

<210> 711
<211> 19
<212> DNA
<213> Homo sapiens

<220>
<223> siRNA target sequence for ERN1 (NM_001433).

<400> 711
gccgaagttc agatggaat 19

<210> 712
<211> 19
<212> DNA
<213> Homo sapiens

<220>
<223> siRNA target sequence for CHUK (NM_001278).

<400> 712
ggagaagttc ggtttagta 19

<210> 713
<211> 19
<212> DNA
<213> Homo sapiens

<220>
<223> siRNA target sequence for CHUK (NM_001278).

<400> 713
ggccctcagt aatatcaaa 19

<210> 714
<211> 19

<212> DNA
<213> Homo sapiens

<220>
<223> siRNA target sequence for CHUK (NM_001278).

<400> 714
gacctgttga ccttacttt 19

<210> 715
<211> 19
<212> DNA
<213> Homo sapiens

<220>
<223> siRNA target sequence for CHUK (NM_001278).

<400> 715
ggccatttaa gcactatta 19

<210> 716
<211> 19
<212> DNA
<213> Homo sapiens

<220>
<223> siRNA target sequence for CHUK (NM_001278).

<400> 716
gccatttaag cactattat 19

<210> 717
<211> 19
<212> DNA
<213> Homo sapiens

<220>
<223> siRNA target sequence for CHUK (NM_001278).

<400> 717
ctggatatag gcctttttt 19

<210> 718
<211> 19
<212> DNA
<213> Homo sapiens

<220>
<223> siRNA target sequence for CHUK (NM_001278).

<400> 718
gttaagtctt cttagatat 19

<210> 719
<211> 19
<212> DNA
<213> Homo sapiens

<220>
<223> siRNA target sequence for CHUK (NM_001278).

<400> 719
gtttatctga ttgtgtaaa 19

<210> 720
<211> 19
<212> DNA
<213> Homo sapiens

<220>
<223> siRNA target sequence for IKBKE (NM_014002).

<400> 720
catcgaacgg ctaaataga 19

<210> 721
<211> 19
<212> DNA
<213> Homo sapiens

<220>
<223> siRNA target sequence for IKBKE (NM_014002).

<400> 721
ctggataagg tgaatttca 19

<210> 722
<211> 19
<212> DNA
<213> Homo sapiens

<220>
<223> siRNA target sequence for IKBKE (NM_014002).

<400> 722
cctgcatccc gacatgtat 19

<210> 723
<211> 19
<212> DNA
<213> Homo sapiens

<220>
<223> siRNA target sequence for IKBKE (NM_014002).

<400> 723
ctgcaggcgg attacaaca 19

<210> 724
<211> 19
<212> DNA
<213> Homo sapiens

<220>
<223> siRNA target sequence for IKBKE (NM_014002).

<400> 724
ggataaggtg aatttcagt 19

<210> 725
<211> 19
<212> DNA
<213> Homo sapiens

<220>
<223> siRNA target sequence for IKBKE (NM_014002).

<400> 725
ccactgccag tgtgtacaa 19

<210> 726
<211> 19
<212> DNA
<213> Homo sapiens

<220>
<223> siRNA target sequence for SYK (NM_003177).

<400> 726
caatgacccc gctcttaaa 19

<210> 727
<211> 19
<212> DNA
<213> Homo sapiens

<220>
<223> siRNA target sequence for SYK (NM_003177).

<400> 727
cagctagtcg agcattatt 19

<210> 728
<211> 19
<212> DNA
<213> Homo sapiens

<220>
<223> siRNA target sequence for SYK (NM_003177).

<400> 728
ggtcagcggg tggaataat 19

<210> 729
<211> 19
<212> DNA
<213> Homo sapiens

<220>
<223> siRNA target sequence for SYK (NM_003177).

<400> 729
gctagtcgag cattattct 19

<210> 730
<211> 19
<212> DNA
<213> Homo sapiens

<220>
<223> siRNA target sequence for SYK (NM_003177).

<400> 730
gacatgtcaa ggataagaa 19

<210> 731
<211> 19
<212> DNA
<213> Homo sapiens

<220>
<223> siRNA target sequence for SYK (NM_003177).

<400> 731
gctgatgaaa actactaca 19

<210> 732
<211> 19
<212> DNA
<213> Homo sapiens

<220>
<223> siRNA target sequence for SYK (NM_003177).

<400> 732
gacacagagg tgtacgaga 19

<210> 733
<211> 19
<212> DNA
<213> Homo sapiens

<220>
<223> siRNA target sequence for SYK (NM_003177).

<400> 733
ctgatgaaaa ctactacaa 19

<210> 734
<211> 19
<212> DNA
<213> Homo sapiens

<220>
<223> siRNA target sequence for PTK2 (NM_153831).

<400> 734
gaagagcgat tatatgtta 19

<210> 735
<211> 19
<212> DNA
<213> Homo sapiens

<220>
<223> siRNA target sequence for PTK2 (NM_153831).

<400> 735
gtaatcggtc gaattgaaa 19

<210> 736
<211> 19
<212> DNA

<213> Homo sapiens

<220>
<223> siRNA target sequence for PTK2 (NM_153831).

<400> 736
caatggagcg agtattaaa 19

<210> 737
<211> 19
<212> DNA
<213> Homo sapiens

<220>
<223> siRNA target sequence for PTK2 (NM_153831).

<400> 737
ctggaccggt cgaatgata 19

<210> 738
<211> 19
<212> DNA
<213> Homo sapiens

<220>
<223> siRNA target sequence for PTK2 (NM_153831).

<400> 738
gcaatggagc gagtattaa 19

<210> 739
<211> 19
<212> DNA
<213> Homo sapiens

<220>
<223> siRNA target sequence for PTK2 (NM_153831).

<400> 739
ctccagagtc aatcaattt 19

<210> 740
<211> 19
<212> DNA
<213> Homo sapiens

<220>
<223> siRNA target sequence for PTK2 (NM_153831).

<400> 740
gctccagagt caatcaatt 19

<210> 741
<211> 19
<212> DNA
<213> Homo sapiens

<220>
<223> siRNA target sequence for PTK2 (NM_153831).

<400> 741
gttggtttaa agcgatttt 19

<210> 742
<211> 19
<212> DNA
<213> Homo sapiens

<220>
<223> siRNA target sequence for PTK2B (NM_173174).

<400> 742
ggtcctgaat cgtattctt 19

<210> 743
<211> 19
<212> DNA
<213> Homo sapiens

<220>
<223> siRNA target sequence for PTK2B (NM_173174).

<400> 743
ccccagagtc cattaactt 19

<210> 744
<211> 19
<212> DNA
<213> Homo sapiens

<220>
<223> siRNA target sequence for PTK2B (NM_173174).

<400> 744
ggacgaggac tattacaaa 19

<210> 745

<211> 19
<212> DNA
<213> Homo sapiens

<220>
<223> siRNA target sequence for PTK2B (NM_173174).

<400> 745
gaccccatgg tttatatga 19

<210> 746
<211> 19
<212> DNA
<213> Homo sapiens

<220>
<223> siRNA target sequence for PTK2B (NM_173174).

<400> 746
ggaggtatga ccttcaaat 19

<210> 747
<211> 19
<212> DNA
<213> Homo sapiens

<220>
<223> siRNA target sequence for PTK2B (NM_173174).

<400> 747
gcagcataga gtcagacat 19

<210> 748
<211> 19
<212> DNA
<213> Homo sapiens

<220>
<223> siRNA target sequence for PTK2B (NM_173174).

<400> 748
gtggaggtat gaccttcaa 19

<210> 749
<211> 19
<212> DNA
<213> Homo sapiens

<220>

<223> siRNA target sequence for ROS1 (NM_002944).

<400> 749
gaagctggac ttatactaa 19

<210> 750
<211> 19
<212> DNA
<213> Homo sapiens

<220>
<223> siRNA target sequence for ROS1 (NM_002944).

<400> 750
gacatggatt ggtataaca 19

<210> 751
<211> 19
<212> DNA
<213> Homo sapiens

<220>
<223> siRNA target sequence for ROS1 (NM_002944).

<400> 751
cgaaaggcga cgttttgt 19

<210> 752
<211> 19
<212> DNA
<213> Homo sapiens

<220>
<223> siRNA target sequence for ROS1 (NM_002944).

<400> 752
caagccaagc gaatcattt 19

<210> 753
<211> 19
<212> DNA
<213> Homo sapiens

<220>
<223> siRNA target sequence for ROS1 (NM_002944).

<400> 753
ggaagctgga cttatacta 19

<210> 754
<211> 19
<212> DNA
<213> Homo sapiens

<220>
<223> siRNA target sequence for ROS1 (NM_002944).

<400> 754
ctgtcactcc ttataccta 19

<210> 755
<211> 19
<212> DNA
<213> Homo sapiens

<220>
<223> siRNA target sequence for ROS1 (NM_002944).

<400> 755
ctttctgtca ctccttata 19

<210> 756
<211> 19
<212> DNA
<213> Homo sapiens

<220>
<223> siRNA target sequence for ROS1 (NM_002944).

<400> 756
caacatgtct gatgtatct 19

<210> 757
<211> 19
<212> DNA
<213> Homo sapiens

<220>
<223> siRNA target sequence for ALK (NM_004304).

<400> 757
ccacctacgt atttaagat 19

<210> 758
<211> 19
<212> DNA
<213> Homo sapiens

<220>
<223> siRNA target sequence for ALK (NM_004304).

<400> 758
cctgtatacc ggataatga 19

<210> 759
<211> 19
<212> DNA
<213> Homo sapiens

<220>
<223> siRNA target sequence for ALK (NM_004304).

<400> 759
gccacctacg tatttaaga 19

<210> 760
<211> 19
<212> DNA
<213> Homo sapiens

<220>
<223> siRNA target sequence for ALK (NM_004304).

<400> 760
cgctttgccg atagaatat 19

<210> 761
<211> 19
<212> DNA
<213> Homo sapiens

<220>
<223> siRNA target sequence for ALK (NM_004304).

<400> 761
gccacgggga agtgaatat 19

<210> 762
<211> 19
<212> DNA
<213> Homo sapiens

<220>
<223> siRNA target sequence for ALK (NM_004304).

<400> 762

ccatcatgac cgactacaa 19

<210> 763
<211> 19
<212> DNA
<213> Homo sapiens

<220>
<223> siRNA target sequence for ALK (NM_004304).

<400> 763
caatgacccc gaaatggat 19

<210> 764
<211> 19
<212> DNA
<213> Homo sapiens

<220>
<223> siRNA target sequence for ALK (NM_004304).

<400> 764
ccggcatcat gattgtgta 19

<210> 765
<211> 19
<212> DNA
<213> Homo sapiens

<220>
<223> siRNA target sequence for MET (NM_000245).

<400> 765
gaacagcgag ctaaatata 19

<210> 766
<211> 19
<212> DNA
<213> Homo sapiens

<220>
<223> siRNA target sequence for MET (NM_000245).

<400> 766
cagcgcgttg acttattca 19

<210> 767
<211> 19

<212> DNA
<213> Homo sapiens

<220>
<223> siRNA target sequence for MET (NM_000245).

<400> 767
gtgcattccc tatcaaata 19

<210> 768
<211> 19
<212> DNA
<213> Homo sapiens

<220>
<223> siRNA target sequence for MET (NM_000245).

<400> 768
gattcttacc ccattaagt 19

<210> 769
<211> 19
<212> DNA
<213> Homo sapiens

<220>
<223> siRNA target sequence for MET (NM_000245).

<400> 769
caaagcgatg aaatatctt 19

<210> 770
<211> 19
<212> DNA
<213> Homo sapiens

<220>
<223> siRNA target sequence for MET (NM_000245).

<400> 770
catttggata ggcttgtaa 19

<210> 771
<211> 19
<212> DNA
<213> Homo sapiens

<220>
<223> siRNA target sequence for MET (NM_000245).

<400> 771
ctctagatgc tcagacttt 19

<210> 772
<211> 19
<212> DNA
<213> Homo sapiens

<220>
<223> siRNA target sequence for MET (NM_000245).

<400> 772
gttaaaggtg aagtgttaa 19

<210> 773
<211> 19
<212> DNA
<213> Homo sapiens

<220>
<223> siRNA target sequence for NTRK1 (NM_002529).

<400> 773
gcatcctgta ccgtaagtt 19

<210> 774
<211> 19
<212> DNA
<213> Homo sapiens

<220>
<223> siRNA target sequence for NTRK1 (NM_002529).

<400> 774
ggctcagtcg cctgaatct 19

<210> 775
<211> 19
<212> DNA
<213> Homo sapiens

<220>
<223> siRNA target sequence for NTRK1 (NM_002529).

<400> 775
ctcagtcgcc tgaatctct 19

<210> 776
<211> 19
<212> DNA
<213> Homo sapiens

<220>
<223> siRNA target sequence for NTRK1 (NM_002529).

<400> 776
ggctccgtgc tcaatgaga 19

<210> 777
<211> 19
<212> DNA
<213> Homo sapiens

<220>
<223> siRNA target sequence for NTRK1 (NM_002529).

<400> 777
ggtcaagatt ggtgatttt 19

<210> 778
<211> 19
<212> DNA
<213> Homo sapiens

<220>
<223> siRNA target sequence for NTRK2 (NM_006180).

<400> 778
caatttacc cgaaacaaa 19

<210> 779
<211> 19
<212> DNA
<213> Homo sapiens

<220>
<223> siRNA target sequence for NTRK2 (NM_006180).

<400> 779
catcaagcga cataacatt 19

<210> 780
<211> 19
<212> DNA
<213> Homo sapiens

<220>
<223> siRNA target sequence for NTRK2 (NM_006180).

<400> 780
gtgatccggt tcctaatat 19

<210> 781
<211> 19
<212> DNA
<213> Homo sapiens

<220>
<223> siRNA target sequence for NTRK2 (NM_006180).

<400> 781
gatccggttc ctaatatgt 19

<210> 782
<211> 19
<212> DNA
<213> Homo sapiens

<220>
<223> siRNA target sequence for NTRK2 (NM_006180).

<400> 782
cttgtgtggc ggaaaatct 19

<210> 783
<211> 19
<212> DNA
<213> Homo sapiens

<220>
<223> siRNA target sequence for NTRK2 (NM_006180).

<400> 783
cctgcagata cccaattgt 19

<210> 784
<211> 19
<212> DNA
<213> Homo sapiens

<220>
<223> siRNA target sequence for NTRK2 (NM_006180).

<400> 784
ctggtgcatt ccattcact 19

<210> 785
<211> 19
<212> DNA
<213> Homo sapiens

<220>
<223> siRNA target sequence for NTRK2 (NM_006180).

<400> 785
cacagggctc cttaaggat 19

<210> 786
<211> 19
<212> DNA
<213> Homo sapiens

<220>
<223> siRNA target sequence for INSR (NM_000208).

<400> 786
gccctgtgac gcatgaaat 19

<210> 787
<211> 19
<212> DNA
<213> Homo sapiens

<220>
<223> siRNA target sequence for INSR (NM_000208).

<400> 787
ctgtgacgca tgaaatctt 19

<210> 788
<211> 19
<212> DNA
<213> Homo sapiens

<220>
<223> siRNA target sequence for INSR (NM_000208).

<400> 788
gcatggtcgc ccatgattt 19

<210> 789
<211> 19
<212> DNA

<213> Homo sapiens

<220>
<223> siRNA target sequence for INSR (NM_000208).

<400> 789
catggtcgcc catgatttt 19

<210> 790
<211> 19
<212> DNA
<213> Homo sapiens

<220>
<223> siRNA target sequence for INSR (NM_000208).

<400> 790
ggatcacgac tgttcttta 19

<210> 791
<211> 19
<212> DNA
<213> Homo sapiens

<220>
<223> siRNA target sequence for INSR (NM_000208).

<400> 791
gattggaagt atttatcta 19

<210> 792
<211> 19
<212> DNA
<213> Homo sapiens

<220>
<223> siRNA target sequence for INSR (NM_000208).

<400> 792
caccaatacg tcattcaca 19

<210> 793
<211> 19
<212> DNA
<213> Homo sapiens

<220>
<223> siRNA target sequence for INSR (NM_000208).

<400> 793
cggacatctt ttgacaaga 19

<210> 794
<211> 19
<212> DNA
<213> Homo sapiens

<220>
<223> siRNA target sequence for RET (NM_000323).

<400> 794
gcttgtcccg agatgttta 19

<210> 795
<211> 19
<212> DNA
<213> Homo sapiens

<220>
<223> siRNA target sequence for RET (NM_000323).

<400> 795
catctgactc cctgattta 19

<210> 796
<211> 19
<212> DNA
<213> Homo sapiens

<220>
<223> siRNA target sequence for RET (NM_000323).

<400> 796
ctgactccct gatttatga 19

<210> 797
<211> 19
<212> DNA
<213> Homo sapiens

<220>
<223> siRNA target sequence for RET (NM_000323).

<400> 797
cttgtcccga gatgtttat 19

<210> 798

<211> 19
<212> DNA
<213> Homo sapiens

<220>
<223> siRNA target sequence for RET (NM_000323).

<400> 798
gggtcggatt ccagttaaa 19

<210> 799
<211> 19
<212> DNA
<213> Homo sapiens

<220>
<223> siRNA target sequence for RET (NM_000323).

<400> 799
ccacatggat tgaaaacaa 19

<210> 800
<211> 19
<212> DNA
<213> Homo sapiens

<220>
<223> siRNA target sequence for RET (NM_000323).

<400> 800
cttccacatg gattgaaaa 19

<210> 801
<211> 19
<212> DNA
<213> Homo sapiens

<220>
<223> siRNA target sequence for RET (NM_000323).

<400> 801
ccttccacat ggattgaaa 19

<210> 802
<211> 19
<212> DNA
<213> Homo sapiens

<220>

<223> siRNA target sequence for TYRO3 (NM_006293).

<400> 802
gcatcagcga tgaactaaa 19

<210> 803
<211> 19
<212> DNA
<213> Homo sapiens

<220>
<223> siRNA target sequence for TYRO3 (NM_006293).

<400> 803
gaaaacgctg agatttaca 19

<210> 804
<211> 19
<212> DNA
<213> Homo sapiens

<220>
<223> siRNA target sequence for TYRO3 (NM_006293).

<400> 804
gccaggaccc cttatacat 19

<210> 805
<211> 19
<212> DNA
<213> Homo sapiens

<220>
<223> siRNA target sequence for TYRO3 (NM_006293).

<400> 805
gaccccttat acatcaaca 19

<210> 806
<211> 19
<212> DNA
<213> Homo sapiens

<220>
<223> siRNA target sequence for TYRO3 (NM_006293).

<400> 806
ggcatcagcg atgaactaa 19

<210> 807
<211> 19
<212> DNA
<213> Homo sapiens

<220>
<223> siRNA target sequence for FLT4 (NM_182925).

<400> 807
gtgtgggctg agtttaact 19

<210> 808
<211> 19
<212> DNA
<213> Homo sapiens

<220>
<223> siRNA target sequence for FLT4 (NM_182925).

<400> 808
ccgtgtgggc tgagtttaa 19

<210> 809
<211> 19
<212> DNA
<213> Homo sapiens

<220>
<223> siRNA target sequence for FLT4 (NM_182925).

<400> 809
ggcctgaggc gcaacatca 19

<210> 810
<211> 19
<212> DNA
<213> Homo sapiens

<220>
<223> siRNA target sequence for FLT4 (NM_182925).

<400> 810
gcaagaacgt gcatctgtt 19

<210> 811
<211> 19
<212> DNA
<213> Homo sapiens

<220>
<223> siRNA target sequence for FLT4 (NM_182925).

<400> 811
gacctgggct cgtatgtgt 19

<210> 812
<211> 19
<212> DNA
<213> Homo sapiens

<220>
<223> siRNA target sequence for FLT4 (NM_182925).

<400> 812
cggctcacgc agaacttga 19

<210> 813
<211> 19
<212> DNA
<213> Homo sapiens

<220>
<223> siRNA target sequence for FLT4 (NM_182925).

<400> 813
gctactacaa gtacatcaa 19

<210> 814
<211> 19
<212> DNA
<213> Homo sapiens

<220>
<223> siRNA target sequence for FLT3 (NM_004119).

<400> 814
gtgagacgat cctttaaa 19

<210> 815
<211> 19
<212> DNA
<213> Homo sapiens

<220>
<223> siRNA target sequence for FLT3 (NM_004119).

<400> 815

gattggctcg agatatcat 19

<210> 816
<211> 19
<212> DNA
<213> Homo sapiens

<220>
<223> siRNA target sequence for FLT3 (NM_004119).

<400> 816
gagacgatcc ttttaaact 19

<210> 817
<211> 19
<212> DNA
<213> Homo sapiens

<220>
<223> siRNA target sequence for FLT3 (NM_004119).

<400> 817
ccgctgctcg ttgtttttt 19

<210> 818
<211> 19
<212> DNA
<213> Homo sapiens

<220>
<223> siRNA target sequence for FLT3 (NM_004119).

<400> 818
gttcacaata gatctaaat 19

<210> 819
<211> 19
<212> DNA
<213> Homo sapiens

<220>
<223> siRNA target sequence for FLT3 (NM_004119).

<400> 819
gtgatcaagt gtgttttaa 19

<210> 820
<211> 19

<212> DNA
<213> Homo sapiens

<220>
<223> siRNA target sequence for FLT3 (NM_004119).

<400> 820
ggtgtcgagc agtactcta 19

<210> 821
<211> 19
<212> DNA
<213> Homo sapiens

<220>
<223> siRNA target sequence for FLT3 (NM_004119).

<400> 821
ggctaacaga aaagtgttt 19

<210> 822
<211> 19
<212> DNA
<213> Homo sapiens

<220>
<223> siRNA target sequence for KDR (NM_002253).

<400> 822
gaaagttacc agtctatta 19

<210> 823
<211> 19
<212> DNA
<213> Homo sapiens

<220>
<223> siRNA target sequence for KDR (NM_002253).

<400> 823
gagcacctta actatagat 19

<210> 824
<211> 19
<212> DNA
<213> Homo sapiens

<220>
<223> siRNA target sequence for KDR (NM_002253).

<400> 824
gaatcagacg acaagtatt 19

<210> 825
<211> 19
<212> DNA
<213> Homo sapiens

<220>
<223> siRNA target sequence for KDR (NM_002253).

<400> 825
gtaaaccgag acctaaaaa 19

<210> 826
<211> 19
<212> DNA
<213> Homo sapiens

<220>
<223> siRNA target sequence for KDR (NM_002253).

<400> 826
ggacagtagc agtcaaaat 19

<210> 827
<211> 19
<212> DNA
<213> Homo sapiens

<220>
<223> siRNA target sequence for KDR (NM_002253).

<400> 827
gtggctaagg gcatggagt 19

<210> 828
<211> 19
<212> DNA
<213> Homo sapiens

<220>
<223> siRNA target sequence for KDR (NM_002253).

<400> 828
ccaaattcca ttatgacaa 19

<210> 829
<211> 19
<212> DNA
<213> Homo sapiens

<220>
<223> siRNA target sequence for KDR (NM_002253).

<400> 829
cccaaattcc attatgaca 19

<210> 830
<211> 19
<212> DNA
<213> Homo sapiens

<220>
<223> siRNA target sequence for PDGFRB (NM_002609).

<400> 830
ggtgggcaca ctacaattt 19

<210> 831
<211> 19
<212> DNA
<213> Homo sapiens

<220>
<223> siRNA target sequence for PDGFRB (NM_002609).

<400> 831
gttgggcgaa ggttacaaa 19

<210> 832
<211> 19
<212> DNA
<213> Homo sapiens

<220>
<223> siRNA target sequence for PDGFRB (NM_002609).

<400> 832
ctttctcacg gaaataact 19

<210> 833
<211> 19
<212> DNA
<213> Homo sapiens

<220>
<223> siRNA target sequence for PDGFRB (NM_002609).

<400> 833
gacacgggag aatacttit 19

<210> 834
<211> 19
<212> DNA
<213> Homo sapiens

<220>
<223> siRNA target sequence for PDGFRB (NM_002609).

<400> 834
gtgacaacga ctatatcat 19

<210> 835
<211> 19
<212> DNA
<213> Homo sapiens

<220>
<223> siRNA target sequence for PDGFRB (NM_002609).

<400> 835
catccatcaa cgtctctgt 19

<210> 836
<211> 19
<212> DNA
<213> Homo sapiens

<220>
<223> siRNA target sequence for PDGFRB (NM_002609).

<400> 836
cctccgacga gatctatga 19

<210> 837
<211> 19
<212> DNA
<213> Homo sapiens

<220>
<223> siRNA target sequence for YES1 (NM_005433).

<400> 837
gaaatcaacg aggtatttt 19

<210> 838
<211> 19
<212> DNA
<213> Homo sapiens

<220>
<223> siRNA target sequence for YES1 (NM_005433).

<400> 838
cacaaccaga gcacaattt 19

<210> 839
<211> 19
<212> DNA
<213> Homo sapiens

<220>
<223> siRNA target sequence for YES1 (NM_005433).

<400> 839
gtatggtcgg tttacaata 19

<210> 840
<211> 19
<212> DNA
<213> Homo sapiens

<220>
<223> siRNA target sequence for YES1 (NM_005433).

<400> 840
ctgtatggtc ggtttacaa 19

<210> 841
<211> 19
<212> DNA
<213> Homo sapiens

<220>
<223> siRNA target sequence for YES1 (NM_005433).

<400> 841
ggttatatcc cgagcaatt 19

<210> 842
<211> 19
<212> DNA

<213> Homo sapiens

<220>
<223> siRNA target sequence for YES1 (NM_005433).

<400> 842
caagaagctc agataatga 19

<210> 843
<211> 19
<212> DNA
<213> Homo sapiens

<220>
<223> siRNA target sequence for YES1 (NM_005433).

<400> 843
gggctgcatt aaaagtaaa 19

<210> 844
<211> 19
<212> DNA
<213> Homo sapiens

<220>
<223> siRNA target sequence for YES1 (NM_005433).

<400> 844
ctgcattaaa agtaaagaa 19

<210> 845
<211> 19
<212> DNA
<213> Homo sapiens

<220>
<223> siRNA target sequence for FES (NM_002005).

<400> 845
gattggacgg gggaacttt 19

<210> 846
<211> 19
<212> DNA
<213> Homo sapiens

<220>
<223> siRNA target sequence for FES (NM_002005).

<400> 846
caccigaggc cctiaacta 19

<210> 847
<211> 19
<212> DNA
<213> Homo sapiens

<220>
<223> siRNA target sequence for FES (NM_002005).

<400> 847
ggctttccta gcattcctt 19

<210> 848
<211> 19
<212> DNA
<213> Homo sapiens

<220>
<223> siRNA target sequence for FES (NM_002005).

<400> 848
gaatacctgg agattagca 19

<210> 849
<211> 19
<212> DNA
<213> Homo sapiens

<220>
<223> siRNA target sequence for FES (NM_002005).

<400> 849
ctactggagg gcatgagaa 19

<210> 850
<211> 19
<212> DNA
<213> Homo sapiens

<220>
<223> siRNA target sequence for BCL2 (NM_000633).

<400> 850
gatgaagtac atccattat 19

<210> 851

<211> 19
<212> DNA
<213> Homo sapiens

<220>
<223> siRNA target sequence for BCL2 (NM_000633).

<400> 851
gtgatgaagt acatccatt 19

<210> 852
<211> 19
<212> DNA
<213> Homo sapiens

<220>
<223> siRNA target sequence for BCL2 (NM_000633).

<400> 852
gagttcggtg ggtcatgt 19

<210> 853
<211> 19
<212> DNA
<213> Homo sapiens

<220>
<223> siRNA target sequence for BCL2 (NM_000633).

<400> 853
gttcggtggg gtcatgtgt 19

<210> 854
<211> 19
<212> DNA
<213> Homo sapiens

<220>
<223> siRNA target sequence for BCL2 (NM_000633).

<400> 854
ggatgactga gtacctgaa 19

<210> 855
<211> 19
<212> DNA
<213> Homo sapiens

<220>

<223> siRNA target sequence for BIRC4 (NM_001167).

<400> 855
gccacgcagt ctacaaatt 19

<210> 856
<211> 19
<212> DNA
<213> Homo sapiens

<220>
<223> siRNA target sequence for BIRC4 (NM_001167).

<400> 856
gaagcacgga tctttactt 19

<210> 857
<211> 19
<212> DNA
<213> Homo sapiens

<220>
<223> siRNA target sequence for BIRC4 (NM_001167).

<400> 857
gaagaagcta gattaaagt 19

<210> 858
<211> 19
<212> DNA
<213> Homo sapiens

<220>
<223> siRNA target sequence for BIRC4 (NM_001167).

<400> 858
cacatgcaga ctatctttt 19

<210> 859
<211> 19
<212> DNA
<213> Homo sapiens

<220>
<223> siRNA target sequence for BIRC4 (NM_001167).

<400> 859
gaagagttta atagattaa 19

<210> 860
<211> 19
<212> DNA
<213> Homo sapiens

<220>
<223> siRNA target sequence for BIRC4 (NM_001167).

<400> 860
gtagaagagt ttaatagat 19

<210> 861
<211> 19
<212> DNA
<213> Homo sapiens

<220>
<223> siRNA target sequence for BIRC4 (NM_001167).

<400> 861
ctgtatggat agaaatatt 19

<210> 862
<211> 19
<212> DNA
<213> Homo sapiens

<220>
<223> siRNA target sequence for BIRC7 (NM_139317).

<400> 862
ctgctccggt caaaaggaa 19

<210> 863
<211> 19
<212> DNA
<213> Homo sapiens

<220>
<223> siRNA target sequence for BIRC7 (NM_139317).

<400> 863
cctgctccgg tcaaaagga 19

<210> 864
<211> 19
<212> DNA
<213> Homo sapiens

<220>
<223> siRNA target sequence for BIRC7 (NM_139317).

<400> 864
gagaggacgt gcaaggtgt 19

<210> 865
<211> 19
<212> DNA
<213> Homo sapiens

<220>
<223> siRNA target sequence for BIRC7 (NM_139317).

<400> 865
ccgtgtccat cgtctttgt 19

<210> 866
<211> 19
<212> DNA
<213> Homo sapiens

<220>
<223> siRNA target sequence for BIRC7 (NM_139317).

<400> 866
cctggacgga gcatgccaa 19

<210> 867
<211> 19
<212> DNA
<213> Homo sapiens

<220>
<223> siRNA target sequence for PPARA (NM_005036).

<400> 867
gctaaaatac ggagtttat 19

<210> 868
<211> 19
<212> DNA
<213> Homo sapiens

<220>
<223> siRNA target sequence for PPARA (NM_005036).

<400> 868

ccacccggac gatatcttt 19

<210> 869
<211> 19
<212> DNA
<213> Homo sapiens

<220>
<223> siRNA target sequence for PPARA (NM_005036).

<400> 869
ctttgtcat acatgatat 19

<210> 870
<211> 19
<212> DNA
<213> Homo sapiens

<220>
<223> siRNA target sequence for PPARA (NM_005036).

<400> 870
cacacaacgc gattcgttt 19

<210> 871
<211> 19
<212> DNA
<213> Homo sapiens

<220>
<223> siRNA target sequence for PPARA (NM_005036).

<400> 871
gctggtagcg tatggaaat 19

<210> 872
<211> 19
<212> DNA
<213> Homo sapiens

<220>
<223> siRNA target sequence for PPARG (NM_138712).

<400> 872
ggagtccacg agatcattt 19

<210> 873
<211> 19

<212> DNA
<213> Homo sapiens

<220>
<223> siRNA target sequence for PPARG (NM_138712).

<400> 873
ctccctcatg gcaattgaa 19

<210> 874
<211> 19
<212> DNA
<213> Homo sapiens

<220>
<223> siRNA target sequence for PPARG (NM_138712).

<400> 874
gagtccacga gatcattta 19

<210> 875
<211> 19
<212> DNA
<213> Homo sapiens

<220>
<223> siRNA target sequence for PPARG (NM_138712).

<400> 875
ctgtcggatc cacaaaaaa 19

<210> 876
<211> 19
<212> DNA
<213> Homo sapiens

<220>
<223> siRNA target sequence for PPARG (NM_138712).

<400> 876
cagattgaag cttatctat 19

<210> 877
<211> 19
<212> DNA
<213> Homo sapiens

<220>
<223> siRNA target sequence for PPARG (NM_138712).

<400> 877
gcatctccac cttattatt 19

<210> 878
<211> 19
<212> DNA
<213> Homo sapiens

<220>
<223> siRNA target sequence for PPARG (NM_138712).

<400> 878
ggcgagggcg atcttgaca 19

<210> 879
<211> 19
<212> DNA
<213> Homo sapiens

<220>
<223> siRNA target sequence for PPARG (NM_138712).

<400> 879
gtccttcccg ctgaccaaa 19

<210> 880
<211> 19
<212> DNA
<213> Homo sapiens

<220>
<223> siRNA target sequence for DVL1 (NM_004421).

<400> 880
ccgtcgtccg ggtcatgca 19

<210> 881
<211> 19
<212> DNA
<213> Homo sapiens

<220>
<223> siRNA target sequence for DVL2 (NM_004422).

<400> 881
gtccatacgg acatggcat 19

<210> 882
<211> 19
<212> DNA
<213> Homo sapiens

<220>
<223> siRNA target sequence for DVL3 (NM_004423).

<400> 882
gcctagacga cttccactt 19

<210> 883
<211> 19
<212> DNA
<213> Artificial

<220>
<223> siRNA target sequence for Human immunodeficiency virus 1.

<400> 883
ggacagatag ggttataga 19

<210> 884
<211> 19
<212> DNA
<213> Artificial

<220>
<223> siRNA target sequence for Human immunodeficiency virus 1.

<400> 884
gcgagagcgt cagtattaa 19

<210> 885
<211> 19
<212> DNA
<213> Artificial

<220>
<223> siRNA target sequence for Human immunodeficiency virus 1.

<400> 885
gtagaccggt tctataaaa 19

<210> 886
<211> 19
<212> DNA
<213> Artificial

<220>
<223> siRNA target sequence for Human immunodeficiency virus 1.

<400> 886
cgacccctcg tcacaataa 19

<210> 887
<211> 19
<212> DNA
<213> Artificial

<220>
<223> siRNA target sequence for Human immunodeficiency virus 1.

<400> 887
gccctaggtg tgaatatca 19

<210> 888
<211> 19
<212> DNA
<213> Artificial

<220>
<223> siRNA target sequence for Human immunodeficiency virus 1.

<400> 888
gcttagggca acatatcta 19

<210> 889
<211> 19
<212> DNA
<213> Artificial

<220>
<223> siRNA target sequence for Human immunodeficiency virus 1.

<400> 889
gaagaactta gatcattat 19

<210> 890
<211> 19
<212> DNA
<213> Artificial

<220>
<223> siRNA target sequence for Human immunodeficiency virus 1.

<400> 890
gaactgtatc ctttaactt 19

<210> 891
<211> 19
<212> DNA
<213> Artificial

<220>
<223> siRNA target sequence for Human immunodeficiency virus 1.

<400> 891
gaaagactcc taaatttaa 19

<210> 892
<211> 19
<212> DNA
<213> Artificial

<220>
<223> siRNA target sequence for Human immunodeficiency virus 1.

<400> 892
cttagggcaa catatctat 19

## Description

## TECHNICAL FIELD

The present invention relates to RNA interference and more particularly, for example, to a method for designing sequences of polynucleotides for causing RNA interference, the method improving efficiency in testing, manufacturing, etc., in which RNA interference is used. Hereinafter, RNA interference may also be referred to as "RNAi".

The present invention further relates to a base sequence processing apparatus, a program for running a base sequence processing method on a computer, a recording medium, and a base sequence processing system. In particular, the invention relates to a base sequence processing apparatus capable of efficiently selecting a base sequence from the base sequences of a target gene, which causes RNA interference in a target gene, a program for running a base sequence processing method on a computer, a recording medium, and a base sequence processing system.

## BACKGROUND ART

RNA interference is a phenomenon of gene destruction wherein double-stranded RNA comprising sense RNA and anti-sense RNA (hereinafter also referred to as "dsRNA") homologous to a specific region of a gene to be functionally inhibited, destructs the target gene by causing interference in the homologous portion of mRNA which is a transcript of the target gene. RNA interference was first proposed in 1998 following an experiment using nematodes. However, in mammals, when long dsRNA with about 30 or more base pairs is introduced into cells, an interferon response is induced, and cell death occurs due to apoptosis. Therefore, it was difficult to apply the RNAi method to mammals.

On the other hand, it was demonstrated that RNA interference could occur in early stage mouse embryos and cultured mammalian cells, and it was found that the induction mechanism of RNA interference also existed in the mammalian cells. At present, it has been demonstrated that short double-stranded RNA with about 21 to 23 base pairs (short interfering RNA, siRNA) can induce RNA interference without exhibiting cytotoxicity even in the mammalian cell system, and it has become possible to apply the RNAi method to mammals.

## DISCLOSURE OF INVENTION

The RNAi method is a technique which is expected to have various applications. However, while dsRNA or siRNA that is homologous to a specific region of a gene, exhibits an RNA interference effect in most of the sequences in drosophila and nematodes, 70% to 80% of randomly selected (21 base) siRNA do not exhibit an RNA interference effect in mammals. This poses a great problem when gene functional analysis is carried out using the RNAi method in mammals.

Conventional designing of siRNA has greatly depended on the experiences and sensory perceptions of the researcher or the like, and it has been difficult to design siRNA actually exhibiting an RNA interference effect with high probability. Other factors that prevent further research being conducted on RNA interference and its various applications are high costs and time consuming procedures required for carrying out an RNA synthesis resulting in part from the unwanted synthesis of siRNA.

Under such circumstances, it is an object of the present invention to provide a more efficient and simplified means for the RNAi method.

In order to achieve the above object, the present inventors have studied a technique for easily obtaining siRNA, which is one of the steps requiring the greatest effort, time, and cost when the RNAi method is used. In view of the fact that preparation of siRNA is a problem especially in mammals, the present inventors have attempted to identify the sequence regularity of siRNA effective for RNA interference using mammalian cultured cell systems. As a result, it has been found that effective siRNA sequences have certain regularity, and thereby, the present invention has been completed. Namely, the present invention is as described below.

[1]A method for searching a target base sequence of RNA interference comprising: searching a sequence segment, conforming to the following rules (a) to (d), from the base sequences of a target gene for RNA interference:

(a) The 3' end base is adenine, thymine, or uracil,
(b) The 5' end base is guanine or cytosine,
(c) A 7-base sequence from the 3' end is rich in one or more types of bases selected from the group consisting of adenine, thymine, and uracil, and
(d) The number of bases is within a range that allows RNA interference to occur without causing cytotoxicity.

[2] The method for searching the target base sequence according to item [1], wherein, in the rule (c), at least three bases among the seven bases are one or more types of bases selected from the group consisting of adenine, thymine, and uracil.

[3] The method for searching the target base sequence according to item [1] or [2], wherein, in the rule (d), the number of bases is 13 to 28.

[4] A method for designing a base sequence of a polynucleotide for causing RNA interference comprising: searching a base sequence, conforming to the rules (a) to (d) below, from the base sequences of a target gene and designing a base sequence homologous to the searched base sequence:

(a) The 3' end base is adenine, thymine, or uracil,
(b) The 5' end base is guanine or cytosine,
(c) A 7-base sequence from the 3' end is rich in one or more types of bases selected from the group consisting of adenine, thymine, and uracil, and
(d) The number of bases is within a range that allows RNA interference to occur without causing cytotoxicity.

[5] The method for designing the base sequence according to item [4], wherein, in the rule (c), at least three bases among the seven bases are one or more types of bases selected from the group consisting of adenine, thymine, and uracil.

[6] The method for designing the base sequence according to item [4] or [5], wherein the number of bases in the homologous base sequence designed is 13 to 28.

[7] The method for designing the base sequence according to any one of items [4] to [6], wherein designing is performed so that at least 80% of bases in the homologous base sequence designed corresponds to the base sequence searched.

[8] The method for designing the base sequence according to any one of items [4] to [7], wherein the 3' end base of the base sequence searched is the same as the 3' end base of the base sequence designed, and the 5' end base of the base sequence searched is the same as the 5' end base of the base sequence designed.

[9] The method for designing the base sequence according to any one of items [4] to [8], wherein an overhanging portion is added to the 3' end of the polynucleotide.

[10] A method for producing a double-stranded polynucleotide comprising: forming one strand by providing an overhanging portion to the 3' end of a base sequence homologous to a prescribed sequence which is contained in the base sequences of a target gene and which conforms to the following rules (a) to (d), and forming the other strand by providing an overhanging portion to the 3' end of a base sequence complementary to the base sequence homologous to the prescribed sequence, wherein the number of bases in each strand is 15 to 30:

(a) The 3' end base is adenine, thymine, or uracil,
(b) The 5' end base is guanine or cytosine,
(c) A 7-base sequence from the 3' end is rich in one or more types of bases selected from the group consisting of adenine, thymine, and uracil, and
(d) The number of bases is within a range that allows RNA interference to occur without causing cytotoxicity.

[11] A double-stranded polynucleotide synthesized by searching a sequence segment having 13 to 28 bases, conforming to the following rules (a) to (d), from the base sequences of a target gene for RNA interference, forming one strand by providing an overhanging portion to the 3' end of a base sequence homologous to a prescribed sequence which is contained in the base sequences of the target gene and which conforms to the following rules (a) to (d), and forming the other strand by providing an overhanging portion to the 3' end of a base sequence complementary to the base sequence homologous to the prescribed sequence, wherein the number of bases in each strand is 15 to 30:

(a) The 3' end base is adenine, thymine, or uracil,
(b) The 5' end base is guanine or cytosine,
(c) A 7-base sequence from the 3' end is rich in one or more types of bases selected from the group consisting of adenine, thymine, and uracil, and
(d) The number of bases is within a range that allows RNA interference to occur without causing cytotoxicity.

[12] A method for inhibiting gene expression comprising the steps of searching a sequence segment having 13 to 28 bases, conforming to the following rules (a) to (d), from the base sequences of a target gene for RNA interference, synthesizing a double-stranded polynucleotide such that one strand is formed by providing an overhanging portion to the 3' end of a base sequence homologous to a prescribed sequence which is contained in the base sequences of the target gene and which conforms to the following rules (a) to (d), the other strand is formed by providing an overhanging portion to the 3' end of a base sequence complementary to the base sequence homologous to the prescribed sequence, and the number of bases in each strand is 15 to 30, and adding the synthesized double-stranded polynucleotide to an expression system of the target gene of which expression is to be inhibited to inhibit the expression of the target gene:

(a) The 3' end base is adenine, thymine, or uracil,
(b) The 5' end base is guanine or cytosine,
(c) A 7-base sequence from the 3' end is rich in one or more types of bases selected from the group consisting of adenine, thymine, and uracil, and
(d) The number of bases is within a range that allows RNA interference to occur without causing cytotoxicity.

[13] A base sequence processing apparatus characterized in that it comprises partial base sequence creation means for acquiring base sequence information of a target gene for RNA interference and creating partial base sequence information corresponding to a sequence segment having a predetermined number of bases in the base sequence information; 3' end base determination means for determining whether the 3' end base in the partial base sequence information created by the partial base sequence creation means is adenine, thymine, or uracil; 5' end base determination means for determining whether the 5' end base in the partial base sequence information created by the partial base sequence creation means is guanine or cytosine; predetermined base inclusion determination means for determining whether base sequence information comprising 7 bases at the 3' end in the partial base sequence information created by the partial base sequence creation means is rich in one or more types of bases selected from the group consisting of adenine, thymine, and uracil; and prescribed sequence selection means for selecting prescribed sequence information which specifically causes RNA interference in the target gene from the partial base sequence information created by the partial base sequence creation means, based on the results determined by the 3' base determination means, the 5' end base determination means, and the predetermined base inclusion determination means.
[14] The base sequence processing apparatus according to item [13], characterized in that the partial base sequence creation means further comprises region-specific base sequence creation means for creating the partial base sequence information having the predetermined number of bases from a segment corresponding to a coding region or transcription region of the target gene in the base sequence information.
[15] The base sequence processing apparatus according to item [13] or [14], characterized in that the partial base sequence creation means further comprises common base sequence creation means for creating the partial base sequence information having the predetermined number of bases which is common in a plurality of base sequence information derived from different organisms.
[16] The base sequence processing apparatus according to any one of items [13] to [15], characterized in that the base sequence information that is rich corresponds to base sequence information comprising the 7 bases containing at least 3 bases which are one or more types of bases selected from the group consisting of adenine, thymine, and uracil.
[17] The base sequence processing apparatus according to any one of items [13] to [16], wherein the predetermined number of bases is 13 to 28.
[18] The base sequence processing apparatus according to any one of items [13] to [17], characterized in that the partial base sequence creation means further comprises overhanging portion-containing base sequence creation means for creating the partial base sequence information containing an overhanging portion.
[19] The base sequence processing apparatus according to any one of items [13] to [17], characterized in that it comprises overhanging-portion addition means for adding an overhanging portion to at least one end of the prescribed sequence information.
[20] The base sequence processing apparatus according to item [18] or [19], wherein the number of bases in the overhanging portion is 2.
[21] The base sequence processing apparatus according to any one of items [13] to [20], characterized in that it comprises identical/similar base sequence search means for searching base sequence information, identical or similar to the prescribed sequence information, from other base sequence information, and unrelated gene target evaluation means for evaluating whether the prescribed sequence information targets genes unrelated to the target gene based on the identical or similar base sequence information searched by the identical/similar base sequence search means.
[22] The base sequence processing apparatus according to item [21], characterized in that the unrelated gene target evaluation means further comprises total sum calculation means for calculating the total sum of reciprocals of the values showing the degree of identity or similarity based on the total amount of base sequence information on the genes unrelated to the target gene in the identical or similar base sequence information searched by the identical/similar base sequence search means and the values showing the degree of identity or similarity attached to the base sequence information on the genes unrelated to the target gene, and total sum-based target evaluation means for evaluating whether the prescribed sequence information targets the genes unrelated to the target gene based on the total sum calculated by the total sum calculation means.

[23] A program for running base sequence processing method on a computer, characterized in that it comprises a partial base sequence creation step of acquiring base sequence information of a target gene for RNA interference and creating partial base sequence information corresponding to a sequence segment having a predetermined number of bases in the base sequence information; a 3' end base determination step of determining whether the 3' end base in the partial base sequence information created in the partial base sequence creation step is adenine, thymine, or uracil; a 5' end base determination step of determining whether the 5' end base in the partial base sequence information created in the partial base sequence creation step is guanine or cytosine; a predetermined base inclusion determination step of determining whether base sequence information comprising 7 bases at the 3' end in the partial base sequence information created in the partial base sequence creation step is rich in one or more types of bases selected from the group consisting of adenine, thymine, and uracil; and a prescribed sequence selection step of selecting, based on the results determined in the 3' base determination step, the 5' end base determination step, and the predetermined base inclusion determination step, prescribed sequence information which specifically causes RNA interference in the target gene from the partial base sequence information created in the partial base sequence creation step.

[24] A computer-readable recording medium characterized in that the program according to item [23] is recorded in the medium.

[25] A base sequence processing system which comprises a base sequence processing apparatus which processing base sequence information of a target gene for RNA interference and a client apparatus, the base sequence processing apparatus and the client apparatus being connected to each other via a network in a communicable manner, characterized in that the client apparatus comprises base sequence transmission means for transmitting a name of the target gene or the base sequence information to the base sequence processing apparatus, and prescribed sequence acquisition means for acquiring prescribed sequence information which is transmitted from the base sequence processing apparatus and which specifically causes RNA interference in the target gene, and the base sequence processing apparatus comprises partial base sequence creation means for acquiring base sequence information corresponding to the name of the target gene or the base sequence information transmitted from the client apparatus and creating partial base sequence information corresponding to a sequence segment having a predetermined number of bases in the base sequence information; 3' end base determination means for determining whether the 3' end base in the partial base sequence information created by the partial base sequence creation means is adenine, thymine, or uracil; 5' end base determination means for determining whether the 5' end base in the partial base sequence information created by the partial base sequence creation means is guanine or cytosine; predetermined base inclusion determination means for determining whether base sequence information comprising 7 bases at the 3' end in the partial base sequence information created by the partial base sequence creation means is rich in one or more types of bases selected from the group consisting of adenine, thymine, and uracil; prescribed sequence selection means for selecting the prescribed sequence information from the partial base sequence information created by the partial base sequence creation means, based on the results determined by the 3' base determination means, the 5' end base determination means, and the predetermined base inclusion determination means; and prescribed sequence transmission means for transmitting the prescribed sequence information selected by the prescribed sequence selection means to the client apparatus.

[26] A base sequence processing method characterized in that it comprises a partial base sequence creation step of acquiring base sequence information of a target gene for RNA interference and creating partial base sequence information corresponding to a sequence segment having a predetermined number of bases in the base sequence information; a 3' end base determination step of determining whether the 3' end base in the partial base sequence information created in the partial base sequence creation step is adenine, thymine, or uracil; a 5' end base determination step of determining whether the 5' end base in the partial base sequence information created in the partial base sequence creation step is guanine or cytosine; a predetermined base inclusion determination step of determining whether base sequence information comprising 7 bases at the 3' end in the partial base sequence information created in the partial base sequence creation step is rich in one or more types of bases selected from the group consisting of adenine, thymine, and uracil; and a prescribed sequence selection step of selecting, based on the results determined in the 3' base determination step, the 5' end base determination step, and the predetermined base inclusion determination step, prescribed sequence information which specifically causes RNA interference in the target gene from the partial base sequence information created in the partial base sequence creation step.

[27] The base sequence processing method according to item [26], characterized in that the partial base sequence creation step further comprises a region-specific base sequence creation step of creating the partial base sequence information having the predetermined number of bases from a segment corresponding to a coding region or transcription region of the target gene in the base sequence information.

[28] The base sequence processing method according to item [26] or [27], characterized in that the partial base sequence creation step further comprises a common base sequence creation step for creating the partial base sequence information having the predetermined number of bases which is common in a plurality of base sequence information derived from different organisms.

[29] The base sequence processing method according to any one of items [26] to [28], characterized in that the base sequence information that is rich corresponds to base sequence information comprising the 7 bases containing at least 3 bases which are one or more types of bases selected from from the group consisting of adenine, thymine, and uracil.

[30] The base sequence processing method according to any one of items [26] to [29], wherein the predetermined number of bases is 13 to 28.

[31] The base sequence processing method according to any one of items [26] to [30], characterized in that the partial base sequence creation step further comprises an overhanging portion-containing base sequence creation step of creating the partial base sequence information containing an overhanging portion.

[32] The base sequence processing method according to any one of items [26] to [30], characterized in that it comprises an overhanging-portion addition step of adding an overhanging portion to at least one end of the prescribed sequence information.

[33] The base sequence processing method according to item [31] or [32], wherein the number of bases in the overhanging portion is 2.

[34] The base sequence processing method according to any one of items [26] to [33], characterized in that it comprises an identical/similar base sequence search step of searching base sequence information identical or similar to the prescribed sequence information from other base sequence information, and unrelated gene target evaluation step of evaluating whether the prescribed sequence information targets genes unrelated to the target gene based on the identical or similar base sequence information searched in the identical/similar base sequence search step.

[35] The base sequence processing method according to item [34], characterized in that the unrelated gene target evaluation step further comprises a total sum calculation step of calculating the total sum of reciprocals of the values showing the degree of identity or similarity based on the total amount of base sequence information on the genes unrelated to the target gene in the identical or similar base sequence information searched in the identical/similar base sequence search step and the values showing the degree of identity or similarity attached to the base sequence information on the genes unrelated to the target gene, and a total sum-based target evaluation step of evaluating whether the prescribed sequence information targets the genes unrelated to the target gene based on the total sum calculated in the total sum calculation step.

[36] The program according to item [23], characterized in that the partial base sequence creation step further comprises a region-specific base sequence creation step of creating the partial base sequence information having the predetermined number of bases from a segment corresponding to a coding region or transcription region of the target gene in the base sequence information.

[37] The program according to item [23] or [36], characterized in that the partial base sequence creation step further comprises a common base sequence creation step of creating the partial base sequence information having the predetermined number of bases which is common in a plurality of base sequence information derived from different organisms.

[38] The program according to any one of items [23], [36], and [37], characterized in that the base sequence information that is rich corresponds to base sequence information comprising the 7 bases containing at least 3 bases which are one or more types of bases selected from the group consisting of adenine, thymine, and uracil.

[39] The program according to any one of items [23], [36], [37], and [38], wherein the predetermined number of bases is 13 to 28.

[40] The program according to any one of items [23], [36], [37], [38], and [39], characterized in that the partial base sequence creation step further comprises an overhanging portion-containing base sequence creation step of creating the partial base sequence information containing an overhanging portion.

[41] The program according to any one of items [23], [36], [37], [38], and [39], characterized in that it comprises an overhanging-portion addition step of adding an overhanging portion to at least one end of the prescribed sequence information.

[42] The program according to item [40] or [41], wherein the number of bases in the overhanging portion is 2.

[43] The program according to any one of items [23], [36], [37], [38], [39], [40], [41], and [42], characterized in that it comprises an identical/similar base sequence search step of searching base sequence information identical or similar to the prescribed sequence information from other base sequence information, and an unrelated gene target evaluation step of evaluating whether the prescribed sequence information targets genes unrelated to the target gene based on the identical or similar base sequence information searched in the identical/similar base sequence search step.

[44] The program according to item [43], characterized in that the unrelated gene target evaluation step further comprises a total sum calculation step of calculating the total sum of reciprocals of the values showing the degree of identity or similarity based on the total amount of base sequence information on the genes unrelated to the target gene in the identical or similar base sequence information searched in the identical/similar base sequence search step and the values showing the degree of identity or similarity attached to the base sequence information on the genes unrelated to the target gene, and a total sum-based target evaluation step of evaluating whether the prescribed sequence information targets the genes unrelated to the target gene based on the total sum calculated in the total sum calculation step.

[45] A computer-readable recording medium characterized in that the program according to any one of items [23] and [36] to [44] is recorded in the medium.

[46] The base sequence processing system according to item [25], characterized in that, in the base sequence processing apparatus, the partial base sequence creation means further comprises region-specific base sequence creation means for creating the partial base sequence information having the predetermined number of bases from a segment corresponding to a coding region or transcription region of the target gene in the base sequence information.

[47] The base sequence processing system according to item [25] or [46], characterized in that, in the base sequence processing apparatus, the partial base sequence creation means further comprises common base sequence creation means for creating the partial base sequence information having the predetermined number of bases which is common in a plurality of base sequence information derived from different organisms.

[48] The base sequence processing system according to any one of items [25], [46], and [47], characterized in that, in the base sequence processing apparatus, the base sequence information that is rich corresponds to base sequence information comprising the 7 bases containing at least 3 bases which are one or more types of bases selected from the group consisting of adenine, thymine, and uracil.

[49] The base sequence processing system according to any one of items [25], [46], [47], and [48], wherein, in the base sequence processing apparatus, the predetermined number of bases is 13 to 28.

[50] The base sequence processing system according to any one of items [25], [46], [47], [48], and [49], characterized in that, in the base sequence processing apparatus, the partial base sequence creation means further comprises overhanging portion-containing base sequence creation means for creating the partial base sequence information containing an overhanging portion.

[51] The base sequence processing system according to any one of items [25], [46], [47], [48], and [49], characterized in that the base sequence processing apparatus comprises overhanging-portion addition means for adding an overhanging portion to at least one end of the prescribed sequence information.

[52] The base sequence processing system according to item [50] or [51], wherein, in the base sequence processing apparatus, the number of bases in the overhanging portion is 2.

[53] The base sequence processing system according to any one of items [25], [46], [47], [48], [49], [50], [51], and [52], characterized in that the base sequence processing apparatus comprises identical/similar base sequence search means for searching base sequence information identical or similar to the prescribed sequence information from other base sequence information, and unrelated gene target evaluation means for evaluating whether the prescribed sequence information targets genes unrelated to the target gene based on the identical or similar base sequence information searched by the identical/similar base sequence search means.

[54] The base sequence processing system according to item [53], characterized in that, in the base sequence processing apparatus, the unrelated gene target evaluation means further comprises total sum calculation means for calculating the total sum of reciprocals of the values showing the degree of identity or similarity based on the total amount of base sequence information on the genes unrelated to the target gene in the identical or similar base sequence information searched by the identical/similar base sequence search means and the values showing the degree of identity or similarity attached to the base sequence information on the genes unrelated to the target gene, and total sum-based target evaluation means for evaluating whether the prescribed sequence information targets the genes unrelated to the target gene based on the total sum calculated by the total sum calculation means.

Brief Description of the Drawings

Fig. 1 is a diagram which shows the designing of siRNA corresponding to sequences common to human and mice. Fig. 2 is a diagram which shows the regularity of siRNA exhibiting an RNAi effect. Fig. 3 is a diagram which shows common segments (shown in bold letters) having prescribed sequences in the base sequences of human FBP1 and mouse Fbpl. Fig. 4 is a diagram listing prescribed sequences common to human FBP1 and mouse Fbp1. Fig. 5 is a diagram in which the prescribed sequences common to human FBP1 and mouse Fbp1 are scored. Fig. 6 is a diagram showing the results of BLAST searches on one of the prescribed sequences performed so that genes other than the target are not knocked out. Fig. 7 is a diagram showing the results of BLAST searches on one of the prescribed sequences performed so that genes other than the target are not knocked out. Fig. 8 is a diagram showing an output result of a program. Fig. 9 is a diagram which shows the designing of RNA fragments (a to p). Fig. 10 is a diagram showing the results of testing whether siRNA a to p exhibited an RNAi effect, in which "B" shows the results in drosophila cultured cells, and "C" shows the results in human cultured cells. Fig. 11 is a diagram showing the analysis results concerning the characteristics of sequences of siRNA a to p. Fig. 12 is a principle diagram showing the basic principle of the present invention. Fig. 13 is a block diagram which shows an example of the configuration of a base sequence processing apparatus 100 of the system to which the present invention is applied. Fig. 14 is a diagram which shows an example of information stored in a target gene base sequence file 106a. Fig. 15 is a diagram which shows an example of information stored in a partial base sequence file 106b. Fig. 16 is a diagram which shows an example of information stored in a determination result file 106c. Fig. 17 is a diagram which shows an example of information stored in a prescribed sequence file 106d. Fig. 18 is a diagram which shows an example of information stored in a reference sequence database 106e. Fig. 19 is a diagram which shows an example of information stored in a degree of identity or similarity file 106f. Fig. 20 is a diagram which shows an example of information stored in an evaluation result file 106g. Fig. 21 is a block diagram which shows an example of the structure of a partial base sequence creation part 102a of the system to which the present invention is applied. Fig. 22 is a block diagram which shows an example of the structure of an unrelated gene target evaluation part 102h of the system to which the present invention is applied. Fig. 23 is a flowchart which shows an example of the main processing of the system in the embodiment. Fig. 24 is a flowchart which shows an example of the unrelated gene evaluation process of the system in the embodiment. Fig. 25 is a diagram which shows the structure of a target expression vector pTREC. Fig. 26 is a diagram which shows the results of PCR in which one of the primers in Example 2, 2. (2) is designed such that no intron is inserted. Fig. 27 is a diagram which shows the results of PCR in which one of the primers in Example 2, 2. (2) is designed such that an intron is inserted. Fig. 28 is a diagram which shows the sequence and structure of siRNA; siVIM35. Fig. 29 is a diagram which shows the sequence and structure of siRNA; siVIM812. Fig. 30 is a diagram which shows the sequence and structure of siRNA; siControl. Fig. 31 is a diagram which shows the results of assay of RNAi activity of siVIM812 and siVIM35. Fig. 32 is a diagram which shows RNAi activity of siControl, siVIM812, and siVIM35 against vimentin. Fig. 33 is a diagram which shows the results of antibody staining. Fig. 34 is a diagram which shows the assay results of RNAi activity of siRNA designed by the program against the luciferase gene. Fig. 35 is a diagram which shows the assay results of RNAi activity of siRNA designed by the program against the sequences of SARS virus.

BEST MODE FOR CARRYING OUT THE INVENTION

The embodiments of the present invention will be described below in the order of the columns <1> to <7>

<1>Method for searching target base sequence of RNA interference
<2> Method for designing base sequence of polynucleotide for causing RNA interference
<3> Method for producing double-stranded polynucleotide
<4> Method for inhibiting gene expression
<5> siRNA sequence design program
<6> siRNA sequence design business model system
<7> Base sequence processing apparatus for running siRNA sequence design program, etc.

<1> Method for searching target base sequence of RNA interference

The search method of the present invention is a method for searching a base sequence, which causes RNA interference, from the base sequences of a target gene. Specifically, in the search method of the present invention, a sequence segment conforming to the following rules (a) to (d) is searched from the base sequences of a target gene for RNA interference.

(a) The 3' end base is adenine, thymine, or uracil.
(b) The 5' end base is guanine or cytosine.
(c) A 7-base sequence from the 3' end is rich in one or more types of bases selected from the group consisting of adenine, thymine, and uracil.
(d) The number of bases is within a range that allows RNA interference to occur without causing cytotoxicity.

The term "gene" in the term "target gene" means a medium which codes for genetic information. The "gene" consists of a substance, such as DNA, RNA, or a complex of DNA and RNA, which codes for genetic information. As the genetic information, instead of the substance itself, electronic data of base sequences can be handled in a computer or the like. The "target gene" may be set as one coding region, a plurality of coding regions, or all the polynucleotides whose sequences have been revealed. When a gene with a particular function is desired to be searched, by setting only the particular gene as the target, it is possible to efficiently search the base sequences which cause RNA interference specifically in the particular gene. Namely, RNA interference is known as a phenomenon which destructs mRNA by interference, and by selecting a particular coding region, search load can be reduced. Moreover, a group of transcription regions may be treated

as the target region to be searched. Additionally, in the present specification, base sequences are shown on the basis of sense strands, i.e., sequences of mRNA, unless otherwise described. Furthermore, in the present specification, a base sequence which satisfies the rules (a) to (d) is referred to as a "prescribed sequence". In the rules, thymine corresponds to a DNA base sequence, and uracil corresponds to an RNA base sequence.

The rule (c) regulates so that a sequence in the vicinity of the 3' end contains a rich amount of type(s) of base (s) selected from the group consisting of adenine, thymine, and uracil, and more specifically, as an index for search, regulates so that a 7-base sequence from the 3' end is rich in one or more types of bases selected from adenine, thymine, and uracil.

In the rule (c), the phrase "sequence rich in" means that the frequency of a given base appearing is high, and schematically, a 5 to 10-base sequence, preferably a 7-base sequence, from the 3' end in the prescribed sequence contains one or more types of bases selected from adenine, thymine, and uracil in an amount of preferably at least 40% or more, and more preferably at least 50%. More specifically, for example, in a prescribed sequence of about 19 bases, among 7 bases from the 3' end, preferably at least 3 bases, more preferably at least 4 bases, and particularly preferably at least 5 bases, are one or more types of bases selected from the group consisting of adenine, thymine, and uracil.

The means for confirming the correspondence to the rule (c) is not particularly limited as long as it can be confirmed that preferably at least 3 bases, more preferably at least 4 bases, and particularly preferably at least 5 bases, among 7 bases are adenine, thymine, or uracil. For example, a case, wherein inclusion of 3 or more bases which correspond to one or more types of bases selected from the group consisting of adenine, thymine, and uracil in a 7-base sequence from the 3' end is defined as being rich, will be described below. Whether the base is any one of the three types of bases is checked from the first base at the 3' end one after another, and when three corresponding bases appear by the seventh base, conformation to the rule (c) is determined. For example, if three corresponding bases appear by the third base, checking of three bases is sufficient. That is, in the search with respect to the rule (c), it is not always necessary to check all of the seven bases at the 3' end. Conversely, non-appearance of three or more corresponding bases by the seventh base means being not rich, thus being determined that the rule (c) is not satisfied.

In a double-stranded polynucleotide, it is well-known that adenine complementarily forms hydrogen-bonds to thymine or uracil. In the complementary hydrogen bond between guanine and cytosine (G-C hydrogen bond), three hydrogen bonding sites are formed. On the other hand, the complementary hydrogen bond between adenine and thymine or uracil (A-(T/U) hydrogen bond) includes two hydrogen bonding sites. Generally speaking, the bonding strength of the A-(T/U) hydrogen bond is weaker than that of the G-C hydrogen bond.

In the rule (d), the number of bases of the base sequence to be searched is regulated. The number of bases of the base sequence to be searched corresponds to the number of bases capable of causing RNA interference. Depending on the conditions, for example the species of an organism, in cases of siRNA having an excessively large number of bases, cytotoxicity is known to occur. The upper limit of the number of bases varies depending on the species of organism to which RNA interference is desired to be caused. The number of bases of the single strand constituting siRNA is preferably 30 or less regardless of the species. Furthermore, in mammals, the number of bases is preferably 24 or less, and more preferably 22 or less. The lower limit, which is not particularly limited as long as RNA interference is caused, is preferably at least 15, more preferably at least 18, and still more preferably at least 20. With respect to the number of bases as a single strand constituting siRNA, searching with a number of 21 is particularly preferable.

Furthermore, although a description will be made below, in siRNA, an overhanging portion is provided at the 3' end of the prescribed sequence. The number of bases in the overhanging portion is preferably 2. Consequently, the upper limit of the number of bases in the prescribed sequence only, excluding the overhanging portion, is preferably 28 or less, more preferably 22 or less, and still more preferably 20 or less, and the lower limit is preferably at least 13, more preferably at least 16, and still more preferably at least 18. In the prescribed sequence, the most preferable number of bases is 19. The target base sequence for RNAi may be searched either including or excluding the overhanging portion.

Base sequences conforming to the prescribed sequence have an extremely high probability of causing RNA interference. Consequently, in accordance with the search method of the present invention, it is possible to search sequences that cause RNA interference with extremely high probability, and designing of polynucleotides which cause RNA interference can be simplified.

In another preferred example, the prescribed sequence does not contain a sequence in which 7 or more bases of guanine (G) and/or cytosine (C) are continuously present. Examples of the sequence in which 7 or more bases of guanine and/or cytosine are continuously present include a sequence in which either guanine or cytosine is continuously present as well as a sequence in which a mixed sequence of guanine and cytosine is present. More specific examples include GGGGGGG, CCCCCCC, and a mixed sequence of GCGGCCC.

Furthermore, in the search of the prescribed sequence, detection can be efficiently performed by using a computer installed with a program which allows a search of segments conforming to the rules (a) to (c), etc., after determining the number of bases. More specific embodiments will be described below in the columns <5> siRNA sequence design program and <7> Base sequence processing apparatus for running siRNA sequence design program.

<2> Method for designing base sequence of polynucleotide for causing RNA interference

In the method for designing a base sequence in accordance with the present invention, a base sequence of polynucleotide which causes RNA interference (siRNA) is designed on the basis of the base sequence searched by the search method described above. siRNA is mainly composed of RNA. siRNA which partially contains DNA, i.e., a hybrid polynucleotide, is also included in the examples of siRNA. In the method for designing a base sequence in accordance with the present invention, a base sequence conforming to the rules (a) to (d) is searched from the base sequences of a target gene, and a base sequence homologous to the searched base sequence is designed. In another preferred design example, it may be possible to take into consideration a case in which the prescribed sequence does not contain a sequence in which 7 or more bases of guanine (G) and/or cytosine (C) are continuously present. The rules (a) to (d) and the search method are the same as those described above regarding the search method of the present invention.

The term "homologous sequence" refers to the same sequence and a sequence in which mutations, such as deletions, substitutions, and additions, have occurred to the same sequence to an extent that the function of causing the RNA interference has not been lost. Although depending on the conditions, such as the type and sequence of the target gene, the range of the allowable mutation, in terms of homology, is preferably 80% or more, more preferably 90% or more, and still more preferably 95% or more. When homology in the range of the allowable mutation is calculated, desirably, the numerical values calculated using the same search algorithm are compared. The search algorithm is not particularly limited. A search algorithm suitable for searching for local sequences is preferable. More specifically, BLAST, search, or the like is preferably used.

As described above, although slight modification of the searched sequence is allowable, it is particularly preferred that the number of bases in the base sequence to be designed be the same as that of the searched sequence. For example, with respect to the allowance for change under the same number of bases, the bases of the base sequence to be designed correspond to those of the sequence searched at a rate of preferably 80% or more, more preferably 90% or more, and particularly preferably 95% or more. For example, when a base sequence having 19 bases is designed, preferably 16 or more bases, more preferably 18 or more bases, correspond to those of the searched base sequence. Furthermore, when a sequence homologous to the searched base sequence is designed, desirably, the 3' end base of the base sequence searched is the same as the 3' end base of the base sequence designed, and also desirably, the 5' end base of the base sequence searched is the same as the 5' end base of the base sequenced designed.

An overhanging portion is usually provided on a siRNA molecule. The overhanging portion is a protrusion provided on the 3' end of each strand in a double-stranded RNA molecule. Although depending on the species of organism, the number of bases in the overhanging portion is preferably 2. Basically, any base sequence is acceptable in the overhanging portion. In some cases, the same base sequence as that of the target gene to be searched, TT, UU, or the like may be preferably used. As described above, by providing the overhanging portion at the 3' end of the prescribed sequence which has been designed so as to be homologous to the base sequence searched, a sense strand constituting siRNA is designed.

Alternatively, it may be possible to search the prescribed sequence with the overhanging portion being included from the start to perform designing. The preferred number of bases in the overhanging portion is 2. Consequently, for example, in order to design a single strand constituting siRNA including a prescribed sequence having 19 bases and an overhanging portion having 2 bases, as the number of bases of siRNA including the overhanging portion, a sequence of 21 bases is searched from the target gene. Furthermore, when a double-stranded state is searched, a sequence of 23 bases may be searched.

In the method for designing a base sequence in accordance with the present invention, as described above, a given sequence is searched from a desired target gene. The target to which RNA interference is intended to be caused does not necessarily correspond to the origin of the target gene, and is also applicable to an analogous species, etc. For example, it is possible to design siRNA used for a second species that is analogous to a first species using a gene isolated from the first species as a target gene. Furthermore, it is possible to design siRNA that can be widely applied to mammals, for example, by searching a common sequence from two or more species of mammals and searching a prescribed sequence from the common sequence to perform designing. The reason for this is that it is highly probable that the sequence common to two or more mammals exists in other mammals.

In order to prevent RNA interference from occurring in genes not related to the target gene, preferably, a search is made to determine whether a sequence that is identical or similar to the designed sequence is included in the other genes. A search for the sequence that is identical or similar to the designed sequence may be performed using software capable of performing a general homology search, etc. By excluding such an identical/similar sequence, it is possible to design a sequence which causes RNA interference specifically to the target gene only.

In the design method of the present invention, RNA molecules that cause RNA interference can be easily designed with high probability. Although synthesis of RNA still requires effort, time, and cost, the design method of the present invention can greatly minimize them.

<3> Method for producing double-stranded polynucleotide

By the method for producing a double-stranded polynucleotide in accordance with the present invention, a double-stranded polynucleotide that has a high probability of causing RNA interference can be produced. For the double-stranded polynucleotide of the present invention, a base sequence of the polynucleotide is designed in accordance with the method for designing the base sequence of the present invention described above, and a double-stranded polynucleotide is synthesized so as to follow the sequence design. Preferred embodiments in the sequence design are the same as those described above regarding the method for designing the base sequence.

The double-stranded polynucleotide synthesized causes RNA interference, and siRNA is known as such a double-stranded polynucleotide. Additionally, the double-stranded polynucleotide produced by the production method of the present invention is preferably composed of RNA, but a hybrid polynucleotide which partially includes DNA may be acceptable. In this specification, double-stranded polynucleotides partially including DNA are also contained in the concept of siRNA. According to the research conducted by the present inventors, siRNA tends to have structural and functional asymmetry, and in view of the object of causing RNA interference, a half of the sense strand at the 5' end side and a half of the antisense strand at the 3' end side are desirably composed of RNA.

In a double-stranded polynucleotide, one strand is formed by providing an overhanging portion to the 3' end of a base sequence homologous to the prescribed sequence conforming to the rules (a) to (d) contained in the base sequence of the target gene, and the other strand is formed by providing an overhanging portion to the 3' end of a base sequence complementary to the base sequence homologous to the prescribed sequence. The number of bases in each strand, including the overhanging portion, is 18 to 24, more preferably 20 to 22, and particularly preferably 21. The number of bases in the overhanging portion is preferably 2. siRNA having 21 bases in total in which the overhanging portion is composed of 2 bases is suitable for causing RNA interference with high probability without causing cytotoxicity even in mammals.

RNA may be synthesized, for example, by chemical synthesis or by standard biotechnology. In one technique, a DNA strand having a predetermined sequence is produced, single-stranded RNA is synthesized using the produced DNA strand as a template in the presence of a transcriptase, and the synthesized single-stranded RNA is formed into double-stranded RNA.

With respect to the basic technique for molecular biology, there are many standard, experimental manuals, for example, BASIC METHODS IN MOLECULAR BIOLOGY (1986); Sambrook et al., MOLECULAR CLONING; A LABORATORY MANUAL, Second Edition, Cold Spring Harbor Laboratory Press, Cold Spring Harbor, N.Y. (1989); Saibo-Kogaku Handbook (Handbook for cell engineering), edited by Toshio Kuroki et al., Yodosha (1992); and Shin-Idenshi-Kogaku Handbook (New handbook for genetic engineering), edited by Muramatsu et al., Yodosha (1999).

One preferred embodiment of polynucleotide produced by the production method of the present invention is a double-stranded polynucleotide produced by a method in which a sequence segment including 13 to 28 bases conforming to the rules (a) to (d) is searched from a base sequence of a target gene for RNA interference, one strand is formed by providing an overhanging portion at the 3' end of a base sequence homologous to the prescribed sequence following the rules (a) to (d), the other strand is formed by providing an overhanging portion at the 3' end of a sequence complementary to the base sequence homologous to the prescribed sequence, and synthesis is performed so that the number of bases in each strand is 15 to 30. The resulting polynucleotide has a high probability of causing RNA interference.

It is also possible to prepare an expression vector which expresses siRNA. By placing a vector which expresses a sequence containing the prescribed sequence under a condition of a cell line or cell-free system in which expression is allowed to occur, it is possible to supply predetermined siRNA using the expression vector.

Since conventional designing of siRNA has depended on the experiences and intuition of the researcher, trial and error have often been repeated. However, by the double-stranded polynucleotide production method in accordance with the present invention, it is possible to produce a double-stranded polynucleotide which causes RNA interference with high probability. In accordance with the search method, sequence design method, or polynucleotide production method of the present invention, it is possible to greatly reduce effort, time, and cost required for various experiments, manufacturing, etc., which use RNA interference. Namely, the present invention greatly simplifies various experiments, research, development, manufacturing, etc., in which RNA interference is used, such as gene analysis, search for targets for new drug development, development of new drugs, gene therapy, and research on differences between species, and thus efficiency can be improved.

<4> Method for inhibiting gene expression

The method for inhibiting gene expression in accordance with the present invention includes a step of searching a predetermined base sequence, a step of designing and synthesizing a base sequence of siRNA based on the searched base sequence, and a step of introducing the resulting siRNA into an expression system containing a target gene.
The step of searching the predetermined base sequence follows the method for searching the target base sequence for RNA interference described above. Preferred embodiments are the same as those described above. The step of designing and synthesizing the base sequence of siRNA based on the searched base sequence can be carried out in accordance with the method for designing the base sequence of the polynucleotide for causing RNA interference and the method for producing the double-stranded polynucleotide described above. Preferred embodiments are the same as those described above.
The resulting double-stranded polynucleotide is added to an expression system for a target gene to inhibit the expression of the target gene. The expression system for a target gene means a system in which the target gene is expressed, and more specifically, a system provided with a reaction system in which at least mRNA of the target gene is formed. Examples of the expression system for the target gene include both in vitro and *in vivo* systems. In addition to cultured cells, cultured tissues, and living bodies, cell-free systems can also be used as the expression system for the target genes. The target gene of which expression is intended to be inhibited (inhibition target gene) is not necessarily a gene of a species corresponding to the origin of the searched sequence. However, as the relationship between the origin of the search target gene and the origin of the inhibition target gene becomes closer, a predetermined gene can be more specifically and effectively inhibited.
Introduction into an expression system means incorporation into the expression reaction system for the target gene. For example, in one method, a double-stranded nucleotide is transfected to a cultured cell including a target gene and incorporated into the cell. In another method, an expression vector having a base sequence comprising a prescribed sequence and an overhanging portion is formed, and the expression vector is introduced into a cell having a target gene.
In accordance with the gene inhibition method of the present invention, since polynucleotides which cause RNA interference can be efficiently produced, it is possible to inhibit genes efficiently and simply.

<5> siRNA sequence design program

Embodiments of the siRNA sequence design program will be described below.

(5-1) Outline of the program

When species whose genomes are not sequenced, for example, horse and swine, are subjected to RNA interference, this program calculates a sequence of siRNA usable in the target species based on published sequence information regarding human beings and mice. If siRNA is designed using this program, RNA interference can be carried out rapidly without sequencing the target gene. In the design (calculation) of siRNA, sequences having RNAi activity with high probability are selected in consideration of the rules of allocation of G or C (the rules (a) to (d) described above), and checking is performed by homology search so that RNA interference does not occur in genes that are not related to the target gene. In this specification, "G or C" may also be written as "G/C", and "A or T" may also be written as "A/T". Furthermore, "T(U)" in "A/T(U)" means T (thymine) in the case of sequences of deoxyribonucleic acid and U (uracil) in the case of sequences of ribonucleic acid.

(5-2) Policy of siRNA design

Sequences of human gene X and mouse gene X which are homologous to the human gene are assumed to be known. This program reads the sequences and searches completely common sequences each having 23 or more bases from the coding regions (CDS). By designing siRNA from the common portions, the resulting siRNA can target both human and mouse gene X (Fig. 1).
Since the portions completely common to human beings and mice are believed to also exist in other mammals with high probability, the siRNA is expected to act not only on gene X of human beings and mice but also on gene X of other mammals. Namely, even if in an animal species in which the sequence of a target gene is not known, if sequence information is known regarding the corresponding homologues of human beings and mice, it is possible to design siRNA using this program.
Furthermore, in mammals, it is known that sequences of effective siRNA have regularity (Fig. 2). In this program, only sequences conforming to the rules are selected. Fig. 2 is a diagram which shows regularity of siRNA sequences exhibiting an RNAi effect (rules of G/C allocation of siRNA). In Fig. 2, with respect to siRNA in which two RNA strands, each having a length of 21 bases and having an overhang of 2 bases on the 3' side, form base pairs between 19 bases at the 5' side of the two strands, the sequence in the coding side among the 19 bases forming the base pairs must satisfy the following conditions: 1) The 3' end is A/U; 2) the 5' end is G/C, and 3) 7 characters on the 3' side has a high ratio of A/U. In particular, the conditions 1) and 2) are important.

(5-3) Structure of program

This program consists of three parts, i.e., (5-3-1) a part which searches sequences of sites common to human beings and mice (partial sequences), (5-3-2) a part which scores the sequences according to the rules of G/C allocation, and (5-3-3) a part which performs checking by homology search so that unrelated genes are not targeted.

(5-3-1) Part which searches common sequences

This part reads a plurality of base sequence files (file 1, file 2, file 3, ...) and finds all sequences of 23 characters that commonly appear in all the files.
(Calculation Example) As file 1, sequences of human gene FBP1 (HM_000507: Homo sapiens fructose-1, 6-bisphosphatase 1) and, as file 2, sequences of mouse gene Fbp1 (NM_019395: Mus musculus fructose bisphosphatase 1) were inputted into the program. As a result, from the sequences of the two (Fig. 3), 15 sequences, each having 23 characters, that were common to the two (sequences common to human FBP1 and mouse Fbp1) were found (Fig. 4).

(5-3-2) Part which scores sequences

This part scores the sequences each having 23 characters in order to only select the sequences conforming to the rules of G/C allocation.
(Method) The sequences each having 23 characters are scored in the following manner.

Score 1: Is the 21st character from the head A/U? [no = 0, yes = 1]
Score 2: Is the third character from the head G/C? [no = 0, yes = 1]
Score 3: The number of A/U among 7 characters between the 15th character and 21st character from the head [0 to 7]
Total score: Product of scores 1 to 3. However, if the product is 3 or less, the total score is considered as zero.

(Calculation Example) With respect to 15 sequences in Fig. 4, the results of calculation are shown in Fig. 5. Fig. 5 is a diagram in which the sequences common to human FBP1 and mouse Fbp1 are scored. Furthermore, score 1, score 2, score 3, and total score are described in this order after the sequences shown in Fig. 5.

(5-3-3) Part which performs checking so that unrelated genes are not targeted

In order to prevent the designed siRNA from acting on genes unrelated to the target gene, homology search is performed against all the published mRNA of human beings and mice, and the degree of unrelated genes being hit is evaluated. Various search algorithms can be used in the homology search. Herein, an example in which BLAST is used will be described. Additionally, when BLAST is used, in view that the sequences to be searched are as short as 23 bases, it is desirable that Word Size be decreased sufficiently.
After the Blast search, among the hits with an E-value of 10.0 or less, with respect to all the hits other than the target gene, the total sum of the reciprocals of the E-values are calculated (hereinafter, the value is referred to as a homology score). Namely, the homology score (X) is found in accordance with the following expression.

$$X = \sum_{\text{all hits}} \frac{1}{E}$$

Note: A lower E value of the hit indicates higher homology to 23 characters of the query and higher risk of being targeted by siRNA. A larger number of hits indicates a higher probability that more unrelated genes are targeted. In consideration of these two respects, the risk that siRNA targets genes unrelated to the target gene is evaluated using the above expression.
(Calculation Example) The results of homology search against the sequences each having 23 characters and the homology scores are shown (Figs. 6 and 7). Fig. 6 shows the results of BLAST searches of a sequence common to human FBP1 and mouse Fbp1, i.e., "caccctgacccgcttcgtcatgg", and the first two lines are the results in which both mouse Fbp1 and human FBP1 are hit. The homology score is 5.9, and this is an example of a small number of hits. The risk that siRNA of this sequence targets the other genes is low. Furthermore, Fig. 7 shows the results of BLAST searches of a sequence common to human FBP1 and mouse Fbp1, i.e., "gccttctgagaaggatgctctgc". This is an example of a large number of hits, and the homology score is 170.8. Since the risk of targeting other genes is high, the sequence is not suitable as siRNA.

In practice, the parts (5-3-1), (5-3-2), and (5-3-3) may be integrated, and when the sequences of human beings and mice shown in Fig. 3 are inputted, an output as shown in Fig. 8 is directly obtained. Herein, after the sequences shown in Fig. 8, score 1, score 2, score 3, total score, and the tenfold value of homology score are described in this order. Additionally, in order to save processing time, the program may be designed so that the homology score is not calculated when the total score is zero. As a result, it is evident that the segment "36 caccctgacccgcttcgtcatgg" can be used as siRNA. Furthermore, one of the parts (5-3-1), (5-3-2), and (5-3-3) may be used independently.

(5-4) Actual calculation

With respect to about 6,400 gene pairs among the homologues between human beings and mice, siRNA was actually designed using this program. As a result, regarding about 70% thereof, it was possible to design siRNA which had a sequence common to human beings and mice and which satisfied the rules of effective siRNA sequence regularity so that unrelated genes were not targeted.

These siRNA sequences are expected to effectively inhibit target genes not only in human beings and mice but also in a wide range of mammals, and are believed to have a high industrial value, such as applications to livestock and pet animals. Moreover, it is possible to design siRNA which simultaneously targets two or more genes of the same species, e.g.,eIF2C1 and eIF2C2, using this program. Thus, the method for designing siRNA provided by this program has a wide range of application and is extremely strong. In further application, by designing a PCR primer using a sequence segment common to human beings and mice, target genes can be amplified in a wide range of mammals.

Additionally, embodiments of the apparatus which runs the siRNA sequence design program will be described in detail below in the column <7> Base sequence processing apparatus for running siRNA sequence design program.

<6> siRNA sequence design business model system

In the siRNA sequence design business model system of the present invention, when the siRNA sequence design program is applied, the system refers to a genome database, an EST database, and a phylogenetic tree database, alone or in combination, according to the logic of this program, and effective siRNA in response to availability of gene sequence information is proposed to the client. The term "availability" means a state in which information is available.

(1) In a case in which it is difficult to specify an ORF although genome information is available, siRNA candidates effective against assumed exon sites are extracted based on EST information, etc., and siRNA sequences in consideration of splicing variants and evaluation results thereof are displayed.

(2) In a case in which a gene sequence and a gene name are known, after the input of the gene sequence or the gene name, effective siRNA candidates are extracted, and siRNA sequences and evaluation results thereof are displayed.

(3) In a case in which genome information is not available, using the gene sequences of a related species storing the same type of gene functions (congeneric or having the same origin) or gene sequences of two or more species which have a short distance in phylogenetic trees and of which genome sequences are available, effective siRNA candidates are extracted, and siRNA sequences and evaluation results thereof are displayed.

(4) In order to analyze functions of genes relating infectious diseases and search for targets for new drug development, a technique is effective in which the genome database and phylogenetic tree database of microorganisms are further combined with apoptosis induction site information and function expression site information of microorganisms to obtain exhaustive siRNA candidate sequences.

<7> Base sequence processing apparatus for running siRNA sequence design program, etc.

Embodiments of the base sequence processing apparatus which is an apparatus for running the siRNA sequence design program described above, the program for running a base sequence processing method on a computer, the recording medium, and the base sequence processing system in accordance with the present invention will be described in detail below with reference to the drawings. However, it is to be understood that the present invention is not restricted by the embodiments.

[Summary of the present invention]

The summary of the present invention will be described below, and then the constitution, processing, etc., of the present invention will be described in detail. Fig. 12 is a principle diagram showing the basic principle of the present invention.

Overall, the present invention has the following basic features. That is, in the present invention, base sequence information of a target gene for RNA interference is obtained, and partial base sequence information corresponding to a sequence segment having a predetermined number of bases in the base sequence information is created (step S-1).

In step S-1, partial base sequence information having a predetermined number of bases may be created from a segment corresponding to a coding region or transcription region of the target gene in the base sequence information. Furthermore, partial base sequence information having a predetermined number of bases which is common in a plurality of base sequence information derived from different organisms (e.g., human base sequence information and mouse base sequence information) may be created. Furthermore, partial base sequence information having a predetermined number of bases which is common in a plurality of analogous base sequence information in the same species may be created. Furthermore, common partial base sequence information having a predetermined number of bases may be created from segments corresponding to coding regions or transcription regions of the target gene in a plurality of base sequence information derived from different species. Furthermore, common partial base sequence information having a predetermined number of bases may be created from segments corresponding to coding regions or transcription regions of the target gene in a plurality of analogous base sequence information in the same species. Consequently, a prescribed sequence which specifically causes RNA interference in the target gene can be efficiently selected, and calculation load can be reduced.

Furthermore, in step S-1, partial base sequence information including an overhanging portion may be created. Specifically, for example, partial base sequence information to which overhanging portion inclusion information, which shows that an overhanging portion is included, is added may be created. Namely, partial base sequence information and overhanging portion inclusion information may be correlated with each other. Thereby, it becomes possible to select the prescribed sequence with the overhanging portion being included from the start to perform designing.

The upper limit of the predetermined number of bases is, in the case of not including the overhanging portion, preferably 28 or less, more preferably 22 or less, and still more preferably 20 or less, and in the case of including the overhanging portion, preferably 32 or less, more preferably 26 or less, and still more preferably 24 or less. The lower limit of the predetermined number of bases is, in the case of not including the overhanging portion, preferably at least 13, more preferably at least 16, and still more preferably at least 18, and in the case of including the overhanging portion, preferably at least 17, more preferably at least 20, and still more preferably at least 22. Most preferably, the predetermined number of bases is, in the case of not including the overhanging portion, 19, and in the case of including the overhanging portion, 23. Thereby, it is possible to efficiently select the prescribed sequence which causes RNA interference without causing cytotoxicity even in mammals.

Subsequently, it is determined whether the 3' end base in the partial base sequence information created in step S-1 is adenine, thymine, or uracil (step S-2). Specifically, for example, when the 3' end base is adenine, thymine, or uracil, "1" may be outputted as the determination result, and when it is not, "0" may be outputted.

Subsequently, it is determined whether the 5' end base in the partial base sequence information created in step S-1 is guanine or cytosine (step S-3). Specifically, for example, when the 5' end base is guanine or cytosine, "1" may be outputted as the determination result, and when it is not, "0" may be outputted.

Subsequently, it is determined whether base sequence information comprising 7 bases at the 3' end in the partial base sequence information created in step S-1 is rich in one or more types of bases selected from the group consisting of adenine, thymine, and uracil (step S-4). Specifically, for example, the number of bases of one or more types of bases selected from the group consisting of adenine, thymine, and uracil contained in the base sequence information comprising 7 bases at the 3' end may be outputted as the determination result. The rule of determination in step S-4 regulates that base sequence information in the vicinity of the 3' end of the partial base sequence information created in step S-1 contains a rich amount of one or more types of bases selected from the group consisting of adenine, thymine, and uracil, and more specifically, as an index for search, regulates that the base sequence information in the range from the 3' end base to the seventh base from the 3' end is rich in one or more types of bases selected from the group consisting of adenine, thymine, and uracil.

In step S-4, the phrase "base sequence information rich in" corresponds to the phrase "sequence rich in" described in the column <1> Method for searching target base sequence for RNA interference. Specifically, for example, when the partial base sequence information created in step S-1 comprises about 19 bases, in the base sequence information comprising 7 bases in the partial base sequence information, preferably at least 3 bases, more preferably at least 4 bases, and particularly preferably at least 5 bases, are one or more types of bases selected from the group consisting of adenine, thymine, and uracil.

Furthermore, in steps S-2 to S-4, when partial base sequence information including the overhanging portion is determined, the sequence segment excluding the overhanging portion in the partial base sequence information is considered as the determination target.

Subsequently, based on the determination results in steps S-2, S-3, and S-4, prescribed sequence information which specifically causes RNA interference in the target gene is selected from the partial base sequence information created in step S-1 (Step S-5).

Specifically, for example, partial base sequence information in which the 3' end base has been determined as adenine, thymine, or uracil in step S-2, the 5' end base has been determined as guanine or cytosine in step S-3, and base sequence information comprising 7 bases at the 3' end in the partial base sequence information has been determined as being rich in one or more types of bases selected from the group consisting of adenine, thymine, and uracil is selected as prescribed sequence information. Specifically, for example, a product of the values outputted in steps S-2, S-3, and S-4 may be calculated, and based on the product, prescribed sequence information may be selected from the partial base sequence information created in step S-1.

Consequently, it is possible to efficiently and easily produce a siRNA sequence which has an extremely high probability of causing RNA interference, i.e., which is effective for RNA interference, in mammals, etc.

Here, an overhanging portion may be added to at least one end of the prescribed sequence information selected in step S-5. Additionally, for example, when a target is searched, the overhanging portion may be added to both ends of the prescribed sequence information. Consequently, designing of a polynucleotide which causes RNA interference can be simplified.

Additionally, the number of bases in the overhanging portion corresponds to the number of bases described in the column <2> Method for designing base sequence of polynucleotide for causing RNA interference. Specifically, for example, 2 is particularly suitable as the number of bases.

Furthermore, base sequence information that is identical or similar to the prescribed sequence information selected in step S-5 may be searched from other base sequence information (e.g., base sequence information published in a public database, such as RefSeq (Reference Sequence project) of NCBI) using a known homology search method, such as BLAST, FASTA, or search, and based on the searched identical or similar base sequence information, evaluation may be made whether the prescribed sequence information targets genes unrelated to the target gene.

Specifically, for example, base sequence information that is identical or similar to the prescribed sequence information selected in step S-5 is searched from other base sequence information (e.g., base sequence information published in a public database, such as RefSeq of NCBI) using a known homology search method, such as BLAST, FASTA, or ssearch. Based on the total amount of base sequence information on the genes unrelated to the target gene in the searched identical or similar base sequence information and the values showing the degree of identity or similarity (e.g., "E value" in BLAST, FASTA, or ssearch) attached to the base sequence information on the genes unrelated to the target gene, the total sum of the reciprocals of the values showing the degree of identity or similarity is calculated, and based on the calculated total sum (e.g., based on the size of the total sum calculated), evaluation may be made whether the prescribed sequence information targets genes unrelated to the target gene.

Consequently, it is possible to select a sequence which specifically causes RNA interference only to the target gene.

If RNA is synthesized based on the prescribed sequence information which is selected in accordance with the present invention and which does not cause RNA interference in genes unrelated to the target gene, it is possible to greatly reduce effort, time, and cost required compared with conventional techniques.

[System configuration]

First, the configuration of this system will be described. Fig. 13 is a block diagram which shows an example of the system to which the present invention is applied and which conceptually shows only the parts related to the present invention.

Schematically, in this system, a base sequence processing apparatus 100 which processes base sequence information of a target gene for RNA interference and an external system 200 which provides external databases regarding sequence information, structural information, etc., and external programs, such as homology search, are connected to each other via a network 300 in a communicable manner.

In Fig. 13, the network 300 has a function of interconnecting between the base sequence processing apparatus 100 and the external system 200, and is, for example, the Internet.

In Fig. 13, the external system 200 is connected to the base sequence processing apparatus 100 via the network 300, and has a function of providing the user with the external databases regarding sequence information, structural information, etc., and Web sites which execute external programs, such as homology search and motif search.

The external system 200 may be constructed as a WEB server, ASP server, or the like, and the hardware structure thereof may include a commercially available information processing apparatus, such as a workstation or a personal computer, and its accessories. Individual functions of the external system 200 are implemented by a CPU, a disk drive, a memory unit, an input unit, an output unit, a communication control unit, etc., and programs for controlling them in the hardware structure of the external system 200.

In Fig. 13, the base sequence processing apparatus 100 schematically includes a controller 102, such as a CPU, which controls the base sequence processing apparatus 100 overall; a communication control interface 104 which is connected to a communication device (not shown in the drawing), such as a router, connected to a communication line or the like; an input-output control interface 108 connected to an input unit 112 and an output unit 114; and a memory 106 which stores various databases and tables. These parts are connected via given communication channels in a communicable manner. Furthermore, the base sequence processing apparatus 100 is connected to the network 300 in a communicable manner via a communication device, such as a router, and a wired or radio communication line.

Various databases and tables (a target gene base sequence file 106a - a target gene annotation database 106h) which are stored in the memory 106 are storage means, such as fixed disk drives, for storing various programs used for various processes, tables, files, databases, files for web pages, etc.

Among these components of the memory 106, the target gene base sequence file 106a is target gene base sequence storage means for storing base sequence information of the target gene for RNA interference. Fig. 14 is a diagram which shows an example of information stored in the target gene base sequence file 106a.

As shown in Fig. 14, the information stored in the target gene base sequence file 106a consists of base sequence identification information which uniquely identifies base sequence information of the target gene for RNA interference (e.g., "NM_000507" in Fig. 14) and base sequence information (e.g., "ATGGCTGA ... AGTGA" in Fig. 14), the base sequence identification information and the base sequence information being associated with each other.

Furthermore, a partial base sequence file 106b is partial base sequence storage means for storing partial base sequence information, i.e., a sequence segment having a predetermined number of bases in base sequence information of the target gene for RNA interference. Fig. 15 is a diagram which shows an example of information stored in the partial base sequence file 106b.

As shown in Fig. 15, the information stored in the partial base sequence file 106b consists of partial base sequence identification information which uniquely identifies partial base sequence information (e.g., "NM_000507:36" in Fig. 15), partial base sequence information (e.g., "caccct ... tcatgg" in Fig. 15), and information on inclusion of an overhanging portion which shows the inclusion of the overhanging portion (e.g., "included" in Fig. 15), the partial base sequence identification information, the partial base sequence information, and the information on inclusion of the overhanging portion being associated with each other.

A determination result file 106c is determination result storage means for storing the results determined by a 3' end base determination part 102b, a 5' end base determination part 102c, and a predetermined base inclusion determination part 102d, which will be described below. Fig. 16 is a diagram which shows an example of information stored in the determination result file 106c.

As shown in Fig. 16, the information stored in the determination result file 106c consists of partial base sequence identification information (e.g., "NM_000507:36" in Fig. 16), determination result on 3' end base corresponding to a result determined by the 3' end base determination part 102b (e.g., "1" in Fig. 16), determination result on 5' end base corresponding to a result determined by the 5' end base determination part 102c (e.g., "1" in Fig. 16), determination result on inclusion of predetermined base corresponding to a result determined by the predetermined base inclusion determination part 102d (e.g., "4" in Fig. 16), and comprehensive determination result corresponding to a result obtained by putting together the results determined by the 3' end base determination part 102b, the 5' end base determination part 102c, and the predetermined base inclusion determination part 102d (e.g., "4" in Fig. 16), the partial base sequence identification information, the determination result on 3' end base, the determination result on 5' end base, the determination result on inclusion of predetermined base, and the comprehensive determination result being associated with each other.

Additionally, Fig. 16 shows an example of the case in which, with respect to the determination result on 3' end base and the determination result on 5' end base, "1" is set when determined as being "included" by each of the 3' end base determination part 102b and the 5' end base determination part 102c and "0" is set when determined as being "not included". Furthermore, Fig. 16 shows an example of the case in which the determination result on inclusion of predetermined base is set as the number of bases corresponding to one or more types of bases selected from the group consisting of adenine, thymine, and uracil contained in the base sequence information comprising 7 bases at the 3' end in the partial base sequence information. Furthermore, Fig. 16 shows an example of the case in which the comprehensive determination result is set as the product of the determination result on 3' end base, the determination result on 5' end base, and the determination result on inclusion of predetermined base. Specifically, for example, when the product is 3 or less, "0" may be set.

Furthermore, a prescribed sequence file 106d is prescribed sequence storage means for storing prescribed sequence information corresponding to partial base sequence information which specifically causes RNA interference in the target gene. Fig. 17 is a diagram which shows an example of information stored in the prescribed sequence file 106d.

As shown in Fig. 17, the information stored in the prescribed sequence file 106d consists of partial base sequence identification information (e.g., "NM_000507:36" in Fig. 17) and prescribed sequence information corresponding to partial base sequence information which specifically causes RNA interference in the target gene (e.g., caccct ... tcatgg" in Fig. 17), the partial base sequence identification information and the prescribed sequence information being associated with each other.

Furthermore, a reference sequence database 106e is a database which stores reference base sequence information corresponding to base sequence information to which reference is made to search base sequence information identical or similar to the prescribed sequence information by an identical/similar base sequence search part 102g, which will be described below. The reference sequence database 106e may be an external base sequence information database accessed via the Internet or may be an in-house database created by copying such a database, storing the original sequence information, or further adding unique annotation information to such a database. Fig. 18 is a diagram which shows an example of information stored in the reference sequence database 106e.

As shown in Fig. 18, the information stored in the reference sequence database 106e consists of reference sequence identification information (e.g., "ref|NM_015820.1|" in Fig. 18) and reference base sequence information (e.g., "caccct ... gcatgg" in Fig. 18), the reference sequence identification information and the reference base sequence information being associated with each other.

Furthermore, a degree of identity or similarity file 106f is degree of identity or similarity storage means for storing the degree of identity or similarity corresponding to a degree of identity or similarity of identical or similar base sequence information searched by an identical/similar base sequence search part 102g, which will be described below. Fig. 19 is a diagram which shows an example of information stored in the degree of identity or similarity file 106f.

As shown in Fig. 19, the information stored in the degree of identity or similarity file 106f consists of partial base sequence identification information (e.g., "NM_000507:36" in Fig. 19), reference sequence identification information (e.g., "ref|NM_015820.1|" and "ref|NM_003837.1|" in Fig. 19), and degree of identity or similarity (e.g., "0.52" in Fig. 19), the partial base sequence identification information, the reference sequence identification information, and the degree of identity or similarity being associated with each other.

Furthermore, an evaluation result file 106g is evaluation result storage means for storing the result of evaluation on whether genes unrelated to the target gene are targeted by an unrelated gene target evaluation part 102h, which will be described below. Fig. 20 is a diagram which shows an example of information stored in the evaluation result file 106g.

As shown in Fig. 20, the information stored in the evaluation result file 106g consists of partial base sequence identification information (e.g., "ink__000507:36" and "no_000507:441" in Fig. 20), total sum calculated by a total sum calculation part 102m, which will be described below, (e.g., "5.9" and "170.8" in Fig. 20), and evaluation result (e.g., "nontarget" and "target" in Fig. 20), the partial base sequence identification information, the total sum, and the evaluation result being associated with each other. Additionally, in Fig. 20, "nontarget" means that the prescribed sequence information does not target genes unrelated to the target gene, and "target" means that the prescribed sequence information targets genes unrelated to the target gene.

A target gene annotation database 106h is target gene annotation storage means for storing annotation information regarding the target gene. The target gene annotation database 106h may be an external annotation database which stores annotation information regarding genes and which is accessed via the Internet or may be an in-house database created by copying such a database, storing the original sequence information, or further adding unique annotation information to such a database.

The information stored in the target gene annotation database 106h consists of target gene identification information which identifies the target gene (e.g., the name of a gene to be targeted, and Accession number (e.g., "NM_000507" and "FBP1" described on the top in Fig. 3)) and simplified information on the target gene (e.g., "Homo sapiens fructose-1,6-bisphosphatase 1" describe on the top in Fig. 3), the target gene identification information and the simplified information being associated with each other.

In Fig. 13, the communication control interface 104 controls communication between the base sequence processing apparatus 100 and the network 300 (or a communication device, such as a router). Namely, the communication control interface 104 performs data communication with other terminals via communication lines.

In Fig. 13, the input-output control interface 108 controls the input unit 112 and the output unit 114. Here, as the output unit 114, in addition to a monitor (including a home television), a speaker may be used (hereinafter, the output unit 114 may also be described as a monitor). As the input unit 112, a keyboard, a mouse, a microphone, or the like may be used. The monitor cooperates with a mouse to implement a pointing device function.

In Fig. 13, the controller 102 includes control programs, such as OS (Operating System), programs regulating various processing procedures, etc., and internal memories for storing required data, and performs information processing for implementing various processes using the programs, etc. The controller 102 functionally includes a partial base sequence creation part 102a, a 3' end base determination part 102b, a 5' end base determination part 102c, a predetermined base inclusion determination part 102d, a prescribed sequence selection part 102e, an overhanging portion-adding part 102f, an identical/similar base sequence search part 102g, and an unrelated gene target evaluation part 102h.

Among them, the partial base sequence creation part 102a is partial base sequence creation means for acquiring base sequence information of a target gene for RNA interference and creating partial base sequence information corresponding to a sequence segment having a predetermined number of bases in the base sequence information. As shown in Fig. 21, the partial base sequence creation part 102a includes a region-specific base sequence creation part 102i, a common base sequence creation part 102j, and an overhanging portion-containing base sequence creation part 102k.

Fig. 21 is a block diagram which shows an example of the structure of the partial base sequence creation part 102a of the system to which the present invention is applied and which shows only the parts related to the present invention.

In Fig. 21, the region-specific base sequence creation part 102i is region-specific base sequence creation means for creating partial base sequence information having a predetermined number of bases from a segment corresponding to a coding region or transcription region of the target gene in the base sequence information.

The common base sequence creation part 102j is common base sequence creation means for creating partial base sequence information having a predetermined number of bases which is common in a plurality of base sequence information derived from different organisms.

The overhanging portion-containing base sequence creation part 102k is overhanging portion-containing base sequence creation means for creating partial base sequence information containing an overhanging portion.

Referring back to Fig. 13, the 3' end base determination part 102b is 3' end base determination means for determining whether the 3' end base in the partial base sequence information is adenine, thymine, or uracil.

Furthermore, the 5' end base determination part 102c is 5' end base determination means for determining whether the 5' end base in the partial base sequence information is guanine or cytosine.

Furthermore, the predetermined base inclusion determination part 102d is predetermined base inclusion determination means for determining whether the base sequence information comprising 7 bases at the 3' end in the partial base sequence information is rich in one or more types of bases selected from the group consisting of adenine, thymine, and uracil.

Furthermore, the prescribed sequence selection part 102e is prescribed sequence selection means for selecting prescribed sequence information, which specifically causes RNA interference in the target gene, from the partial base sequence information based on the results determined by the 3' end base determination part 102b, the 5' end base determination part 102c, and the predetermined base inclusion determination part 102c.

Furthermore, the overhanging portion-adding part 102f is overhanging portion addition means for adding an overhanging portion to at least one end of the prescribed sequence information.

Furthermore, the identical/similar base sequence search part 102g is identical/similar base sequence search means for searching base sequence information, identical or similar to the prescribed sequence information, from other base sequence information.

Furthermore, the unrelated gene target evaluation part 102h is unrelated gene target evaluation means for evaluating whether the prescribed sequence information targets genes unrelated to the target gene based on the identical or similar base sequence information. As shown in Fig. 22, the unrelated gene target evaluation part 102h further includes a total sum calculation part 102m and a total sum-based evaluation part 102n.

Fig. 22 is a block diagram which shows an example of the structure of the unrelated gene target evaluation part 102h of the system to which the present invention is applied and which schematically shows only the parts related to the present invention.

In Fig. 22, the total sum calculation part 102m is total sum calculation means for calculating the total sum of reciprocals of the values showing the degree of identity or similarity based on the total amount of base sequence information on the genes unrelated to the target gene in identical or similar base sequence information and the values showing the degree of identity or similarity attached to the base sequence information on the genes unrelated to the target gene (identity or similarity).

Furthermore, the total sum-based evaluation part 102n is total sum-based target evaluation means for evaluating whether the prescribed sequence information targets genes unrelated to the target gene based on the total sum calculated by the total sum calculation part 102m.

The details of processing of each part will be described later.

[Processing of the system]

An example of processing of the system having the configuration described above in this embodiment will be described in detail with reference to Figs. 23 and 24.

[Main processing]

First, the details of the main processing will be described with reference to Fig. 23, etc. Fig. 23 is a flowchart which shows an example of the main processing of the system in this embodiment.

The base sequence processing apparatus 100 acquires base sequence information of a target gene for RNA interference by the partial base sequence creation process performed by the partial base sequence creation part 102a, stores it in a predetermined memory region of the target gene base sequence file 106a, creates partial base sequence information corresponding to a sequence segment having a predetermined number of bases in the base sequence information, and stores the created partial base sequence information in a predetermined memory region of the partial base sequence file 106b (step SA-1).

In step SA-1, the partial base sequence creation part 102a may create partial base sequence information having a predetermined number of bases from a segment corresponding to a coding region or transcription region of the target gene in the base sequence information by the processing of the region-specific base sequence creation part 102i and may store the created partial base sequence information in a predetermined memory region of the partial base sequence file 106b.

In step SA-1, the partial base sequence creation part 102a may create partial base sequence information having a predetermined number of bases which is common in a plurality of base sequence information derived from different organisms (e.g., human base sequence information and mouse base sequence information) by the processing of the common base sequence creation part 102j and may store the created partial base sequence information in a predetermined memory region of the partial base sequence file 106b. Furthermore, common partial base sequence information having a predetermined number of bases which is common in a plurality of analogous base sequence information in the same species may be created.

In step SA-1, the partial base sequence creation part 102a may create partial base sequence information having a predetermined number of bases from segments corresponding to coding regions or transcription regions of the target gene in a plurality of base sequence information derived from different species by the processing of the region-specific base sequence creation part 102i and the common base sequence creation part 102j and may store the created partial base sequence information in a predetermined memory region of the partial base sequence file 106b. Furthermore, common partial base sequence information having a predetermined number of bases may be created from segments corresponding to coding regions or transcription regions of the target gene in a plurality of analogous base sequence information in the same species.

Furthermore, in step SA-1, the partial base sequence creation part 102a may create partial base sequence information containing an overhanging portion by the processing of the overhanging portion-containing base sequence creation part 102k. Specifically, for example, the partial base sequence creation part 102a may create partial base sequence information to which the overhanging portion inclusion information which shows the inclusion of the overhanging portion by the processing of the overhanging portion-containing base sequence creation part 102k and may store the created partial base sequence information and the overhanging portion inclusion information so as to be associated with each other in a predetermined memory region of the partial base sequence file 106b.

The upper limit of the predetermined number of bases is, in the case of not including the overhanging portion, preferably 28 or less, more preferably 22 or less, and still more preferably 20 or less, and in the case of including the overhanging portion, preferably 32 or less, more preferably 26 or less, and still more preferably 24 or less. The lower limit of the predetermined number of bases is, in the case of not including the overhanging portion, preferably at least 13, more preferably at least 16, and still more preferably at least 18, and in the case of including the overhanging portion, preferably at least 17, more preferably at least 20, and still more preferably at least 22. Most preferably, the predetermined number of bases is, in the case of not including the overhanging portion, 19, and in the case of including the overhanging portion, 23.

Subsequently, the base sequence processing apparatus 100 determines whether the 3' end base in the partial base sequence information created in step SA-1 is adenine, thymine, or uracil by the processing of the 3' end base determination part 102b and stores the determination result in a predetermined memory region of the determination result file 106c (step SA-2). Specifically, for example, the base sequence processing apparatus 100 may store "1" when the 3' end base in the partial base sequence information created in step SA-1 is adenine, thymine, or uracil, by the processing of the 3' end base determination part 102b, and "0" when it is not, in a predetermined memory region of the determination result file 106c.

Subsequently, the base sequence processing apparatus 100 determines whether the 5' end base in the partial base sequence information created in step SA-1 is guanine or cytosine by the processing of the 5' end base determination part 102c and stores the determination result in a predetermined memory region of the determination result file 106c (step SA-3). Specifically, for example, the base sequence processing apparatus 100 may store "1" when the 5' end base in the partial base sequence information created in step SA-1 is guanine or cytosine, by the processing of the 5' end base determination part 102c, and "0" when it is not, in a predetermined memory region of the determination result file 106c.

Subsequently, the base sequence processing apparatus 100 determines whether the base sequence information comprising 7 bases at the 3' end in the partial base sequence information created in step SA-1 is rich in one or more types of bases selected from the group consisting of adenine, thymine, and uracil by the processing of the predetermined base inclusion determination part 102d and stores the determination result in a predetermined memory region of the determination result file 106c (step SA-4). Specifically, for example, the base sequence processing apparatus 100, by the processing of the predetermined base inclusion determination part 102d, may store the number of bases corresponding to one or more types of bases selected from the group consisting of adenine, thymine, and uracil contained in the base sequence information comprising 7 bases at the 3' end in the partial base sequence information created in step SA-1 in a predetermined memory region of the determination result file 106c. The rule of determination in step SA-4 regulates that base sequence information in the vicinity of the 3' end of the partial base sequence information created in step SA-1 contains a rich amount of one or more types of bases selected from the group consisting of adenine, thymine, and uracil, and more specifically, as an index for search, regulates that the base sequence information in the range from the 3' end base to the seventh base from the 3' end is rich in one or more types of bases selected from the group consisting of adenine, thymine, and uracil.

In step SA-4, the phrase "base sequence information rich in" corresponds to the phrase "sequence rich in" described in the column <1> Method for searching target base sequence for RNA interference. Specifically, for example, when the partial base sequence information created in step SA-1 comprises about 19 bases, in the base sequence information comprising 7 bases at the 3' end in the partial base sequence information, preferably at least 3 bases, more preferably at least 4 bases, and particularly preferably at least 5 bases, are one or more types of bases selected from the group consisting of adenine, thymine, and uracil.

Furthermore, in steps SA-2 to SA-4, when partial base sequence information including the overhanging portion is determined, the sequence segment excluding the overhanging portion in the partial base sequence information is considered as the determination target.

Subsequently, based on the determination results in steps SA-2, SA-3, and SA-4, the base sequence processing apparatus 100, by the processing of the prescribed sequence selection part 102e, selects prescribed sequence information which specifically causes RNA interference in the target gene from the partial base sequence information created in step SA-1 and stores it in a predetermined memory region of the prescribed sequence file 106d (Step SA-5).

Specifically, for example, the base sequence processing apparatus 100, by the processing of the prescribed sequence selection part 102e, selects partial base sequence information, in which the 3' end base has been determined as adenine, thymine, or uracil in step SA-2, the 5' end base has been determined as guanine or cytosine in step SA-3, and base sequence information comprising 7 bases at the 3' end in the partial base sequence information has been determined as being rich in one or more types of bases selected from the group consisting of adenine, thymine, and uracil, as prescribed sequence information, and stores it in a predetermined memory region of the prescribed sequence file 106d. Specifically, for example, the base sequence processing apparatus 100, by the processing of the prescribed sequence selection part 102e, may calculate a product of the values outputted in steps SA-2, SA-3, and SA-4 and, based on the product, select prescribed sequence information from the partial base sequence information created in step SA-1.

Here, the base sequence processing apparatus 100 may add an overhanging portion to at least one end of the prescribed sequence information selected in step SA-5 by the processing of the overhanging portion-adding part 102f, and may store it in a predetermined memory region of the prescribed sequence file 106d. Specifically, for example, by the processing of the overhanging portion-adding part 102f, the base sequence processing apparatus 100 may change the prescribed sequence information stored in the prescribed sequence information section in the prescribed sequence file 106d to prescribed sequence information in which an overhanging portion is added to at least one end. Additionally, for example, when a target is searched, the overhanging portion may be added to both ends of the prescribed sequence information.

Additionally, the number of bases in the overhanging portion corresponds to the number of bases described in the column <2> Method for designing base sequence of polynucleotide for causing RNA interference. Specifically, for example, 2 is particularly suitable as the number of bases.

Furthermore, the base sequence processing apparatus 100, by the processing of the identical/similar base sequence search part 102g, may search base sequence information that is identical or similar to the prescribed sequence information selected in step SA-5 from other base sequence information (e.g., base sequence information published in a public database, such as Ref Seq of NCBI) using a known homology search method, such as BLAST, FASTA, or ssearch, and based on the searched identical or similar base sequence information, by the unrelated gene target evaluation process performed by the unrelated gene target evaluation part 102h, may evaluate whether the prescribed sequence information targets genes unrelated to the target gene.

Specifically, for example, the base sequence processing apparatus 100, by the processing of the identical/similar base sequence search part 102g, may search base sequence information that is identical or similar to the prescribed sequence information selected in step SA-5 from other base sequence information (e.g., base sequence information published in a public database, such as RefSeq of NCBI) using a known homology search method, such as BLAST, FASTA, or ssearch. The unrelated gene target evaluation part 102h, by the processing of the total sum calculation part 102m, may calculate the total sum of the reciprocals of the values showing the degree of identity or similarity based on the total amount of base sequence information on the genes unrelated to the target gene in the searched identical or similar base sequence information and the values showing the degree of identity or similarity (e.g., "E value" in BLAST, FASTA, or ssearch) attached to the base sequence information on the genes unrelated to the target gene. The unrelated gene target evaluation part 102h, by the processing of the total sum-based evaluation part 102n, may evaluate whether the prescribed sequence information targets genes unrelated to the target gene based on the calculated total sum.

Here, the details of the unrelated gene target evaluation process performed by the unrelated gene target evaluation part 102h will be described with reference to Fig. 24.

Fig. 24 is a flowchart which shows an example of the unrelated gene evaluation process of the system in this embodiment.

First, the base sequence processing apparatus 100, by the processing of the identical/similar base sequence search part 102g, searches base sequence information that is identical or similar to the prescribed sequence information selected in step SA-5 from other base sequence information (e.g., base sequence information published in a public database, such as RefSeq of NCBI) using a known homology search method, such as BLAST, FASTA, or ssearch, and stores identification information of the prescribed sequence information ("partial base sequence identification information" in Fig. 19), identification information of the searched identical or similar base sequence information ("reference sequence identification information" in Fig. 19), and the value showing the degree of identity or similarity (e.g., "E value" in BLAST, FASTA, or ssearch) ("degree of identity or similarity" in Fig. 19) attached to the searched identical or similar base sequence information so as to be associated with each other in a predetermined memory region of the degree of identity or similarity file 106f.

Subsequently, the unrelated gene target evaluation part 102h, by the processing of the total sum calculation part 102m, calculates the total sum of reciprocals of the values showing the degree of identity or similarity based on the total amount of base sequence information on the genes unrelated to the target gene in the searched identical or similar base sequence information and the values showing the degree of identity or similarity (e.g., "E value" in BLAST, FASTA, or ssearch) attached to the base sequence information on the genes unrelated to the target gene, and stores identification information of the prescribed sequence information ("partial base sequence identification information" in Fig. 20) and the calculated total sum ("total sum" in Fig. 20) so as to be associated with each other in a predetermined memory region of the evaluation result file 106g (step SB-1).

Subsequently, the unrelated gene target evaluation part 102h, by the processing of the total sum-based evaluation part 102n, evaluates whether the prescribed sequence information targets genes unrelated to the target gene based on the total sum calculated in step SB-1 (e.g., based on the size of the total sum calculated in step SB-1), and stores the evaluation results ("nontarget" and "target" in Fig. 20) in a predetermined memory region of the evaluation result file 106g (Step SB-2).

The main process is thereby completed.

[Other embodiments]

One preferred embodiment of the present invention has been described above. However, it is to be understood that the present invention can be carried out in various embodiments other than the embodiment described above within the scope of the technical idea described in the claims.

For example, although the case in which the base sequence processing apparatus 100 performs processing on a stand-alone mode has been described, construction may be made such that processing is performed in accordance with the request from a client terminal which is constructed separately from the base sequence processing apparatus 100, and the processing results are sent back to the client terminal. Specifically, for example, the client terminal transmits a name of the target gene for RNA interference (e.g., gene name or accession number) or base sequence information regarding the target gene to the base sequence processing apparatus 100, and the base sequence processing apparatus 100 performs the processes described above in the controller 102 on base sequence information corresponding to the name or the base sequence information transmitted from the client terminal to select prescribed sequence information which specifically causes RNA interference in the target gene and transmits it to the client terminal. In such a case, for example, by acquiring sequence information from a public database, siRNA against the gene in query may be selected. Alternatively, for example, siRNA for all the genes may be calculated and stored preliminarily, and siRNA may be immediately selected in response to the request from the client terminal (e.g., gene name or accession number) and the selected siRNA may be sent back to the client terminal.

Furthermore, the base sequence processing apparatus 100 may check the specificity of prescribed sequence information with respect to genes unrelated to the target gene. Thereby, it is possible to select prescribed sequence information which specifically causes RNA interference only in the target gene.

Furthermore, in the system comprising a client terminal and the base sequence processing apparatus 100, an interface function may be introduced in which, for example, the results of RNA interference effect of siRNA (e.g., "effective" or "not effective") are fed back from the Web page users on the Web, and the experimental results fed back from the users are accumulated in the base sequence processing apparatus 100 so that the sequence regularity of siRNA effective for RNA interference is improved.

Furthermore, the base sequence processing apparatus 100 may calculate base sequence information of a sense strand of siRNA and base sequence information of an antisense strand complementary to the sense strand from the prescribed sequence information. Specifically, for example, when "caccctgacccgcttcgtcatgg" is selected as 23-base sequence information wherein 2-base overhanging portions are added to both ends of the prescribed sequence as a result of the processes described above, the base sequence processing apparatus 100 calculates the base sequence information of a sense strand "5'-CCCUGACCCGCUUCGUCAUGG-3"' and the base sequence information of an antisense strand "5'-AUGACGAAGCGGGUCAGGGUG-3"'. Consequently, it is not necessary to manually arrange the sense strand and the antisense strand when a polynucleotide is ordered, thus improving convenience.

Furthermore, in the processes described in the embodiment, the processes described as being automatically performed may be entirely or partially performed manually, or the processes described as being manually performed may be entirely or partially performed automatically by a known method.

In addition, processing procedures, control procedures, specific names, information including various registration data and parameters, such as search conditions, examples of display screen, and database structures may be changed in any manner except when otherwise described.

Furthermore, with respect to the base sequence processing apparatus 100, the components are shown in the drawings only based on the functional concept, and it is not always necessary to physically construct the components as shown in the drawings.

For example, the process functions of the individual parts or individual units of the base sequence processing apparatus 100, in particular, the process functions performed in the controller 102, may be entirely or partially carried out by a CPU (Central Processing Unit) or programs which are interpreted and executed by the CPU. Alternatively, it may be possible to realize the functions based on hardware according to a wired logic. Additionally, the program is recorded in a recording medium which will be described below and is mechanically read by the base sequence processing apparatus 100 as required.

Namely, the memory 106, such as a ROM or HD, records a computer program which, together with OS (Operating System), gives orders to the CPU to perform various types of processing. The computer program is executed by being loaded into a RAM or the like, and, together with the CPU, constitutes the controller 102. Furthermore, the computer program may be recorded in an application program server which is connected to the base sequence processing apparatus 100 via any network 300, and may be entirely or partially downloaded as required.

The program of the present invention may be stored in a computer-readable recording medium. Here, examples of the "recording medium" include any "portable physical medium", such as a flexible disk, an optomagnetic disk, a ROM, an EPROM, an EEPROM, a CD-ROM, a MO, a DVD, or a flash disk; any "fixed physical medium", such as a ROM, a RAM, or a HD which is incorporated into various types of computer system; and a "communication medium" which holds the program for a short period of time, such as a communication line or carrier wave, in the case when the program is transmitted via a network, such as a LAN, a WAN, or Internet.

Furthermore, the "program" means a data processing method described in any language or by any description method, and the program may have any format (e.g., source code or binary code). The "program" is not always limited to the one having a single system configuration, and may have a distributed system configuration including a plurality of modules or libraries, or may achieve its function together with another program, such as OS (Operating System). With respect to specific configurations and procedures for reading the recording medium in the individual units shown in the embodiment, or installation procedures after reading, etc., known configurations and procedures may be employed.

The various types of databases, etc. (target gene base sequence file 106a - target gene annotation database 106h) stored in the memory 106 are storage means, such as memories (e.g., RAMs and ROMs), fixed disk drives (e.g., hard disks), flexible disks, and optical disks, which store various types of programs used for various processes and Web site provision, tables, files, databases, files for Web pages, etc.

Furthermore, the base sequence processing apparatus 100 may be produced by connecting peripheral apparatuses, such as a printer, a monitor, and an image scanner, to a known information processing apparatus, for example, an information processing terminal, such as a personal computer or a workstation, and installing software (including programs, data, etc.) which implements the method of the present invention into the information processing apparatus.

Furthermore, specific modes of distribution/integration of the base sequence processing apparatus 100, etc. are not limited to those shown in the specification and the drawings, and the base sequence processing apparatus 100, etc., may be entirely or partially distributed/integrated functionally or physically in any unit corresponding to various types of loading, etc. (e.g., grid computing). For example, the individual databases may be independently constructed as independent database units, or processing may be partially performed using CGI (Common Gateway Interface).

Furthermore, the network 300 has a function of interconnecting between the base sequence processing apparatus 100 and the external system 200, and for example, may include any one of the Internet, intranets, LANs (including both wired and radio), VANs , personal computer communication networks, public telephone networks (including both analog and digital), dedicated line networks (including both analog and digital), CATV networks, portable line exchange networks/portable packet exchange networks of the IMT2000 system, CSM system, or PDC/PDC-P system, radio paging networks, local radio networks, such as the Bluetooth, PHS networks, and satellite communication networks, such as CS, BS, and ISDB. Namely, the present system can transmit and receive various types of data via any network regardless of wired or radio.

EXAMPLES

The present invention will be described in more detail with reference to the examples. However, it is to be understood that the present invention is not restricted by the examples.

[Example 1]

<1> Gene for measuring RNAi effect and expression vector

As a target gene for measuring an RNAi effect by siRNA, a firefly (Photinus pyralis, P. pyralis) luciferase (luc) gene (P. pyralis luc gene: accession number: U47296) was used, and as an expression vector containing this gene, a pGL3-Control Vector (manufactured by Promega Corporation) was used. The segment of the P. pyralis luc gene is located between an SV40 promoter and a poly A signal within the vector. As an internal control gene, a luc gene of sea pansy (Renilla reniformis, R. reniformis) was used, and as an expression vector containing this gene, pRL-TK (manufactured by Promega Corporation) was used.

<2> Synthesis of 21-base double-stranded RNA (siRNA)

Synthesis of 21-base sense strand and 21-base antisense strand RNA (located as shown in Fig. 9; a to p) was entrusted to Genset Corporation through Hitachi Instrument Service Co., Ltd.

The double-stranded RNA used for inhibiting expression of the P. pyralis luc gene was prepared by associating sense and antisense strands. In the association process, the sense strand RNA and the antisense strand RNA were heated for 3 minutes in a reaction liquid of 10 mM Tris-HC1 (pH 7.5) and 20 mM NaCl, incubated for one hour at 37°C, and left to stand until the temperature reached room temperature. Formation of double-stranded polynucleotides was assayed by electrophoresis on 2% agarose gel in a TBE buffer, and it was confirmed that almost all the single-stranded polynucleotides were associated to form double-stranded polynucleotides.

<3> Mammalian cell cultivation

As mammalian cultured cells, human HeLa cells and HEK293 cells and Chinese hamster CHO-KI cells (RIKEN Cell bank) were used. As a medium, Dulbecco's modified Eagle's medium (manufactured by Gibco BRL) to which a 10% inactivated fetal bovine serum (manufactured by Mitsubishi Kasei) and as antibiotics, 10 units/ml of penicillin (manufactured by Meiji) and 50 μg/ml of streptomycin (manufactured by Meiji) had been added was used. Cultivation was performed at 37°C in the presence of 5% $CO_2$.

<4> Transfection of target gene, internal control gene, and siRNA into mammalian cultured cells

The mammalian cells were seeded at a concentration of 0.2 to 0.3 x $10^6$ cells/ml into a 24-well plate, and after one day, using a Ca-phosphate precipitation method (Saibo-Kogaku Handbook (Handbook for cell engineering), edited by Toshio Kuroki et al., Yodosha (1992)), 1.0 μg of pGL3-Control DNA, 0.5 or 1.0 μg of pRL-TK DNA, and 0.01, 0.1, 1, 10 or 100 nM of siRNA were introduced.

<5> Drosophila cell cultivation

As drosophila cultured cells, S2 cells (Schneider, I., et al., J. Embryol. Exp. Morph., 27, 353-365 (1972)) were used. As a medium, Schneider's Drosophila medium (manufactured by Gibco BRL) to which a 10% inactivated fetal bovine serum (manufactured by Mitsubishi Kasei) and as antibiotics, 10 units/ml of penicillin (manufactured by Meiji) and 50 μg/ml of streptomycin (manufactured by Meiji) had been added was used. Cultivation was performed at 25°C in the presence of 5% $CO_2$.

<6> Transfection of target gene, internal control gene, and siRNA into drosophila cultured cells

The S2 cells were seeded at a concentration of 1.0 x $10^6$ cells/ml into a 24-well plate, and after one day, using a Ca-phosphate precipitation method (Saibo-Kogaku Handbook (Handbook for cell engineering), edited by Toshio Kuroki et al., Yodosha (1992)), 1.0 μg of pGL3-Control DNA, 0.1 μg of pRL-TK DNA, and 0.01, 0.1, 1, 10 or 100 nM of siRNA were introduced.

<7> Measurement of RNAi effect

The cells transfected with siRNA were recovered 20 hours after transfection, and using a Dual-Luciferase Reporter Assay System (manufactured by Promega Corporation), the levels of expression (luciferase activities) of two types of luciferase (P. pyralis luc and reniformis luc) protein were measured. The amount of luminescence was measured using a Lumat LB9507 luminometer (EG&G Berthold).

<8> Results

The measurement results on the luciferase activities are shown in Fig. 10. Furthermore, the results of study on correspondence between the luciferase activities and the individual base sequences are shown in Fig. 11.

In Fig. 10, the graph represented by B shows the results in the drosophila cells, and the graph represented by C shows the results in the human cells. As shown in Fig. 10, in the drosophila cells, by creating RNA with a base number of 21, it was possible to inhibit the luciferase activities in almost all the sequences. On the other hand, in the human cells, it was evident that it was difficult to obtain sequences which could inhibit the luciferase activities simply by setting the base number at 21.

Analysis was then conducted on the regularity of base sequence with respect to RNA a to p. As shown in Fig. 11, with respect to 5 points of the double-stranded RNA, the base sequence was analyzed. With respect to siRNA a in the top row of the table shown in Fig. 11, the relative luciferase activity (RLA) is 0.03. In the antisense strand, from the 3' end, the base sequence of the overhanging portion (OH) is UC; the G/C content (content of guanine or cytosine) in the subsequent 7 bases (3'- T in Fig. 11) is 57%: the G/C content in the further subsequent 5 bases (M in Fig. 11) is 20; the G/C content in the further subsequent 7 bases (5'-T in Fig. 11) is 14%; the 5' end is U; and the G/C content in total is 32%. In the table, a lower RLA value indicates lower RLA activity, i.e., inhibition of the expression of luciferase.

As is evident from the results, in the base sequence of polynucleotides for causing RNA interference, it is highly probable that the 3' end is adenine or uracil and that the 5' end is guanine or cytosine. Furthermore, it has become clear that the 7-base sequence from the 3' end is rich in adenine or uracil.

[Example 2]

1. Construction of target expression vector pTREC

A target expression vector was constructed as follows. A target expression molecule is a molecule which allows expression of RNA having a sequence to be targeted by RNAi (hereinafter, also referred to as a "target sequence").

A target mRNA sequence was constructed downstream of the CMV enhancer/promoter of pCI-neo (GenBank Accession No. U47120, manufactured by Promega Corporation) (Fig. 25). That is, the following double-stranded oligomer was synthesized, the oligomer including a Kozak sequence (Kozak), an ATG sequence, a cloning site having a 23 base-pair sequence to be targeted (target), and an identification sequence for restriction enzyme (NheI, EcoRI, XhoI) for recombination. The double-stranded oligomer consists of a sequence shown in SEQ ID NO: 1 in the sequence listing and its complementary sequence. The synthesized double-stranded oligomer was inserted into the NheI/XbaI site of the pCI--nea to construct a target expression vector pTREC (Fig. 25). With respect to the intron, the intron site derived from β-globin originally incorporated in the pCI-neo was used. 5'-gctagccaccatggaattcacgcgtctcgagtctaga-3' (SEQ ID NO: 1)

The pTREC shown in Fig. 25 is provided with a promoter and an enhancer (pro/enh) and regions PAR(F) 1 and PAR(R) 1 corresponding to the PCR primers. An intron (Intron) is inserted into PAR(F) 1, and the expression vector is designed such that the expression vector itself does not become a template of PCR. After transcription of RNA, in an environment in which splicing is performed in eukaryotic cultured cells or the like, the intron site of the pTREC is removed to join two neighboring PAR(F) 1's. RNA produced from the pTREC can be amplified by RT-PCR. With respect to the intron, the intron site derived from β-globin originally incorporated in the pCI-neo was used.

The pTREC is incorporated with a neomycin-resistant gene (neo) as a control, and by preparing PCR primers corresponding to a part of the sequence in the neomycin-resistant gene and by subjecting the part of the neomycin-resistant gene to RT-PCR, the neomycin-resistant gene can be used as an internal standard control (internal control). PAR(F) 2 and PAR(R) 2 represent the regions corresponding to the PCR primers in the neomycin-resistant gene. Although not shown in the example of Fig. 25, an intron may be inserted into at least one of PAR(F) 2 and PAR(R) 2.
2. Effect of primer for detecting target mRNA (1) Transfection into cultured cells

HeLa cells were seeded at 0.2 to 0.3 x $10^6$ cells per well of a 24-well plate, and after one day, using Lipofectamine 2000 (manufactured by Invitrogen Corp.), 0.5 μg of pTREC vector was transfected according to the manual.

(2) Recovery of cells and quantification of mRNA

One day after the transfection, the cells were recovered and total RNA was extracted with Trizol (manufactured by Invitrogen Corp.). One hundred nanograms of the resulting RNA was reverse transcribed by Superscript II RT (manufactured by Invitrogen Corp.), using oligo (dT) primers, to synthesize cDNA. A control to which no reverse transcriptase was added was prepared. Using one three hundred and twentieth of the amount of the resulting cDNA as a PCR template, quantitative PCR was carried out in a 50-μl reaction system using SYBR Green PCR Master Mix (manufactured by Applied Biosystems Corp.) to quantify target mRNA (referred to as mRNA (T)) and, as an internal control, mRNA derived from the neomycin-resistant gene in the pTREC (referred to as mRNA (C)). A real-time monitoring apparatus ABI PRIZM7000 (manufactured by Applied Biosystems) was used for the quantitative PCR. A primer pair T (SEQ ID NOs: 2 and 3 in the sequence listing) and a primer pair C (SEQ ID NOs: 4 and 5 in the sequence listing) were used for the quantification of mRNA (T) and mRNA (C), respectively.
Primer pair T:

aggcactgggcaggtgtc (SEQ ID NO: 2)
tgctcgaagcattaaccctcacta (SEQ ID NO: 3)

Primer pair C

atcaggatgatctggacgaag (SEQ ID NO: 4) ctcttcagcaatatcacgggt (SEQ ID NO: 5)

Figs. 26 and 27 show the results of PCR. Each of Figs. 26 and 27 is a graph in which the PCR product is taken on the axis of ordinate and the number of cycles of PCR is taken on the axis of abscissa. In the neomycin-resistant gene, there is a small difference in the amplification of the PCR product between the case in which cDNA was synthesized by the reverse transcriptase (+RT) and the control case which no reverse transcriptase was added (-RT) (Fig. 26). This indicates that not only cDNA but also the vector remaining in the cells also acted as a template and was amplified. On the other hand, in target sequence mRNA, there is a large difference between the case in which the reverse transcriptase was added (+RT) and the case in which no transcriptase was added (-RT) (Fig. 27). This result indicates that since one member of the primer pair T is designed so as to sandwich the intron, cDNA derived from intron-free mRNA is efficiently amplified, while the remaining vector having the intron does not easily become a template.

3. Inhibition of expression of target mRNA by siRNA (1) Cloning of evaluation sequence to target expression vector

Sequences corresponding to the coding regions 812-83v and 35-57 of a human vimentin (VIM) gene (Ref Seq ID: NM_003380) were targeted for evaluation. The following synthetic oligonucleotides (evaluation sequence fragments) of SEQ ID NOs: 6 and 7 in the sequence listing were produced, the synthetic oligonucleotides including these sequences and identification sequences for EcoRI and XhoI. Evaluation sequence VIM35 (corresponding to 35-57 of VIM) 5'-gaattcgcaggatgttcggcggcccgggcctcgag-3' (SEQ ID NO: 6) Evaluation sequence VIM812 (corresponding to 812-834 of VIM) 5'-gaattcaagtacgtcagcaatatgaaagtctcgag-3' (SEQ ID NO: 7)
Using the EcoRI and XhoI sites located on both ends of each of the evaluation sequence fragments, each fragment was cloned as a new target sequence between the EcoRI and XhoI sites of the pTREC, and thereby pTREC-VIM35 and pTREC-VIM812 were constructed.

(2) Production of siRNA

siRNA fragments corresponding to the evaluation sequence VIM35 (SEQ ID NO: 8 in the sequence list, Fig. 28), the evaluation sequence VIM812 (SEQ ID NO: 9, Fig. 29), and a control sequence (siContorol, SEQ ID NO: 10, Fig. 30) were synthesized, followed by annealing. Each of the following siRNA sequences is provided with an overhanging portion on the 3' end.

siVIM35 5'-aggauguucggcggcccgggc-3' (SEQ ID NO: 8)
siVIM812 5'-guacgucagcaauaugaaagu-3' (SEQ ID NO: 9)

As a control, siRNA for the luciferase gene was used.

siControl 5'-cauucuauccgcuggaagaug-3' (SEQ ID NO: 10)

(3) Transfection into cultured cells

HeLa cells were seeded at 0.2 to 0.3 x $10^6$ cells per well of a 24-well plate, and after one day, using Lipofectamine 2000 (manufactured by Invitrogen Corp.), 0.5 μ]g of pTREC-VIM35 or pTREC-VIM812, and 100 nM of siRNA corresponding to the sequence derived from each VIM (siVIM35, siVIM812) were simultaneously transfected according to the manual. Into the control cells, 0.5 μg of pTREC-VIM35 or pTREC-VIM812 and 100 nM of siRNA for the luciferase gene (siControl) were simultaneously transfected.

(4) Recovery of cells and quantification of mRNA

One day after the transfection, the cells were recovered and total RNA was extracted with Trizol (Invitrogen). One hundred nanograms of the resulting RNA was reverse transcribed by SuperScript II RT (manufactured by Invitrogen Corp.), using oligo (dT) primers, to synthesize cDNA. Using one three hundred and twentieth of the amount of the resulting cDNA as a PCR template, quantitative PCR was carried out in a 50-μ1 reaction system using SYBR Green PCR Master Mix (manufactured by Applied Biosystems Corp.) to quantify mRNA (referred to as mRNA (T)) including the sequence derived from VIM to be evaluated and, as an internal control, mRNA derived from the neomycin-resistant gene in the pTREC (referred to as mRNA (C)).

A real-time monitoring apparatus ABI PRIZM7000 (manufactured by Applied Biosystems) was used for the quantitative PCR. The primer pair T (SEQ ID NOs: 2 and 3 in the sequence listing) and the primer pair C (SEQ ID NOs: 4 and 5 in the sequence listing) were used for the quantification of mRNA (T) and mRNA (C), respectively. The ratio (T/C) of the resulting values of mRNA was taken on the axis of ordinate (relative amount of target mRNA (%)) in a graph (Fig. 31).

In the control cells, since siRNA for the luciferase gene does not affect target mRNA, the ratio T/C is substantially 1. In VIM812 siRNA, the ratio T/C is extremely decreased. The reason for this is that VIM812 siRNA cut mRNA having the corresponding sequence, and it was shown that VIM812 siRNA has the RNAi effect. On the other hand, in VIM35 siRNA, the T/C ratio was substantially the same as that of the control, and thus it was shown that the sequence of VIM35 does not substantially have the RNAi effect.

[Example 31

1. Inhibition of expression of endogenous vimentin by siRNA

(1) Transfection into cultured cells

HeLa cells were seeded at 0.2 to 0.3 x $10^6$ cells per well of a 24-well plate, and after one day, using Lipofectamine 2000 (manufactured by Invitrogen Corp.), 100 nM of siRNA for VIM (siVIM35 or siVIM812) or control siRNA (siControl) and, as a control for transfection efficiency, 0.5 μg of pEGFP (manufactured by Clontech) were simultaneously transfected according to the manual. pEGFP is incorporated with EGFP.

(2) Assay of endogenous vimentin mRNA

Three days after the transfection, the cells were recovered and total RNA was extracted with Trizol (manufactured by Invitrogen Corp.). One hundred nanograms of the resulting RNA was reverse transcribed by Superscript II RT (manufactured by Invitrogen Corp.), using oligo (dT) primers, to synthesize cDNA. PCR was carried out using the cDNA product as a template and using primers for vimentin, VIM-F3-84 and VIM-R3-274 (SEQ ID NOs: 11 and 12).
VIM-F3-84; gagctacgtgactacgtcca (SEQ ID NO: 11)
VIM-R3-274; gttcttgaactcggtgttgat (SEQ ID NO: 12)

Furthermore, as a control, PCR was carried out using β-actin primers ACTB-F2-481 and ACTB-R2-664 (SEQ ID NOs: 13 and 14). The level of expression of vimentin was evaluated under the common quantitative value of β-actin for each sample.
ACTB-F2-481; cacactgtgcccatctacga (SEQ ID NO: 13)
ACTB-R2-664; gccatctcttgctcgaagtc (SEQ ID NO: 14)

The results are shown in Fig. 32. In Fig. 32, the case in which siControl (i.e., the sequence unrelated to the target) is incorporated is considered as 100% for comparison, and the degree of decrease in mRNA of VIM when siRNA is incorporated into VIM is shown. siVIM-812 was able to effectively inhibit VIM mRNA. In contrast, use of siVIM-35 did not substantially exhibit the RNAi effect.

(3) Antibody staining of cells

Three days after the transfection, the cells were fixed with 3.7% formaldehyde, and blocking was performed in accordance with a conventional method. Subsequently, a rabbit anti-vimentin antibody (α-VIM) or, as an internal control, a rabbit anti-Yes antibody (α-Yes) was added thereto, and reaction was carried out at room temperature. Subsequently, the surfaces of the cells were washed with PBS (Phosphate Buffered Saline), and as a secondary antibody, a fluorescently-labeled anti-rabbit IgG antibody was added thereto. Reaction was carried out at room temperature. After the surfaces of the cells were washed with PBS, observation was performed using a fluorescence microscope.

The fluorescence microscope observation results are shown in Fig. 33. In the nine frames of Fig. 33, the parts appearing white correspond to fluorescent portions. In EGFP and Yes, substantially the same expression was confirmed in all the cells. In the cells into which siControl and siVIM35 were introduced, fluorescence due to antibody staining of vimentin was observed, and the presence of endogenous vimentin was confirmed. On the other hand, in the cells into which siVIM812 was introduced, fluorescence was significantly weaker than that of the cells into which siControl and siVIM35 were introduced. The results show that endogenous vimentin mRNA was interfered by siVIM812, and consequently, the level of expression of vimentin protein was decreased. It has become evident that siVIM812 also has the RNAi effect against endogenous vimentin mRNA.

The results obtained in the assay system of the present invention [Example 2] matched well with the results obtained in the cases in which endogenous genes were actually treated with corresponding siRNA [Example 31. Consequently, it has been confirmed that the assay system is effective as a method for evaluating the RNAi activity of any siRNA.

[Example 4]

Base sequences were designed based on the predetermined rules (a) to (d). The base sequences were designed by a base sequence processing apparatus which runs the siRNA sequence design program. As the base sequences, 15 sequences (SEQ ID NOs: 15 to 29) which were expected to have RNAi activity and 5 sequences (SEQ ID NOs: 30 to 34) which were not expected to have RNAi activity were prepared.

RNAi activity was evaluated by measuring the luciferase activity as in Example 1 except that the target sequence and siRNA to be evaluated were prepared based on each of the designed sequences. The results are shown in Fig. 34. A low luciferase relative activity value indicates an effective state, i.e., siRNA provided with RNAi activity. All of the siRNA which was expected to have RNAi activity by the program effectively inhibited the expression of luciferase.
[Sequences which exhibited RNAi activity; prescribed sequence portions, excluding overhanging portions]
5,gacgccaaaaacataaaga (SEQ ID NO: 15)
184,gttggcagaagctatgaaa (SEQ ID NO: 16)
272,gtgttgggcgcgttattta (SEQ ID NO: 17)
309,ccgcgaacgacatttataa (SEQ ID NO: 18)
428,ccaatcatccaaaaaatta (SEQ ID NO: 19)
515,cctcccggttttaatgaat (SEQ ID NO: 20)
658,gcatgccagagatcctatt (SEQ ID NO: 21)
695,ccggatactgcgattttaa (SEQ ID NO: 22)
734,ggttttggaatgtttacta (SEQ ID NO: 23)
774,gatttcgagtcgtcttaat (SEQ ID NO: 24)
891,gcactctgattgacaaata (SEQ ID NO: 25)
904,caaatacgatttatctaat (SEQ ID NO: 26)
1186,gattatgtccggttatgta (SEQ ID NO: 27)
1306,ccgcctgaagtctctgatt (SEQ ID NO: 28)
1586,ctcgacgcaagaaaaatca (SEQ ID NO: 29)
[Sequences which did not exhibit RNAi activity; prescribed sequence portions, excluding overhanging portions]
14,aacataaagaaaggcccgg (SEQ ID NO: 30)
265,tatgccggtgttgggcgcg (SEQ ID NO: 31)
295,agttgcagttgcgcccgcg (SEQ ID NO: 32)
411,acgtgcaaaaaaagctccc (SEQ ID NO: 33)
1044,ttctgattacacccgaggg (SEQ ID NO: 34)

[Example 5]

siRNA sequences for the SARS virus were designed and the RNAi activities thereof were investigated. The RNAi activity was evaluated by the same assay as used in Example 2 except that the target sequences and the sequences to be evaluated were changed.

The siRNA sequences were designed with respect to 3CL-PRO, RdRp, Spike glycoprotein, Small envelope E protein, Membrane glycoprotein M, Nucleocapsid protein, and s2m motif from the genome of the SARS virus, using the siRNA sequence design program, so as to conform to the predetermined regularity.

As a result of the assay shown in Fig. 35, 11 siRNA sequences designed so as to conform to the regularity effectively inhibited RNA in which corresponding siRNA sequences were incorporated as targets. The case in which siControl (the sequence unrelated to SARS) is incorporated is considered as being 100%, and the relative amount of target mRNA in the case in which each siRNA sequence of SARS is incorporated is shown. When each siRNA sequence is incorporated, the amount of target RNA was decreased to about 10% or less, and the presence of the RNAi activity was confirmed.

[siRNA sequences designed (prescribed sequence portions, excluding overhanging portions)]
sicontrol;gggcgcggtcggtaaagtt (SEQ ID NO: 35)
3CL-PRO;SARS-10754;ggaattgccgtcttagata (SEQ ID NO: 36)
3CL-PRO;SARS-10810;gaatggtcgtactatcctt (SEQ ID NO: 37)
RdRp;SARS-14841;ccaagtaatcgttaacaat (SEQ ID NO: 38)
Spike glycoprotein;SARS-23341;gcttggcgcatatattcta (SEQ ID NO: 39)
Spike glycoprotein;SARS-24375;cctttcgcgacttgataaa (SEQ ID NO: 40)
Small envelope E protein;SARS-26233:gtgcgtactgctgcaatat (SEQ ID NO: 41)
Small envelope E protein;SARS-26288;ctactcgcgtgttaaaaat (SEQ ID NO: 42)
Membrane glycoprotein M;SARS-26399;gcagacaacggtactatta (SEQ ID NO: 43)
Membrane glycoprotein M;SARS-27024;ccggtagcaacgacaatat (SEQ ID NO: 44)
Nucleocapsid protein;SARS-28685;cgtagtcgcggtaattcaa (SEQ ID NO: 45) s2m motif;SARS-29606;gatcgagggtacagtgaat (SEQ ID NO: 46)

[Example 6]

The following siRNA sequences were designed in accordance with the columns "<5> siRNA sequence design program" and "<7> Base sequence processing apparatus for running siRNA sequence design program, etc.". The designed siRNA sequences are shown under SEQ ID NOs: 47 to 892 in the sequence listing.

(Target gene of RNAi) NM_000604, Homo sapiens fibroblast growth factor receptor 1 (fms-related tyrosine kinase 2, Pfeiffer syndrome) (FGFR1).
(Target sequences)
NM_000604-807,gtagcaacgtggagttcat (SEQ ID NO: 47)
NM_000604-806,ggtagcaacgtggagttca (SEQ ID NO: 48)
NM_000604-811,caacgtggagttcatgtgt (SEQ ID NO: 49)
NM_000604-880,ggtgaatgggagcaagatt (SEQ ID NO: 50)
NM_000604-891,gcaagattggcccagacaa (SEQ ID NO: 51)
(Target sequence effective for mouse homolog)
NM_000604-818,gagttcatgtgtaaggtgt (SEQ ID NO: 52)
(Target gene of RNAi)
NM_000141, Homo sapiens fibroblast growth factor receptor 2 (bacteria-expressed kinase, keratinocyte growth factor receptor, craniofacial dysostosis 1, Crouzon syndrome, Pfeiffer syndrome, Jackson-Weiss syndrome) (FGFR2).
(Target sequences)
NM_000141-612,gaggctacaaggtacgaaa (SEQ ID NO: 53)
NM_000141-615,gctacaaggtacgaaacca (SEQ ID NO: 54)
NM_000141-637,ctggagcctcattatggaa (SEQ ID NO: 55)
NM_000141-574, gaaaaacgggaaggagttt (SEQ ID NO: 56)
(Target sequences effective for mouse homolog)
NM_000141-595, gcaggagcatcgcattgga (SEQ ID NO: 57)
NM_000141-69, ccttcagtttagttgagga (SEQ ID NO: 58)
NM_000141-70, cttcagtttagttgaggat (SEQ ID NO: 59)
(Target gene of RNAi)
NM_000142, Homo sapiens fibroblast growth factor receptor 3 (achondroplasia, thanatophoric dwarfism) (FGFR3).
(Target sequences)
NM_000142-899, gacggcacaccctacgtta (SEQ ID NO: 60)
NM_000142-1925, cacaacctcgactactaca (SEQ ID NO: 61)
NM_000142-2154, gcacacacgacctgtacat (SEQ ID NO: 62)
NM_000142-678, cctgcgtcgtggagaacaa (SEQ ID NO: 63)

NM_000142-2157, cacacgacctgtacatgat (SEQ ID NO: 64)
(Target sequence effective for mouse homolog)
NM_000142-812, gagttccactgcaaggtgt (SEQ ID NO: 65)
(Target gene of RNAi)
NM_004448, Homo sapiens v-erb-b2 erythroblastic leukemia viral oncogene homolog 2, neuro/glioblastoma derived oncogene homolog (avian) (ERBB2).
(Target sequences)
NM_004448-356, ggagacccgctgaacaata (SEQ ID NO: 66)
NM_004448-3645, ccttcgacaacctctatta (SEQ ID NO: 67)
NM_004448-3237, gggctggctccgatgtatt (SEQ ID NO: 68)
NM_004448-3238, ggctggctccgatgtattt (SEQ ID NO: 69)
NM_004448-3240, ctggctccgatgtatttga (SEQ ID NO: 70)
(Target gene of RNAi)
NM_001982, Homo sapiens v-erb-b2 erythroblastic leukemia viral oncogene homolog 3 (avian) (ERBB3).
(Target sequences)
NM_001982-1347, gtgctgggcgtatctatat (SEQ ID NO: 71)
NM_001982-1349, gctgggcgtatctatataa (SEQ ID NO: 72)
NM_001982-1548, gcttgtcctgtcgaaatta (SEQ ID NO: 73)
NM_001982-1549, cttgtcctgtcgaaattat (SEQ ID NO: 74)
NM_001982-2857, cattcgcccaacctttaaa (SEQ ID NO: 75)
(Target gene of RNAi)
NM_005235, Homo sapiens v-erb-a erythroblastic leukemia viral oncogene homolog 4 (avian) (ERBB4).
(Target sequences)
NM_005235-295, ggagaatttacgcattatt (SEQ ID NO: 76)
NM_005235-2120, gctcaacttcgtattttga (SEQ ID NO: 77)
NM_005235-2940, ctcaaagatacctagttat (SEQ ID NO: 78)
NM_005235-2121, ctcaacttcgtattttgaa (SEQ ID NO: 79)
NM_005235-2880, ctgacagtagacctaaatt (SEQ ID NO: 80)
(Target gene of RNAi)
NM_002227, Homo sapiens Janus kinase 1 (a protein tyrosine kinase) (JAK1).
(Target sequences)
NM_002227-441, ctcagggacagtatgattt (SEQ ID NO: 81)
NM_002227-1299, cagaatacgccatcaataa (SEQ ID NO: 82)
NM_002227-673, gatgcggataaataatgtt (SEQ ID NO: 83)
NM_002227-672, ggatgcggataaataatgt (SEQ ID NO: 84)
NM_002227-3385, ctttcagaaccttattgaa (SEQ ID NO: 85)
(Target sequences effective for mouse homolog)
NM_002227-607, cagctacaagcgatatatt (SEQ ID NO: 86)
NM_002227-3042, caattgaaaccgataagga (SEQ ID NO: 87)
NM_002227-2944, gggttctcggcaatacgtt (SEQ ID NO: 88)
(Target gene of RNAi)
NM_004972, Homo sapiens Janus kinase 2 (a protein tyrosine kinase) (JAK2).
(Target sequences)
NM_004972-2757, ctggtcggcgtaatctaaa (SEQ ID NO: 89)
NM_004972-2759, ggtcggcgtaatctaaaat (SEQ ID NO: 90)
NM_004972-2760, gtcggcgtaatctaaaatt (SEQ ID NO: 91)
NM_004972-3175, ggaatttatgcgtatgatt (SEQ ID NO: 92)
NM_004972-1452, ctgttcgctcagacaatat (SEQ ID NO: 93)
(Target sequences effective for mouse homolog)
NM_004972-872, ggaaacggtggaattcagt (SEQ ID NO: 94)
NM_004972-870, ctggaaacggtggaattca (SEQ ID NO: 95)
NM_004972-847, gattttgcaaccattata (SEQ ID NO: 96)
(Target gene of RNAi)
NM_000215, Homo sapiens Janus kinase 3 (a protein tyrosine kinase, leukocyte) (JAK3).
(Target sequences)
NM_000215-2315, gtcattcgtgacctcaata (SEQ ID NO: 97)
NM_000215-2522, gacccgctagcccacaata (SEQ ID NO: 98)
NM_000215-2524, cccgctagcccacaataca (SEQ ID NO: 99)

NM_000215-1788, ccatggtgcaggaatttgt (SEQ ID NO: 100)
NM_000215-1825, catgtatctgcgaaaacgt (SEQ ID NO: 101)
(Target gene of RNAi)
NM_003331 , Homo sapiens tyrosine kinase 2 (TYK2).
(Target sequences)
NM_003331-3213, gcctgaaggagtataagtt (SEQ ID NO: 102)
NM_003331-2658, cggaccctacggttttcca (SEQ ID NO: 103)
NM_003331-299, ctatatttccgcataaggt (SEQ ID NO: 104)
(Target sequences effective for mouse homolog)
NM_003331-2674, ccacaagcgctatttgaaa (SEQ ID NO: 105)
NM_003331-2675, cacaagcgctatttgaaaa (SEQ ID NO: 106)
NM_003331-328, gaactggcatggcatgaat (SEQ ID NO: 107)
(Target gene of RNAi)
NM_001079, Homo sapiens zeta-chain (TCR) associated protein kinase 70kDa (ZAP70).
(Target sequences)
NM_001079-512, gaggccgagcgcaaacttt (SEQ ID NO: 108)
NM_001079-1512, ggtacgcacccgaatgcat (SEQ ID NO: 109)
NM_001079-242, gagctctgcgagttctact (SEQ ID NO: 110)
NM_001079-929, gacacgagcgtgtatgaga (SEQ ID NO: 111)
NM_001079-1412, cggcactacgccaagatca (SEQ ID NO: 112)
(Target sequence effective for mouse homolog)
NM_001079-1566, ggagctatggggtcaccat (SEQ ID NO: 113)
(Target gene of RNAi)
NM_005417, Homo sapiens v-src sarcoma (Schmidt-Ruppin A-2) viral oncogene homolog (avian) (SRC).
(Target sequences)
NM_005417-185, ctgttcggaggcttcaact (SEQ ID NO: 114)
NM_005417-685, ggtggcctactactccaaa (SEQ ID NO: 115)
NM_005417-474, gggagtcagagcggttact (SEQ ID NO: 116)
NM_005417-480, cagagcggttactgctcaa (SEQ ID NO: 117)
NM_005417-567, cagtgtctgacttcgacaa (SEQ ID NO: 118)
(Target sequence effective for mouse homolog)
NM_005417-651, cctcccgcacccagttcaa (SEQ ID NO: 119)
(Target gene of RNAi)
NM_002350, Homo sapiens v-yes-1 Yamaguchi sarcoma viral related oncogene homolog (LYN).
(Target sequences)
NM_002350-610, cagcgacatgattaaacat (SEQ ID NO: 120)
NM_002350-533, gttattaagcactacaaaa (SEQ ID NO: 121)
NM_002350-606, gtatcagcgacatgattaa (SEQ ID NO: 122)
(Target sequences effective for mouse homolog)
NM_002350-783, ggatgggttactataacaa (SEQ ID NO: 123)
NM_002350-694, gaagccatgggataaagat (SEQ ID NO: 124)
NM_002350-541, gcactacaaaattagaagt (SEQ ID NO: 125)
(Target gene of RNAi)
NM_005157, Homo sapiens v-abl Abelson murine leukemia viral oncogene homolog 1 (ABL1).
(Target sequences)
NM_005157-232, cactctaagcataactaaa (SEQ ID NO: 126)
NM_005157-770, gagggcgtgtggaagaaat (SEQ ID NO: 127)
NM_005157-262, ccgggtcttaggctataat (SEQ ID NO: 128)
NM_005157-264, gggtcttaggctataatca (SEQ ID NO: 129)
NM_005157-484, catctcgctgagatacgaa (SEQ ID NO: 130)
(Target sequences effective for mouse homolog)
NM_005157-217, ggccagtggagataacact (SEQ ID NO: 131)
NM_005157-1227, gcctggcctacaacaagtt (SEQ ID NO: 132)
NM_005157-680, gtgtcccccaactacgaca (SEQ ID NO: 133)
(Target gene of RNAi)
NM_005158, Homo sapiens v-abl Abelson murine leukemia viral oncogene homolog 2 (arg, Abelson-related gene) (ABL2).
(Target sequences)

NM_005158-3273, ctcaaactcgcaacaaatt (SEQ ID NO: 134)
NM_005158-3272, cctcaaactcgcaacaaat (SEQ ID NO: 135)
NM_005158-1425, ctaaggtttatgaacttat (SEQ ID NO: 136)
NM_005158-448, gctcagcagtctaatcaat (SEQ ID NO: 137)
NM_005158-3110, caggccgctgagaaaatct (SEQ ID NO: 138)
(Target gene of RNAi)
NM_004071, Homo sapiens CDC-like kinase 1 (CLK1).
(Target sequences)
NM_004071-1215, ccaggaaacgtaaatattt (SEQ ID NO: 139)
NM_004071-774, catttcgactggatcatat (SEQ ID NO: 140)
NM_004071-1216, caggaaacgtaaatatttt (SEQ ID NO: 141)
NM_004071-973, ctttggtagtgcaacatat (SEQ ID NO: 142)
NM_004071-463, cgtactaagtgcaagatat (SEQ ID NO: 143)
(Target gene of RNAi)
NM_001291, Homo sapiens CDC-like kinase 2 (CLK2).
(Target sequences)
NM_001291-202, gtatgaccggcgatactgt (SEQ ID NO: 144)
NM_001291-225, gctacagacgcaacgatta (SEQ ID NO: 145)
NM_001291-226, ctacagacgcaacgattat (SEQ ID NO: 146)
NM_001291-45, ggagttaccgtgaacacta (SEQ ID NO: 147)
NM_001291-45, gagttaccgtgaacactat (SEQ ID NO: 148)
(Target gene of RNAi)
NM_001292, Homo sapiens CDC-like kinase 3 (CLK3).
(Target sequences)
NM_001292-189, gccgtgacagcgatacata (SEQ ID NO: 149)
NM_001292-72, cctacagtcgggaacatga (SEQ ID NO: 150)
NM_001292-73, ctacagtcgggaacatgaa (SEQ ID NO: 151)
NM_001292-188, cgccgtgacagcgatacat (SEQ ID NO: 152)
NM_001292-121, gcctcccccacgaagatct (SEQ ID NO: 153)
(Target sequence effective for mouse homolog)
NM_001292-388, ggtgaaggcacctttggca (SEQ ID NO: 154)
(Target gene of RNAi)
NM_020666, Homo sapiens CDC-like kinase 4 (CLK4).
(Target sequences)
NM_020666-617, gtattagagcacttaaata (SEQ 1D NO: 155)
NM_020666-1212, gaaaacgcaagtattttca (SEQ ID NO: 156)
NM_020666-1348, cctggttcgaagaatgtta (SEQ ID NO: 157)
NM_020666-181, cttgaatgagcgagattat (SEQ ID NO: 158)
NM_020666-803, cagatctgccagtcaataa (SEQ ID NO: 159)
(Target sequences effective for mouse homolog)
NM_020666-457, cgttctaagagcaagatat (SEQ ID NO: 160)
NM_020666-446, caaagtggagacgttctaa (SEQ ID NO: 161)
NM_020666-461, ctaagagcaagatatgaaa (SEQ ID NO: 162)
(Target gene of RNAi)
NM_002093, Homo sapiens glycogen synthase kinase 3 beta (GSK3B).
(Target sequences)
NM_002093-326, gtccgattgcgttatttct (SEQ ID NO: 163)
NM_002093-307, gctagatcactgtaacata (SEQ ID NO: 164)
NM_002093-451, gacgctccctgtgatttat (SEQ ID NO: 165)
NM_002093-632, cccaatgtttcgtatatct (SEQ ID NO: 166)
NM_002093-623, cgaggagaacccaatgttt (SEQ ID NO: 167)
(Target sequences effective for mouse homolog)
NM_002093-206, gtatatcaagccaaacttt (SEQ ID NO: 168)
NM_002093-195, catttggtgtggtatatca (SEQ ID NO: 169)
NM_002093-205, ggtatatcaagccaaactt (SEQ ID NO: 170)
(Target gene of RNAi)
NM_182691, Homo sapiens SFRS protein kinase 2 (SRPK2).
(Target sequences)

NM_182691-1312, gccaaatggacgacataaa (SEQ ID NO: 171)
NM_182691-1313, ccaaatggacgacataaaa (SEQ ID NO: 172)
NM_182691-1314, caaatggacgacataaaat (SEQ ID NO: 173)
NM_182691-1985, ctgatcccgatgttagaaa (SEQ ID NO: 174)
NM_182691-233, ggccggtatcatgttatta (SEQ ID NO: 175)
(Target gene of RNAi)
NM_005430, Homo sapiens wingless-type MMTV integration site family, member 1 (WNT1).
(Target sequences)
NM_005430-614, ggccgtacgaccgtattct (SEQ ID NO: 176)
NM_005430-205, gcgtctgatacgccaaaat (SEQ ID NO: 177)
NM_005430-855, cccacgacctcgtctactt (SEQ ID NO: 178)
NM_005430-196, caaacagcggcgtctgata (SEQ ID NO: 179)
(Target sequences effective for mouse homolog)
NM_005430-875, gagaaatcgcccaacttct (SEQ ID NO: 180)
NM_005430-863, ctcgtctacttcgagaaat (SEQ ID NO: 181)
NM_005430-860, gacctcgtctacttcgaga (SEQ ID NO: 182)
(Target gene of RNAi)
NM_003391, Homo sapiens wingless-type MMTV integration site family member 2 (WNT2).
(Target sequences)
NM_003391-111, gggtgatgtgcgataatgt (SEQ ID NO: 183)
NM_003391-681, ggaaaacgggcgattatct (SEQ ID NO: 184)
NM_003391-764, gctaacgagaggtttaaga (SEQ ID NO: 185)
NM_003391-765, ctaacgagaggtttaagaa (SEQ ID NO: 186)
NM_003391-295, ggtcctactccgaagtagt (SEQ ID NO: 187)
(Target sequences effective for mouse homolog)
NM_003391-797, gacctcgtgtattttgaga (SEQ ID NO: 188)
NM_003391-790, gaaaaatgacctcgtgtat (SEQ ID NO: 189)
NM_003391-789, cgaaaaatgacctcgtgta (SEQ ID NO: 190)
(Target gene of RNAi)
NM_004625, Homo sapiens wingless-type MMTV integration site family, member 7A (WNT7A).
(Target sequences)
NM_004625-92, ctgggcgcaagcatcatct (SEQ ID NO: 191)
NM_004625-313, gttcacctacgccatcatt (SEQ ID NO: 192)
NM_004625-524, gcccggactctcatgaact (SEQ ID NO: 193)
NM_004625-480, gcttcgccaaggtctttgt (SEQ ID NO: 194)
(Target sequences effective for mouse homolog)
NM_004625-205, cctggacgagtgtcagttt (SEQ ID NO: 195)
NM_004625-209, gacgagtgtcagtttcagt (SEQ ID NO: 196)
NM_004625-172, catcatcgtcataggagaa (SEQ ID NO: 197)
(Target gene of RNAi)
NM_004626, Homo sapiens wingless-type MMTV integration site family, member 11 (WNT11).
(Target sequences)
NM_004626-543, gatcccaagccaataaact (SEQ ID NO: 198)
NM_004626-917, gacagctgcgaccttatgt (SEQ ID NO: 199)
NM_004626-915, gcgacagctgcgaccttat (SEQ ID NO: 200)
NM_004626-54, ccggcgtgtgctatggcat (SEQ ID NO: 201)
(Target sequences effective for mouse homolog)
NM_004626-59, gtgtgctatggcatcaagt (SEQ ID NO: 202)
NM_004626-560, ctgatgcgtctacacaaca (SEQ ID NO: 203)
NM_004626-562, gatgcgtctacacaacagt (SEQ ID NO: 204)
(Target gene of RNA)
NM_030753, Homo sapiens wingless-type MMTV integration site family, member 3 (WNT3).
(Target sequences)
NM_030753-417, gctgtgactcgcatcataa (SEQ ID NO: 205)
NM_030753-483, ctgacttcggcgtgttagt (SEQ ID NO: 206)
NM_030753-485, gacttcggcgtgttagtgt (SEQ ID NO: 207)
(Target sequences effective for mouse homolog)
NM_030753-887, gaccggacttgcaatgtca (SEQ ID NO: 208)

NM_030753-56, ctcgctggctacccaattt (SEQ ID NO: 209)
NM_030753-59, gctggctacccaatttggt (SEQ ID NO: 210)
(Target gene of RNAi)
NM_033131, Homo sapiens wingless-type MMTV integration site family, member 3A (WNT3A).
(Target sequences)
NM_033131-2, gccccactcggatacttct (SEQ ID NO: 211)
NM_033131-3, ccccactcggatacttctt (SEQ ID NO: 212)
NM_033131-4, cccactcggatacttctta (SEQ ID NO: 213)
NM_033131-77, gctgttgggccacagtatt (SEQ ID NO: 214)
NM_033131-821, gaggcctcgcccaacttct (SEQ ID NO: 215)
(Target sequences effective for mouse homolog)
NM_033131-168, ggaactacgtggagatcat (SEQ ID NO: 216)
NM_033131-50, ggcagctacccgatctggt (SEQ ID NO: 217)
NM_033131-165, gcaggaactacgtggagat (SEQ ID NO: 218)
(Target gene of RNAi)
NM_003392, Homo sapiens wingless-type MMTV integration site family, member 5A (WNT5A).
(Target sequences)
NM_003392-91, gtggtcgctaggtatgaat (SEQ ID NO: 219)
NM_003392-93, ggtcgctaggtatgaataa (SEQ ID NO: 220)
NM_003392-307, ggataacacctctgttttt (SEQ ID NO: 221)
NM_003392-57, ccttcgcccaggttgtaat (SEQ ID NO: 222)
NM_003392-87, cttggtggtcgctaggtat (SEQ ID NO: 223)
(Target sequences effective for mouse homolog)
NM_003392-163, ccaactggcaggactttct (SEQ ID NO: 224)
NM_003392-116, gttcagatgtcagaagtat (SEQ ID NO: 225)
NM_003392-102, gtatgaataaccctgttca (SEQ ID NO: 226)
(Target gene of RNAi)
NM_004196, Homo sapiens cyclin-dependent kinase-like 1 (CDC2-related kinase) (CDKL1).
(Target sequences)
NM_004196-405, cgaaacattccgtgattaa (SEQ ID NO: 227)
NM_004196-305, ctcgtgaagagcataactt (SEQ ID NO: 228)
NM_004196-458, ggaccgagtgactactata (SEQ ID NO: 229)
NM_004196-844, gttgcatcacccatatttt (SEQ ID NO: 230)
NM_004196-330, cactgcaagctgtaaattt (SEQ ID NO: 231)
(Target sequence effective for mouse homolog)
NM_004196-119, gatgaccctgtcataaaga (SEQ ID NO: 232)
(Target gene of RNAi)
NM_003948, Homo sapiens cyclin-dependent kinase-like 2 (CDC2-related kinase) (CDKL2).
(Target sequences)
NM_003948-623, gatcagctatatcatatta (SEQ ID NO: 233)
NM_003948-1379, ccatcaggcatttataaca (SEQ ID NO: 234)
NM_003948-1380, catcaggcatttataacat (SEQ ID NO: 235)
NM_003948-768, ctgaagtggtgatagattt (SEQ ID NO: 236)
NM_003948-626, cagctatatcatattatga (SEQ ID NO: 237)
(Target sequences effective for mouse homolog)
NM_003948-325, gattattaatggaattgga (SEQ ID NO: 238)
NM_003948-1012, ggtacaggataccaatgct (SEQ ID NO: 239)
(Target gene of RNAi)
NM_016508, Homo sapiens cyclin-dependent kinase-like 3 (CDKL3).
(Target sequences)
NM_016508-498, gagctcccgaattagtatt (SEQ ID NO: 240)
NM_016508-500, gctcccgaattagtattaa (SEQ ID NO: 241)
NM_016508-1290, cacccatcaatctaactaa (SEQ ID NO: 242)
NM_016508-1301, ctaactaacagtaatttga (SEQ ID NO: 243)
NM_016508-501, ctcccgaattagtattaaa (SEQ ID NO: 244)
(Target sequences effective for mouse homolog)
NM_016508-785, gttcatgcttgtttacaaa (SEQ ID NO: 245)
NM_016508-555, ctttgggctgtatgatcat (SEQ ID NO: 246)

NM_016508-776, gcagatatagttcatgctt (SEQ ID NO: 247)
(Target gene of RNAi)
NM_002745, Homo sapiens mitogen-activated protein kinase 1 (MAPK1).
(Target sequences)
NM_002745-746, gaagacctgaattgtataa (SEQ ID NO: 248)
NM_002745-276, caaccatcgagcaaatgaa (SEQ ID NO: 249)
NM_002745-849, ccaaagctctggacttatt (SEQ ID NO: 250)
NM_002745-749, gacctgaattgtataataa (SEQ ID NO: 251)
NM_002745-113, gtgtgctctgcttatgata (SEQ ID NO: 252)
(Target sequences effective for mouse homolog)
NM_002745-220, cttactgcgcttcagacat (SEQ ID NO: 253)
NM_002745-228, gcttcagacatgagaacat (SEQ ID NO: 254)
NM_002745-224, ctgcgcttcagacatgaga (SEQ ID NO: 255)
(Target gene of RNAi)
NM_016231, Homo sapiens nemo-like kinase (NLK).
(Target sequences)
NM_016231-450, gagtagcgctcaaaaagat (SEQ ID NO: 256)
NM_016231-1074, gcgctaaggcacatatact (SEQ ID NO: 257)
NM_016231-962, ctactaggacgaagaatat (SEQ ID NO: 258)
NM_016231-579, ctccacacattgactattt (SEQ ID NO: 259)
(Target sequences effective for mouse homolog)
NM_016231-703, gattttgcgaggtttgaaa (SEQ ID NO: 260)
NM_016231-1382, gtccgacaggttaaagaaa (SEQ ID NO: 261)
NM_016231-1384, ccgacaggttaaagaaatt (SEQ ID NO: 262)
(Target gene of RNAi)
NM_001315, Homo sapiens mitogen-activated protein kinase 14 (MAPK14).
(Target sequences)
NM_001315-401, ctccgaggtctaaagtata (SEQ ID NO: 263)
NM_001315-403, ccgaggtctaaagtatata (SEQ ID NO: 264)
NM_001315-251, ggtctgttggacgttttta (SEQ ID NO: 265)
NM_001315-212, ctgcggttacttaaacata (SEQ ID NO: 266)
NM_001315-405, gaggtctaaagtatataca (SEQ ID NO: 267)
(Target sequence effective for mouse homolog)
NM_001315-664, gtttcctggtacagaccat (SEQ ID NO: 268)
(Target gene of RNAi)
NM_002751, Homo sapiens mitogen-activated protein kinase 11 (MAPK11).
(Target sequences)
NM_002751-366, gcgacgagcacgttcaatt (SEQ ID NO: 269)
NM_002751-667, cccgggaagcgactacatt (SEQ ID NO: 270)
NM_002751-669, cgggaagcgactacattga (SEQ ID NO: 271)
NM_002751-731, gaggttctggcaaaaatct (SEQ ID NO: 272)
NM_002751-729, ctgaggttctggcaaaaat (SEQ ID NO: 273)
(Target gene of RNAi)
NM_002969, Homo sapiens mitogen-activated protein kinase 12 (MAPK12).
(Target sequences)
NM_002969-1018, gaagcgtgttacttacaaa (SEQ ID NO: 274)
NM_002969-262, gctgctggacgtattcact (SEX ID NO: 275)
NM_002969-1017, ggaagcgtgttacttacaa (SEQ ID NO: 276)
NM_002969-578, cccgaggtcatcttgaatt (SEQ ID NO: 277)
NM_002969-1013, gaatggaagcgtgttactt (SEQ ID NO: 278)
(Target gene of RNAi)
NM_002754, Homo sapiens mitogen-activated protein kinase 13 (MAPK13).
(Target sequences)
NM_002754-164, ctgagccgaccctttcagt (SEQ ID NO: 279)
NM_002754-174, cctttcagtccgagatctt (SEQ ID NO: 280)
NM_002754-978, ccttagaacacgagaaact (SEQ ID NO: 281)
NM_002754-285, ccctgcgcaacttctatga (SEQ ID NO: 282)
NM_002754-287, ctgcgcaacttctatgact (SEQ ID NO: 283)

(Target gene of RNAi)
NM_139049, Homo sapiens mitogen-activated protein kinase 8 (MAPK8).
(Target sequences)
NM_139049-449, gacttaaagcccagtaata (SEQ ID NO: 284)
NM_139049-213, gagagctagttcttatgaa (SEQ ID NO: 285)
NM_139049-451, cttaaagcccagtaatata (SEQ ID NO: 286)
(Target sequences effective for mouse homolog)
NM_139049-525, caggaacgagttttatgat (SEQ ID NO: 287)
NM_139049-524, gcaggaacgagttttatga (SEQ ID NO: 288)
NM_139049-283, gaaatccctagaagaattt (SEQ ID NO: 289)
(Target gene of RNAi)
NM_002752, Homo sapiens mitogen-activated protein kinase 9 (MAPK9).
(Target sequences)
NM_002752-116, gtttgtgctgcatttgata (SEQ ID NO: 290)
NM_002752-204, gagcttatcgtgaacttgt (SEQ ID NO: 291)
(Target sequences effective for mouse homolog)
NM_002752-878, gccagagatctgttatcaa (SEQ ID NO: 292)
NM_002752-879, ccagagatctgttatcaaa (SEQ ID NO: 293)
NM_002752-880, cagagatctgttatcaaaa (SEQ ID NO: 294)
(Target gene of RNAi)
NM_002753, Homo sapiens mitogen-activated protein kinase 10 (MAPK10).
(Target sequences)
NM_002753-668, gtggtgacacgttattaca (SEQ ID NO: 295)
NM_002753-957, cggactccgagcacaataa (SEQ ID NO: 296)
NM_002753-958, ggactccgagcacaataaa (SEQ ID NO: 297)
MM_002753-811, gtggaataaggtaattgaa (SEQ ID NO: 298)
NM_002753-1212, ctaaaaatggtgtagtaaa (SEQ ID NO: 299)
(Target sequences effective for mouse homolog)
NM_002753-1167, ggaaagaacttatctacaa (SEQ ID NO: 300)
NM_002753-584, gtagtcaagtctgattgca (SEQ ID NO: 301)
NM_002753-761, gaaatggttcgccacaaaa (SEQ ID NO: 302)
(Target gene of RNAi)
NM_001786, Homo sapiens cell division cycle 2, G1 to S and G2 to M (CDC2).
(Target sequences)
NM_001786-782, gatttgctctcgaaaatgt (SEQ ID NO: 303)
NM_001786-788, ctctcgaaaatgttaatct (SEQ ID NO: 304)
NM_001786-658, gggcactcccaataatgaa (SEQ ID NO: 305)
NM_001786-696, ctttacaggactataagaa (SEQ ID NO: 306)
NM_001786-562, gagtataggcaccatattt (SEQ ID NO: 307)
(Target sequence effective for mouse homolog)
NM_001786-869, gacaatcagattaagaaga (SEQ ID NO: 308)
(Target gene of RNAi)
NM_001798, Homo sapiens cyclin-dependent kinase 2 (CDK2).
(Target sequences)
NM_001798-224, ctctacctggtttttgaat (SEQ ID NO: 309)
NM_001798-690, cttctatgcctgattacaa (SEQ ID NO: 310)
NM_001798-770, gatggacggagcttgttat (SEQ ID NO: 311)
NM_001798-226, ctacctggtttttgaattt (SEQ ID NO: 312)
NM_001798-36, gcacgtacggagttgtgta (SEQ ID NO: 313)
(Target gene of RNAi)
NM_000075, Homo sapiens cyclin-dependent kinase 4 (CDK4).
(Target sequences)
NM_000075-45, cctatgggacagtgtacaa (SEQ ID NO: 314)
NM_000075-616, gatgtttcgtcgaaagcct (SEQ ID NO: 315)
NM_000075-161, cgtgaggtggctttactga (SEQ ID NO: 316)
NM_000075-35, ggtgtcggtgcctatggga (SEQ ID NO: 317)
NM_000075-242, cgaactgaccgggagatca (SEQ ID NO: 318)
(Target gene of RNAi)

NM_052984, Homo sapiens cyclin-dependent kinase 4 (CDK4), transcript variant 2, mRNA., 228.. 563,0
(Target sequences)
NM_052984-248, gaccgggagatcaagagat (SEQ ID NO: 319)
NM_052984-251, cgggagatcaagagatgtt (SEQ ID NO: 320)
(Target gene of RNAi)
NM_001799, Homo sapiens cyclin-dependent kinase 7 (MO15 homolog, Xenopus laevis, cdk-activating kinase) (CDK7).
(Target sequences)
NM_001799-242, ggacataaatctaatatta (SEQ ID NO: 321)
NM_001799-104, caaattgtcgccattaaga (SEQ ID NO: 322)
NM_001799-490, ccccaatagagcttataca (SEQ ID NO: 323)
NM_001799-20, cgggcaaagcgttatgaga (SEQ ID NO: 324)
NM_001799-21, gggcaaagcgttatgagaa (SEQ ID NO: 325)
(Target sequence effective for mouse homolog)
NM_001799-345, cctacatgttgatgactct (SEQ ID NO: 326)
(Target gene of RNAi)
NM_000455, Homo sapiens serine/threonine kinase 11 (Peutz-Jeghers syndrome) (STK11).
(Target sequences)
NM_000455-306, ggaggttacggcacaaaaa (SEQ ID NO: 327)
NM_000455-307, gaggttacggcacaaaaat (SEQ ID NO: 328)
NM_000455-309, ggttacggcacaaaaatgt (SEQ ID NO: 329)
NM_000455-1157, cccaaggccgtgtgtatga (SEQ ID NO: 330)
NM_000455-1158, ccaaggccgtgtgtatgaa (SEQ ID NO: 331)
(Target sequence effective for mouse homolog)
NM_000455-916, cagctggttccggaagaaa (SEQ ID NO: 332)
(Target gene of RNAi)
NM_001274, Homo sapiens CHK1 checkpoint homolog (S. pombe) (CHEK1).
(Target sequences)
NM_001274-456, cagtatttcggtataataa (SEQ ID NO: 333)
NM_001274-361, gcatggtattggaataact (SEQ ID NO: 334)
NM_001274-990, gcccctcatacattgataa (SEQ ID NO: 335)
NM_001274-1038, ccacatgtcctgatcatat (SEQ ID NO: 336)
NM_001274-227, ggcaatatccaatatttat (SEQ ID NO: 337)
(Target sequences effective for mouse homolog)
NM_001274-573, ggtcctgtggaatagtact (SEQ ID NO: 338)
NM_001274-416, gaaagggataacctcaaaa (SEQ ID NO: 339)
NM_001274-577, ctgtggaatagtacttact (SEQ ID NO: 340)
(Target gene of RNAi)
NM_002648, Homo sapiens pim-1 oncogene (PIM1).
(Target sequences)
NM_002648-831, ggccaaccttcgaagaaat (SEQ ID NO: 341)
NM_002648-601, cgatgggacccgagtgtat (SEQ ID NO: 342)
NM_002648-602, gatgggacccgagtgtata (SEQ ID NO: 343)
NM_002648-293, ggtttctccggcgtcatta (SEQ ID NO: 344)
NM_002648-834, caaccttcgaagaaatcca (SEQ ID NO: 345)
(Target sequences effective for mouse homolog)
NM_002648-96, ccctggagtcgcagtacca (SEQ ID NO: 346)
NM_002648-203, gtggagaaggaccggattt (SEQ ID NO: 347)
(Target gene of RNAi)
NM_006875, Homo sapiens pim-2 oncogene (PIM2).
(Target sequences)
NM_006875-698, ggggacattccctttgaga (SEQ ID NO: 348)
NM_006875-242, ctcgaagtcgcactgctat (SEQ ID NO: 349)
NM_006875-245, gaagtcgcactgctatgga (SEQ ID NO: 350)
NM_006875-499, gaacatcctgatagaccta (SEQ ID NO: 351)
NM_006875-468, gtggagttgtccatcgtga (SEQ ID NO: 352)
(Target gene of RNAi)
NM_021643, Homo sapiens tribbles homolog 2 (TRB2).
(Target sequences)

NM_021643-174, cttgtatcgggaaatactt (SEQ ID NO: 353)
NM_021643-71, gaagagttgtcgtctataa (SEQ ID NO: 354)
NM_021643-177, gtatcgggaaatacttatt (SEQ ID NO: 355)
NM_021643-524, ctcaagctgcggaaattca (SEQ ID NO: 356)
(Target sequences effective for mouse homolog)
NM_021643-41, gggagatcgcggaacaaaa (SEQ ID NO: 357)
NM_021643-382, gttctttgagcgaagctat (SEQ ID NO: 358)
NM_021643-143, cccgagactccgaacttgt (SEQ ID NO: 359)
(Target gene of RNAi)
NM_007118, Homo sapiens triple functional domain (PTPRF interacting) (TRIO).
(Target sequences)
NM_007118-1684, caccaatgcggataaatta (SEQ ID NO: 360)
NM_007118-1686, ccaatgcggataaattact (SEQ ID NO: 361)
NM_007118-3857, gaaatctacgaatttcata (SEQ ID NO: 362)
NM_007118-6395, gagcagatcgtcatattca (SEQ ID NO: 363)
NM_007118-8531, cctatccgtagcattaaaa (SEQ ID NO: 364)
(Target gene of RNAi)
NM_004938, Homo sapiens death-associated protein kinase 1 (DAPK1).
(Target sequences)
NM_004938-917, caatccgttcgcttgatat (SEQ ID NO: 365)
NM_004938-1741, ggtgtttcgtcgattatca (SEQ ID NO: 366)
NM_004938-1702, gtgtttcgtcgattatcaa (SEQ ID NO: 367)
NM_004938-2824, gaaggtacttcgaaatcat (SEQ ID NO: 368)
NM_004938-668, gaaacgttagcaaatgtat (SEQ ID NO: 369)
(Target sequences effective for mouse homolog)
NM_004938-609, gggtaataacctatatcct (SEQ ID NO: 370)
NM_004938-2697, gaggcgagtttggatatga (SEQ ID NO: 371)
NM_004938-490, ggcccataaaattgacttt (SEQ ID NO: 372)
(Target gene of RNAi)
NM_006252, Homo sapiens protein kinase, AMP-activated, alpha 2 catalytic subunit (PRKAA2).
(Target sequences)
NM_006252-760, gaaacgagcaactatcaaa (SEQ ID NO: 373)
NM_006252-148, gaagattcgcagtttagat (SEQ ID NO: 374)
NM_006252-1227, gcaaaccgtatgacattat (SEQ ID NO: 375)
NM_006252-1338, ctggcaattacgtgaaaat (SEQ ID NO: 376)
NM_006252-1340, ggcaattacgtgaaaatga (SEQ ID NO: 377)
(Target gene of RNAi)
NM_002742, Homo sapiens protein kinase C, mu (PRKCM).
(Target sequences)
NM_002742-508, ggtacgtcaaggtcttaaa (SEQ ID NO: 378)
NM_002742-1332, gattggatagcaaatgtat (SEQ ID NO: 379)
NM_002742-509, gtacgtcaaggtcttaaat (SEQ ID NO: 380)
NM_002742-370, ggaaggcgatcttattgaa (SEQ ID NO: 381)
(Target sequences effective for mouse homolog)
NM_002742-1913, caccctggtgttgtaaatt (SEQ ID NO: 382)
NM_002742-2041, cataacgaagtttttaatt (SEQ ID NO: 383)
NM_002742-2521, ctatcagacctggttagat (SEQ ID NO: 384)
(Target gene of RNAi)
NM_003684, Homo sapiens MAP kinase-interacting serine/threonine kinase 1 (MKNK1).
(Target sequences)
NM_003684-218, gagtatgccgtcaaaatca (SEQ ID NO: 385)
NM_003684-229, caaaatcatcgagaaacaa (SEQ ID NO: 386)
NM_003684-344, gatgacacaaggttttact (SEQ ID NO: 387)
NM_003684-192, gtgccgtgagcctacagaa (SEQ ID NO: 388)
NM_003684-379, gcaaggaggttccatctta (SEQ ID NO: 389)
(Target gene of RNAi)
NM_004759, Homo sapiens mitogen-activated protein kinase-activated protein kinase 2 (MAPKAPK2).
(Target sequences)

NM_004759-942, ccatcaccgagtttatgaa (SEQ ID NO: 390)
NM_004759-836, cgaatgggccagtatgaat (SEQ ID NO: 391)
NM_004759-563, cctgagaatctcttataca (SEQ ID NO: 392)
NM_004759-669, gttatacaccgtactatgt (SEQ ID NO: 393)
NM_004759-362, gatgtgtacgagaatctgt (SEQ ID NO: 394)
(Target gene of RNAi)
NM_172171, Homo sapiens calcium/calmodulin-dependent protein kinase (CaM kinase) II gamma (CAMK2G).
(Target sequences)
NM_172171-113, gagtacgcagcaaaaatca (SEQ ID NO: 395)
NM_172171-422, ctgctgctggcgagtaaat (SEQ ID NO: 396)
NM_172171-1075, ggtacacaacgctacagat (SEQ ID NO: 397)
NM_172171-474, gcctagccatcgaagtaca (SEQ ID NO: 398)
(Target sequences effective for mouse homolog)
NM_172171-425, ctgctggcgagtaaatgca (SEQ ID NO: 399)
NM_172171-260, ctcgtgtttgaccttgtta (SEQ ID NO: 400)
NM_172171-597, gcggggtcatcctgtatat (SEQ ID NO: 401)
(Target gene of RNAi)
NM_015981, Homo sapiens calcium/calmodulin-dependent protein kinase (CaM kinase) II alpha (CAMK2A).
(Target sequences)
NM_015981-1213, ccatcgattctattttgaa (SEQ ID NO: 402)
NM_015981-1210, cttccatcgattctatttt (SEQ ID NO: 403)
NM_015981-1067, cggaaacaggaaattataa (SEQ ID NO: 404)
NM_015981-1066, gcggaaacaggaaattata (SEQ ID NO: 405)
NM_015981-754, gaccattaacccatccaaa (SEQ ID NO: 406)
(Target sequences effective for mouse homolog)
NM_015981-1130, gagtcctacacgaagatgt (SEQ ID NO: 407)
NM_015981-1416, ggcagatcgtccacttcca (SEQ ID NO: 408)
NM_015981-1418, cagatcgtccacttccaca (SEQ ID NO: 409)
(Target gene of RNAi)
NM_020439, Homo sapiens calcium/calmodulin-dependent protein kinase IG (CAMK1G).
(Target sequences)
NM_020439-1354, ggtcatggtaccagttaaa (SEQ ID NO: 410)
NM_020439-1409, ggagtctgtctcattatgt (SEQ ID NO: 411)
NM_020439-639, gtggatacccccattcta (SEQ ID NO: 412)
NM_020439-823, ctggattgacggaaacaca (SEQ ID NO: 413)
NM_020439-662, gaaacggagtataagcttt (SEQ ID NO: 414)
(Target sequences effective for mouse homolog)
NM_020439-85, gggatcaggagctttctca (SEQ ID NO: 415)
NM_020439-903, gcaagtggaggoaagcctt (SEQ ID NO: 416)
(Target gene of RNAi)
NM_007194, Homo sapiens CHK2 checkpoint homolog (S. pombe) (CHEK2).
(Target sequences)
NM_007194-460, ctcttacattgcatacata (SEQ ID NO: 417)
NM_007194-201, ctcaggaactctattctat (SEQ ID NO: 418)
NM_007194-1233, gtttaggagttattctttt (SEQ ID NO: 419)
NM_007194-398, gataaataccgaacataca (SEQ ID NO: 420)
NM_007194-396, cagataaataccgaacata (SEQ ID NO: 421)
(Target sequences effective for mouse homolog)
NM_007194-614, gtagatgatcagtcagttt (SEQ ID NO: 422)
NM_007194-620, gatcagtcagtttatccta (SEQ ID NO: 423)
NM_007194-612, ctgtagatgatcagtcagt (SEQ ID NO: 424)
(Target gene of RNAi)
NM_002610, Homo sapiens pyruvate dehydrogenase kinase, isoenzyme 1 (PDK1).
(Target sequences)
NM_002610-1194, gactcccagtgtataacaa (SEQ ID NO: 425)
NM_002610-553, catgagtcgcatttcaatt (SEQ ID NO: 426)
NM_002610-306, ggacaccatccgttcaatt (SEQ ID NO: 427)
NM_002610-1086, gtctttacgcacaatactt (SEQ ID NO: 428)

NM_002610-388, ggatgctaaagctatttat (SEQ ID NO: 429)
(Target gene of RNAi)
NM_001619, Homo sapiens adrenergic, beta, receptor kinase 1 (ADKBK1).
(Target sequences)
NM_001619-474, gggacgtgttccagaaatt (SEQ ID NO: 430)
NM,001619-317, gagatcttcgactcataca (SEQ ID NO: 431)
NM_001619-665, gacaaaaagcgcatcaaga (SEQ ID NO: 432)
NM_001619-439, gccatacatcgaagagatt (SEQ ID NO: 433)
NM_001619-476, gacgtgttccagaaattca (SEQ ID NO: 434)
(Target sequences effective for mouse homolog)
NM_001619-1476, caaaaggaatcaagttact (SEQ ID NO: 435)
NM_001619-l474, cacaaaaggaatcaagtta (SEQ ID NO: 436)
MM_001619-1171, ccggcagcacaagaccaaa (SEQ ID NO: 437)
(Target gene of RNAi)
NM_005160, Homo sapiens adrenergic, beta, receptor kinase 2 (ADRBK2).
(Target sequences)
NM_005160-1779, gagagtccoggcaaaattt (SEQ ID NO: 438)
NM_005160-1778, ggagagtcccggcaaaatt (SEQ ID NO: 439)
NM_005160-1373, cagcatgtctacttacaaa (SEQ ID NO: 440)
NM_005160-307, cagaagtcgacaaatttat (SEQ ID NO: 441)
NM_005160-306, gcagaagtcgacaaattta (SEQ ID NO: 442)
(Target gene of RNAi)
NM_003161, Homo sapiens ribosomal protein S6 kinase, 70kDa, polypeptide 1 (RPS6KB1).
(Target sequences)
NM_003161-1294, ccgatcacctcgaagattt (SEQ ID NO: 443)
NM_003161-1556, cacctgcgtatgaatctat (SEQ ID NO: 444)
NM_003161-1296, gatcacctcgaagatttat (SEQ ID NO: 445)
NM_D03161-831, gtttgggagcattaatgta (SEQ ID NO: 446)
NM_003161-1295,cgatcacctcgaagattta (SEQ ID NO: 447)
(Target gene of RNAi)
NM_014496, Homo sapiens ribosomal protein S6 kinase, 90kDa, polypeptide 6 (RPS6KA6).
(Target sequences)
NM_014496-682, gaaggcttactcattttgt (SEQ ID NO: 448)
NM_014496-1552, ggaggctagtgatatacta (SEQ ID NO: 449)
NM_014496-1553, gaggctagtgatatactat (SEQ ID NO: 450)
NM_014496-1551, gggaggctagtgatatact (SEQ ID NO: 451)
NM_014496-1481, cttgttacggatttaatga (SEQ ID NO: 452)
(Target sequences effective for mouse homolog)
NM_014496-831, gaaatgagaccatgaatat (SEQ ID NO: 453)
NM_014496-1411, gatgcgctatggacaacat (SEQ ID NO: 454)
NM_014496-927, ggaatccagcaaatagatt (SEQ ID NO: 455)
(Target gene of RNAi)
NM_002953, Homo sapiens ribosomal protein S6 kinase, 90kDa, polypeptide 1 (RPS6KA1).
(Target sequences)
NM_002953-739, ctatggggtgttgatgttt (SEQ ID NO: 456)
NM_002953-1331, gctgtcaaggtcattgata (SEQ ID NO: 457)
NM_002953-1332, ctgtcaaggtcattgataa (SEQ ID NO: 458)
NM_002953-735, ggtcctatggggtgttgat (SEQ ID NO: 459)
NM_002953-738, cctatggggtgttgatgtt (SEQ ID NO: 460)
(Target sequences effective for mouse homolog)
NM_002953-666, gcgggacagtggagtacat (SEQ ID NO: 461)
NM_002953-832, gctaggcatgccccagttt (SEQ ID NO: 462)
NM_002953-1315, caccaacatggagtatgct (SEQ ID NO: 463)
(Target gene of RNAi)
NM_001626, Homo sapiens v-akt murine thymoma viral oncogene homolog 2 (AKT2).
(Target sequences)
NM_001626-141, ctctacccccttaaacaa (SEQ ID NO: 464)
NM_001626-35, cacaagcgtggtgaataca (SEQ ID NO: 465)

NM_001626-143, ctacccccctaaacaact (SEQ ID NO: 466)
NM_001626-41, cgtggtgaatacatcaaga (SEQ ID NO: 467)
NM_001626-420, gcaaggcacgggctaaagt (SEQ ID NO: 468)
(Target gene of RNAi)
NM_005163, Homo sapiens v-akt murine thymoma viral oncogene homolog 1 (AKT1).
(Target sequences)
NM_005163-1294, gactgacaccaggtatttt (SEQ ID NO: 469)
NM_005163-1296, ctgacaccaggtattttga (SEQ ID NO: 470)
NM_005163-1292, gagactgacaccaggtatt (SEQ ID NO: 471)
NM_005163-751, cttctatggcgctgagatt (SEQ ID NO: 472)
NM_005163-630, cagccctgaagtactcttt (SEQ ID NO: 473)
(Target gene of RNAi)
NM_005465, Homo sapiens v-akt murine thymoma viral oncogene homolog 3 (protein kinase B, gamma) (AKT3).
(Target sequences)
NM_005465-229, ccagtggactactgttata (SEQ ID NO: 474)
NM_005465-99, cattcataggatataaaga (SEQ ID NO: 475)
NM_005465-402, cctctacaacccatcataa (SEQ ID NO: 476)
NM_005465-1283, gagacagatactagatatt (SEQ ID NO: 477)
(Target sequences effective for mouse homolog)
NM_005465-733, ggaccgcacacgtttctat (SEQ ID NO: 478)
NM_005465-1317, cagctcagactattacaat (SEQ ID NO: 479)
NM_005465-1319, gctcagactattacaataa (SEQ ID NO: 480)
(Target gene of RNAi)
NM_005627, Homo sapiens serum/glucocorticoid regulated kinase (SGK).
(Target sequences)
NM_005627-875, ggcctgccgcctttttata (SEQ ID NO: 481)
NM_005627-97, gggtctgaacgactttatt (SEQ ID NO: 482)
NM_005627-99, gtctgaacgactttattca (SEQ ID NO: 483)
NM_005627-190, ggagcctgagcttatgaat (SEQ ID NO: 484)
NM_005627-413, gaggagaagcatattatgt (SEQ ID NO: 485)
(Target sequences effective for mouse homolog)
NM_005627-649, catcgtttatagagactta (SEQ ID NO: 486)
NM_005627-367, ctatgcagtcaaagtttta (SEQ ID NO: 487)
NM_005627-307, gatcggaaagggcagtttt (SEQ ID NO: 488)
(Target gene of RNAi)
NM_170693, Homo sapiens serum/glucocorticoid regulated kinase 2 (SGK2).
(Target sequences)
NM_170693-163, gtctgatggggcgttctat (SEQ ID NO: 489)
NM_170693-840, cagactttcttgagattaa (SEQ ID NO: 490)
NM_170693-842, gactttcttgagattaaga (SEQ ID NO: 491)
NM_170693-582, gtggtacccctgagtactt (SEQ ID NO: 492)
NM_170693-183, cagtgaaggtactacagaa (SEQ ID NO: 493)
(Target sequence effective for mouse homolog)
NM_170693-287, gtgggcctgcgctactcct (SEQ ID NO: 494)
(Target gene of RNAi)
NM_013257, Homo sapiens serum/glucocorticoid regulated kinase-like (SGKL).
(Target sequences)
NM_013257-273, caggactaaacgaattcat (SEQ ID NO: 495)
NM_013257-944, gacaccactaccacatttt (SEQ ID NO: 496)
NM_013257-1388, gtatcttctgactattcta (SEQ ID NO: 497)
NM_013257-946, caccactaccacattttgt (SEQ ID NO: 498)
NM_013257-790, gttttacgctgctgaaatt (SEQ ID NO: 499)
(Target sequences effective for mouse homolog)
NM_013257-693, caactgaaaagctttattt (SEQ ID NO: 500)
NM_013257-225, gaatatttggtgataattt (SEQ ID NO: 501)
NM_013257-38, ccaagtgtaagcattccca (SEQ ID NO: 502)
(Target gene of RNAi)
NM_002744, Homo sapiens protein kinase C, zeta (PRKCZ).

(Target sequences)
NM_002744-1233, gcggaaccccgaattacat (SEQ ID NO: 503)
NM_002744-398, caagccaagcgctttaaca (SEQ ID NO: 504)
NM_002744-1447, caaagcctcccatgtttta (SEQ ID NO: 505)
NM_002744-823, ccaaatttacgccatgaaa (SEQ ID NO: 506)
NM_002744-1100, cacgagagggggatcatct (SEQ ID NO: 507)
(Target gene of RNAi)
NM_006254, Homo sapiens protein kinase C, delta (PRKCD).
(Target sequences)
NM_006254-1524, gcggcacccctgactatat (SEQ ID NO: 508)
NM_006254-1339, ctaccgtgccacgttttat (SEQ ID NO: 509)
NM_006254-992, gggacctacggcaagatct (SEQ ID NO: 510)
(Target sequences effective for mouse homolog)
NM_006254-172, gttcgacgcccacatctat (SEQ ID NO: 511)
NM_006254-659, cagaaagaacgcttcaaca (SEQ ID NO: 512)
NM_006254-761,gtgaagcagggattaaagt (SEQ ID NO: 513)
(Target gene of RNAi)
NM_002737, Homo sapiens protein kinase C, alpha (PRKCA).
(Target sequences)
NM_002737-1571, ggcgtcctgttgtatgaaa (SEQ ID NO: 514)
NM_002737-393, gtgacacctgcgatatgaa (SEQ ID NO: 515)
NM_002737-711, gacgactgtctgtagaaat (SEQ ID NO: 516)
NM_002737-1085, gaactgtatgcaatcaaaa (SEQ ID NO: 517)
NM_002737-1924, gctggttattgctaacata (SEQ ID NO: 518)
(Target sequences effective for mouse homolog)
NM_002737-1958, gaagggttctcgtatgtca (SEQ ID NO: 519)
NM_002737-1835, ccattcaagcccaaagtgt (SEQ ID NO: 520)
NM_002737-1234, gctgtacttcgtcatggaa (SEQ ID NO: 521)
(Target gene of RNAi)
NM_002738, Homo sapiens protein kinase C, beta 1 (PRKCB1).
(Target sequences)
NM_002738-573, cagatccctacgtaaaact (SEQ ID NO: 522)
NM_002738-1791, catttttccggtatattga (SEQ ID NO: 523)
NM_002738-1384, catttaccgtgacctaaaa (SEQ ID NO: 524)
NM_002738-575, gatccctacgtaaaactga (SEQ ID NO: 525)
NM_002738-1315, ggagccccatgctgtattt (SEQ ID NO: 526)
(Target sequences effective for mouse homolog)
NM_002738-1006, gatgaaactgaccgatttt (SEQ ID NO: 527)
NM_002738-1961, gaattcgaaggattttcct (SEQ ID NO: 528)
NM_002738-1233, ccatggaccgcctgtactt (SEQ ID NO: 529)
(Target gene of RNAi)
NM_015282, Homo sapiens cytoplasmic linker associated protein 1 (CLASP1).
(Target sequences)
NM_015282-2447, gagccgtatgggatgtatt (SEQ ID NO: 530)
NM_015282-4151, gccgagctgacgattatga (SEQ ID NO: 531)
NM_015282-4152, ccgagctgacgattatgaa (SEQ ID NO: 532)
NM_015282-1786, gcgatctcgaagtgatatt (SEQ ID NO: 533)
NM_015282-635, cagtcccggttgaatgtaa (SEQ ID NO: 534)
(Target gene of RNAi)
NM-006287, Homo sapiens tissue factor pathway inhibitor (lipoprotein-associated coagulation inhibitor) (TFPI).
(Target sequences)
NM_006287-225, ctcgacagtgcgaagaatt (SEQ ID NO: 535)
NM_006287-227, cgacagtgcgaagaattta (SEQ ID NO: 536)
NM_006287-228, gacagtgcgaagaatttat (SEQ ID NO: 537)
NM_006287-230, cagtgcgaagaatttatat (SEQ ID NO: 538)
NM_006287-393, gaatatgtcgaggttatat (SEQ ID NO: 539)
(Target gene of RNAi)
NM_004073, Homo sapiens cytokine-inducible kinase (CNK).

(Target sequences)
NM_004073-1283, gttgactactccaataagt (SEQ ID NO: 540)
NM_004073-138, gcgcctacgctgtcaaagt (SEQ ID NO: 541)
NM_004073-239, cgccacatcgtgcgttttt (SEQ ID NO: 542)
NM_004073-1281, gggttgactactccaataa (SEQ ID NO: 543)
(Target sequences effective for mouse homolog)
NM_004073-192, gcgagaagatcctaaatga (SEQ ID NO: 544)
NM_004073-183, cgcatcagcgcgagaagat (SEQ ID NO: 545)
NM_004073-190, gcgcgagaagatcctaaat (SEQ ID NO: 546)
(Target gene of RNAi)
NM_003384, Homo sapiens vaccinia related kinase 1 (VRK1).
(Target sequences)
NM_003384-776, ccttgggaggataatttga (SEQ ID NO: 547)
NM_003384-773, cttccttgggaggataatt (SEQ ID NO: 548)
NM_003384-195, caccttgtgttgtaaaagt (SEQ ID NO: 549)
NM_003384-777, cttgggaggataatttgaa (SEQ ID NO: 550)
(Target sequences effective for mouse homolog)
NM_003384-372, gttacaggtttatgataat (SEQ ID NO: 551)
NM_003384-463, gcagctaagcttaagaatt (SEQ ID NO: 552)
NM_003384-977, ggactaaaagctataggaa (SEQ ID NO: 553)
(Target gene of RNAi)
NM_006296, Homo sapiens vaccinia related kinase 2 (VRK2).
(Target sequences)
NM_006296-366, gactaggaatagatttaca (SEQ ID NO: 554)
NM_006296-165, caagacatgtagtaaaagt (SEQ ID NO: 555)
NM_006296-874, ggtatgtgctcatagttta (SEQ ID NO: 556)
NM_006296-541, ggtttatcttgcagattat (SEQ ID NO: 557)
NM_006296-113, ggatttggattgatatatt (SEQ ID NO: 558)
(Target sequences effective for mouse homolog)
NM_006296-560, ggactttcctacagatatt (SEQ ID NO: 559)
NM_006296-626, cataatgggacaatagagt (SEQ ID NO: 560)
NM_006296-568, ctacagatattgtcccaat (SEQ ID NO: 561)
(Target gene of RNAi)
NM_004672, Homo sapiens mitogen-activated protein kinase kinase kinase 6 (MAP3K6).
(Target sequences)
NM_004672-2221, ctttctcctccgaactttt (SEQ ID NO: 562)
NM_004672-1489, gatgttggagtttgattat (SEQ ID NO: 563)
NM_004672-814, caaagagctccggctaata (SEQ ID NO: 564)
NM_004672-51, ccctgcgggaggatgtttt (SEQ ID NO: 565)
NM_004672-503, gccgagcagcataatgtct (SEQ ID NO: 566)
(Target sequences effective for mouse homolog)
NM_004672-442, ggactactcggccatcatt (SEQ ID NO: 567)
NM_004672-277, ctatttccgggagaccatt (SEQ ID NO: 568)
NM_004672-1929, ggctgctcaagatttctga (SEQ ID NO: 569)
(Target gene of RNAi)
NM_005923, Homo sapiens mitogen-activated protein kinase kinase kinase 5 (MAP3K5).
(Target sequences)
NM_005923-3294, gatccactgaccgaaaaat (SEQ ID NO: 570)
NM_005923-838, caggaaagctcgtaattta (SEQ ID NO: 571)
NM_005923-840, ggaaagctcgtaatttata (SEQ ID NO: 572)
NM_005923-1525, gtacctcaagtctattgta (SEQ ID NO: 573)
NM_005923-2517, ctggtaccctccagtatat (SEQ ID NO: 574)
(Target gene of RNAi)
NM_020998, Homo sapiens macrophage stimulating 1 (hepatocyte growth factor-like) (MST1).
(Target sequences)
NM_020998-943, ccgatttacgccagaaaaa (SEQ ID NO: 575)
NM_020998-944, cgatttacgccagaaaaat (SEQ ID NO: 576)
NM_020998-945, gatttacgccagaaaaata (SEQ ID NO: 577)

NM_020998-698, ggtctggacgacaactatt (SEQ ID NO: 578)
NM_020998-1827, ccaaaggtacgggtaatga (SEQ ID NO: 579)
(Target gene of RNAi)
NM_003576, Homo sapiens serine/threonine kinase 24 (STE20 homolog, yeast) (STK24).
(Target sequences)
NM_003576-348, gctccgcactagatctatt (SEQ ID NO: 580)
NM_003576-349, ctccgcactagatctatta (SEQ ID NO: 581)
NM_003576-351, ccgcactagatctattaga (SEQ ID NO: 582)
NM_003576-352, cgcactagatctattagaa (SEQ ID NO: 583)
NM_003576-437, ctccattcggagaagaaaa (SEQ ID NO: 584)
(Target sequence effective for mouse homolog)
NM_003576-148, gttcaaaggcattgacaat (SEQ ID NO: 585)
(Target gene of RNAi)
NM_016542, Homo sapiens Mst3 and SOK1-related kinase (MST4).
(Target sequences)
NM_016542-857, ctgatagatcgttttaaga (SEQ ID NO: 586)
NM_016542-139, gcaagtcgttgctattaaa (SEQ ID NO: 587)
NM_016542-1133, gaagaactcgagaaaagta (SEQ ID NO: 588)
NM_016542-556, ggctcctgaagttattcaa (SEQ ID NO: 589)
(Target sequences effective for mouse homolog)
NM_016542-613, gggaattactgctattgaa (SEQ ID NO: 590)
NM_016542-669, caatgagagttctgtttct (SEQ ID NO: 591)
NM_016542-1063, gataatcacacctgcattt (SEQ ID NO: 592)
(Target gene of RNAi)
NM_002576, Homo sapiens p21/Cdc42/Racl-activated kinase 1 (STE20 homolog, yeast) (PAK1).
(Target sequences)
NM_002576-38, gcccctccgatgagaaata (SEQ ID NO: 593)
NM_002576-788, ggcgatcctaagaagaaat (SEQ ID NO: 594)
NM_002576-3, caaataacggcctagacat (SEQ ID NO: 595)
NM_002576-154, ccgattttaccgatccatt (SEQ ID NO: 596)
(Target sequences effective for mouse homolog)
NM_002576-1020, gggttgttatggaatactt (SEQ ID NO: 597)
NM_002576-1165, catcaagagtgacaatatt (SEQ ID NO: 598)
NM_002576-1015, gctgtgggttgttatggaa (SEQ ID NO: 599)
(Target gene of RNAi)
NM_002577, Homo sapiens p21 (CDKN1A)-activated kinase 2 (PAK2).
(Target sequences)
NM_002577-721, cataggtgaccctaagaaa (SEQ ID NO: 600)
NM_002577-908, cccaacatcgttaactttt (SEQ ID NO: 601)
NM_002577-909, ccaacatcgttaacttttt (SEQ ID NO: 602)
NM_002577-557, ccggatcatacgaaatcaa (SEQ ID NO: 603)
NM_002577-558, cggatcatacgaaatcaat (SEQ ID NO: 604)
(Target gene of RNAi)
NM_002578, Homo sapiens p21 (CDKN1A)-activated kinase 3 (PAK3).
(Target sequences)
NM_002578-458, catccttcgagtacaaaaa (SEQ ID NO: 605)
NM_002578-1467, ctgtattccgtgacttttt (SEQ ID NO: 606)
NM_002578-1469, gtattccgtgactttttaa (SEQ ID NO: 607)
NM_002578-706, cacagatcggcaaagaaaa (SEQ ID NO: 608)
NM_002578-3, ctgacggtctggataatga (SEQ ID NO: 609)
(Target sequences effective for mouse homolog)
NM_002578-1376, ccccttaccttaatgaaa (SEQ ID NO: 610)
NM_002578-219, cagactttgagcatacgat (SEQ ID NO: 611)
NM_002578-254, gcagtcaccggggaattca (SEQ ID NO: 612)
(Target gene of RNAi)
NM_005884, Homo sapiens p21(CDKN1A)-activated kinase 4 (PAK4).
(Target sequences)
NM_005884-1502, gggataatggtgattgaga (SEQ ID NO: 613)

NM_005884-1503, ggataatggtgattgagat (SEQ ID NO: 614)
NM_005884-883, gccacagcgagtatcccat (SEQ ID NO: 615)
NM_005884-77, cagcacgagcagaagttca (SEQ ID NO: 616)
NM_005884-1494, ggtcgctggggataatggt (SEQ ID NO: 617)
(Target gene of RNAi)
NM_002755, Homo sapiens mitogen-activated protein kinase kinase 1 (MAP2K1).
(Target sequences)
NM_002755-280, ggccagaaagctaattcat (SEQ ID NO: 618)
NM_002755-402, gcgatggcgagatcagtat (SEQ ID NO: 619)
NM_002755-404, gatggcgagatcagtatct (SEQ ID NO: 620)
NM_002755-682, ctacatgtcgccagaaaga (SEQ ID NO: 621)
NM_002755-1128, ccaccatcggccttaacca (SEQ ID NO: 622)
(Target sequences effective for mouse homolog)
NM_002755-912, gacctcccatggcaatttt (SEQ ID NO: 623)
NM_002755-915, ctcccatggcaatttttga (SEQ ID NO: 624)
NM_002755-911, cgacctcccatggcaattt (SEQ ID NO: 625)
(Target gene of RNAi)
NM_030662, Homo sapiens mitogen-activated protein kinase kinase 2 (MAP2K2).
(Target sequences)
NM_030662-1136, gccggctggttgtgtaaaa (SEQ ID NO: 626)
NM_030662-184, caaggtcggcgaactcaaa (SEQ ID NO: 627)
NM_030662-959, ctcctggactatattgtga (SEQ ID NO: 628)
NM_030662-183, ccaaggtcggcgaactcaa (SEQ ID NO: 629)
NM_030662-711, ggttgcagggcacacatta (SEQ ID NO: 630)
(Target gene of RNAi)
NM_002756, Homo sapiens mitogen-activated protein kinase kinase 3 (MAP2K3).
(Target sequences)
NM_002756-257, cgcacggtcgactgtttct (SEQ ID NO: 631)
NM_002756-258, gcacggtcgactgtttcta (SEQ ID NO: 632)
NM_002756-289, ctacggggcactattcaga (SEQ ID NO: 633)
NM_002756-285, ccttctacggggcactatt (SEQ ID NO: 634)
NM_002756-44, gactcccggaccttcatca (SEQ ID NO: 635)
(Target sequences effective for mouse homolog)
NM_002756-129, gagcctatggggtggtaga (SEQ ID NO: 636)
NM_002756-41, ctggactcccggaccttca (SEQ ID NO: 637)
NM_002756-89, gaggctgatgacttggtga (SEQ ID NO: 638)
(Target gene of RNAi)
NM_002758, Homo sapiens mitogen-activated protein kinase kinase 6 (MAP2K6).
(Target sequences)
NM_002758-394, ggatacatcactagataaa (SEQ ID NO: 639)
NM_002758-395, gatacatcactagataaat (SEQ ID NO: 640)
NM_002758-755, cttcgatttccctatgatt (SEQ ID NO: 641)
NM_002758-340, cttttatggcgcactgttt (SEQ ID NO: 642)
NM_002758-399, catcactagataaattcta (SEQ ID NO: 643)
(Target sequences effective for mouse homolog)
NM_002758-312, ggacggtggactgtccatt (SEQ ID NO: 644)
NM_002758-418, caaacaagttattgataaa (SEQ ID NO: 645)
NM_002758-415, ctacaaacaagttattgat (SEQ ID NO: 646)
(Target gene of RNAi)
NM_003010, Homo sapiens mitogen-activated protein kinase kinase 4 (MAP2K4).
(Target sequences)
NM_003010-543, ctacctcgtttgataagtt (SEQ ID NO: 647)
NM_003010-1130, gcatgctatgtttgtaaaa (SEQ ID NO: 648)
NM_003010-1056, ccaaaaggccaaagtataa (SEQ ID NO: 649)
(Target sequences effective for mouse homolog)
NM_003010-1129, cgcatgctatgtttgtaaa (SEQ ID NO: 650)
NM_003010-1057, caaaaggccaaagtataaa (SEQ ID NO: 651)
NM_003010-452, gtaatgcggagtagtgatt (SEQ ID NO: 652)

(Target gene of RNAi)
NM_016123, Homo sapiens interleukin-1 receptor-associated kinase 4 (IRAK4).
(Target sequences)
NM_016123-1299, gccaatgtcggcatgaaaa (SEQ ID NO: 653)
NM_016123-1073, gctttgcgtggagaaataa (SEQ ID NO: 654)
NM_016123-38, ctcaatgttggactaatta (SEQ ID NO: 655)
NM_016123-769, cctctgcttagtatatgtt (SEQ ID NO: 656)
NM_016123-1180, gttattgctagatattaaa (SEQ ID NO: 657)
(Target gene of RNAi)
NM_002880, Homo sapiens v-raf-1 murine leukemia viral oncogene homolog 1 (RAF1).
(Target sequences)
NM_002880-1703, gatcttagtaagctatata (SEQ ID NO: 658)
NM_002880-232, gcatgactgccttatgaaa (SEQ ID NO: 659)
NM_002880-1597, ctatggcatcgtattgtat (SEQ ID NO: 660)
NM_002880-1706, cttagtaagctatataaga (SEQ ID NO: 661)
NM_002880-568, cagacaactcttattgttt (SEQ ID NO: 662)
(Target gene of RNAi)
NM_000020, Homo sapiens activin A receptor type II-like 1 (ACVRL1).
(Target sequences)
NM_000020-1453, caagaagacactacaaaaa (SEQ ID NO: 663)
NM_000020-722, gagactgagatatataaca (SEQ ID NO: 664)
NM_000020-1456, gaagacactacaaaaaatt (SEQ ID NO: 665)
NM_000020-728, gagatctataacacagtat (SEQ ID NO: 666)
NM_000020-846, gctccctctacgactttct (SEQ ID NO: 667)
(Target gene of RNAi)
NM_001105, Homo sapiens activin A receptor, type I (ACVR1).
(Target sequences)
NM_001105-1456, cacagcactgcgtatcaaa (SEQ ID NO: 668)
NM_001105-428, gttgctctccgaaaattta (SEQ ID NO: 669)
NM_001105-431, gctctccgaaaatttaaaa (SEQ ID NO: 670)
NM_001105-1460, gcactgcgtatcaaaaaga (SEQ ID NO: 671)
NM_001105-1458, cagcactgcgtatcaaaaa (SEQ ID NO: 672)
(Target sequences effective for mouse homolog)
NM_001105-1306, caatgacccaagttttgaa (SEQ ID NO: 673)
NM_001105-1381, gttctcagacccgacatta (SEQ ID NO: 674)
NM_001105-281, caaggggactggtgtaaca (SEQ ID NO: 675)
(Target gene of RNAi)
NM_004302, Homo sapiens activin A receptor, type IB (ACVR1B).
(Target sequences)
NM_004302-609, cccgaaccatcgttttaca (SEQ ID NO: 676)
NM_004302-610, ccgaaccatcgttttacaa (SEQ ID NO: 677)
NM_004302-897 ,caattgaggggatgattaa (SEQ ID NO: 678)
NM_004302-857, cacgggtccctgtttgatt (SEQ ID NO: 679)
NM_004302-859, cgggtccctgtttgattat (SEQ ID NO: 680)
(Target sequences effective for mouse homolog)
NM_004302-1119, gggtggggaccaaacgata (SEQ ID NO: 681)
NM_004302-1063, cctggctgtccgtcatgat (SEQ ID NO: 682)
NM_004302-1121, gtggggaccaaacgataca (SEQ ID NO: 683)
(Target gene of RNAi)
NM_145259, Homo sapiens activin A receptor, type IC (ACVRIC).
(Target sequences)
NM_145259-1419, ctgctcttcgtattaagaa (SEQ ID NO: 684)
NM_145259-956, gctcatcgagacataaaat (SEQ ID NO: 685)
NM_145259-825, gctccttatatgactattt (SEQ ID NO: 686)
NM_145259-959, catcgagacataaaatcaa (SEQ ID NO: 687)
NM_145259-1237, gtaccaattgccttattat (SEQ ID NO: 688)
(Target gene of RNAi)
NM_004612, Homo sapiens transforming growth factor, beta receptor I (activin A receptor type II-like kinase, 53kDa) (TGFBR1).
(Target sequences)
NM_004612-236, cgagataggccgtttgtat (SEQ ID NO: 689)
NM_004612-1451, gcattgcggattaagaaaa (SEQ ID NO: 690) NM_004612-463, ccatcgagtgccaaatgaa (SEQ ID NO: 691)
NM_004612-492, cattagatcgcccttttat (SEQ ID NO: 692)
NM_004612-1449, cagcattgcggattaagaa (SEQ ID NO: 693)
(Target sequences effective for mouse homolog)
NM_004632-829,gttggtgtcagattatcat (SEQ ID NO: 694)
NM_004612-288, caacatattgctgcaatca (SEQ ID NO: 695)
NM_004612-839, gattatcatgagcatggat (SEQ ID NO: 696)
(Target gene of RNAi)
NM_004836, Homo sapiens eukaryotic translation initiation factor 2-alpha kinase 3 (EIF2AK3).
(Target sequences)
NM_004836-1594, catagcaacaacgtttatt (SEQ ID NO: 697)
NM_004836-1419, catatgataatggttatta (SEQ ID NO: 698)
NM_004836-1900, ggtaatgcgagaagttaaa (SEQ ID NO: 699)
NM_004836-1248, ctaatgaaaacgcaattat (SEQ ID NO: 700)
(Target sequences effective for mouse homolog)
NM_004836-784, ctttgaacttcggtatatt (SEQ ID NO: 701)
NM_004836-782, cactttgaacttcggtata (SEQ ID NO: 702)
NM_004836-983, gaatgggagtaccagtttt (SEQ ID NO: 703)
(Target gene of RNAi)
NM_001433, Homo sapiens ER to nucleus signalling 1 (ERN1).
(Target sequences)
NM_ODi433-24D7, cattgcacgagaattgata (SEQ ID NO: 704)
NM_001433-2277, caggctgcgtcttttacta (SEQ ID NO: 705)
NM_001433-2530, cgtgagcgacagaatagaa (SEQ ID NO: 706)
NM_001433-1149, ccaaacatcgggaaaatgt (SEQ ID NO: 707)
NM_001433-364, ggacatctggtatgttatt (SEQ ID NO: 708)
(Target sequences effective for mouse homolog)
NM_001433-319, cccatgccgaagttcagat (SEQ ID NO: 709)
NM_001433-2254, ctacacggtggacatcttt (SEQ ID NO: 710)
NM_001433-324, gccgaagttcagatggaat (SEQ ID NO: 711)
(Target gene of RNAi)
NM_001278, Homo sapiens conserved helix-loop-helix ubiquitous kinase (CHUK).
(Target sequences)
NM_fl01278-746, ggagaagttcggtttagta (SEQ ID NO: 712)
NM_001278-1879, ggccctcagtaatatcaaa (SEQ ID NO: 713)
NM_001278-864, gacctgttgaccttacttt (SEQ ID NO: 714)
NM_001278-2150, ggccatttaagcactatta (SEQ ID NO: 715)
NM_001278-2151, gccatttaagcactattat (SEQ ID NO: 716)
(Target sequences effective for mouse homolog)
NK_001278-645, ctggatataggoctttttt (SEQ ID NO: 717)
NM_001278-1354, gttaagtcttcttagatat (SEQ ID NO: 718)
NM_001278-1203, gtttatctgattgtgtaaa (SEQ ID NO: 719)
(Target gene of RNAi)
NM_014002, Homo sapiens inhibitor of kappa light polypeptide gene enhancer in B-cells, kinase epsilon (IKBKE).
(Target sequences)
NM_014002-2107, catcgaacggctaaataga (SEQ ID NO: 720)
NM_014002-1724, ctggataaggtgaatttca (SEQ ID NO: 721)
NM_014002-535, cctgcatcccgacatgtat (SEQ ID NO: 722)
NM_014002-1220, ctgcaggcggattacaaca (SEQ ID NO: 723)
NM_014002-1726, ggataaggtgaatttcagt (SEQ ID NO: 724)
(Target sequence effective for mouse homolog)
NM_014002-54, ccactgccagtgtgtacaa (SEQ ID NO: 725)
(Target gene of RNAi)
NM_003177, Homo sapiens spleen tyrosine kinase (SYK).
(Target sequences)

NM_003177-1222, caatgaccccgctcttaaa (SEQ ID NO: 726)
NM_003177-713, cagctagtcgagcattatt (SEQ ID NO: 727)
NM_003177-849, ggtcagcgggtggaataat (SEQ ID NO: 728)
NM_003177-715, gctagtcgagcattattct (SEQ ID NO: 729)
NM_003177-1389, gacatgtcaaggataagaa (SEQ ID NO: 730)
(Target sequences effective for mouse homolog)
NM_003177-1559, gctgatgaaaactactaca (SEQ ID NO: 731)
NM_003177-1028, gacacagaggtgtacgaga (SEQ ID NO: 732)
NM_003177-1560, ctgatgaaaactactacaa (SEQ ID NO: 733)
(Target gene of RNAi)
NM_153831, Homo sapiens PTK2 protein tyrosine kinase 2 (PTK2).
(Target sequences)
NM_153831-451, gaagagcgattatatgtta (SEQ ID NO: 734)
NM_153831-1889, gtaatcggtcgaattgaaa (SEQ ID NO: 735)
NM_153831-93, caatggagcgagtattaaa (SEQ ID NO: 736)
NM_153831-2747, ctggaccggtcgaatgata (SEQ ID NO: 737)
NM_153831-92, gqaatggagcgagtattaa (SEQ ID NO: 738)
(Target sequences effective for mouse homolog)
NM.153831-1767, ctccagagtcaatcaattt (SEQ ID NO: 739)
NK_153831-1766, gctccagagtcaatcaatt (SEQ ID NO: 740)
NM_153831-599, gttggtttaaagcgatttt (SEQ ID NO: 741)
(Target gene of RNAi)
NM_173174, Homo sapiens PTK2B protein tyrosine kinase 2 beta (PTK2B).
(Target sequences)
NM_173174-1273, ggtcctgaatcgtattctt (SEQ ID NO: 742)
NM_173174-1776, ccccagagtccattaactt (SEQ ID NO: 743)
NM_173174-1723, ggacgaggactattacaaa (SEQ ID NO: 744)
NM_173174-2486, gaccccatggtttatatga (SEQ ID NO: 745)
(Target sequences effective for mouse homolog)
NM_173174-378, ggaggtatgaccttcaaat (SEQ ID NO: 746)
NM_173174-1182, gcagcatagagtcagaeat (SEQ ID NO: 747)
NM_173174-376, gtggaggtatgaccttcaa (SEQ ID NO: 748)
(Target gene of RNAi)
NM_002944, Homo sapiens v-ros UR2 sarcoma virus oncogene homolog 1 (avian) (ROS1).
(Target sequences)
NM_002944-417, gaagctggacttatactaa (SEQ ID NO: 749)
NM_002944-2123, gacatggattggtataaca (SEQ ID NO: 750)
NM_002944-2163, cgaaaggcgacgtttttgt (SEQ ID NO: 751)
NM_002944-1385, caagccaagcgaatcattt (SEQ ID NO: 752)
NM_002944-416, ggaagctggacttatacta (SEQ ID NO: 753)
(Target sequences effective for mouse homolog)
NM_002944-3048, ctgtcactccttataccta (SEQ ID NO: 754)
NM_002944-3044, ctttctgtcactccttata (SEQ ID NO: 755)
NM_002944-1051, caacatgtctgatgtatct (SEQ ID NO: 756)
(Target gene of RNAi)
NM_004304, Homo sapiens anaplastic lymphoma kinase (Ki-1) (ALK).
(Target sequences)
NM_004304-2469, ccacctacgtatttaagat (SEQ ID NO: 757)
NM_004304-4067, cctgtataccggataatga (SEQ ID NO: 758)
NM_004304-2468, gccacctacgtatttaaga (SEQ ID NO: 759)
NM_004304-4183, cgctttgccgatagaatat (SEQ ID NO: 760)
NM_004304-2922, gccacggggaagtgaatat (SEQ ID NO: 761)
(Target sequences effective for mouse homolog)
NM_004304-3258, ccatcatgaccgactacaa (SEQ ID NO: 762)
NM_004304-2833, caatgaccccgaaatggat (SEQ ID NO: 763)
NM_004304-3156, ccggcatcatgattgtgta (SEQ ID NO: 764)
(Target gene of RNAi)
NM_000245, Homo sapiens met proto-oncogene (hepatocyte growth factor receptor) (MET).

(Target sequences)
NM_000245-2761, gaacagcgagctaaatata (SEQ ID NO: 765)
NM_000245-1271, cagcgcgttgacttattca (SEQ ID NO: 766)
NM_000245-1086, gtgcattccctatcaaata (SEQ ID NO: 767)
NM_000245-725, gattcttaccccattaagt (SEQ ID NO: 768)
NM_000245-3619, caaagcgatgaaatatctt (SEQ ID NO: 769)
(Target sequences effective for mouse homolog)
NM_000245-2987, catttggataggcttgtaa (SEQ ID NO: 770)
NM_000245-801, ctctagatgctcagacttt (SEQ ID NO: 771)
NM_000245-2660, gttaaaggtgaagtgttaa (SEQ ID NO: 772)
(Target gene of RNAi)
NM_002529, Homo sapiens neurotrophic tyrosine kinase, receptor, type 1 (NTRK1).
(Target sequences)
NM_002529-2091, gcatcctgtaccgtaagtt (SEQ ID NO: 773)
NM_002529-345, ggctcagtcgcctgaatct (SEQ ID NO: 774)
NM_002529-347, ctcagtcgcctgaatctct (SEQ ID NO: 775)
NM_002529-953, ggctccgtgctcaatgaga (SEQ ID NO: 776)
NM_002529-1987, ggtcaagattggtgatttt (SEQ ID NO: 777)
(Target gene of RNAi)
NM_006180, Homo sapiens neurotrophic tyrosine kinase, receptor, type 2 (NTRK2).
(Target sequences)
NM_006180-358, caattttacccgaaacaaa (SEQ ID NO: 778)
NM_006180-1642, catcaagcgacataacatt (SEQ ID NO: 779)
NM_006180-663, gtgatccggttcctaatat (SEQ ID NO: 780)
NM_006180-665, gatccggttcctaatatgt (SEQ ID NO: 781)
NM_006180-792, cttgtgtggcggaaaatct (SEQ ID NO: 782)
(Target sequences effective for mouse homolog)
NM_006180-562, cctgcagatacccaattgt (SEQ ID NO: 783)
NM_006180-898, ctggtgcattccattcact (SEQ ID NO: 784) NM_006180-735,cacagggctccttaaggat (SEQ ID NO: 785)
(Target gene of RNAi)
NM_000208, Homo sapiens insulin receptor (INSR).
(Target sequences)
NM_000208-2562, gccctgtgacgcatgaaat (SEQ ID NO: 786)
NM_000208-2565, ctgtgacgcatgaaatctt (SEQ ID NO: 787)
NM_000208-3492, gcatggtcgcccatgattt (SEQ ID NO: 788)
NM_000208-3493, catggtcgcccatgatttt (SEQ ID NO: 789)
NM_000208-329, ggatcacgactgttcttta (SEQ ID NO: 790)
(Target sequences effective for mouse homolog)
NM_000208-2911, gattggaagtatttatcta (SEQ ID NO: 791)
NM_000208-902, caccaatacgtcattcaca (SEQ ID NO: 792)
NM_000208-1514, cggacatcttttgacaaga (SEQ ID NO: 793)
(Target gene of RNAi)
NM_000323, Homo sapiens ret proto-oncogene (multiple endocrine neoplasia and medullary thyroid carcinoma 1, Hirschsprung disease) (RET).
(Target sequences)
NM_000323-2679, gcttgtcccgagatgttta (SEQ ID NO: 794)
NM_000323-3066, catctgactccctgattta (SEQ ID NO: 795)
NM_000323-3069, ctgactccctgatttatga (SEQ ID NO: 796)
NM_000323-2680, cttgtcccgagatgtttat (SEQ ID NO: 797)
NM_000323-2728, gggtcggattccagttaaa (SEQ ID NO: 798)
(Target sequences effective for mouse homolog)
NM_000323-3159, ccacatggattgaaaacaa (SEQ ID NO: 799)
NM_000323-3156, cttccacatggattgaaaa (SEQ ID NO: 800)
NM_000323-3155, ccttccacatggattgaaa (SEQ ID NO: 801)
(Target gene of RNAi)
NM_006293, Homo sapiens TYR03 protein tyrosine kinase (TYRO3).
(Target sequences)
NM_006293-1494, gcatcagcgatgaactaaa (SEQ ID NO: 802)

NM_006293-2207, gaaaacgctgagatttaca (SEQ ID NO: 803)
NM_006293-2394, gccaggaccccttatacat (SEQ ID NO: 804)
NM_006293-2399, gacccettatacatcaaca (SEQ ID NO: 805)
NM_006293-1493, ggcatcagcgatgaactaa (SEQ ID NO: 806)
(Target gene of RNAi)
NM_182925, Homo sapiens fms-related tyrosine kinase 4 (FLT4).
(Target sequences)
NM_182925-758,gtgtgggctgagtttaact (SEQ ID NO: 807)
NM_182925-756,ccgtgtgggctgagtttaa (SEQ ID NO: 808)
NM_182925-1217,ggcctgaggcgcaacatca (SEQ ID NO: 809)
NM_182925-1827.gcaagaacgtgcatctgtt (SEQ ID NO: 810)
NM_182925-908,gacctgggctcgtatgtgt (SEQ ID NO: 811)
(Target sequences effective for mouse homolog)
NM_182925-2033,cggctcacgcagaacttga (SEQ ID NO: 812)
NM_182925-330, gctactacaagtacatcaa (SEQ ID NO: 813)
(Target gene of RNAi)
NM_004119, Homo sapiens fms-related tyrosine kinase 3 (FLT3).
(Target sequences)
NM_004119-1569, gtgagacgatccttttaaa (SEQ ID NO: 814)
NM_004119-2490, gattggctcgagatatcat (SEQ ID NO: 815)
NM_004119-1571, gagacgatccttttaaact (SEQ ID NO: 816)
NM_004119-32, ccgctgctcgttgtttttt (SEQ ID NO: 817)
NM_004119-730, gttcacaatagatctaaat (SEQ ID NO: 818)
(Target sequences effective for mouse homolog)
NM_004119-92, gtgatcaagtgtgttttaa (SEQ ID NO: 819)
NM_004119-1483, ggtgtcgagcagtactcta (SEQ ID NO: 820)
NM_004119-1456, ggctaacagaaaagtgttt (SEQ ID NO: 821)
(Target gene of RNAi)
NM_002253, Homo sapiens kinase insert domain receptor (a type III receptor tyrosine kinase) (KDR).
(Target sequences)
NM_002253-617, gaaagttaccagtctatta (SEQ ID NO: 822)
NM_002253-865, gagcaccttaactatagat (SEQ ID NO: 823)
NM_002253-2020, gaatcagacgacaagtatt (SEQ ID NO: 824)
NM_002253-815, gtaaaccgagacctaaaaa (SEQ ID NO: 825)
NM_002253-2586, ggacagtagcagtcaaaat (SEQ ID NO: 826)
(Target sequences effective for mouse homolog)
NM_002253-3032, gtggctaagggcatggagt (SEQ ID NO: 827)
NM_002253-3627, ccaaattccattatgacaa (SEQ ID NO: 828)
NM_002253-3626, cccaaattccattatgaca (SEQ ID NO: 829)
(Target gene of RNAi)
NM_002609, Homo sapiens platelet-derived growth factor receptor, beta polypeptide (PDGFRB).
(Target sequences)
NM_002609-961, ggtgggcacactacaattt (SEQ ID NO: 830)
NM_002609-2881, gttgggcgaaggttacaaa (SEQ ID NO: 831)
NM_002609-409, ctttctcacggaaataact (SEQ ID NO: 832)
NM_002609-278, gacacgggagaatactttt (SEQ ID NO: 833)
NM_002609-3048, gtgacaacgactatatcat (SEQ ID NO: 834)
(Target sequences effective for mouse homolog)
NM_002609-633, catccatcaacgtctctgt (SEQ ID NO: 835)
NM_002609-2784, cctccgacgagatctatga (SEQ ID NO: 836)
(Target gene of RNAi)
NM_005433, Homo sapiens v-yes-1 Yamaguchi sarcoma viral oncogene homolog 1 (YES1).
(Target sequences)
NM_005433-525, gaaatcaacgaggtatttt (SEQ ID NO: 837)
NM_005433-670, cacaaccagagcacaattt (SEQ ID NO: 838)
NM_005433-1333, gtatggtcggtttacaata (SEQ ID NO: 839)
NM_005433-1331, ctgtatggtcggtttacaa (SEQ ID NO: 840)
NM_005433-416, ggttatatcccgagcaatt (SEQ ID NO: 841)

(Target sequences effective for mouse homolog)
NM_005433-953, caagaagctcagataatga (SEQ ID NO: 842)
NM_005433-1, gggctgcattaaaagtaaa (SEQ ID NO: 843)
NM_005433-4, ctgcattaaaagtaaagaa (SEQ ID NO: 844)
(Target gene of RNAi)
NM_002005, Homo sapiens feline sarcoma oncogene (FES).
(Target sequences)
NM_002005-1696, gattggacgggggaacttt (SEQ ID NO: 845)
NM_002005-2181, cacctgaggcccttaacta (SEQ ID NO: 846)
NM_002005-1553, ggctttcctagcattcctt (SEQ ID NO: 847)
NM_002005-683, gaatacctggagattagca (SEQ ID NO: 848)
NM_002005-74, ctactggagggcatgagaa (SEQ ID NO: 849)
(Target gene of RNAi)
NM_000633, Homo sapiens B-cell CLL/lymphoma 2 (BCL2).
(Target sequences)
NM_000633-43, gatgaagtacatccattat (SEQ ID NO: 850)
NM_000633-41, gtgatgaagtacatccatt (SEQ ID NO: 851)
(Target sequences effective for mouse homolog)
NM_000633-452, gagttcggtggggtcatgt (SEQ ID NO: 852)
NM_000633-454, gttcggtggggtcatgtgt (SEQ ID NO: 853)
NM_000633-525, ggatgactgagtacctgaa (SEQ ID NO: 854)
(Target gene of RNAi)
MM_001167, Homo sapiens baculoviral IAP repeat-containing 4 (BIRC4).
(Target sequences)
NM_001167-302, gccacgcagtctacaaatt (SEQ ID NO: 855)
NM_001167-794, gaagcacggatctttactt (SEQ ID NO: 856)
NM_001167-485, gaagaagctagattaaagt (SEQ ID NO: 857)
NM_001167-402, cacatgcagactatctttt (SEQ ID NO: 858)
(Target sequences effective for mouse homolog)
NM_001167-71, gaagagtttaatagattaa (SEQ ID NO: 859)
NM_001167-68, gtagaagagtttaatagat (SEQ ID NO: 860)
NM_001167-1354, ctgtatggatagaaatatt (SEQ ID NO: 861)
(Target gene of RNAi)
NM_139317, Homo sapiens baculoviral IAP repeat-containing 7 (livin) (BIRC7).
(Target sequences)
NM_139317-458, ctgctccggtcaaaaggaa (SEQ ID NO: 862)
NM_139317-457, cctgctccggtcaaaagga (SEQ ID NO: 863)
NM_139317-743, gagaggacgtgcaaggtgt (SEQ ID NO: 864)
NM_139317-774, ccgtgtccatcgtctttgt (SEQ ID NO: 865)
NM_139317-417, cctggacggagcatgccaa (SEQ ID NO: 866)
(Target gene of RNAi)
NM_005036, Homo sapiens peroxisome proliferative activated receptor, alpha (PPARA).
(Target sequences)
NM_005036-922, gctaaaatacggagtttat (SEQ ID NO: 867)
NM_005036-1243, ccacccggacgatatcttt (SEQ ID NO: 868)
NM_005036-711, cttttgtcatacatgatat (SEQ ID NO: 869)
NM_005036-498, cacacaacgcgattcgttt (SEQ ID NO: 870)
NM_005036-988, gctggtagcgtatggaaat (SEQ ID NO: 871)
(Target gene of RNAi)
NM_138712, Homo sapiens peroxisome proliferative activated receptor, gamma (PPARG).
(Target sequences)
NM_138712-953, ggagtccacgagatcattt (SEQ ID NO: 872)
NM_138712-304, ctccctcatggcaattgaa (SEQ ID NO: 873)
NM_138712-954, gagtccacgagatcattta (SEQ ID NO: 874)
NM_138712-445, ctgtcggatccacaaaaaa (SEQ ID NO: 875)
NM_138712-409, cagattgaagcttatctat (SEQ ID NO: 876)
(Target sequences effective for mouse homolog)
NM_138712-239, gcatctccaccttattatt (SEQ ID NO: 877)

NM_138712-688, ggcgagggcgatcttgaca (SEQ ID NO: 878)
NM_138712-664, gtccttcccgctgaccaaa (SEQ ID NO: 879)
(Target gene of RNAi)
NM_004421, Homo sapiens dishevelled, dsh homolog 1 (Drosophila) (DVL1).
(Target sequences)
NM_004421-1173, ccgtcgtccgggtcatgca (SEQ ID NO: 880)
(Target gene of RNAi)
NM_004422, Homo sapiens dishevelled, dsh homolog 2 (Drosophila) (DVL2).
(Target sequences)
NM_004422-1253, gtccatacggacatggcat (SEQ ID NO: 881)
(Target gene of RNAi)
NM_004423, Homo sapiens dishevelled, dsh homolog 3 (Drosophila) (DVL3).
(Target sequences)
NM_004423-1197, gcctagacgacttccactt (SEQ ID NO: 882)
(Target gene of RNAi)
NC_001802, Human immunodeficiency virus 1, complete genome.
(Target sequences)
NC_001802-8242, ggacagatagggttataga (SEQ ID NO: 883)
NC_001802-340, gcgagagcgtcagtattaa (SEQ ID NO: 884)
NC_001802-1222, gtagaccggttctataaaa (SEQ ID NO: 885)
NC_001802-1818, cgacccctcgtcacaataa (SEQ ID NO: 886)
NC_001802-4973, gccctaggtgtgaatatca (SEQ ID NO: 887)
NC_001802-5224, gcttagggcaacatatcta (SEQ ID NO: 888)
NC_001802-550, gaagaacttagatcattat (SEQ ID NO: 889)
NC_001802-1777, gaactgtatcctttaactt (SEQ ID NO: 890)
NC_001802-3244, gaaagactcctaaatttaa (SEQ ID NO: 891)
NC_001802-5225, cttagggcaacatatctat (SEQ ID NO: 892)

[Advantages of the Invention]

According to the present invention, siRNA actually having an RNAi effect can be obtained with high probability. Thus, when preparing novel siRNA, it is possible to greatly reduce the effort required to carry out repeated tests of trial and error, based on the experiences of the researcher, in actually synthesizing siRNA and in confirming whether the synthesized product has an RNAi effect. Namely, the present invention is extremely preferred for carrying out a search or for creation of siRNA having a novel sequence. Furthermore, by using the present invention, a wide variety of desired siRNA can be obtained in a short time. Since necessity for actual preparation of siRNA in a trial-and-error manner has been reduced, it becomes possible to greatly reduce the cost required for testing and manufacturing techniques, in which RNA interference is used. Additionally, the present invention not only greatly simplifies all testing and manufacturing techniques, in which the RNAi effect is used, but also significantly improves their reliability as techniques. The present invention is particularly effective in performing RNA interference in higher animals such as mammals.

INDUSTRIAL APPLICABILITY

As described above, the present invention relates to RNA interference and more particularly, for example, to a method for designing sequences of polynucleotides for causing RNA interference, the method improving efficiency in testing, manufacturing, etc., in which RNA interference is used.

## Claims

1. A double stranded polynucleotide produced by a method comprising:

   i) producing a double-stranded polynucleotide of a sequence segment having 19 bases, conforming to the following rules (a) to (c), from the base sequence of a target gene for RNA interference;

      (a) The 3' end base of a sense strand is adenine, thymine or uracil,
      (b) The 5' end base of a sense strand is guanine or cytosine, and
      (c) In a 7-base sequence from the 3' end of a sense strand, at least five bases among the seven bases are one or more types of bases selected from the group consisting of adenine, thymine, and uracil,
      (d) wherein the searched target sequence has a following general formula.
      5' - S NNNNNNNNNNNNN XXXXXX W - 3'
      3' - S NNNNNNNNNNN XXXXXX W -5'
      S is G or C
      N is G, C, A, T or U
      at least three of X is A, T or U
      W is A, T or U

   ii) forming a sense strand by providing an overhanging portion of 2 bases to the 3' end thereof; and
   iii) forming an antisense strand by providing an overhanging portion of 2 bases to the 3' end thereof, wherein the number of bases in each strand is 21.

2. A double stranded polynucleotide of Claim 1, consisting of a base sequence selected the SEQ ID NOs: 15-29 and 35-892, and a base sequence complementary to the selected base sequence.

Fig.1

```
Human
sequence    GACACGGACGTCAACACCCTGACCCGCTTCGTCATGGAGGAGGGCAGGAAGGCCCGCG
Mouse
sequence    GAAACGGATATCAGCACCCTGACCCGCTTCGTCATGGAGCAGGGCAGGAAGGCTCAGG
            ** *****  *** ************************* ************* *  *
                                   ↓
                      5'-CCCUGACCCGCUUCGUCAUGG-3'
                    3'-GUGGGACUGGGCGAAGCAGUA-5'
```

Fig.2

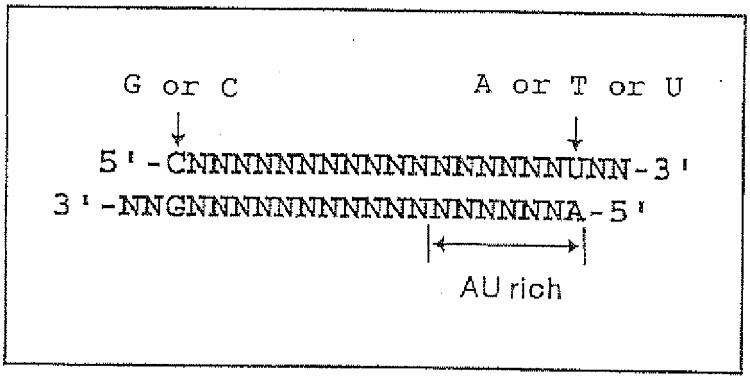
G or C
A or T or U
5'-CNNNNNNNNNNNNNNNNNNNNNUNN-3'
3'-NNGNNNNNNNNNNNNNNNNNNNA-5'
AU rich

# *Fig.3*

NM_000507: *Homo sapiens* fructose-1,6-bisphosphatase 1 (*FBP1*)
NM_019395: *Mus musculus* fructose bisphosphatase 1 (*Fbp1*)

```
NM_000507    1 ATGGCTGACCAGGCGCCCTTCGACACGGACGTCAACACCCTGACCCGCTTCGTCATGGAG   60
NM_019395    1 ATGGCGAACCATGCGCCCTTCGAAACGGATATCAGCACCCTGACCCGCTTCGTCATGGAG   60

NM_000507   61 GAGGGCAGGAAGGCCCGCGGCACGGGCGAGTTGACCCAGCTGCTCAACTCGCTCTGCACA  120
NM_019395   61 CAGGGCAGGAAGGCTCAGGGCACGGGGGAGTTGACCCAGCTGCTGAATTCGCTCTGCACC  120

                                                            ▼ intron
NM_000507  121 GCAGTCAAAGCCATCTCTTCGGCGGTGCGCAAGGCGGGCATCGCGCACCTCTATGGCATT  180
NM_019395  121 GCGATCAAAGCCATCTCGTCTGCGGTGCGCCAGGCGGGCATCGCACAGCTCTATGGTATC  180

NM_000507  181 GCTGGTTCTACCAACGTGACAGGTGATCAAGTTAAGAAGCTGGACGTCCTCTCCAACGAC  240
NM_019395  181 GCTGGCTCAACCAATGTGACTGGGGATCAAGTAAAGAAGCTGGACATACTTTCCAATGAC  240

NM_000507  241 CTGGTTATGAACATGTTAAAGTCATCCTTTGCCACGTGTGTTCTCGTGTCAGAAGAAGAT  300
NM_019395  241 CTGGTGATCAATATGCTGAAGTCGTCCTACGCTACCTGTGTTCTTGTGTCTGAAGAAAAC  300

                                               ▼ intron
NM_000507  301 AAACACGCCATCATAGTGGAACCGGAGAAAAGGGGTAAATATGTGGTCTGTTTTGATCCC  360
NM_019395  301 ACAAATGCCATCATAATCGAACCTGAGAAGAGGGGCAAATATGTTGTCTGTTTCGATCCC  360

NM_000507  361 CTTGATGGATCTTCCAACATCGATTGCCTTGTGTCCGTTGGAACCATTTTTGGCATCTAT  420
NM_019395  361 CTTGATGGCTCATCCAACATTGACTGCCTTGTGTCCATCGGAACCATTTTTGGCATTTAC  420

                       ▼ intron
NM_000507  421 AGAAAGAAATCAACTGATGAGCCTTCTGAGAAGGATGCTCTGCAACCAGGCCGGAACCTG  480
NM_019395  421 AGAAAGAAAAGTACTGATGAGCCTTCTGAGAAGGATGCTCTGCAGCCCGGCCGGGACCTG  480

NM_000507  481 GTGGCAGCCGGCTACGCACTGTATGGCAGTGCCACCATGCTGGTCCTTGCCATGGACTGT  540
NM_019395  481 GTGGCAGCCGGGTATGCGCTCTATGGCAGTGCCACCATGTTGGTCCTTGCCATGGATTGT  540

                                          ▼ intron
NM_000507  541 GGGGTCAACTGCTTCATGCTGGACCCGGCCATCGGGGAGTTCATTTTGGTGGACAAGGAT  600
NM_019395  541 GGTGTCAACTGCTTCATGCTGGACCCGTCCATTGGAGAATTCATTATGGTGGACAGGGAC  600

NM_000507  601 GTGAAGATAAAAAAGAAAGGTAAAATCTACAGCCTTAACGAGGGCTACGCCAGGGACTTT  660
NM_019395  601 GTGAAGATGAAGAAGAAAGGTAACATCTACAGCCTTAATGAGGGTTATGCCAAGGACTTT  660

                                                        ▼ intron
NM_000507  661 GACCCTGCCGTCACTGAGTACATCCAGAGGAAGAAGTTCCCCCCAGATAATTCAGCTCCT  720
NM_019395  661 GACCCTGCCATCAATGAGTATCTCCAGAGGAAAAAGTTCCCTCCGGATGGTTCAGCCCCC  720

NM_000507  721 TATGGGGCCCGGTATGTGGGCTCCATGGTGGCTGATGTTCATCGCACTCTGGTCTACGGA  780
NM_019395  721 TATGGTGCCCGGTATGTGGGGTCCATGGTGGCTGATATTCACCGCACTCTGGTATATGGA  780

                                                        ▼ intron
NM_000507  781 GGGATATTTCTGTACCCCGCTAACAAGAAGAGCCCCAATGGAAAGCTGAGACTGCTGTAC  840
NM_019395  781 GGGATCTTTTTATACCCCGCCAACAAGAAAAGCCCAAGTGGAAAGCTGCGGCTGCTGTAT  840

NM_000507  841 GAATGCAACCCCATGGCCTACGTCATGGAGAAGGCTGGGGGAATGGCCACCACTGGGAAG  900
NM_019395  841 GAGTGCAACCCCATAGCTTATGTCATGGAGAAGGCCGGTGGGCTCGCCACCACGGGGGAC  900

NM_000507  901 GAGGCCGTGTTAGACGTCATTCCCACAGACATTCACCAGAGGGCGCCGGTGATCTTGGGA  960
NM_019395  901 AAAGATATATTAGACATCGTTCCCACCGAGATCCACCAGAAGGCACCAGTCGTCATGGGG  960

NM_000507  961 TCCCCCGACGACGTGCTCGAGTTCCTGAAGGTGTATGAGAAGCACTCTGCCCAGTGA   1017
NM_019395  961 TCCTCTGAAGATGTGCAGGAGTTCCTGGAGATCTACAGGAAGCACAAAGCCAAGTGA   1017
```

Fig.4

| | |
|---|---|
| NM_000507: 36 | caccctgacccgcttcgtcatgg |
| NM_000507: 37 | accctgacccgcttcgtcatgga |
| NM_000507: 38 | ccctgacccgcttcgtcatggag |
| NM_000507:433 | actgatgagccttctgagaagga |
| NM_000507:434 | ctgatgagccttctgagaaggat |
| NM_000507:435 | tgatgagccttctgagaaggatg |
| NM_000507:436 | gatgagccttctgagaaggatgc |
| NM_000507:437 | atgagccttctgagaaggatgct |
| NM_000507:438 | tgagccttctgagaaggatgctc |
| NM_000507:439 | gagccttctgagaaggatgctct |
| NM_000507:440 | agccttctgagaaggatgctctg |
| NM_000507:441 | gccttctgagaaggatgctctgc |
| NM_000507:442 | ccttctgagaaggatgctctgca |
| NM_000507:544 | gtcaactgcttcatgctggaccc |
| NM_000507:545 | tcaactgcttcatgctggacccg |

Fig.5

| | | | | | |
|---|---|---|---|---|---|
| NM_000507: 36 | caccctgacccgcttcgtcatgg | 1 | 1 | 4 | 4 |
| NM_000507: 37 | accctgacccgcttcgtcatgga | 0 | 1 | 3 | 0 |
| NM_000507: 38 | ccctgacccgcttcgtcatggag | 0 | 1 | 3 | 0 |
| NM_000507:433 | actgatgagccttctgagaagga | 0 | 0 | 4 | 0 |
| NM_000507:434 | ctgatgagccttctgagaaggat | 0 | 1 | 3 | 0 |
| NM_000507:435 | tgatgagccttctgagaaggatg | 1 | 0 | 4 | 0 |
| NM_000507:436 | gatgagccttctgagaaggatgc | 1 | 0 | 4 | 0 |
| NM_000507:437 | atgagccttctgagaaggatgct | 0 | 1 | 4 | 0 |
| NM_000507:438 | tgagccttctgagaaggatgctc | 0 | 0 | 3 | 0 |
| NM_000507:439 | gagccttctgagaaggatgctct | 1 | 1 | 3 | 0 |
| NM_000507:440 | agccttctgagaaggatgctctg | 0 | 1 | 3 | 0 |
| NM_000507:441 | gccttctgagaaggatgctctgc | 1 | 1 | 4 | 4 |
| NM_000507:442 | ccttctgagaaggatgctctgca | 0 | 0 | 3 | 0 |
| NM_000507:544 | gtcaactgcttcatgctggaccc | 0 | 1 | 2 | 0 |
| NM_000507:545 | tcaactgcttcatgctggacccg | 0 | 0 | 2 | 0 |

Fig.6

| Sequences producing significant alignments: | Score (bits) | E Value |
|---|---|---|
| ref\|NM_019395.1\| Mus musculus fructose bisphosphatase 1 (Fbp1), ... | 46 | 9e-06 |
| ref\|NM_000507.2\| Homo sapiens fructose-1,6-bisphosphatase 1 (FBP... | 46 | 9e-06 |
| ref\|NM_015820.1\| Mus musculus heparan sulfate 6-O-sulfotransfera... | 30 | 0.52 |
| ref\|NM_003837.1\| Homo sapiens fructose-1,6-bisphosphatase 2 (FBP... | 30 | 0.52 |
| ref\|NM_145960.1\| Mus musculus hypothetical protein MGC19099 (MGC... | 28 | 2.0 |
| ref\|NM_010884.1\| Mus musculus N-myc downstream regulated 1 (Ndr1... | 28 | 2.0 |
| ref\|NM_025405.1\| Mus musculus RIKEN cDNA 1110033J19 gene (111003... | 26 | 8.1 |
| ref\|NM_029098.1\| Mus musculus RIKEN cDNA 1110013E13 gene (111001... | 26 | 8.1 |
| ref\|NM_012258.2\| Homo sapiens hairy/enhancer-of-split related wi... | 26 | 8.1 |
| ref\|NM_018113.1\| Homo sapiens lipocalin-interacting membrane rec... | 26 | 8.1 |
| ref\|NM_014780.1\| Homo sapiens KIAA0076 gene product (KIAA0076), ... | 26 | 8.1 |
| ref\|NM_012218.1\| Homo sapiens interleukin enhancer binding facto... | 26 | 8.1 |
| ref\|NM_004516.1\| Homo sapiens interleukin enhancer binding facto... | 26 | 8.1 |
| ref\|NM_000875.2\| Homo sapiens insulin-like growth factor 1 recep... | 26 | 8.1 |
| ref\|NM_001188.1\| Homo sapiens BCL2-antagonist/killer 1 (BAK1), mRNA | 26 | 8.1 |

# Fig.7

| Sequences producing significant alignments: | | Score (bits) | E Value |
|---|---|---|---|
| ref\|NM_019395.1\| | Mus musculus fructose bisphosphatase 1 (Fbp1), ... | 46 | 9e-06 |
| ref\|NM_000507.2\| | Homo sapiens fructose-1,6-bisphosphatase 1 (FBP... | 46 | 9e-06 |
| ref\|NM_007994.1\| | Mus musculus fructose bisphosphatase 2 (Fbp2), ... | 36 | 0.008 |
| ref\|NM_139045.1\| | Homo sapiens SWI/SNF related, matrix associated... | 32 | 0.13 |
| ref\|NM_003070.2\| | Homo sapiens SWI/SNF related, matrix associated... | 32 | 0.13 |
| ref\|NM_022026.1\| | Mus musculus aquaporin 9 (Aqp9), mRNA | 30 | 0.52 |
| ref\|NM_011834.1\| | Mus musculus kynurenine aminotransferase II (Ka... | 30 | 0.52 |
| ref\|NM_008632.1\| | Mus musculus microtubule-associated protein 2 (... | 30 | 0.52 |
| ref\|NM_023266.1\| | Mus musculus RIKEN cDNA 1200003I07 gene (120000... | 30 | 0.52 |
| ref\|NM_144499.1\| | Homo sapiens guanine nucleotide binding protein... | 30 | 0.52 |
| ref\|NM_000172.2\| | Homo sapiens guanine nucleotide binding protein... | 30 | 0.52 |
| ref\|NM_015125.2\| | Homo sapiens capicua homolog (Drosophila) (CIC)... | 30 | 0.52 |
| ref\|NM_146249.1\| | Mus musculus hypothetical protein MGC18735 (MGC... | 28 | 2.0 |
| ref\|NM_027017.1\| | Mus musculus RIKEN cDNA 3300002I08 gene (330000... | 28 | 2.0 |
| ref\|NM_145490.1\| | Mus musculus similar to zinc finger protein 97 ... | 28 | 2.0 |
| ref\|NM_145528.1\| | Mus musculus similar to Hypothetical protein KI... | 28 | 2.0 |
| ref\|NM_144546.1\| | Mus musculus expressed sequence AL024077 (AL024... | 28 | 2.0 |
| ref\|NM_144852.1\| | Mus musculus hypothetical protein MGC27672 (MGC... | 28 | 2.0 |
| ref\|NM_080467.1\| | Mus musculus ATPase, H+ transporting, lysosomal... | 28 | 2.0 |
| ref\|NM_029813.1\| | Mus musculus RIKEN cDNA 2210418O10 gene (221041... | 28 | 2.0 |
| ref\|NM_029583.1\| | Mus musculus RIKEN cDNA 2810408B13 gene (281040... | 28 | 2.0 |
| ref\|NM_001231.2\| | Homo sapiens calsequestrin 1 (fast-twitch, skel... | 28 | 2.0 |
| ref\|NM_020824.1\| | Homo sapiens Rho-GTPase activating protein 10 (... | 28 | 2.0 |

··· [Following 78 lines are omitted]

*Fig.8*

| | | | | | | |
|---|---|---|---|---|---|---|
| NM_000507: 36 | caccctgacccgcttcgtcatgg | 1 | 1 | 4 | 4 | 59 |
| NM_000507: 37 | accctgacccgcttcgtcatgga | 0 | 1 | 3 | 0 | - |
| NM_000507: 38 | ccctgacccgcttcgtcatggag | 0 | 1 | 3 | 0 | - |
| NM_000507:433 | actgatgagccttctgagaagga | 0 | 0 | 4 | 0 | - |
| NM_000507:434 | ctgatgagccttctgagaaggat | 0 | 1 | 3 | 0 | - |
| NM_000507:435 | tgatgagccttctgagaaggatg | 1 | 0 | 4 | 0 | - |
| NM_000507:436 | gatgagccttctgagaaggatgc | 1 | 0 | 4 | 0 | - |
| NM_000507:437 | atgagccttctgagaaggatgct | 0 | 1 | 4 | 0 | - |
| NM_000507:438 | tgagccttctgagaaggatgctc | 0 | 0 | 3 | 0 | - |
| NM_000507:439 | gagccttctgagaaggatgctct | 1 | 1 | 3 | 0 | - |
| NM_000507:440 | agccttctgagaaggatgctctg | 0 | 1 | 3 | 0 | - |
| NM_000507:441 | gccttctgagaaggatgctctgc | 1 | 1 | 4 | 4 | 1708 |
| NM_000507:442 | ccttctgagaaggatgctctgca | 0 | 0 | 3 | 0 | - |
| NM_000507:544 | gtcaactgcttcatgctggaccc | 0 | 1 | 2 | 0 | - |
| NM_000507:545 | tcaactgcttcatgctggacccg | 0 | 0 | 2 | 0 | - |

Fig.9

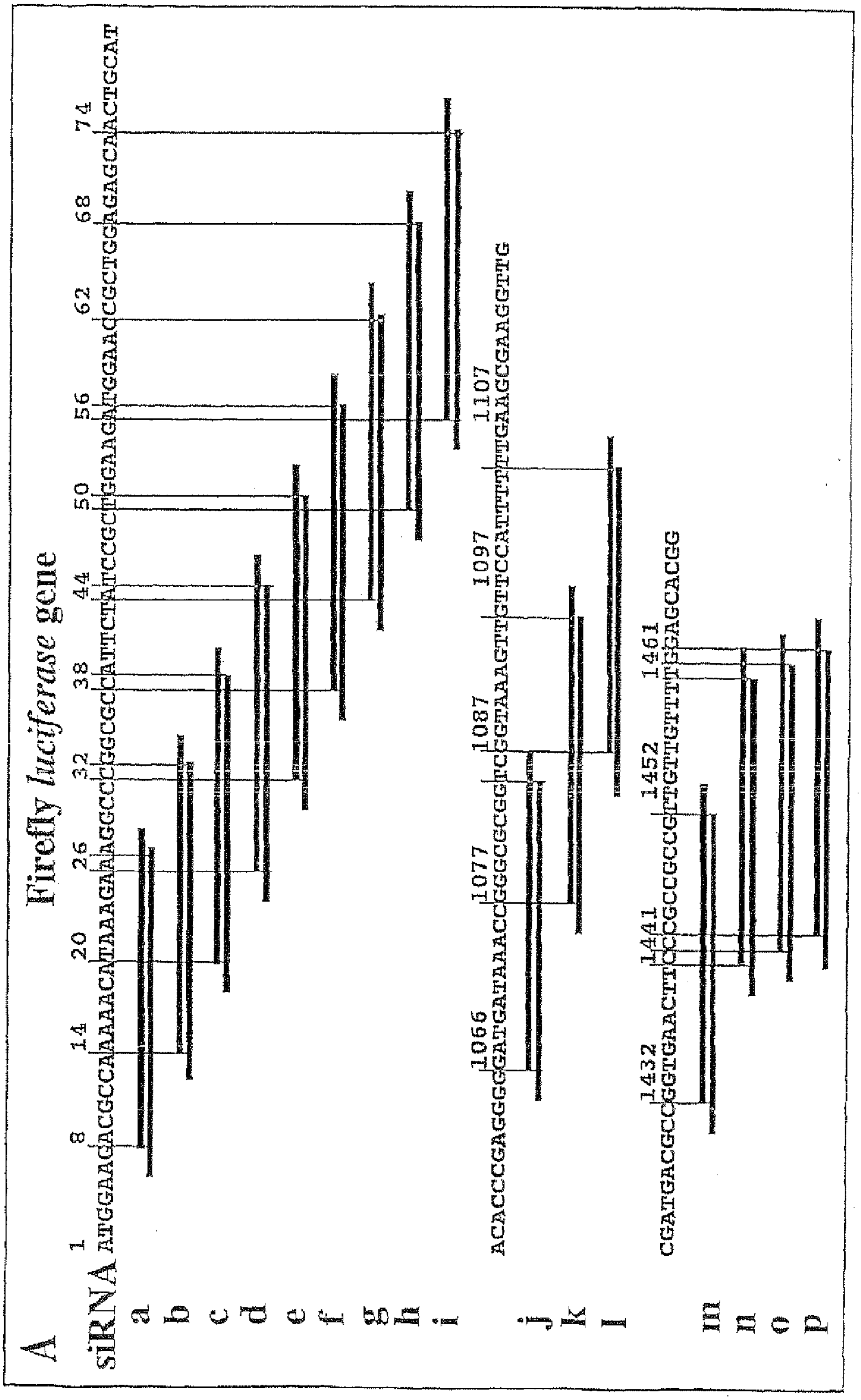
A
Firefly *luciferase* gene
siRNA
a
b
c
d
e
f
g
h
i
j
k
l
m
n
o
p
1 8 14 20 26 32 38 44 50 56 62 68 74
ATGGAAGACGCCAAAAACATAAAGAAAGGCCCGGCGCCATTCTATCCGCTGGAAGATGGAACCGCTGGAGAGCAACTGCAT
1066 1077 1087 1097 1107
ACACCCGAGGGGGATGATAAACCGGGCGCGGTCGGTAAAGTTGTTCCATTTTTTGAAGCGAAGGTTG
1432 1441 1452 1461
CGATGACGCCGGTGAACTTCCCGCCGCCGTTGTTGTTTTGGAGCACGG

Fig.10

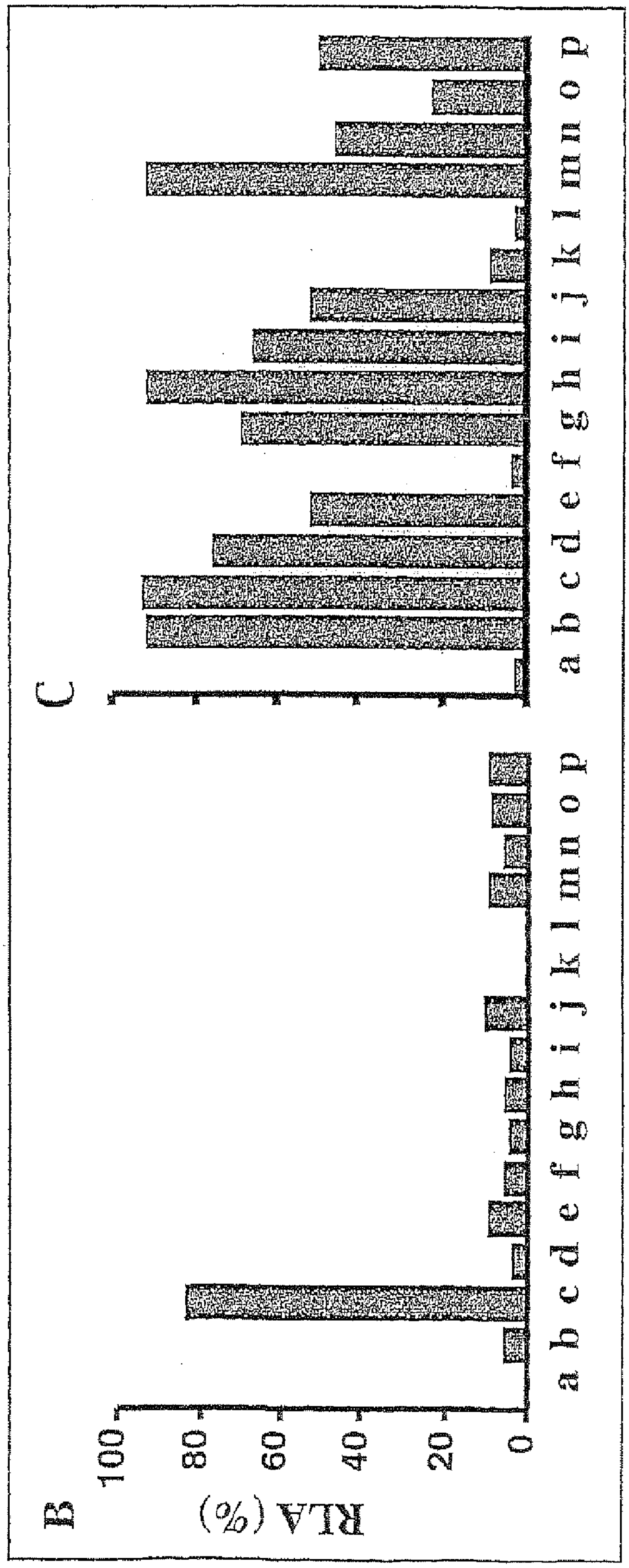

Fig.11

D

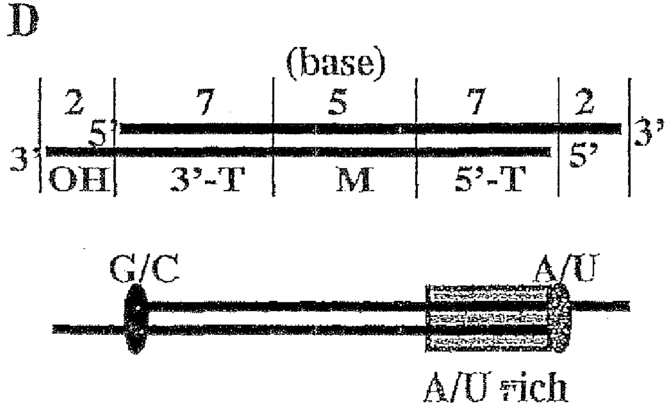


| RLA* | siRNA | G/C content (%) | | | | |
|---|---|---|---|---|---|---|
| | | OH | 3'-T | M | 5'-T | Total |
| 0.03 | a | UC | U-57 | 20 | 14-U | 32 |
| 0.03 | l | AG | C-43 | 20 | 29-A | 32 |
| 0.04 | f | CG | G-29 | 80 | 43-U | 47 |
| 0.09 | k | GG | C-100 | 80 | 14-A | 63 |
| 0.23 | o | AG | G-100 | 60 | 14-A | 58 |
| | | | 66 | 52 | 23 | 46 |
| 0.46 | n | AA | G-100 | 60 | 29-A | 63 |
| 0.50 | p | GG | G-100 | 40 | 29-C | 58 |
| 0.52 | e | GG | G-100 | 20 | 57-A | 63 |
| 0.52 | j | GG | C-29 | 60 | 100-C | 68 |
| 0.66 | i | UC | U-43 | 80 | 57-U | 58 |
| | | | 74 | 52 | 54 | 62 |
| 0.69 | g | GA | U-57 | 60 | 43-G | 53 |
| 0.76 | d | CU | U-71 | 100 | 29-U | 63 |
| 0.92 | m | GG | C-57 | 60 | 100-C | 74 |
| 0.92 | h | CG | A-43 | 40 | 86-C | 58 |
| 0.92 | b | GU | U-14 | 20 | 71-G | 37 |
| 0.93 | c | GU | A-29 | 80 | 100-G | 63 |
| | | | 45 | 60 | 72 | 58 |

Fig. 12

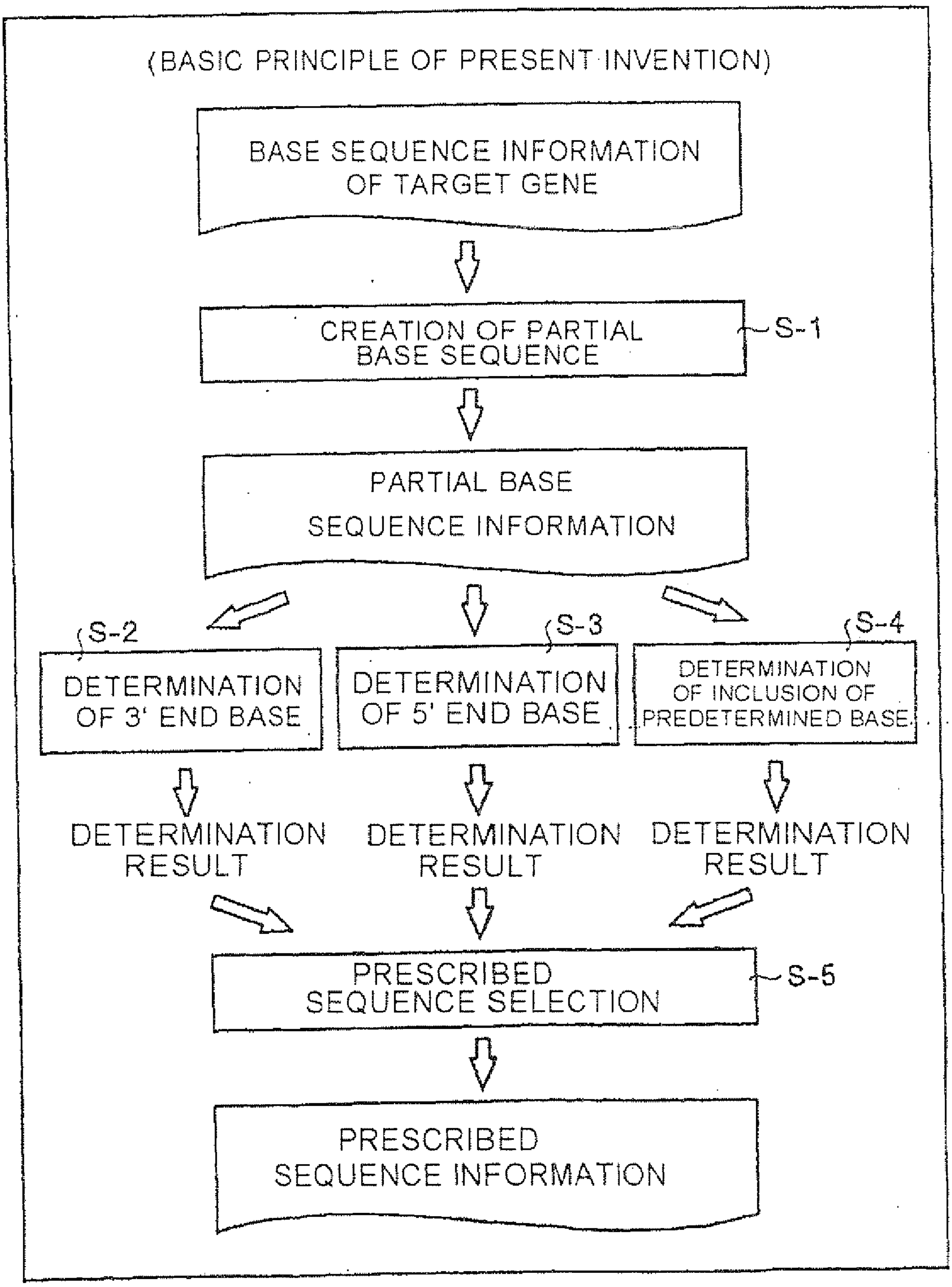
(BASIC PRINCIPLE OF PRESENT INVENTION)
BASE SEQUENCE INFORMATION OF TARGET GENE
CREATION OF PARTIAL BASE SEQUENCE
S-1
PARTIAL BASE SEQUENCE INFORMATION
S-2
DETERMINATION OF 3' END BASE
S-3
DETERMINATION OF 5' END BASE
S-4
DETERMINATION OF INCLUSION OF PREDETERMINED BASE
DETERMINATION RESULT
DETERMINATION RESULT
DETERMINATION RESULT
PRESCRIBED SEQUENCE SELECTION
S-5
PRESCRIBED SEQUENCE INFORMATION

Fig. 13

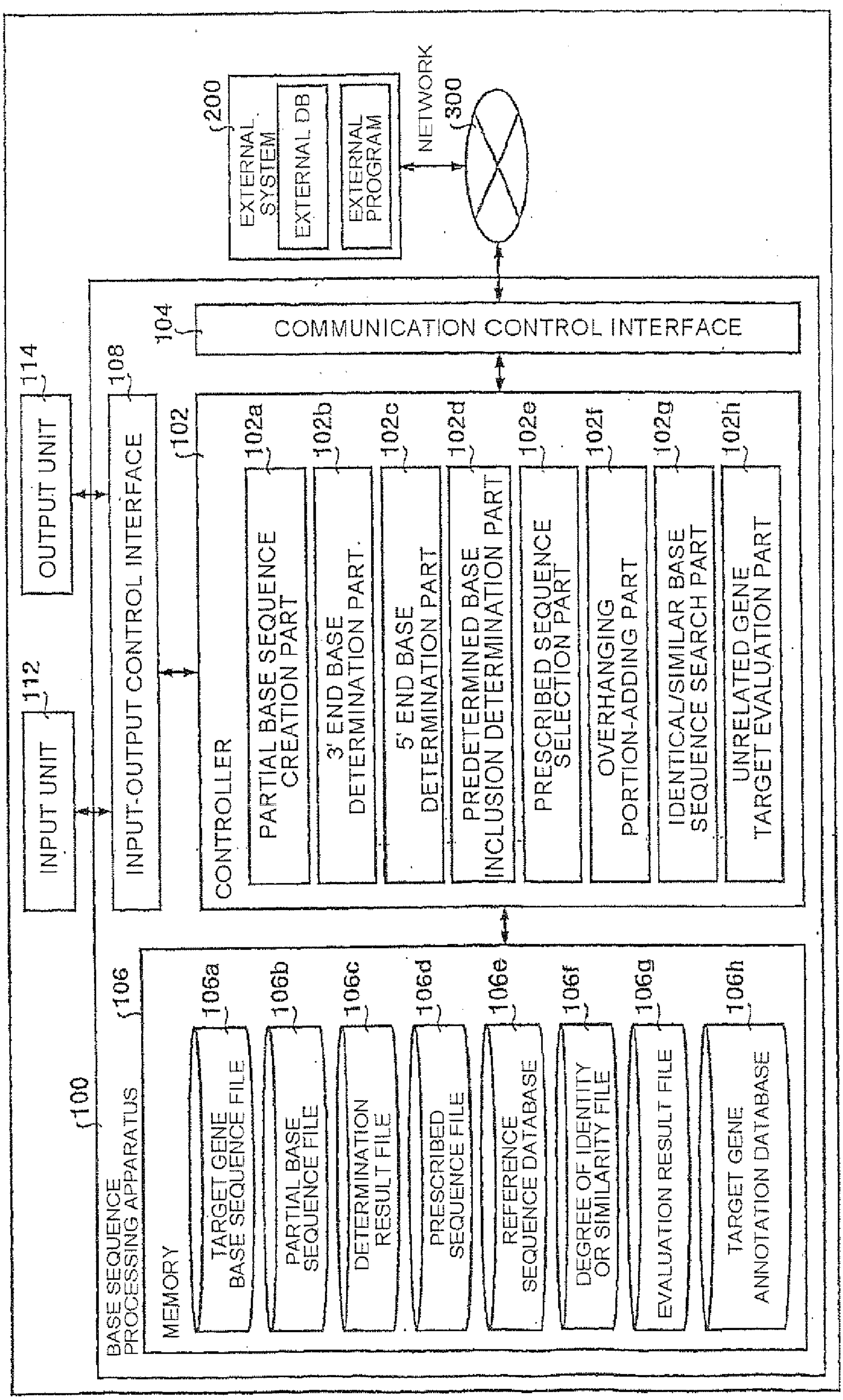
100
BASE SEQUENCE PROCESSING APPARATUS
106
MEMORY
106a
TARGET GENE BASE SEQUENCE FILE
106b
PARTIAL BASE SEQUENCE FILE
106c
DETERMINATION RESULT FILE
106d
PRESCRIBED SEQUENCE FILE
106e
REFERENCE SEQUENCE DATABASE
106f
DEGREE OF IDENTITY OR SIMILARITY FILE
106g
EVALUATION RESULT FILE
106h
TARGET GENE ANNOTATION DATABASE
112
INPUT UNIT
114
OUTPUT UNIT
108
INPUT-OUTPUT CONTROL INTERFACE
102
CONTROLLER
102a
PARTIAL BASE SEQUENCE CREATION PART
102b
3' END BASE DETERMINATION PART
102c
5' END BASE DETERMINATION PART
102d
PREDETERMINED BASE INCLUSION DETERMINATION PART
102e
PRESCRIBED SEQUENCE SELECTION PART
102f
OVERHANGING PORTION-ADDING PART
102g
IDENTICAL/SIMILAR BASE SEQUENCE SEARCH PART
102h
UNRELATED GENE TARGET EVALUATION PART
104
COMMUNICATION CONTROL INTERFACE
200
EXTERNAL SYSTEM
EXTERNAL DB
EXTERNAL PROGRAM
NETWORK
300

Fig. 14

TARGET BASE SEQUENCE FILE

106a

| BASE SEQUENCE IDENTIFICATION INFORMATION | BASE SEQUENCE INFORMATION |
|---|---|
| NM_000507 | ATGGCTGA ··· AGTGA |
| ⋮ | ⋮ |

Fig. 15

PARTIAL BASE SEQUENCE FILE

106b

| PARTIAL BASE SEQUENCE IDENTIFICATION INFORMATION | PARTIAL BASE SEQUENCE INFORMATION | INFORMATION ON INCLUSION OF OVERHANGING PORTION |
|---|---|---|
| NM_000507:36 | caccct ··· tcatgg | INCLUDED |
| ⋮ | ⋮ | ⋮ |

Fig. 16

DETERMINATION RESULT FILE

106c

| PARTIAL BASE SEQUENCE IDENTIFICATION INFORMATION | DETERMINATION RESULT ON 3' END BASE | DETERMINATION RESULT ON 5' END BASE | DETERMINATION RESULT ON INCLUSION OF PREDETERMINED BASE | COMPREHENSIVE DETERMINATION RESULT |
|---|---|---|---|---|
| NM_000507:36 | 1 | 1 | 4 | 4 |
| ⋮ | ⋮ | ⋮ | ⋮ | ⋮ |

Fig. 17

PRESCRIBED SEQUENCE FILE

106d

| PARTIAL BASE SEQUENCE IDENTIFICATION INFORMATION | PRESCRIBED SEQUENCE INFORMATION |
|---|---|
| NM_000507:36 | caccct ··· tcatgg |
| ⋮ | ⋮ |

Fig. 18

REFERENCE SEQUENCE DATABASE

106e

| REFERENCE SEQUENCE IDENTIFICATION INFORMATION | REFERENCE BASE SEQUENCE INFORMATION |
|---|---|
| ref\|NM_015820.1\| | caccct ··· gcatgg |
| ⋮ | ⋮ |

Fig. 19

DEGREE OF IDENTITY OR SIMILARITY FILE
106f

| PARTIAL BASE SEQUENCE IDENTIFICATION INFORMATION | PRESCRIBED IDENTIFICATION INFORMATION | DEGREE OF IDENTITY OR SIMILARITY |
|---|---|---|
| NM_000507:36 | ref\|NM_015820.1\| | 0.52 |
| | ref\|NM_003837.1\| | 0.52 |
| ⋮ | ⋮ | ⋮ |

Fig. 20

EVALUATION RESULT FILE
106g

| PARTIAL BASE SEQUENCE IDENTIFICATION INFORMATION | REFERENCE SEQUENCE IDENTIFICATION INFORMATION | EVALUATION INFORMATION |
|---|---|---|
| NM_000507:36 | 5.9 | NONTARGET |
| NM_000507:441 | 170.8 | TARGET |
| ⋮ | ⋮ | ⋮ |

Fig. 21

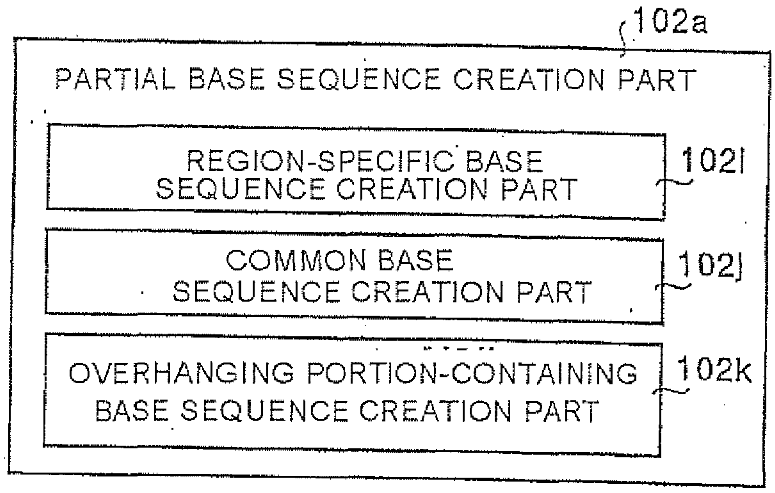
102a
PARTIAL BASE SEQUENCE CREATION PART
REGION-SPECIFIC BASE SEQUENCE CREATION PART
102l
COMMON BASE SEQUENCE CREATION PART
102j
OVERHANGING PORTION-CONTAINING BASE SEQUENCE CREATION PART
102k

Fig. 22

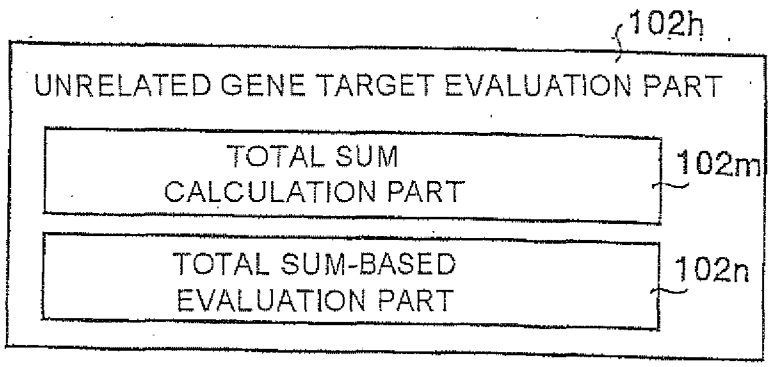
102h
UNRELATED GENE TARGET EVALUATION PART
TOTAL SUM CALCULATION PART
102m
TOTAL SUM-BASED EVALUATION PART
102n

Fig. 23

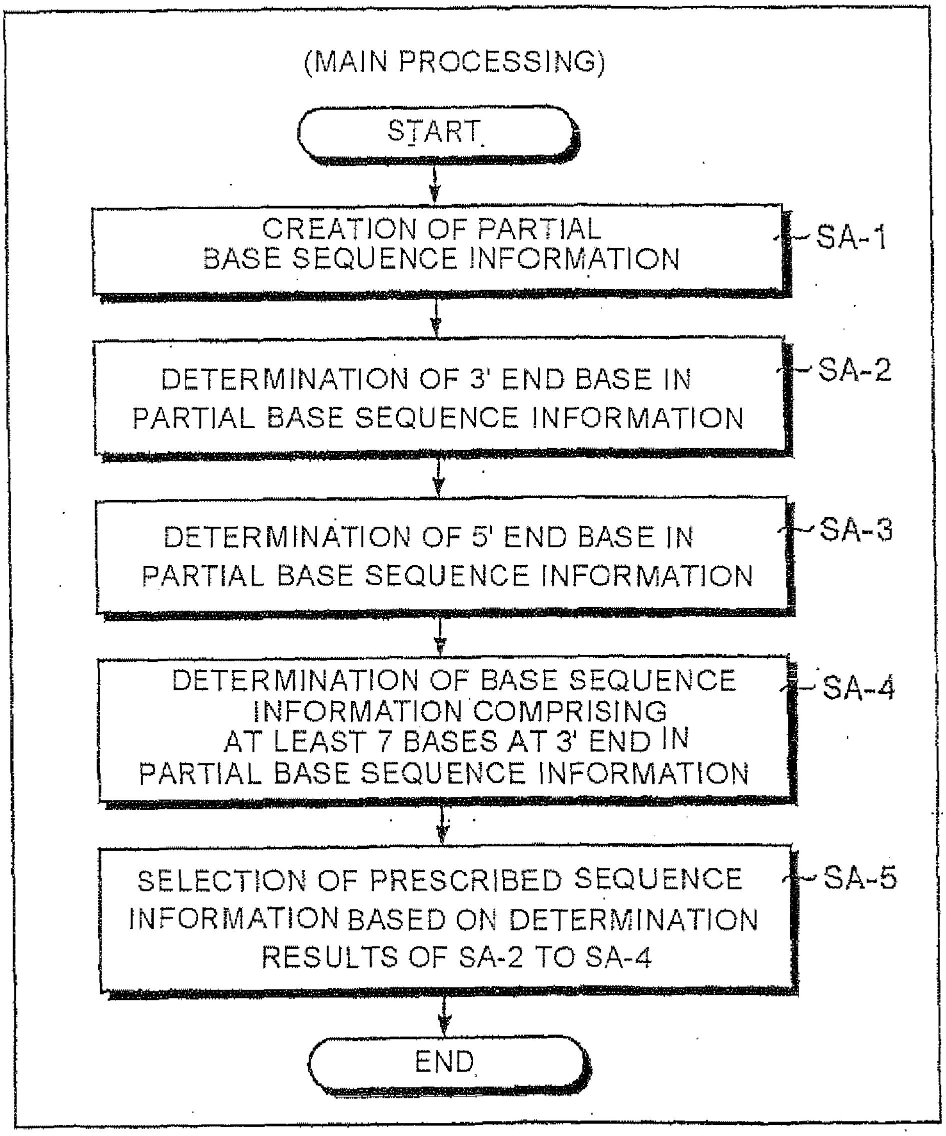
(MAIN PROCESSING)
START
CREATION OF PARTIAL BASE SEQUENCE INFORMATION
SA-1
DETERMINATION OF 3' END BASE IN PARTIAL BASE SEQUENCE INFORMATION
SA-2
DETERMINATION OF 5' END BASE IN PARTIAL BASE SEQUENCE INFORMATION
SA-3
DETERMINATION OF BASE SEQUENCE INFORMATION COMPRISING AT LEAST 7 BASES AT 3' END IN PARTIAL BASE SEQUENCE INFORMATION
SA-4
SELECTION OF PRESCRIBED SEQUENCE INFORMATION BASED ON DETERMINATION RESULTS OF SA-2 TO SA-4
SA-5
END

Fig. 24

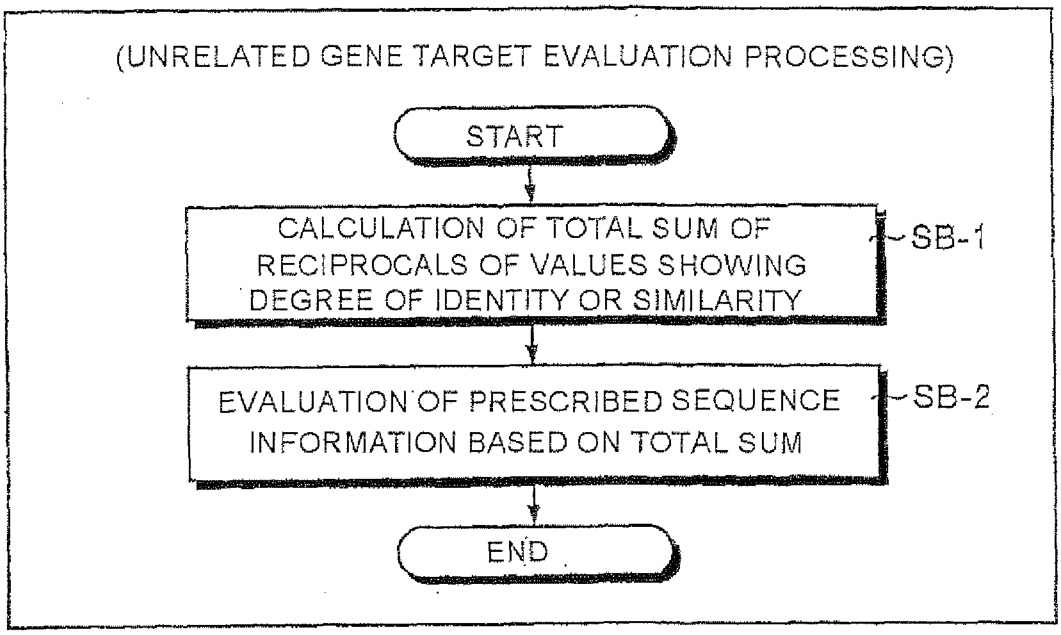
(UNRELATED GENE TARGET EVALUATION PROCESSING)
START
CALCULATION OF TOTAL SUM OF RECIPROCALS OF VALUES SHOWING DEGREE OF IDENTITY OR SIMILARITY
SB-1
EVALUATION OF PRESCRIBED SEQUENCE INFORMATION BASED ON TOTAL SUM
SB-2
END

*Fig.25*

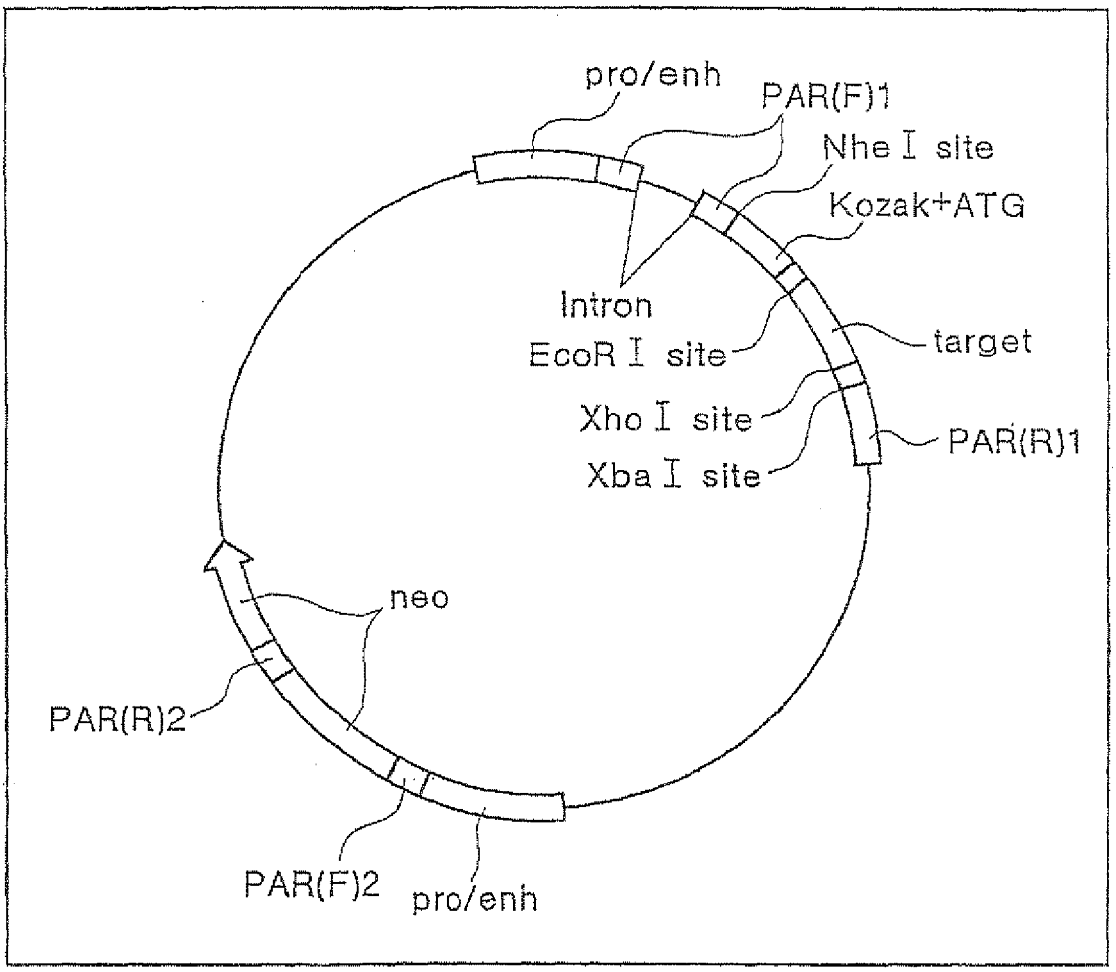
pro/enh
PAR(F)1
Nhe I site
Kozak+ATG
Intron
EcoR I site
target
Xho I site
PAR(R)1
Xba I site
neo
PAR(R)2
PAR(F)2
pro/enh

Fig.26

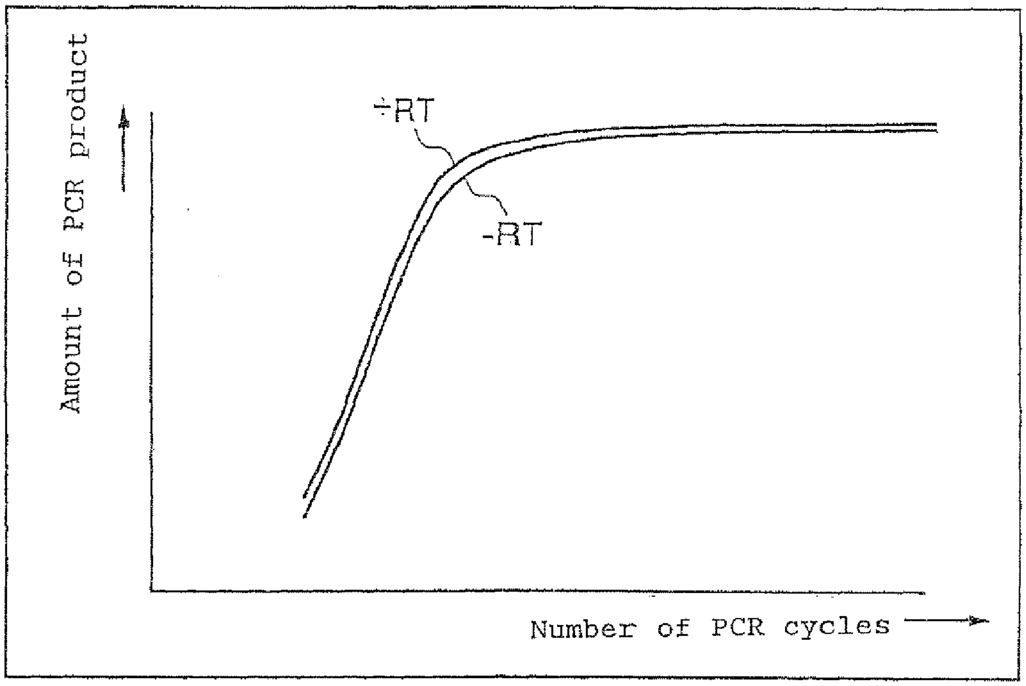
+RT
-RT
Amount of PCR product
Number of PCR cycles

Fig.27

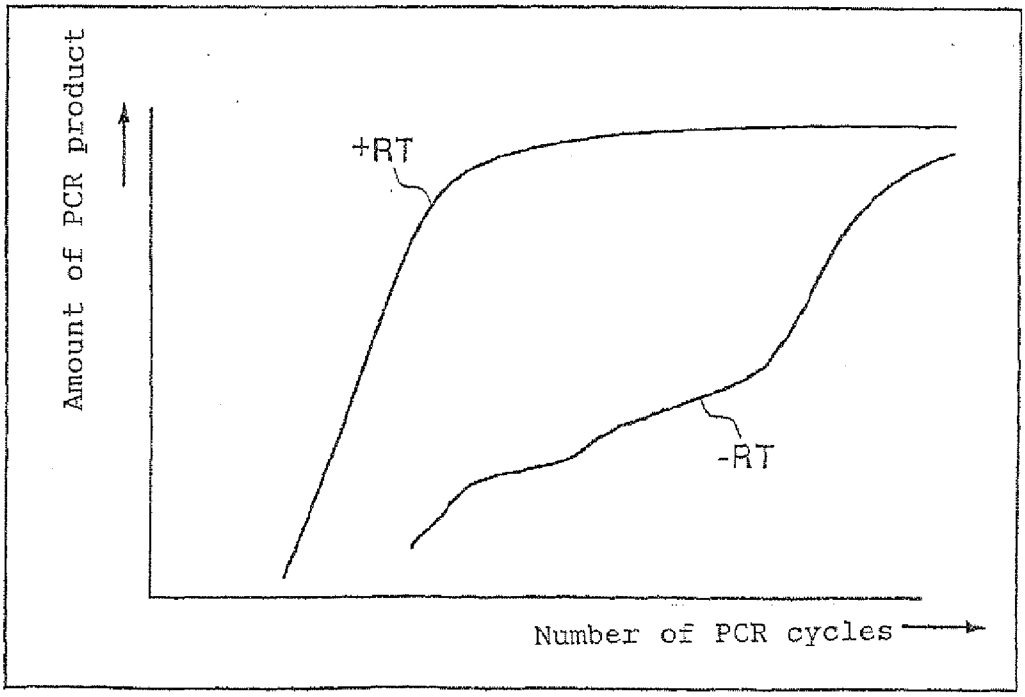
+RT
-RT
Amount of PCR product
Number of PCR cycles

*Fig.28*

```
5' -   aggauguucggcggcccgggc -3'
3' - cguccuacaagccgccgggcc   -5'
```

*Fig.29*

```
5' -   guacgucagcaauaugaaagu -3'
3' - ugcaugcagucguuauacuuu   -5'
```

Fig.30

5'- CAUUCUAUCCGCUGGAAGAUG -3'
3'- CGGUAAGAUAGGCGACCUUCU -5'

Fig.31

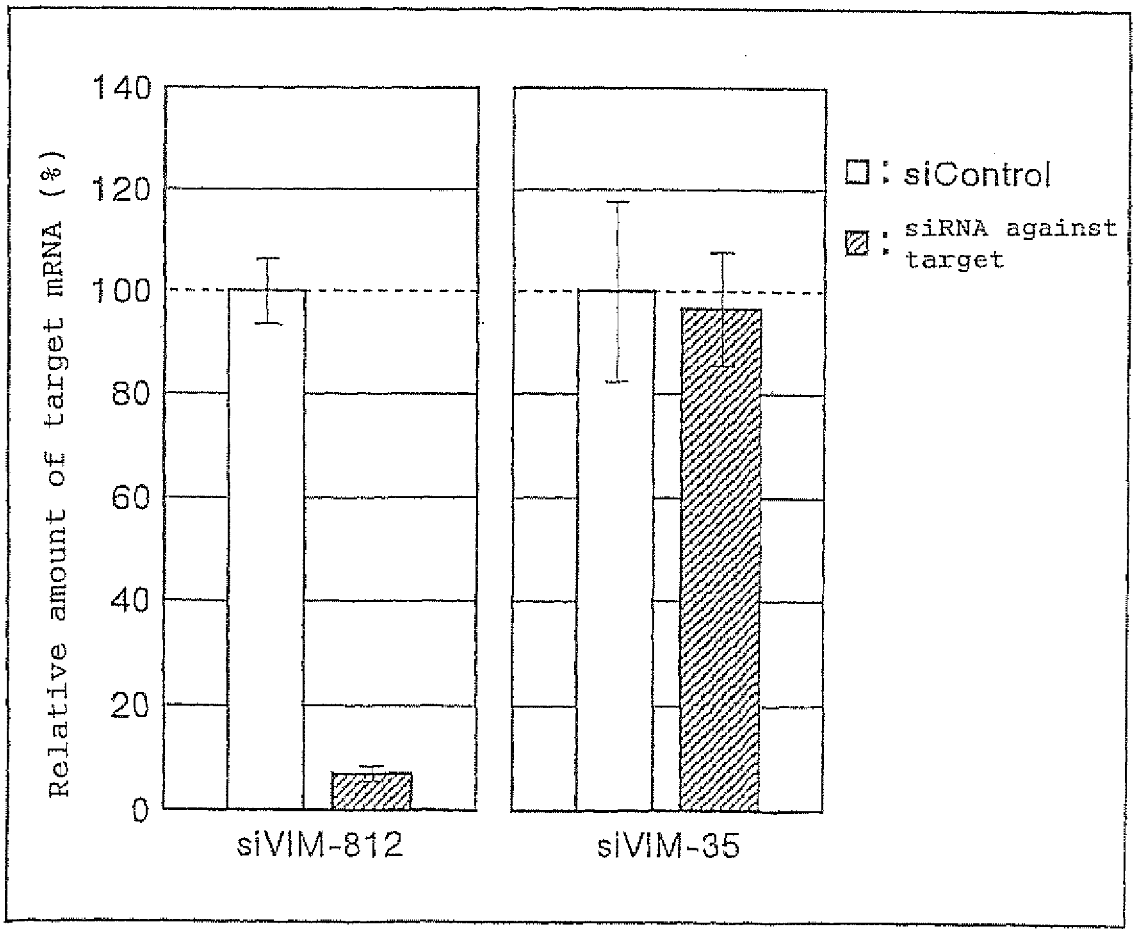

Fig.32

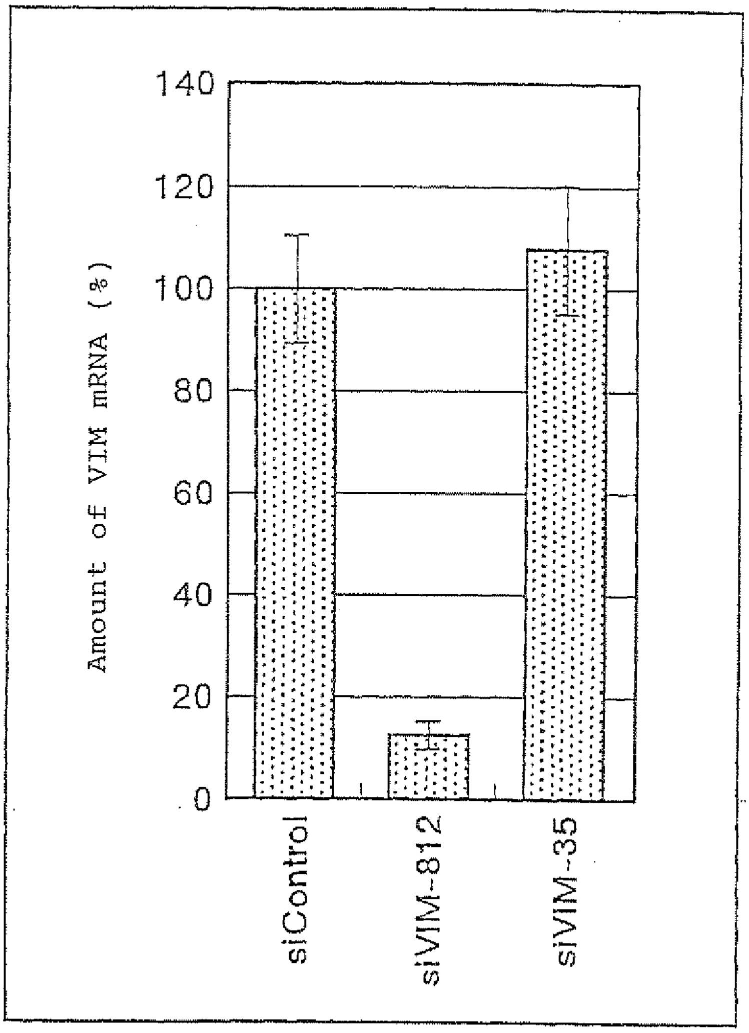
Amount of VIM mRNA (%)
140
120
100
80
60
40
20
0
siControl
siVIM-812
siVIM-35

Fig.33

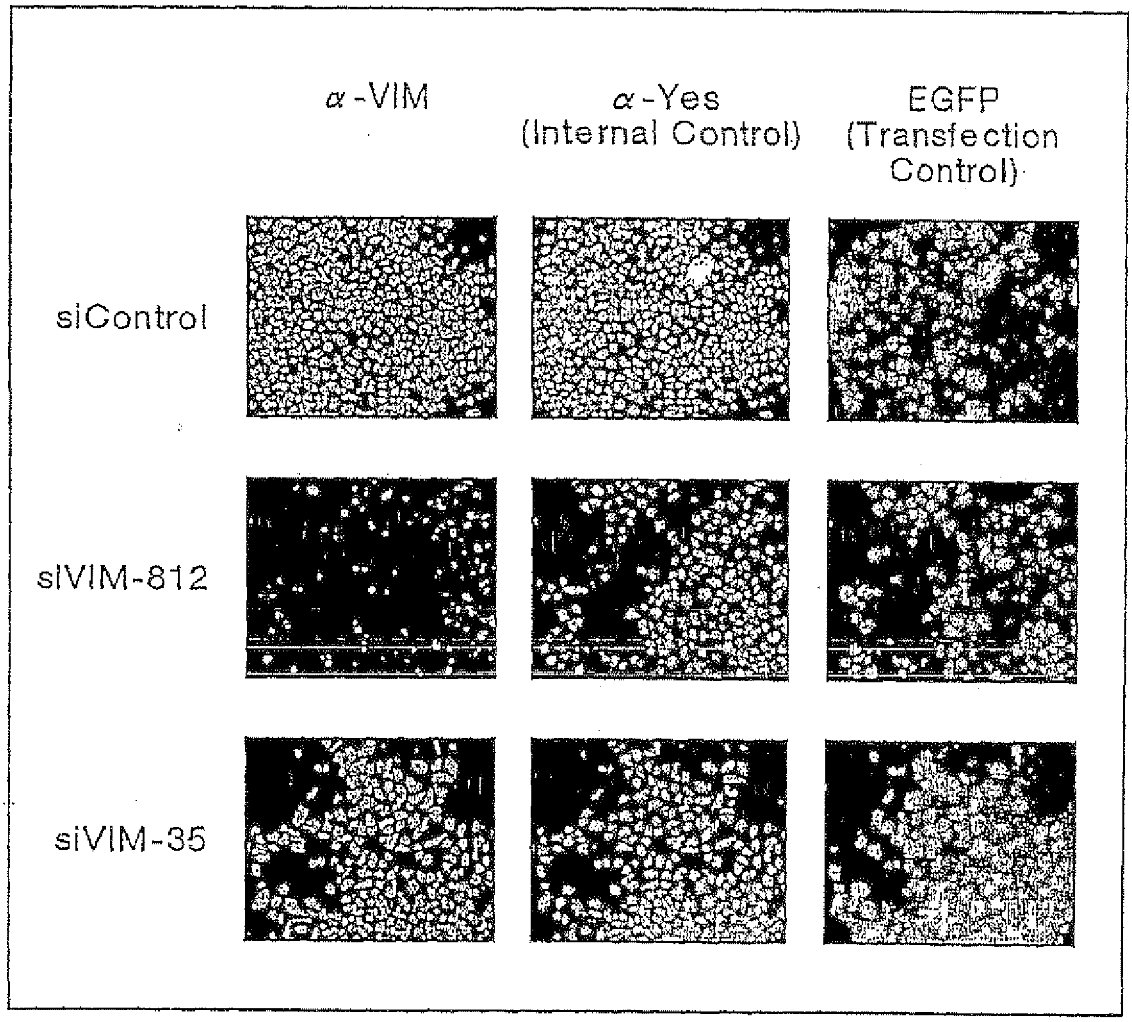
α-VIM
α-Yes
(Internal Control)
EGFP
(Transfection
Control)
siControl
sIVIM-812
siVIM-35

# Fig.34

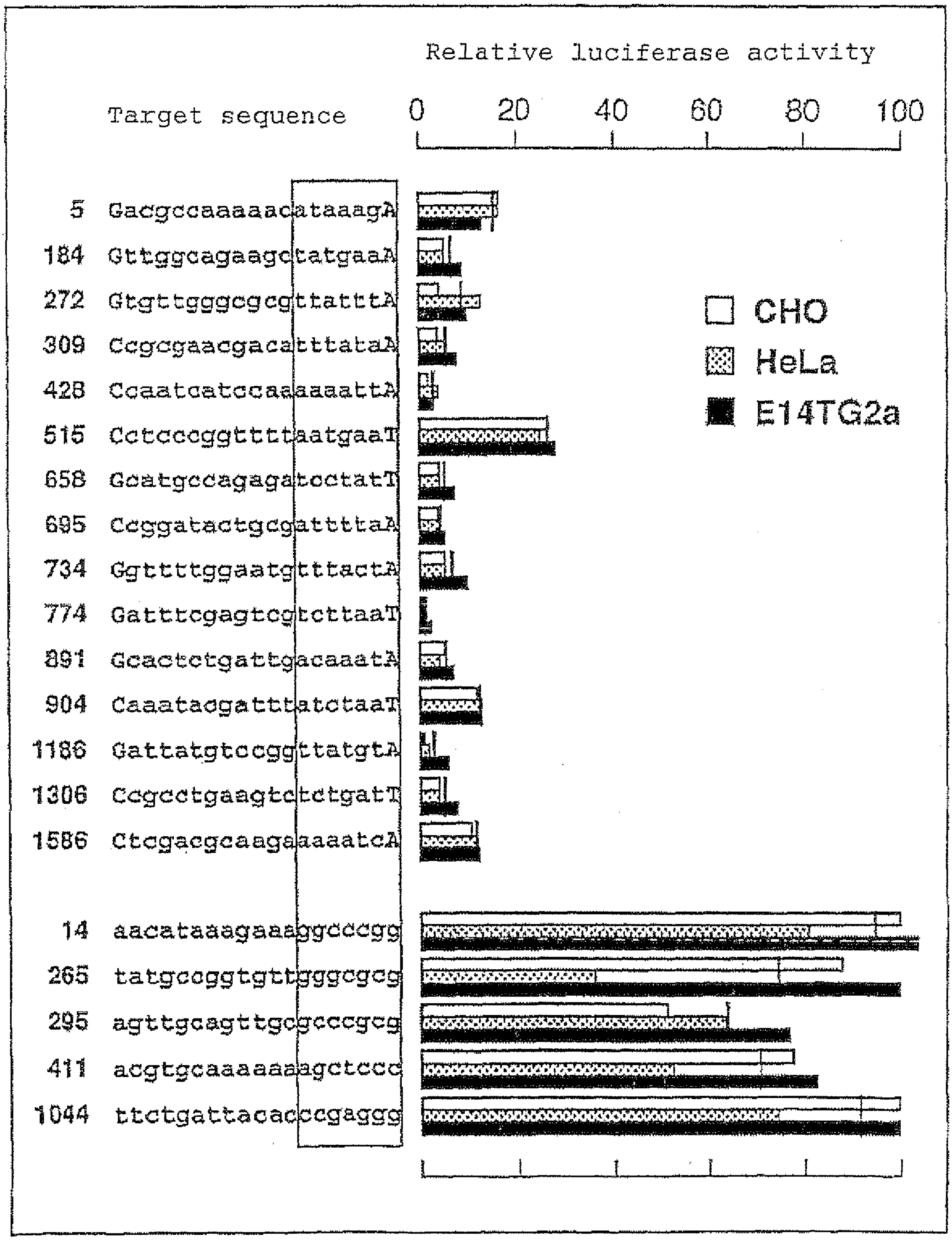
Relative luciferase activity
Target sequence
0
20
40
60
80
100
5 GacgccaaaaacataaagA
184 GttggcagaagctatgaaA
272 GtgttgggcgcgttatttA
309 CcgcgaacgacatttataA
428 CcaatcatccaaaaaattA
515 CctcccggttttaatgaaT
658 GcatgccagagatcctatT
695 CcggatactgcgattttaA
734 GgttttggaatgtttactA
774 GatttcgagtcgtcttaaT
891 GcactctgattgacaaatA
904 CaaatacgatttatctaaT
1186 GattatgtccggttatgtA
1306 CcgcctgaagtctctgatT
1586 CtcgacgcaagaaaaatcA
14 aacataaagaaaggcccgg
265 tatgccggtgttgggcgcg
295 agttgcagttgcgcccgcg
411 acgtgcaaaaaaagctccc
1044 ttctgattacacccgaggg
CHO
HeLa
E14TG2a

Fig.35

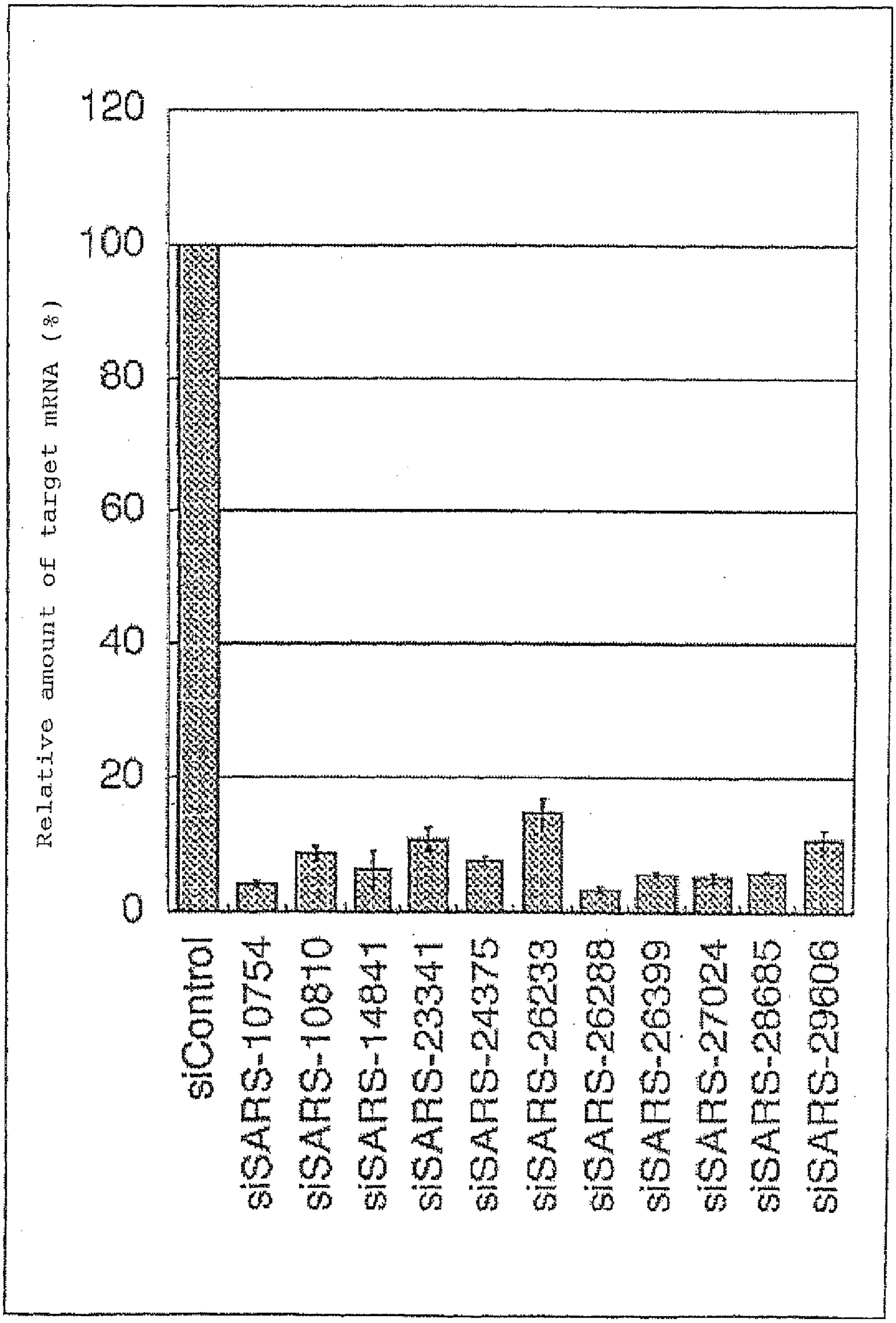
Relative amount of target mRNA (%)
0
20
40
60
80
100
120
siControl
siSARS-10754
siSARS-10810
siSARS-14841
siSARS-23341
siSARS-24375
siSARS-26233
siSARS-26288
siSARS-26399
siSARS-27024
siSARS-28685
siSARS-29606

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- BASIC METHODS IN MOLECULAR BIOLOGY
- **SAMBROOK et al.** MOLECULAR CLONING; A LABORATORY MANUAL. Cold Spring Harbor Laboratory Press, 1989
- Saibo-Kogaku Handbook. Yodosha, 1992
- Shin-Idenshi-Kogaku Handbook. Yodosha, 1999
- **SCHNEIDER et al.** *J. Embryol. Exp. Morph.,* 1972, vol. 27, 353-365